# EUROPEAN PATENT SPECIFICATION

(11) **EP 2 059 604 B1**
(45) Date of publication and mention of the grant of the patent: **01.05.2013**
(21) Application number: 07838012.8
(22) Date of filing: 11.09.2007
(51) Int. Cl.: C12P 7/06, C12N 9/00, C12N 9/14, C12N 9/24, C07K 1/00

(54) **ENZYME SYSTEMS FOR SACCHARIFICATION OF PLANT CELL WALL POLYSACCHARIDES**
ENZYMSYSTEME ZUR VERZUCKERUNG VON POLYSACCHARIDEN AUS PFLANZENZELLWÄNDEN
SYSTÈMES ENZYMATIQUES POUR LA SACCHARIFICATION DE POLYSACCHARIDES DE PAROIS CELLULAIRES DE PLANTES

(30) Priority: 12.09.2006 US 519104
(43) Date of publication of application: 20.05.2009
(73) Proprietor: University Of Maryland, College Park, MD 20742-1001 (US)
(72) Inventor: HUTCHESON, Steven Wayne, Columbia, MD 21046 (US); WEINER, Ronald M., Potomac, MD 20854 (US)
(74) Representative: Gitto, Serena
(86) International application number: PCT/US2007/019708
(87) International publication number: WO 2008/033330

(56) References cited:
- US-A1- 2006 105 914
- BHAT M K: "Cellulases and related enzymes in biotechnology" BIOTECHNOLOGY ADVANCES, ELSEVIER PUBLISHING, BARKING, GB, vol. 18, no. 5, 1 August 2000 (2000-08-01), pages 355-383, XP004211815 ISSN: 0734-9750
- TAYLOR LARRY E II ET AL: "Complete cellulase system in the marine bacterium Saccharophagus degradans strain 2-40(T)" JOURNAL OF BACTERIOLOGY,, vol. 188, no. 11, 1 June 2006 (2006-06-01), pages 3849-3861, XP002525799
- GRAY K.A. ET AL.: 'Bioethanol' CURR. OPIN. CHEM. BIOL. vol. 10, no. 2, April 2006, pages 141 - 146, XP025155166
- KATAHIRA S. ET AL.: 'Ethanol fermentation from lignocellulosic hydrolysate by a recombinant xylose- and cellooligosaccharide-assimilating yeast strain' APPL. MICROBIOL. BIOTECHNOL. vol. 72, no. 6, October 2006, pages 1136 - 1143, XP019441681
- PERCIVAL ZHANG Y.Z. ET AL.: 'Outlook for cellulase improvement: Screening and selection strategies' BIOTECHNOL. ADV. vol. 24, no. 5, September 2006 - October 2006, pages 452 - 481, XP025146608
- PATEL-PREDD P. ET AL.: 'Overcoming the hurdles to producing ethanol from cellulose' ENVIRON. SCI. TECHNOL. vol. 40, no. 13, 01 July 2006, pages 4052 - 4053, XP008104516

## Description

### BACKGROUND OF THE INVENTION

### 1. Field of the Invention:

The invention is generally directed to degradative enzymes and systems. In particular, the present invention is directed to plant cell wall degrading enzymes and associated proteins found in *Microbulbifer degradans,* systems containing such enzymes and/or proteins, and methods of using the systems to obtain ethanol.

### 2. Background of the Invention:

Cellulases and related enzymes have been utilized in food, beer, wine, animal feeds, textile production and laundering, pulp and paper industry, and agricultural industries. Various such uses are described in the paper "Cellulases and related enzymes in biotechnology" by M.K. Bhat (Biotechnical Advances 18 (2000) 355-383)

The cell walls of plants are composed of a heterogenous mixture of complex polysaccharides that interact through covalent and noncovalent means. Complex polysaccharides of higher plant cell walls include, for example, cellulose (β-1, 4 glucan) which generally makes up 35-50% of carbon found in cell wall components. Cellulose polymers self associate through hydrogen bonding, van der Waals interactions and hydrophobic interactions to form semi-crystalline cellulose microfibrils. These microfibrils also include noncrystalline regions, generally known as amorphous cellulose. The cellulose microfibrils are embedded in a matrix formed of hemicelluloses (including, e.g., xylans, arabinans, and mannans), pectins (e.g., galacturonans and galactans), and various other β-1, 3 and β-1, 4 glucans. These matrix polymers are often substituted with, for example, arabinose, galactose and/or xylose residues to yield highly complex arabinoxylans, arabinogalactans, galactomannans, and xyloglucans. The hemicellulose matrix is, in turn, surrounded by polyphenolic lignin.

The complexity of the matrix makes it difficult to degrade by microorganisms as lignin and hemicellulose components must be degraded before enzymes can act on the core cellulose microfibrils. Ordinarily, a consortium of different microorganisms is required to degrade cell wall polymers to release the constituent monosaccharides. For saccharification of plant cell walls, the lignin must be permeabilized and hemicellulose removed to allow cellulose-degrading enzymes to act on their substrate. For industrial saccharification of cell walls, large amounts of primarily fungal cellulases are added to processed feedstock that has been treated with dilute sulfuric acid at high temperature and pressure to permeabilize the lignin and partially saccharify the hemicellulose constituents.

*Saccharophagus degradans* strain 2-40 (herein referred to as "*S. degradans* 2-40" or "2-40") is a representative of an emerging group of marine bacteria that degrade complex polysaccharides (CP). *S*. *degradans* has been deposited at the American Type Culture Collection and bears accession number ***ATCC*** 43961. *S. degradans* 2-40, formerly known and referred to synonymously herein as *Microbulbifer degradans* strain 2-40 ("M. *degradans* 2-40"), is a marine □-proteobacterium that was isolated from decaying *Sparina alterniflora,* a salt marsh cord grass in the Chesapeake Bay watershed. Consistent with its isolation from decaying plant matter, *S*. *degradans* strain 2-40 is able to degrade many complex polysaccharides, including cellulose, pectin, xylan, and chitin, which are common components of the cell walls of higher plants. *S*. *degradans* strain 2-40 is also able to depolymerize algal cell wall components, such as agar, agarose, and laminarin, as well as protein, starch, pullulan, and alginic acid. In addition to degrading this plethora of polymers, *S. degradans* strain 2-40 can utilize each of the polysaccharides as the sole carbon source. Therefore, *S. degradans* strain 2-40 is not only an excellent model of microbial degradation of insoluble complex polysaccharides (ICPs) but can also be used as a paradigm for complete metabolism of these ICPs. ICPs are polymerized saccharides that are used for form and structure in animals and plants. They are insoluble in water and therefore are difficult to break down.

*Microbulbifer degradans* strain 2-40 requires at least 1 % sea salts for growth and will tolerate salt concentrations as high as 10%. It is a highly pleomorphic, Gram-negative bacterium that is aerobic, generally rod-shaped, and motile by means of a single polar flagellum. Previous work has determined that 2-40 can degrade at least 10 different carbohydrate polymers (CP), including agar, chitin, alginic acid, carboxymethylcellulose (CMC), β-glucan, laminarin, pectin, pullulan, starch and xylan (Ensor, Stotz et al. 1999). In addition, it has been shown to synthesize a true tyrosinase (Kelley, Coyne et al. 1990). 16S rDNA analysis shows that 2-40 is a member of the gamma-subclass of the phylum *Proteobacteria,* related to *Microbulbifer hydrolyticus* (Gonzalez and Weiner 2000) and to *Teridinibacter sp*.,(Distel, Morrill et al. 2002) cellulolytic nitrogen-fixing bacteria that are symbionts of shipworms.

The agarase, chitinase and alginase systems have been generally characterized. Zymogram activity gels indicate that all three systems are comprised of multiple depolymerases and multiple lines of evidence suggest that at least some of these depolymerases are attached to the cell surface (Stotz 1994; Whitehead 1997; Chakravorty 1998). Activity assays reveal that the majority of 2-40 enzyme activity resides with the cell fraction during logarithmic growth on CP, while in later growth phases the bulk of the activity is found in the supernatant and cell-bound activity decreases dramatically (Stotz 1994). Growth on CP is also accompanied by dramatic alterations in cell morphology. Glucose-grown cultures of 2-40 are relatively uniform in cell size and shape, with generally smooth and featureless cell surfaces. However, when grown on agarose, alginate, or chitin, 2-40 cells exhibit novel surface structures and features.

These exo- and extra-cellular structures (ES) include small protuberances, larger bleb-like structures that appear to be released from the cell, fine fimbrae or pili, and a network of fibril-like appendages which may be tubules of some kind. Immunoelectron microscopy has shown that agarases, alginases and/or chitinases are localized in at least some types of 2-40 ES. The surface topology and pattern of immunolocalization of 2-40 enzymes to surface protuberances are very similar to what is seen with cellulolytic members of the genus *Clostridium.*

The oldest methods studied to convert lignocellulosic materials to saccharides are based on acid hydrolysis (see, e.g., review by Grethlein, Chemical Breakdown Of Cellulosic Materials, J.APPL.CHEM. BIOTECHNOL. 28:296-308 (1978)). This process can involve the use of concentrated or dilute acids. For example, U.S. Patent Nos. 5,221,537 and 5,536,325, describe a two-step process for the acid hydrolysis of lignocellulosic material to glucose. These processes have numerous disadvantages including, for example, recovery of the acid, the specialized materials of construction required, the need to minimize water in the system, and the high production of degradation products which can inhibit the fermentation to ethanol.

To overcome the problems of the acid hydrolysis process, cellulose conversion processes are being developed using enzymatic hydrolysis. See, for example, U.S. Patent No. 5,916,780, which discloses enzymatic hydrolysis with a pre-treatment step to break down the integrity of the fiber structure and make the cellulose more accessible to attack by cellulase enzymes in the treatment phase.

U.S. Patent No. 6,333,181, discloses production of ethanol from lignocellulosic material by treatment of a mixture of lignocellulose, cellulose, and an ethanologenic microorganism with ultrasound.

There exists a need to identify enzyme systems that use cellulose as a substrate, express the genes encoding the proteins using suitable vectors, identify and isolate the amino acid products (enzymes and non-enzymatic products), and use these products as well as organisms containing these genes for purposes, such as the production of ethanol and uses described in the Bhat paper. There is also a need in the art of using lignocellulosic materials for production of ethanol, to develop more effective treatment methods that result in greater yields of ethanol.

### SUMMARY OF THE INVENTION

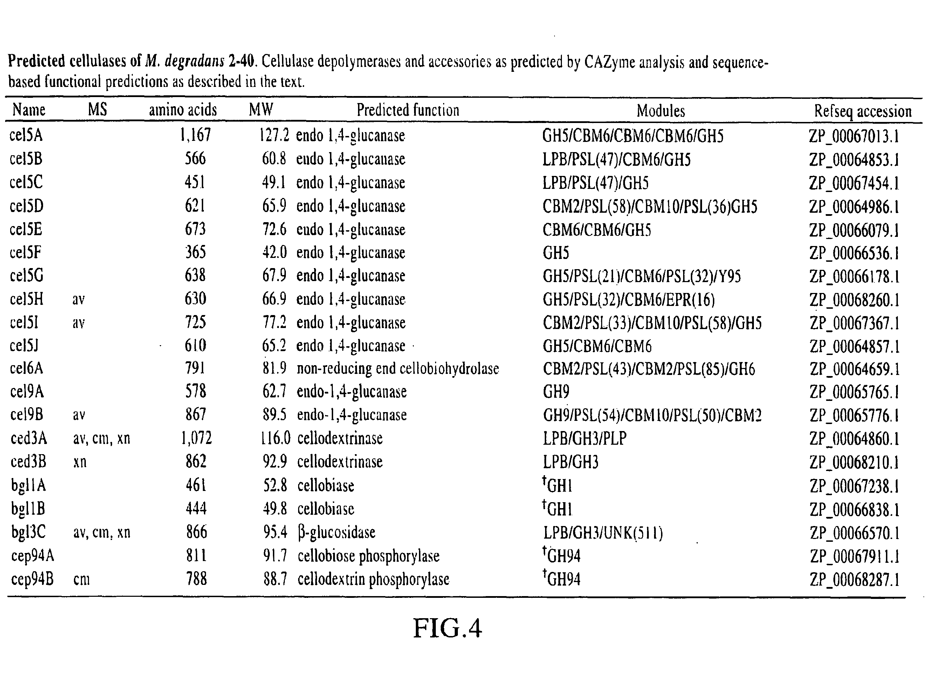

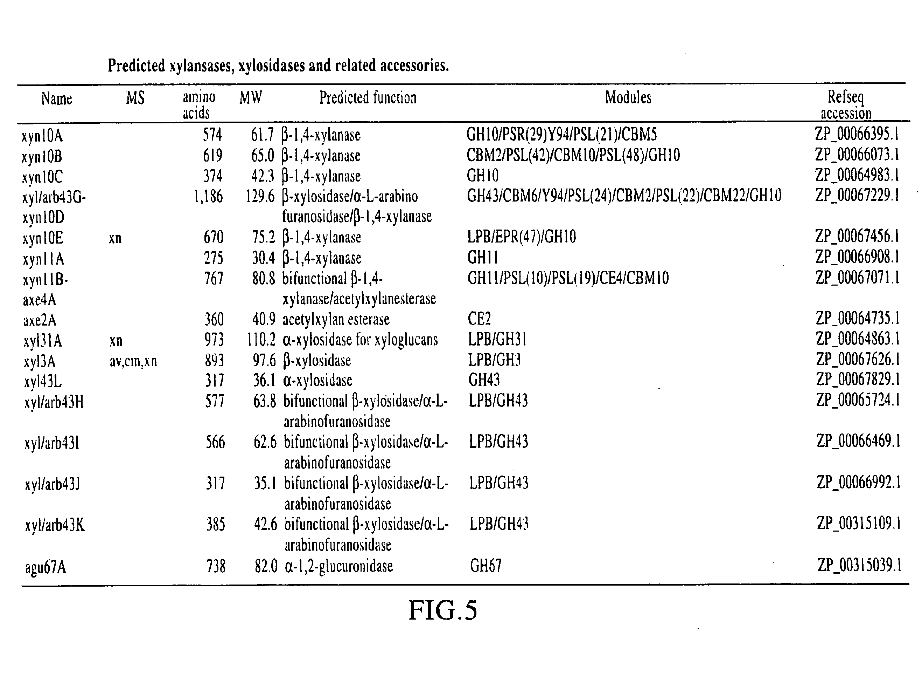

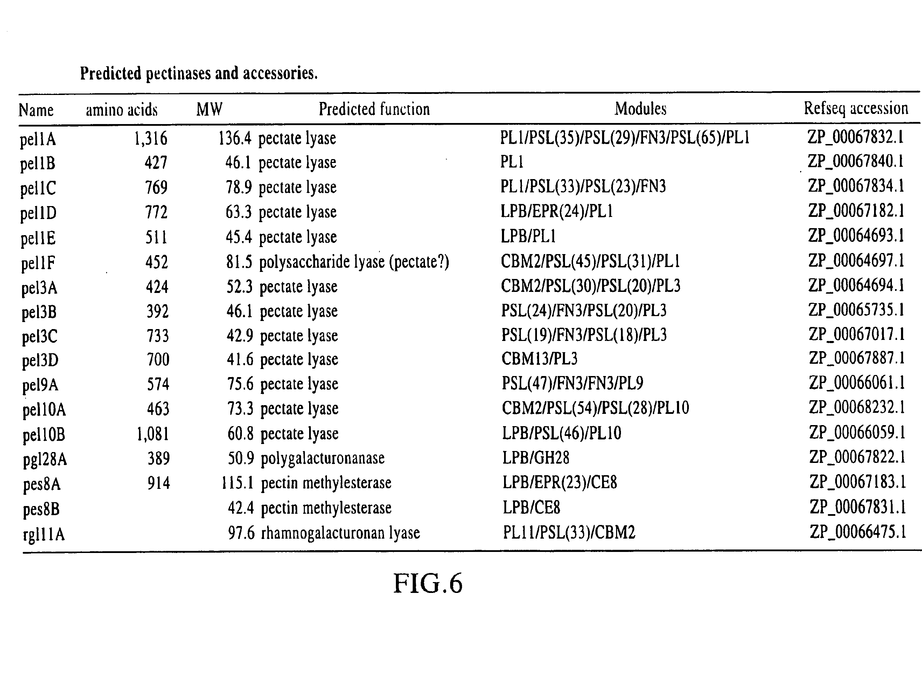

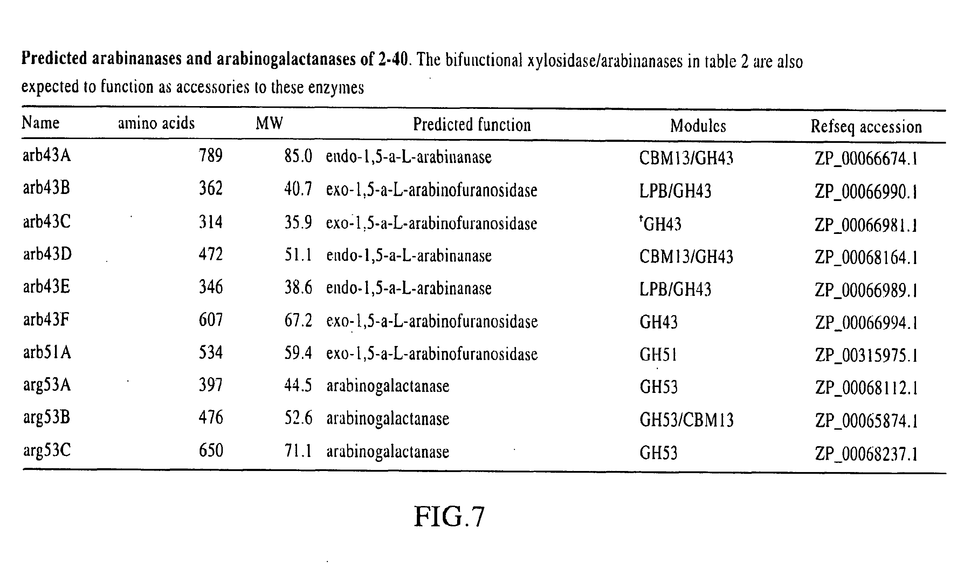

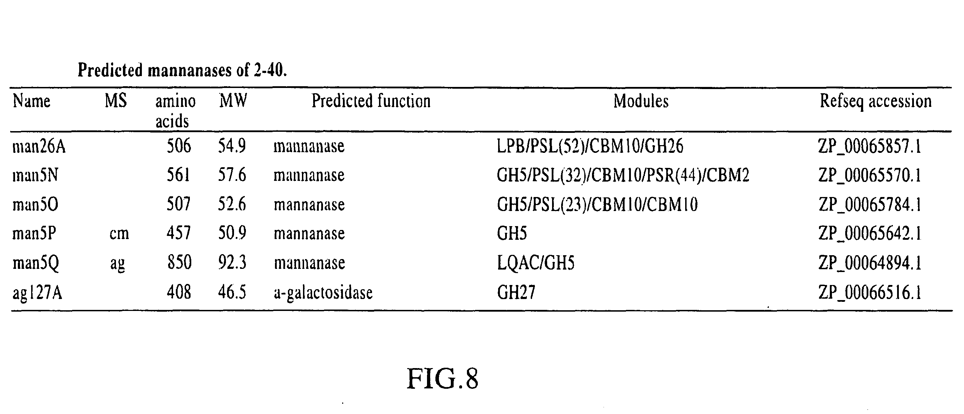

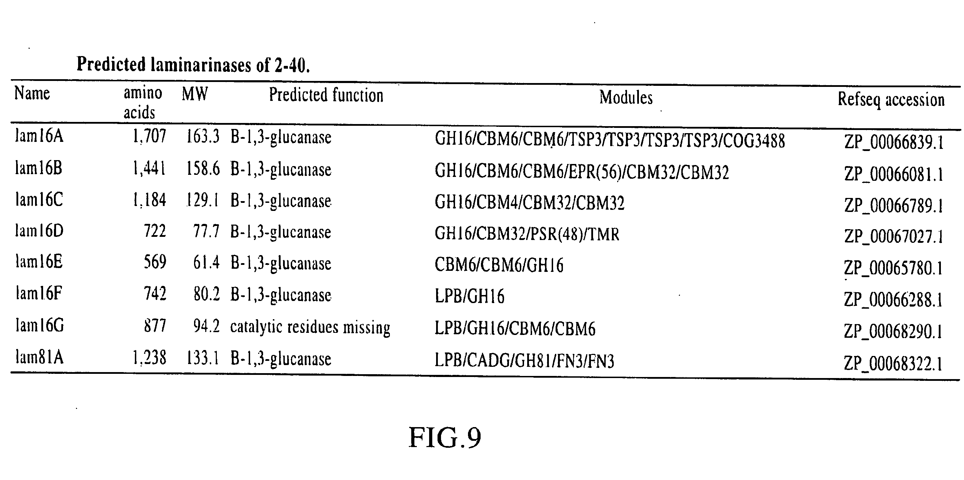

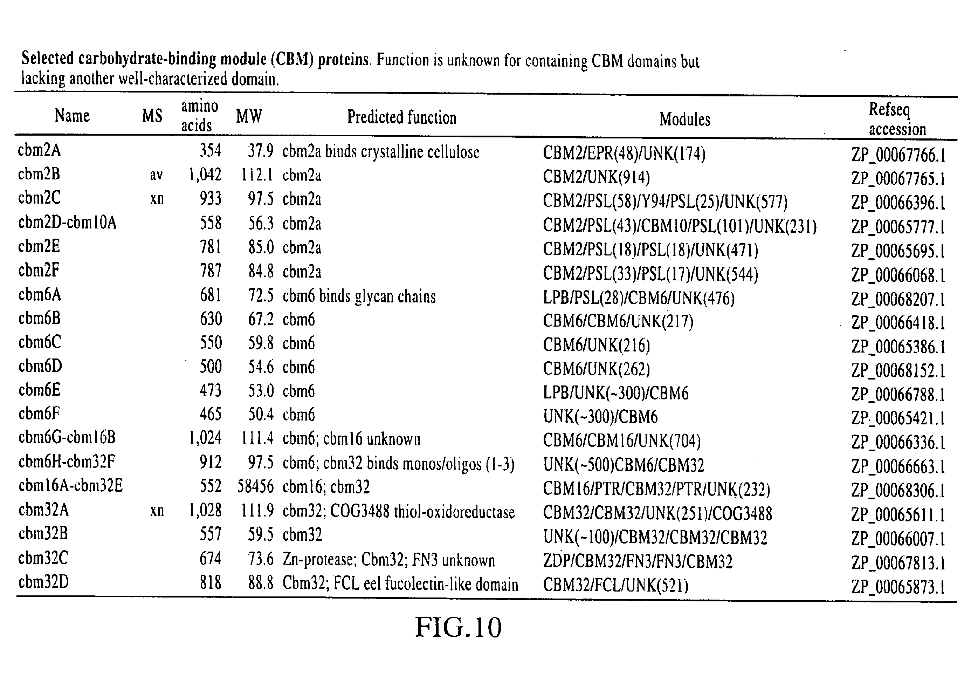

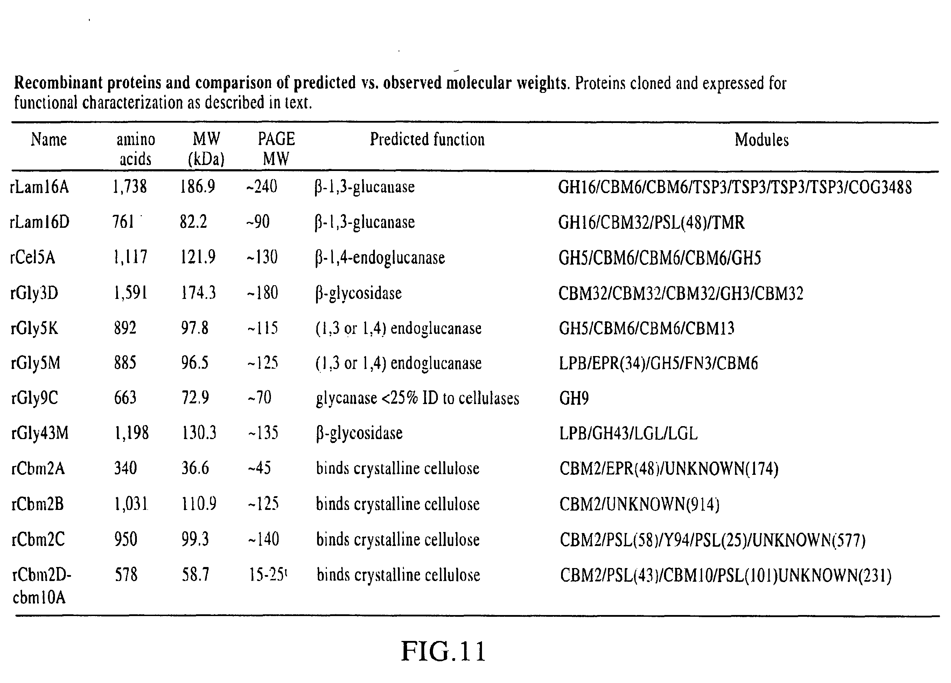

One aspect of the present invention concerns a method for producing ethanol from lignocellulosic material, comprising: contacting lignocellulosic material with *Saccharophagus degradans* expressing an effective saccharifying amount of one or more compounds listed in Figures 4-11, to obtain saccharides; and converting the saccharides to produce ethanol, as defined in claim 1. Specific embodiments of the present invention are defined in the dependent claims 2-10.

In addition, the present invention concerns a method for producing saccharides from lignocellulosic material, comprising: contacting lignocellulosic material with *Saccharophagus degradans* expressing an effective saccharifying amount of one or more compounds listed in Figures 4-11 to obtain saccharides as defined in claim 12.

Specific aspects of said method are defined in the dependent claim 13.

The present invention further concerns the use of *Saccharophagus degradans* expressing an effective saccharifying amount of one or more compounds listed in Figures 4-11 for the production of ethanol from lignocellulosic material.

Other embodiment is directed to the use of *Saccharophagus degradans* expressing an effective saccharifying amount of one or more compounds listed in Figures 4-11 to obtain saccharides from lignocellulosic material.

Conversion of sugars to ethanol and recovery may be accomplished by, but are not limited to, any of the well-established methods known to those of skill in the art. For example, through the use of an ethanologenic microorganism, such as *Zymomonas, Erwinia, Klebsiella, Xanthomonas, and Escherichia,* preferably *Escherichia coli* K011 and *Klebsiella oxytoca* P2.

Such an ethanologenic microorganism expresses an effective amount of one or more compounds listed in Figures 4-11 to saccharify the lignocellulosic material and an effective amount of one or more enzymes or enzyme systems which, in turn, catalyze (individually or in concert) the conversion of the saccharides to ethanol.

The cellulose degrading substances can be used in food, beer, wine, animal feeds, textile production and laundering, pulp and paper industry, and agricultural industries. The present invention is advantageous in that saccharification of plant cell walls and ethanol production processes including saccharification may be obtained without permeabilizing lignin and/or removing or partially saccharifying the hemicellulose or hemicellulose constituents before the cellulose-degrading enzymes can act on their substrate. The present invention also allows for saccharification and ethanol production processes including saccharification without or with a reduced amount of fungal cellulases, acids (e.g., sulfuric acid), high temperatures, and high pressures in the saccharification process.

### BRIEF DESCRIPTION OF THE FIGURES

Figure 1A shows the chemical formula of cellulose;

Figure 1 B illustrates the physical structure of cellulose;

Figure 2A illustrates the degradation of cellulose fibrils;

Figure 2B shows the chemical representation of cellulose degradation to cellobiose and glucose;

Figure 3 shows SDS-PAGE and Zymogram analysis of 2-40 culture supernatants;

Figure 4 lists the predicted cellulases of *S*. *degradans* 2-40 (the sequences from Figures 4-10 are disclosed as SEQ ID NOs 1-214, respectively in order of appearance in the appendix);

Figure 5 lists the predicted xylanases, xylosidases and related accessories of *M*. *degradans* 2-40;

Figure 6 lists the predicted pectinases and related accessories of *S*. *degradans* 2-40;

Figure 7 lists the arabinanases and arabinogalactanases of *S*. *degradans 2-40;*

Figure 8 lists the mannanases of *S*. *degradans* 2-40;

Figure 9 lists the laminarinases of *S*. *degradans 2-40;*

Figure 10 lists selected carbohydrate-binding module proteins of *S. degradans* 2-40; and

Figure 11 lists the recombinant proteins of *S*. *degradans* 2-40 and a comparison of predicted vs. observed molecular weights thereof.

### DETAILED DESCRIPTION

Analysis of the genome sequence of *S. degradans* 2-40 reveals an abundance of genes coding for enzymes that are predicted to degrade plant-derived carbohydrates. To date, 2-40 is the only sequenced marine bacterium with apparently complete cellulase and xylanase systems, as well as a number of other systems containing plant-wall active carbohydrases.

Thus it appears that 2-40 can play a significant role in the marine carbon cycle, functioning as a "super-degrader" that mediates the breakdown of CP from various algal, plantal, and invertebrate sources. The remarkable enzymatic diversity, novel surface features (ES), and the apparent localization of carbohydrases to ES make *S*. *degradans* 2-40 an intriguing organism in which to study the cell biology of CP metabolism and surface enzyme attachment.

It has now been discovered that 2-40 has a complete complement of enzymes, suitably positioned, to degrade plant cell walls. This has been accomplished by the following approaches: a) annotation and genomic analysis of 2-40 plant-wall active enzyme systems, b) identification of enzymes and other proteins which contain domains or motifs that may be involved in surface enzyme display, c) the development of testable models based on identified protein motifs, and d) cloning and expression of selected proteins for the production of antibody probes to allow testing of proposed models of surface enzyme display using immunoelectron microscopy.

These efforts have been greatly facilitated by the recent sequencing of the genome of 2-40, allowing a strategy where genes which code for proteins with potential involvement in surface attachment may be identified based on sequence homology with modules or domains known to function in surface attachment and/or adhesion.

Enzymatic and non-enzymatic ORFs with compelling sequence elements are identified using BLAST and other amino acid sequence alignment and analysis tools. Genes of interest can be cloned into *E coli,* expressed with in-frame polyhistidine affinity tag fusions and purified by nickel ion chromatography, thus providing the means of identifying and producing recombinant 2-40 proteins for study and antibody probe production.

The genome sequence of 2-40 was recently obtained in conjunction with the Department of Energy's Joint Genome Initiative (JGI). The finished draft sequence dated January 19, 2005 comprises 5.1Mbp contained in a single contiguous sequence. Automated annotation of open reading frames (ORFs) was performed by the computational genomics division of the Oak Ridge National Laboratory (ORNL), and the annotated sequence is available on the World Wide Web (hftp://genome.ornl.gov/microbial/mdeg).

The initial genome annotation has revealed a variety of carbohydrases, including a number of agarases, alginases and chitinases. Remarkably, the genome also contains an abundance of enzymes with predicted roles in the degradation of plant cell wall polymers, including a number of ORFs with homology to cellulases, xylanases, pectinases, and other glucanases and glucosidases. In all, over 180 open reading frames with a probable role in carbohydrate catabolism were identified in the draft genome.

To begin to define the cellulase, xylanase and pectinase systems of 2-40, genes were initially classified as belonging to one of those systems by BLAST homology. Ambiguous ORFs were tentatively assigned to the class of the best known hit. Other tools used to refine this tentative classification include Pfam (Protein families database of alignments and HMMs; http:/www.sanger.ac.uk/Software/Pfam/) and SMART (Simple Modular Architecture Research Tool; http://smart.embl-heidelberg.de/) which use multiple alignments and hidden Markov models (statistical models of sequence consensus homology) to identify discreet modular domains within a protein sequence. These analyses were relatively successful; however, a number of ORFs remained difficult to classify based on sequence homology alone.

Enzymes have traditionally been classified by substrate specificity and reaction products. In the pre-genomic era, function was regarded as the most amenable (and perhaps most useful) basis for comparing enzymes and assays for various enzymatic activities have been well-developed for many years, resulting in the familiar EC classification scheme. Cellulases and other *O*-Glycosyl hydrolases, which act upon glycosidic bonds between two carbohydrate moieties (or a carbohydrate and non-carbohydrate moiety as occurs in nitrophenol-glycoside derivatives) are designated as EC 3.2.1.-, with the final number indicating the exact type of bond cleaved. According to this scheme an endo-acting cellulase (1,4-β-endoglucanase) is designated EC 3.2.1.4.

With the advent of widespread genome sequencing projects and the ease of determining the nucleotide sequence of cloned genes, ever-increasing amounts of sequence data have facilitated analyses and comparison of related genes and proteins on an unprecedented scale. This is particularly true for carbohydrases; it has become clear that classification of such enzymes according to reaction specificity, as is seen in the E.C. nomenclature scheme, is limited by the inability to convey sequence similarity. Additionally, a growing number of carbohydrases have been crystallized and their 3-D structures solved.

One of the major revelations of carbohydrase sequence and structure analyses is that there are discreet families of enzymes with related sequence, which contain conserved three-dimensional folds that can be predicted based on their amino acid sequence. Further, it has been shown that enzymes with the same three-dimensional fold exhibit the same stereospecificity of hydrolysis, even when they catalyze different reactions (Henrissat, Teeri et al. 1998; Coutinho and Henrissat 1999).

These findings form the basis of a sequence-based classification of carbohydrase modules which is available in the form of an internet database, the Carbohydrate-Active enZYme server (CAZy), at http://afmb.cnrs-mrs.fr/CAZY/index:html (Coutinho and Henrissat 1999; Coutinho and Henrissat 1999).

CAZy defines four major classes of carbohydrases, based on the type of reaction catalyzed: Glycosyl Hydrolases (GH's), Glycosyltransferases (GT's), Polysaccharide Lyases (PL's), and Carbohydrate Esterases (CE's). GH's cleave glycosidic bonds through hydrolysis. This class includes many familiar polysaccharidases such as cellulases, xylanases, and agarases. GT's generally function in polysaccharide synthesis, catalyzing the formation of new glycosidic bonds through the transfer of a sugar molecule from an activated carrier molecule, such as uridine diphosphate (UDP), to an acceptor molecule. While GT's often function in biosynthesis, there are examples where the mechanism is exploited for bond cleavage, as occurs in the phosphorolytic cleavage of cellobiose and cellodextrins (Lou, Dawson et al. 1996). PL's use a β-elimination mechanism to mediate bond cleavage and are commonly involved in alginate and pectin depolymerization. CE's generally act as deacetylases on *O*- or *N-*substituted polysaccharides. Common examples include xylan and chitin deacetylases. Sequence-based families are designated by number within each class, as is seen with GH5: glycosyl hydrolase family 5. Members of GH5 hydrolyze β-1,4 bonds in a retaining fashion, using a double-displacement mechanism which results in retention of the original bond stereospecificity. Retention or inversion of anomeric configuration is a general characteristic of a given GH family (Henrissat and Bairoch 1993; Coutinho and Henrissat 1999). Many examples of endocellulases, xylanases and mannanases belonging to GH5 have been reported, illustrating the variety of substrate specificity possible within a GH family. Also, GH5s are predominantly endohydrolases cleaving chains of their respective substrates at random locations internal to the polymer chains. While true for GH5, this generalization does not hold for many other GH families. In addition to carbohydrases, the CAZy server defines numerous families of Carbohydrate Binding Modules (CBM). As with catalytic modules, CBM families are designated based on amino acid sequence similarity and conserved three-dimensional folds.

The CAZyme structural families have been incorporated into a new classification and nomenclature scheme, developed by Bernard Henrissat and colleagues (Henrissat, Teeri et al. 1998). Traditional gene/protein nomenclature assigns an acronym indicating general function and order of discovery; in this scheme an organism's cellulase genes are designated celA, celB, etc., regardless of their actual mechanism of action on cellulose. Some researchers have attempted to convey more information by naming cellulases as endoglucanases (engA, engB) or cellobiohydrolases (cbhA, cbhB), however this requires determination of function *in vitro* and still fails to convey relatedness of protein sequence and structure. CAZyme nomenclature retains the familiar acronym to indicate the functional system a gene belongs to and incorporates the family number designation. Capital letters after the family number indicate the order of report within a given organism system. An example is provided by two endoglucanases, CenA and CenB, of *Cellulomonas fimi.* In the old nomenclature nothing can be deduced from the names except order of discovery. Naming them Cel6A and Cel9A, respectively, makes it immediately clear that these two cellulases are unrelated in sequence, and so belong to different GH families (where Cel stands for cellulase, and 9 for glycosyl hydrolase family nine). While this scheme does not distinguish between endo- and exo- activity, these designations are not absolute and can be included in discussion of an enzyme when relevant (i.e. the cellobiohydrolase Cel6A, the endoxylanase Xyn10B). Catalytic modules take precedence in naming carbohydrases; since many (or even most) carbohydrases contain at least one CBM, they are named for their enzymatic module. If more than one catalytic domain is present, they are named in order from N-terminus to C-terminus, i.e. cel9A-cel48A contains a GH9 at the amino-terminus and a GH48 at the carboxy-terminus. Both domains act against cellulose. There are, however, many examples of CBM modules occurring on proteins with no predicted carbohydrase module. In the absence of some other predicted functional domain (like a protease) these proteins are named for the CBM module family. If there are multiple CBM families present, then naming is again from amino to carboxy end, i.e. cbm2D-cbm10A (Henrissat, Teeri et al. 1998). This nomenclature has been widely accepted and will be used in the naming of all 2-40 plant-wall active carbohydrases and related proteins considered as part of this study.

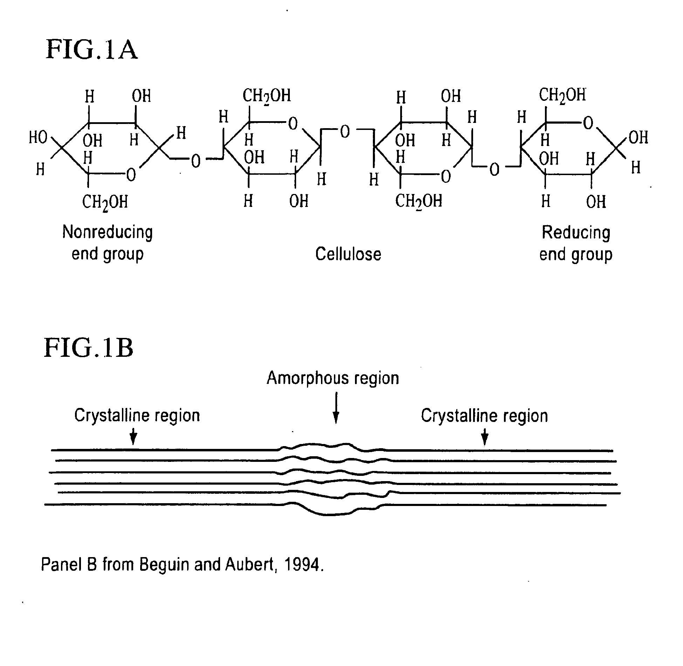

The cell walls of higher plants are comprised of a variety of carbohydrate polymer (CP) components. These CP interact through covalent and non-covalent means, providing the structural integrity plants required to form rigid cell walls and resist turgor pressure. The major CP found in plants is cellulose, which forms the structural backbone of the cell wall. See Fig. 1A. During cellulose biosynthesis, chains of poly-β-1,4-D-glucose self associate through hydrogen bonding and hydrophobic interactions to form cellulose microfibrils which further self-associate to form larger fibrils. Cellulose microfibrils are somewhat irregular and contain regions of varying crystallinity. The degree of crystallinity of cellulose fibrils depends on how tightly ordered the hydrogen bonding is between its component cellulose chains. Areas with less-ordered bonding, and therefore more accessible glucose chains, are referred to as amorphous regions (Figure 1 B). The relative crystallinity and fibril diameter are characteristic of the biological source of the cellulose (Beguin and Aubert 1994; Tomme, Warren et al. 1995; Lynd, Weimer et al. 2002). The irregularity of cellulose fibrils results in a great variety of altered bond angles and steric effects which hinder enzymatic access and subsequent degradation.

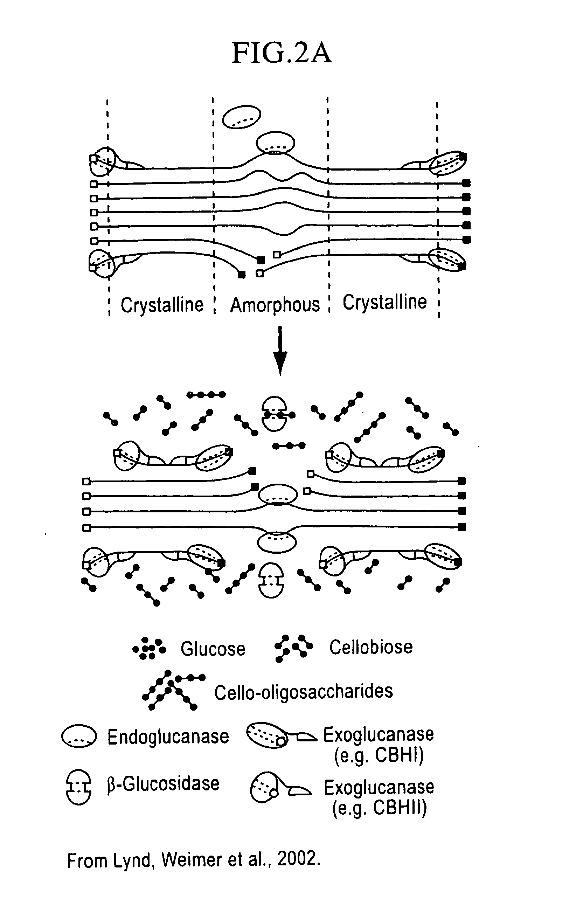

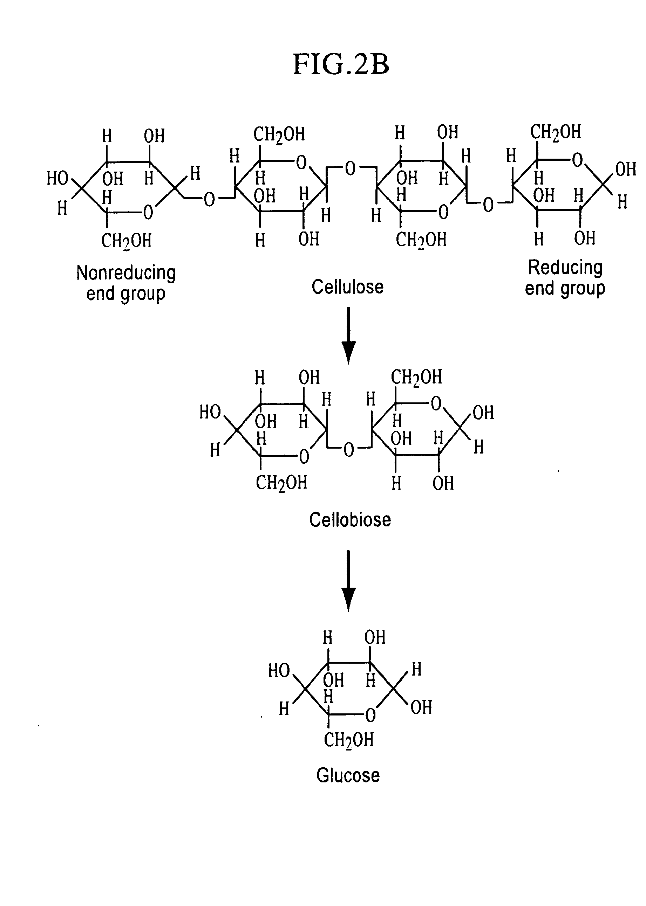

The general model for cellulose depolymerization to glucose involves a minimum of three distinct enzymatic activities (See Figures 2A and 2B). Endoglucanases cleave cellulose chains internally to generate shorter chains and increase the number of accessible ends, which are acted upon by exoglucanases. These exoglucanases are specific for either reducing ends or non-reducing ends and frequently liberate cellobiose, the dimer of cellulose (cellobiohydrolases). The accumulating cellobiose is cleaved to glucose by cellobiases (β-1,4-glucosidases). In many systems an additional type of enzyme is present: cellodextrinases are β-1,4-glucosidases which cleave glucose monomers from cellulose oligomers, but not from cellobiose. Because of the variable crystallinity and structural complexity of cellulose, and the enzymatic activities required for is degradation, organisms with "complete" cellulase systems synthesize a variety of endo and/or exo-acting β-1,4-glucanases.

For example, *Cellulomonas fimi* and *Thermomonospora fusca* have each been shown to synthesize six cellulases while *Clostridium thermocellum* has as many as 15 or more (Tomme, Warren et al. 1995). Presumably, the variations in the shape of the substrate-binding pockets and/or active sites of these numerous cellulases facilitate complete cellulose degradation (Warren 1996). Organisms with complete cellulase systems are believed to be capable of efficiently using plant biomass as a carbon and energy source while mediating cellulose degradation. The ecological and evolutionary role of incomplete cellulose systems is less clear, although it is believed that many of these function as members of consortia (such as ruminal communities) which may collectively achieve total or near-total cellulose hydrolysis (Ljungdahl and Eriksson 1985; Tomme, Warren et al. 1995).

In the plant cell wall, microfibrils of cellulose are embedded in a matrix of hemicelluloses (including xylans, arabinans and mannans), pectins (galacturonans and galactans), and various β-1,3 and β-1,4 glucans. These matrix polymers are often substituted with arabinose, galactose and/or xylose residues, yielding arabinoxylans, galactomannans and xytoglucans to name a few (Tomme, Warren et al. 1995; Warren 1996; Kosugi, Murashima et al. 2002; Lynd, Weimer et al. 2002). The complexity and sheer number of different glycosyl bonds presented by these non-cellulosic CP requires specific enzyme systems which often rival cellulase systems in enzyme count and complexity. Because of its heterogeneity, plant cell wall degradation often requires consortia of microorganisms (Ljungdahl and Eriksson 1985; Tomme, Warren et al. 1995).

Objectives -*S*. *degradans* and *M degradans* synthesize complete multi-enzyme systems that degrade the major structural polymers of plant cell walls. A) define cellulase and xylanase systems, determining the activities of genes for which function cannot be predicted by sequence homology; and B) genomic identification and annotation of other plant-degrading enzyme systems by sequence homology (i.e. pectinases, laminarinases, etc.).

All publications, patents and patent applications are herein expressly incorporated by reference in their entirety to the same extent as if each individual publication, patent or patent application was specifically and indicated individually to be incorporated by reference in its entirety.

The following examples illustrate but are not intended in any way to limit the scope of the invention.

Experimental Results

I: Genomic, proteomic and functional analyses of 2-40 plant-wall active enzymes

From the ORNL annotation it is clear that the 2-40 genome contains numerous enzymes with predicted activity against plant cell wall polymers. This is particularly surprising since 2-40 is an estuarine bacterium with several complex enzyme systems that degrade common marine polysaccharides such as agar, alginate, and chitin. Defining multienzyme systems based on automated annotations is complicated by the presence of poorly conserved domains and/or novel combinations of domains. There are many examples of this in the plant-wall active enzymes of 2-40. Accordingly, the ORNL annotations of carbohydrase ORFs were manually reviewed with emphasis on the modular composition and then assigned to general groups based on the substrate they were likely to be involved with (i.e. cellulose or xylan degradation). These genomic sequence analyses resulted in a pool of about 25 potential cellulases, 11 xylanases and 17 pectinases.

When sequence homology is well-conserved, highly accurate predictions of function are possible. Therefore, to verify the presence of functioning cellulase and xylanase systems in *M degradans,* zymograms and enzyme activity assays were performed as discussed below. Also, attempts were made to identify enzymes from 2-40 culture supernatants using Mass Spectrometry based proteomics.

Next, more sophisticated genomic analyses were used to predict function where possible and to identify ORFs which require functional characterization to determine their roles, if any, in the cellulase and xylanase systems. ORFs which belong to other plant wall-active enzyme systems were tentatively classified based on the sequence analyses and functional predictions of B. Henrissat.

To gain insight into the induction and expression of 2-40 cellulases and xylanases, specific activities were determined for avicel and xylan-grown cells and supernatants by dinitrosalicylic acid reducing-sugar assays (DNSA assays), as discussed in the Experimental Protocols section at the end of this proposal. Xylanase activity was measured for avicel-grown cultures, and vice versa, in order to investigate possible co-induction of activity by these two substrates which occur together in the plant cell wall.

Growth on either avicel or xylan yields enzymatic activity against both substrates, suggesting co-induction of the cellulase and xylanase systems. As with other 2-40 carbohydrase systems, highest levels of activity were induced by the homologous substrate. The results also reveal some key differences in the expression of these two systems. When grown on avicel, cellulase activity is cell-associated in early growth and accumulates significantly in late-stage supernatants. Cell and supernatant fractions exhibit low levels of xylanase activity that remain roughly equal throughout all growth phases. In contrast, xylan-grown cultures exhibit the majority of xylanase and cellulase activity in the cellular fraction throughout the growth cycle. Cellulase activity does not accumulate in the supernatant and xylanase activity accumulates modestly, but still remains below the cell-bound activity.

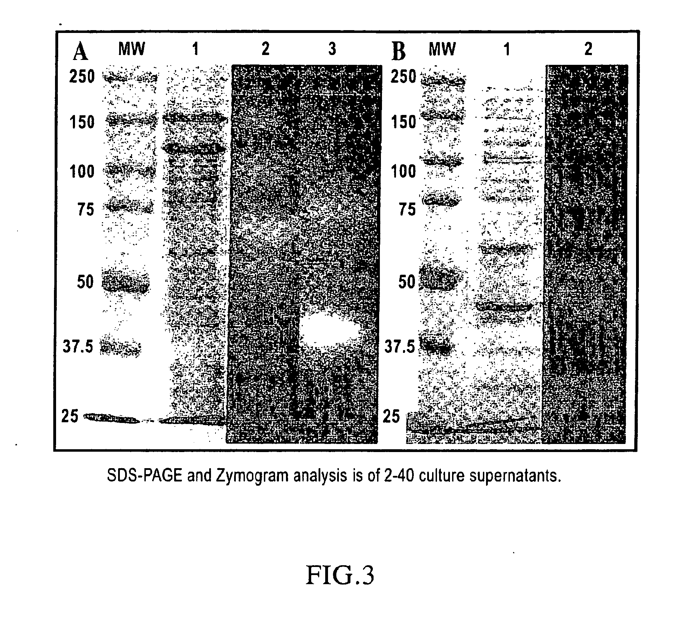

Enzyme activity gels (zymograms) of avicel and xylan grown cell pellets and culture supernatants were analyzed to visualize and identify expressed cellulases and xylanases. The zymograms revealed five xylanolytic bands in xylan-grown supernatants (Figure 3), four of which correspond well with the calculated MW of predicted xylanases (xyl/arb43G-xyn10D: 129.6kDa, xyn10E: 75.2kDa, xyn10C: 42.3kDa, and xyn11A: 30.4kDa; see Table 2). Avicel-grown cultures showed eight active bands with MWs ranging from 30-150kDa in CMC zymograms. CMC is generally a suitable substrate for endocellulase activity. These zymograms clearly demonstrate that 2-40 synthesizes a number of endocellulases of varied size during growth on avice indicative of a functioning multienzyme cellulase system. Together, the CMC and xylan zymograms confirm the results of the genomic analyses and the inducible expression of multienzyme cellulase and xylanase systems in *M degradans* 2-40.

To identify individual cellulases and xylanases produced during growth on CP, culture supernatants were subjected to proteomic analysis using reversed-phase high-performance liquid chromatography (RP-HPLC) coupled with tandem Mass Spectrometry (MS/MS). The power resulting from separating the peptides on the RP-HPLC column prior to electrospray ionization and MS/MS analysis allows the identification of a great number of proteins from complex samples (Smith, Loo et al. 1990; Shevchenko, Wilm et al. 1996; Jonsson, Aissouni et al. 2001). These analyses confidently identified over 100 different non-enzymatic proteins and a number of carbohydrases, including a xylanase, two xylosidases, a cellulase, and two cellodextrinases. An agarase was identified during additional analyses of agarose-grown supernatant.

Gel-slice digestion, extraction, and MS/MS analyses performed at the Stanford University Mass Spectrometry facility identified two annotated cellulases from an avicel-grown supernatant sample. One, designated cel5H, has a predicted MW of 67kDa and was identified from a band with an apparent MW of 75kDa. The other, cel9B, has a predicted MW of 89kDa, but an apparent MW of 120kDa. The discrepancy between the predicted and apparent MW of cel9B is consistent with similar instances where certain 2-40 proteins, cloned and expressed in *E coli,* exhibit apparent MWs which are 30-40% higher than their predicted MW.

The amino acid translations of all gene models in the 2-40 draft genome were analyzed on the CAZy ModO (Carbohydrase Active enZyme Modular Organization) server at AFMB-CRNS. This analysis identified all gene models that contain a catalytic module (GH, GT, PL, or CE) and/or a CBM. In all, the genome contains 222 gene models containing CAZy domains, most of which have modular architecture. Of these, 117 contain a GH module, 39 have GTs, 29 PLs, and 17 CE. Many of these carry one or more CBM from various families. There are also 20 proteins that contain a CBM but no predicted carbohydrase domain.

Detailed comparisons of 2-40 module sequences to those in the ModO database allowed specific predictions of function for modules where the sequence of the active site is highly conserved. For example, Cel9B (from the gel slice MS/MS) contains a GH9 module which is predicted to function as an endocellulase, a CBM2 and a CBM10 module.

When catalytic module sequences are less conserved, only a general mechanism can be predicted. This is the case with gly5M which contains a GH5 predicted to be either a 1,3 or 1,4 glucanase sequence analysis cannot be certain which, and so the acronym designation "gly" for glycanase.

The results of this detailed evaluation and analysis were used to assign genes to cellulase, xylanase, pectinase, laminarinase, arabinanase and mannanase systems. Each system was also assigned the relevant accessory enzymes, i.e. cellobiases belong to the cellulase system and xylosidases belong to the xylanase system. Genes with less-conserved GH modules which have the most potential to function as cellulases, xylanases or accessories were identified and designated as needing demonstration of function.

The results of the ORNL annotation, follow-up annotation analyses, proteomic (mass spectrometry) analyses, CAZyme modular analyses and functional predictions have been incorporated into Figures 4-11, which contain tables that summarize the predicted plant wall active carbohydrases and selected CBM only genes of 2-40.

The genes chosen for cloning and functional analysis include the carbohydrases gly3C, gly5K, gly5M, gly9C, and gly43M. Because the active site of gly5L is highly homologous to that of gly5K, its activity is inferred from the results obtained from gly5K. Four of the 20 "CBM only" proteins, cbm2A, cbm2B, cbm2C and cbm2D-cbm10A are included in activity assays to investigate their predicted lack of enzymatic function. These four contain CBM2 modules that are predicted to bind to crystalline cellulose. This predicted affinity is the reason for their inclusion in activity assays; those proteins that bind to cellulose are most likely to contain cellulase or xylanase modules which were not detected by sequence analysis. With CBM only proteins, a lack of detected enzyme activity will confirm the absence of a catalytic domain (CD).

In order to define the complete cellulase and xylanase systems of *M degradans,* those enzymes which may belong to the systems but cannot be confidently assigned based on sequence homology will be expressed, purified and assayed for activity as described in the Experimental Protocols. To date, gly3C, gly5K, gly5M, gly9C and gly43M, as well as cbm2A, cbm2B, cbm2C and cbm2D-cbm10A, have been cloned into expression strains as pETBlue2 (Novagen) constructs. This vector places expression under the control an inducible T7 *lac* promoter and incorporates a C-terminal 6xHistidine tag, allowing purification of the recombinant protein by nickel ion affinity. Successful cloning and expression of these proteins was confirmed by western blots using α-HisTag^{®} monoclonal antibody (Novagen). All expressed proteins have apparent MWs which are close to, or larger, than their predicted MW (Table 8) except for Cbm2D-Cbm10A which appears to be unstable; two separate attempts to clone and express this protein have resulted in HisTag® containing bands which occur near the dye front in western blots, suggesting proteolytic degradation of this gene product. An additional enzyme, Cel5A, has been cloned and expressed for use as an endocellulase positive control in activity assays. Cel5A has a predicted MW of 129 kDa, contains two GH5 modules, and is highly active in HE-cellulose zymograms.

The major criteria for assigning function will be the substrate acted upon, and the type of activity detected. As such, the various enzyme activity assays will focus on providing a qualitative demonstration of function rather than on rigorously quantifying relative activity levels. The assays required are dictated by the substrate being tested, and are discussed in more detail in Experimental Protocols. For cellulose it is important to distinguish between β-1,4-endoglucanase (endocellulase), β-1,4-exoglucanase (cellobiohydrolase), and β-1,4-glucosidase (cellobiase) activities. This will be accomplished using zymograms to assay for endocellulase, DNSA reducing-sugar assays for cellobiohydrolase, and p-nitrophenol-β-1,4-cellobioside (pnp-cellobiose) for cellobiase activity. The combined results from all three assays will allow definition of function as follows: a positive zymogram indicates endocellulase activity, a negative zymogram combined with a positive DNSA assay and a negative pnp-cellobiose assay indicates an exocellulase, while a negative zymogram and DNSA with a positive pnp-cellobiose result will imply that the enzyme is a cellobiase.

Xylanase (β-1,4-xylanase), laminarinase (β-1,3-glucanase), and mixed glucanase (β-1,3(4)-glucanase) activity will be determined by xylan, laminarin and barley glucan zymograms, respectively. Unlike cellulose, there do not appear to be any reports of "xylobiohydrolases" or other exo-acting enzymes which specifically cleave dimers from these substrates. Thus zymograms will suffice for demonstrating depolymerase (endo) activity and pnp-derivatives will detect monosaccharide (exo) cleavage. The pnp-derivatives used in this study will include pnp-α-L-arabinofuranoside, -α-L-arabinopyranoside, -β-L-arabinopyranoside, -β-D-cellobioside, -α-D-xylopyranoside and -β-D-xylopyranoside. These substrates were chosen based on the possible activities of the domains in question. The assays will allow determination of function for any α- and β-arabinosidases, β-cellobiases, β-xylosidases, bifunctional α-arabinosidase/β-xylosidases, and α-xylosidases which cleave α-linked xylose substituents from xyloglucans. The pnp-derivative assays will be run in 96-well microtiter plates using a standard curve of p-nitrophenol concentrations, as discussed in Experimental Protocols.

The combination of assays for β-1,4-, β-1,3-, and β-1,3(4)- glucanase activities, as well as for β-1,4-xylanase and the various exo-glycosidase activities should clearly resolve the function of the ambiguous carbohydrases. Proteins with demonstrated activity will be assigned to the appropriate enzyme system.

Experimental Protocols

Zymograms

All activity gels were prepared as standard SDS-PAGE gels with the appropriate CP substrate incorporated directly into the separating gel. Zymograms are cast with 8% polyacrylamide concentration and the substrate dissolved in dH₂O and/or gel buffer solution to give a final concentration of 0.1 % (HE-cellulose), 0.15% (barley β-glucan), or 0.2% (xylan). Gels are run under discontinuous conditions according to the procedure of Laemmli (Laemmli 1970) with the exception of an 8 minute treatment at 95°C in sample buffer containing a final concentration of 2% SDS and 100mM dithiothreitol (DTT). After electrophoresis, gels are incubated at room temperature for 1 hour in 80ml of a renaturing buffer of 20mM PIPES buffer pH 6.8 which contains 2.5% Triton X-100, 2mM DTT and 2.5mM CaCl₂. The calcium was included to assist the refolding of potential calcium-binding domains such as the tsp3s of Lam16A.

After the 1 hour equilibration, gels were placed in a fresh 80ml portion of renaturing buffer and held overnight at 4°C with gentle rocking. The next morning gels were equilibrated in 80ml of 20mM PIPES pH6.8 for 1 hour at room temperature, transferred to a clean container, covered with the minimal amount of PIPES buffer and incubated at 37°C for 4 hours. Following incubation gels were stained for 30 minutes with a solution of either 0.25% Congo red in dH₂O (HE-cellulose, β-glucan and xylan) or 0.01% Toluidine blue in 7% acetic acid. Gels were destained with 1M NaCl for Congo red and dH₂O for Toluidine blue until clear bands were visible against a stained background.

Nelson-Somogyi reducing-sugar assays

Purified proteins were assayed for activity using a modification of the Nelson-Somogyi reducing sugar method adapted for 96-well microtiter plates, using 50ul reaction volumes (Green, Clausen et al. 1989). Test substrates included avicel, CMC, phosphoric-acid swollen cellulose (PASC), Barley glucan, laminarin, and xylan dissolved at 1% in 20mM PIPES pH 6.8 (Barley glucan and laminarin, 0.5%). Barley glucan, laminarin and xylan assays were incubated 2 hours at 37°C; avicel, CMC and PASC assays were incubated 36 hours at 37°C. Samples were assayed in triplicate, corrected for blank values, and levels estimated from a standard curve. Protein concentration of enzyme assay samples was measured in triplicate using the Pierce BCA protein assay according to the manufacture's instructions. Enzymatic activity was calculated, with one unit (U) defined as 1µM of reducing sugar released/minute and reported as specific activity in U/mg protein.

Exoglycosidase activity assays: pnp-derivatives

Purified proteins were assayed for activity against pNp derivatives of α-L-arabinofuranoside, -α-L-arabinopyranoside, -β-L-arabinopyranoside, -β-D-cellobioside, - α-D-glucopyranoside, -β-D-glucopyranoside, -α-D-xylopyranoside and -β-D-xylopyranoside. 25µl of enzyme solution was added to 125µl of 5mM substrate solution in 20mM PIPES pH 6.8, incubated for 30 min at 37°C, and A₄₀₅ was determined. After correcting for blank reactions, readings were compared to a p-nitrophenol standard curve and reported as specific activities in U/mg protein, with one unit (U) defined as 1µmol p-Np/min.

Mass Spectrometry and proteomic analyses

Stationary-phase supernatants from avicel, CMC, and xylan-grown cultures were concentrated to ~25X by centrifugal ultrafiltration using microcon or centricon devices (Millipore). Sample protein concentrations were determined by the BCA protein assay. Samples were exchanged into 100mM Tris buffer, pH 8.5, which also contained 8M urea and 10mM DTT. Samples were incubated 2 hours at 37°C with shaking to denature the proteins and reduce disulfide bonds. After reduction, 1 M iodoacetate was added to a final concentration of 50mM and the reaction was incubated 30 minutes at 25°C in the dark. This step alkylates the reduced cysteine residues, thereby preventing reformation of disulfide bonds. The samples are then exchanged into 50mM Tris, 1mM CaCl₂, pH 8.5 using microcon devices. The denatured, reduced, and alkylated sample is digested into peptide fragments using proteomics-grade trypsin (Promega) at a 1:50 enzyme (trypsin) to substrate (supernatant) ratio. Typical digestion reactions were around 150µl total volume. Digestions were incubated overnight at 37°C, stopped by addition of 99% formic acid to a final concentration of ~1% and analyzed by RPHPLC-MS/MS at the UMCP College of Life Sciences CORE Mass Spectrometry facility.

Peptide fragments were loaded onto a Waters 2960 HPLC fitted with a 12cm microbore column containing C₁₈ as the adsorbent and eluted with a linear gradient of increasing acetonitrile (CH₃CN) concentration into an electrospray ionization apparatus. The electrospray apparatus ionized and injected the peptides into a Finnagin LCQ tandem Mass Spectrometer. Automated operating software controlled the solvent gradient and continually scanned the eluted peptides. The program identifies each of the three most abundant ion species in a survey scan, isolates each of them in the Mass Spectrometer's ion trap and fragments them by inducing collisions with helium molecules. The resulting sub-fragment masses are recorded for further analysis by peptide analysis packages like SEQUEST and MASCOT. After the three subscan and collision cycles have completed, the MS takes another survey scan and the cycle repeats until the end of the run, usually about three hours. The raw MS reads are used by the analysis software to generate peptide fragment sequences, which were compared to amino acid sequence translations of all gene models in the 2-40 draft genome. Peptide identity matches were evaluated using accepted thresholds of statistical significance which are specific for each program.

Cloning and expression of 2-40 proteins in E coli

The basic cloning and expression system uses pETBlue2 (Novagen) as the vector, *E coli* DH5α (Invitrogen) as the cloning strain, and *E coli* BL-21(DE3) Tuner® cells (Novagen) for protein expression strain. This system allows the cloning of toxic or otherwise difficult genes because the vector places expression under the control of a T7 *lac* promoter which is lacking in the cloning strain DH5α, thereby abolishing even low-level expression during plasmid screening and propagation. After the blue/white screen, plasmids are purified from DH5α and transformed into the expression host (Tuners). The Tuner strain has the T7 *lac* promoter, allowing IPTG-inducible expression of the vector-coded protein and lacks the *Lon* and *Omp* proteases.

The nucleotide sequences of gene models were obtained from the DOE JGl's *Microbulbifer degradans* genome web server and entered into the PrimerQuest^{™} design tool provided on Integrated DNA Technologies web page located at http://biotools.idtdna.com/Primerquest/. The design parameters were Optimum Tₘ 60°C, Optimum Primer Size 20nt, Optimum GC% = 50, and the product size ranges were chosen so that the primers were selected within the first and last 100 nucleotides of each ORF in order to clone as much of the gene as reasonably possible. The cloning and expression vector, pETBlue2, provides a C-terminal 6xHistidine fusion as well as the start and stop codon for protein expression. Thus, careful attention to the frame of the vector and insert sequences is required when adding 5' restriction sites to the PCR primers. The resulting "tailed primers" were between 26 to 30nt long, and their sequences were verified by "virtual cloning" analysis using the PDRAW software package (http://www.acaclone.com). This program allows vector and insert DNA sequences to be cut with standard restriction enzymes and ligated together. The amino acid translations of the resulting sequences were examined to detect any frame shifts introduced by errors in primer design. Following this verification, the primers were purchased from Invitrogen (Frederick, MD).

PCR reactions contained 10pMol of forward and reverse primers, 1µl of 10mM DNTPs, 1.5µl of 100mM MgCl₂, and 1µl Proof Pro® *Pfu* Polymerase in a 50µl reaction with 0.5µl of 2-40 genomic DNA as the template. PCRs conditions used standard parameters for tailed primers and *Pfu* DNA polymerase. PCR products were cleaned up with the QIAGEN QIAquick PCR Cleanup kit and viewed in 0.8% agarose gels. Following cleanup and confirmation of size, PCR products and pETBlue2 are digested with appropriate restriction enzymes, usually *AscI* and *ClaI* at 37°C for 1 to 4 hours, cleaned up using the QIAquick kit, and visualized in agarose gels. Clean digestions are ligated using T4 DNA ligase for at least 2 hours in the dark at room temperature. Ligations are then transformed into *E coli* DH5α by electroporation. Transformants are incubated one hour at 37°C in non-selective media, and then plated onto LB agar containing ampicillin and X-gal. As pETBlue2 carries an Amp^{r} gene and inserts are cloned into the *lac*Z ORF, white colonies contain the insert sequence. White colonies are picked with toothpicks and patched onto a new LB/Amp/X-gal plate, with three of the patched colonies also being used to inoculate 3ml overnight broths. Plasmids are prepped from broths which correspond to patched colonies which remained white after overnight outgrowth. These plasmid preps are then singly digested with an appropriate restriction enzyme and visualized by agarose electrophoresis for size confirmation.

The plasmids are then heat-shock transformed into the Tuner® strain, which carries a chromosomal chloramphenicol resistance gene (*Cm^{r}*). The Transformants are incubated 1 hour at 37°C in non-selective rescue medium, plated on LB agar with Amp and Cm (Tuner medium) and incubated overnight at 37°C. Any colonies thus selected should contain the vector and insert. This is confirmed by patching three colonies onto a Tuner medium plate and inoculating corresponding 3ml overnight broths. The next morning the broths are used to inoculate 25ml broths which are grown to an OD₆₀₀ of around 0.6 (2-3 hours). At this point a 1ml aliquot is removed from the culture, pelleted and resuspended in 1/10 volume 1X SDS-PAGE treatment buffer. This pre-induced sample is frozen at -20°C for later use in western blots. The remaining broth is then amended to 1mM IPTG and incubated 4 hours at 37°C. Induced pellet samples are collected at hourly intervals. These samples and the pre-induced control are run in standard SDS-PAGE gels and electroblotted onto PVDF membrane. The membranes are then processed as western blots using a 1/5000 dilution of monoclonal mouse α-HisTag® primary antibodies followed by HRP-conjugated goat α-mouse IgG secondary antibodies. Bands are visualized colorimetrically using BioRad's Opti-4CN substrate kit. Presence of His tagged bands in the induced samples, but not in uninduced controls, confirms successful expression and comparison of bands from the hourly time points are used to optimize induction parameters in later, larger-scale purifications.

Production and purification of recombinant proteins

Expression strains are grown to an OD₆₀₀ of 0.6 to 0.8 in 500ml or 1 liter broths of tuner medium. At this point a non-induced sample is collected and the remaining culture induced by addition of 100mM IPTG to a final concentration of 1 mM. Induction is carried out for four hours at 37°C or for 16 hours at 25°C. Culture pellets are harvested and frozen overnight at -20°C for storage and to aid cell lysis. Pellets are then thawed on ice for 10 minutes and transferred to pre-weighed falcon tubes and weighed. The cells are then rocked for 1 hour at 25°C in 4ml of lysis buffer (8M Urea, 100mM NaH₂PO₄, 25mM Tris, pH 8.0) per gram wet pellet weight. The lysates are centrifuged for 30 minutes at 15,000g to pellet cell debris. The cleared lysate (supernatant) is pipetted into a clean falcon tube, where 1ml of QIAGEN 50% Nickel-NTA resin is added for each 4ml cleared lysate. This mixture is gently agitated for 1 hour at room temperature to facilitate binding between the Ni⁺² ions on the resin and the His tags of the recombinant protein. After binding, the slurry is loaded into a disposable mini column and the flow thru (depleted lysate) is collected and saved for later evaluation. The resin is washed twice with lysis buffer that has been adjusted to pH 7.0; the volume of each of these washes is equal to the original volume of cleared lysate. The flow thru of these two washes is also saved for later analysis in western blots to evaluate purification efficiency.

At this point the columns contain relatively purified recombinant proteins which are immobilized by the His tags at their C-terminus. This is an ideal situation for refolding, so the column is moved to a 4°C room and a series of renaturation buffers with decreasing urea concentrations are passed through the column. The renaturation buffers contain varying amounts of urea in 25mM Tris pH 7.4, 500mM NaCl, and 20% glycerol. This buffer is prepared as stock solutions containing 6M, 4M, 2M and 1M urea. Aliquots of these can be easily mixed to obtain 5M and 3M urea concentrations thus providing a descending series of urea concentrations in 1 M steps. One volume (the original lysate volume) of 6M buffer is passed through the column, followed by one volume of 5M buffer, continuing on to the 1M buffer which is repeated once to ensure equilibration of the column at 1 M urea. At this point the refolded proteins are eluted in 8 fractions of 1/10^{th} original volume using 1 M urea, 25mM Tris pH 7.4, 500mM NaCl, 20% glycerol containing 250mM imidazole. The imidazole disrupts the Nickel ion-His tag interaction, thereby releasing the protein from the column.

Western blots are used to evaluate the amount of His tagged protein in the depleted lysate, the two washes, and the eluted fractions. If there is an abundance of recombinant protein in the depleted lysate and/or washes it is possible to repeat the process and "scavenge" more protein. Eluate fractions that contain the protein of interest are pooled and then concentrated and exchanged into storage buffer (20mM Tris pH 7.4, 10mM NaCl, 10% glycerol) using centricon centrifugal ultrafiltration devices (Millipore). The enzyme preparations are then aliquoted and frozen at -80°C for use in activity assays.

The cellulose degrading enzymes, related proteins and systems containing thereof, of this invention, for example including one or more enzymes or cellulose -binding proteins, have a number of uses. Many possible uses of the cellulases of the present invention are the same as described for other cellulases in the paper "Cellulases and related enzymes in biotechnology" by M.K. Bhat (Biotechnical Advances 18 (2000) 355-383). For examples, the cellulases and systems thereof of this invention can be utilized in food, beer, wine, animal feeds, textile production and laundering, pulp and paper industry, and agricultural industries.

These systems can be used to degrade cellulose to produce short chain peptides for use in medicine.

These systems are used to break down cellulose in the extraction and/or clarification of fruit and vegetable juices, in the production and preservation of fruit nectars and purees, in altering the texture, flavor and other sensory properties of food, in the extraction of olive oil, in improving the quality of bakery products, in brewing beer and making wine, in preparing monogastic and ruminant feeds, in textile and laundry technologies including "fading" denim material, defibrillation of lyocell, washing garments and the like, preparing paper and pulp products, and in agricultural uses.

Cellulose may be used to absorb environmental pollutants and waste spills. The cellulose may then be degraded by the cellulase degrading systems of the present invention. Bacteria that can metabolize environmental pollutants and can degrade cellulose may be used in bioreactors that degrade toxic materials. Such a bioreactor would be advantageous since there would be no need to add additional nutrients to maintain the bacteria - they would use cellulose as a carbon source.

The present invention concerns a method for producing ethanol from lignocellulosic material, comprising: contacting lignocellulosic material with *Saccharophagus degradans* expressing an effective saccharifying amount of one or more compounds listed in Figures 4-11, preferably cellulase cel5A listed in Figure 4, to obtain saccharides; and converting the saccharides to produce ethanol. The contacting can be conducted in a marine environment, such as underwater.

In a further aspect of the present invention, the *Saccharophagus degradans* expresses an effective saccharifying amount of all of the compounds listed in Figures 4-11.

In a further aspect of the present invention, the one or more compounds listed in Figures 4-11 are present in dry form, in a buffer, or in the form of a paste, paint, or micelle. In addition, the one or more compounds listed in Figures 4-11 can be in a system consisting essentially of one or more compounds listed in Figures 4-11. According to a further aspect of the present invention, the one or more compounds listed in Figures 4-11 are present in a system further comprising metal ions, chelators, detergents, organic ions, inorganic ions, or one or more additional proteins, such as biotin and/or albumin.

The ethanologenic microorganism expresses an effective amount of one or more compounds listed in Figures 4-11 to saccharify the lignocellulosic material and an effective amount of one or more enzymes or enzyme systems which, in turn, catalyze (individually or in concert) the conversion of the saccharides (e.g., sugars such as xylose and/or glucose) to ethanol. The one or more enzymes or enzyme systems of the ethanologenic organism may be expressed naturally or by, but not limited to, any of the methods known to those of skill in the art. For example, release of the one or more enzymes or enzyme systems may be obtained through the use of ultrasound.

Conversion of sugars to ethanol and recovery may be accomplished by, but are not limited to, any of the well-established methods known to those of skill in the art. For example, through the use of an ethanologenic microorganism, such as Zymomonas, Erwinia, Klebsiella, Xanthomonas, and Escherichia, preferably Escherichia coli K011 and Klebsiella oxytoca P2.

The present invention further concerns the use of *Saccharophagus degradans* expressing an effective saccharifying amount of one or more compounds listed in Figures 4-11 for the production of ethanol from lignocellulosic material.

In addition, the present invention concerns a method for producing saccharides from lignocellulosic material, comprising:
contacting lignocellulosic material with *Saccharophagus degradans* expressing an effective saccharifying amount of one or more compounds listed in Figures 4-11 to obtain saccharides. For example, release of the one or more compounds listed in Figures 4-11 may be obtained subjecting *Saccharophagus degradans* to ultrasound.
Other embodiment is directed to the use of *Saccharophagus degradans* expressing an effective saccharifying amount of one or more compounds listed in Figures 4-11 to obtain saccharides from lignocellulosic material.

### References Cited

Andrykovitch, G. and I. Marx (1988) "Isolation of a new polysaccharide-digesting bacterium from a salt marsh." Applied and Environmental Microbiology 54: 3-4.
Beguin, P. and J. P. Aubert (1994) "The biological degradation of cellulose." FEMS Microbiol Rev 13(1): 25-58.
Chakravorty, D. (1998). Cell Biology of Alginic Acid degradation by Marine Bacterium 2-40. College Park, University of Maryland.
Coutinho, P. M. and B. Henrissat (1999) Carbohydrate-active enzyme server. http://afmb.cnrs-mrs.fr/~cazyl/CAZY/index.html Accessed Jan 21, 2004
Coutinho, P. M. and B. Henrissat (1999) The modular structure of cellulases and other carbohydrate-active enzymes: an integrated database approach. Genetics, biochemistry and ecology of cellulose degradation. T. Kimura. Tokyo, Uni Publishers Co: 15-23.
Distel, D. L., W. Morrill, et al. (2002) "Teredinibacter turnerae gen. nov., sp. nov., a dinitrogen-fixing, cellulolytic, endosymbiotic gamma-proteobacterium isolated from the gills of wood-boring molluscs (Bivalvia: Teredinidae)." Int J Syst Evol Microbiol 52(6): 2261-2269.
Ensor, L., S. K. Stotz, et al. (1999) "Expression of multiple insoluble complex polysaccharide degrading enzyme systems by a marine bacterium." J Ind Microbiol Biotechnol 23: 123-126.
Gonzalez, J. and R. M. Weiner (2000) "Phylogenetic characterization of marine bacterium strain 2-40, a degrader of complex polysaccharides." International journal of systematic evolution microbiology 50: 831-834.
Henrissat, B. and A. Bairoch (1993) "New families in the classification of glycosyl hydrolases based on amino acid sequence similarities." Biochem J 293 (Pt 3): 781-8.
Henrissat, B., T. T. Teeri, et al. (1998) "A scheme for designating enzymes that hydrolyse the polysaccharides in the cell walls of plants." FEBS Lett 425(2): 352-4.
Jonsson, A. P., Y. Aissouni, et al. (2001) "Recovery of gel-separated proteins for insolution digestion and mass spectrometry." Anal Chem 73(22): 5370-7.
Kelley, S. K., V. Coyne, et al. (1990) "Identification of a tyrosinase from a periphytic marine bacterium." FEMS Microbiol Lett 67: 275-280.
Kosugi, A., K. Murashima, et al. (2002) "Characterization of two noncellulosomal subunits, ArfA and BgaA, from Clostridium cellulovorans that cooperate with the cellulosome in plant cell wall degradation." J Bacteriol 184(24): 6859-65.
Laemmli, U. K. (1970). "Cleavage of structural proteins during the assembly of the head of the bacteriophage T4." Nature 277: 680-685.
Ljungdahl, L. G. and K. E. Eriksson (1985) Ecology of Microbial Cellulose Degradation. Advances in Microbial Ecology. New York, Plenum Press. 8: 237-299.
Lou, J., K. Dawson, et al. (1996) "Role of phosphorolytic cleavage in cellobiose and cellodextrin metabolism by the ruminal bacterium Prevotella ruminicola." Appl. Environ. Microbiol. 62(5): 1770-1773.
Lynd, L. R., P. J. Weimer, et al. (2002) "Microbial cellulose utilization: fundamentals and biotechnology." Microbiol Mol Biol Rev 66(3): 506-77, table of contents.
Shevchenko, A., M. Wilm, et al. (1996) "Mass spectrometric sequencing of proteins silver-stained polyacrylamide gels." Anal Chem 68(5): 850-8.
Smith, R. D., J. A. Loo, et al. (1990) "New developments in biochemical mass spectrometry: electrospray ionization." Anal Chem 62(9): 882-99.
Stotz, S. K. (1994). An agarase system from a periphytic prokaryote. College Park, University of Maryland.
Sumner, J. B. and E. B. Sisler (1944) "A simple method for blood sugar." Archives of Biochemistry 4: 333-336.
Tomme, P., R. A. Warren, et al. (1995) "Cellulose hydrolysis by bacteria and fungi." Adv Microb Physiol 37: 1-81.
Warren, R. A. (1996) "Microbial hydrolysis of polysaccharides." Annu Rev Microbiol 50: 183-212.
Whitehead, L. (1997). Complex Polysaccharide Degrading Enzyme Arrays Synthesized By a Marine Bacterium. College Park, University of Maryland.

### LISTING OF AMINO ACID SEQUENCES OF THE COMPOUNDS IN FIGURES 4-10, AND THE CORRESPONDING NUCLEOTIDE SEQUENCES (SEQ ID NOs 1-214, RESPECTIVELY IN ORDER OF APPEARANCE)

LOCUS NZ_AABI02000010 3504 bp DNA linear BCT 23-SEP-2003
DEFINITION Microbulbifer degradans 2-40 Mdeg_14, whole genome shotgun sequence.
ACCESSION NZ AABI02000010 REGION: 24249..27752
VERSION NZ-AABI02000010.1 Gl:28878292
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3504)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028613. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review.
   DNA sequence and predicted proteins are available for BLAST at
   http://www.ncbi.nlm.nih.gov/sutils/genom table.cgi.
   URL -- http://www.igi.doe.gov
   Contact: Paul Richardson
   microbes@cuba.jgi-psf.org.
COG assignment was automatically updated on Sep 23, 2003. FEATURES Location/Qualifiers
   source 1..3504
      /organism="Microbulbifer degradans 2-40"
      /mol_type="genomic DNA"
      /strain="2-40"
      /db_xref="taxon:203122"
   gene 1..3504
      /gene="Mdeg2412"
   CDS 1..3504
      /gene="Mdeg2412"
      /codon_start=1
      /transl_table=11
      /product="COG2730: Endoglucanase"
      /protein_id="ZP 00067013.1"
      /db_xref="Gl:23028625"
      /db_xref="COG:COG2730" 
/translation="MTIKRWPFDRKGPPKKPNAKKLLASLAAALSLTAMQSTAAVEPL
QTSGNQILVGNQAKALGGHSLFWHNVPAAGSLYNADTVSRLKNDWNSKVIRAAIGVEV
PFNSENTYIGNKGSSLAAIDRVVNAAVANDMYVIIDFHTHHADQVENVAHDFFNEVSS
RYGHLNNVIYEVFNEPEWCGEHGRWASTIKPYAERVIQTIRNNDPDNLVIVGTTCFSQ
DVDVAAADPINDVNVAYTLHFYAATPAHQQPLRDKAQTALDRGAPLFVTEWGTTTFTG
DGFVDEAQTRTWINWLNERGISHVNWSASTQPESSAIWNGDMTYKHSGLLVGELVQQT
NGTTTPPTGEISGPCDLHFVPAKAEAESFCTAKGIQFETTTDTGGGQNMGWLDAGDWV
TFDVDVPASGQYLIDYRVASELGDGRFRTEAANGTALGTISVPNTGGWQNWQTHTHTV
QLSQGTQTVKLVAETGGWNLNWFEVRAGEVCEGADCPCEGAECPCPDCNGTPVKFEAE
 
TFVAMQGVQLENTSDVGGGQNVGYIDSGDWITYNGALPASADNRYVVSYRVARQPSGN
AKFKIEQPGGAAVYGEISVPSTGGWQTWTTISHTITIPANANGFALAAIDGGWNINWI
EIKPATTQPPEPINPLKLQAEDYINFNDTTPGNEGGAHRSDDVDIQATTDTGGGFNVG
WVDAGEWLEYEFFLESPDFYAADVRVASDQTGGALQLQIDGQNVGQAITVGNTGGWQA
WTTKNTLIGDLSAGTHTLRVYAQSGPLNLNWVELKRTTPAPATSCFNIAEDRLNVHLD
AHCTAGSNLQYNWDFGDGNSATGVATSHSYYTSGTYTITLTVSDTRTTDTSSQQVTVD
 
FSAPAGPVDFYGELMVNGNRIHGEKTGEPAQVRGMSFFWSNTGWGQEKWWNASTVDRM
VDEFKVELVRGAMGTDEGGGYLHDASNKARLQAVVEQAIARNVYVIIDWHTHHAEDNI
AEAITFFSEMAQLYGHHDNVIFEIYNEPLNTTSWGTIKHYAEQVIPAIRAHSDNLIVV
GTRTWSQNVDEAAFDKINDSNTAYALHFYVGSHGNHVRNLAQTALNNGAAIFASEWGI
WPNNNYDGMNADDWMNFLDQNKISWANWAISDKVDPNTGQLEPPSMFNPDGSLSSNGQ
YVVNKLNEYAAQAPWREAIAN"
ORIGIN 
1 atgacaatta aacgttggcc gttcgaccga aaaggcccac ctaaaaaacc taacgctaaa
61 aaattactcg caagcttagc ggctgcacta agcttaaccg ccatgcaaag cactgcagcg
121 gtagagccat tacaaaccag cggcaatcaa attcttgttg gcaaccaagc caaagccctt
181 ggcggccaca gcttgttttg gcataacgtg ccggcagcag gcagcttata caatgcagat
241 acagtaagca ggcttaagaa tgattggaac tccaaggtta ttcgggccgc aattggggtt
301 gaagtacctt tcaattcaga aaacacctac ataggcaata agggcagctc gctggccgca
361 atagaccgcg tagttaatgc cgctgttgcc aacgatatgt atgtgattat cgattttcat
421 actcaccatg cagatcaagt agaaaacgtt gcccacgact tttcaacgaa agtttctagc
481 cgttacggtc atttaaacaa tgttatttat gaagtattta acgagccaga atggtgtggc
541 gagcacggtc ggtgggcatc taccattaag ccctacgccg agcgcgttat ccaaaccatt
601 cgcaacaatg acccagacaa cctagtaata gtaggcacta cctgtttctc gcaagatgta
661 gatgtagccg cagccgaccc cattaacgat gtaaacgtgg cctatacgct acacttttac
721 gcagccaccc ctgcccacca gcaacccttg cgcgacaagg cccaaaccgc gctcgaccgc
781 ggcgcgccac tatttgtaac cgaatggggt acaaccacat ttacaggtga tggttttgta
841 gatgaggcgc aaacgcgcac atggattaac tggttaaacg aacgcggtat tagccacgtt
901 aactggtcgg cgtctaccca gccagaaagc tcagctatat ggaatggcga catgacctac
961 aagcattcgg gcttattggt tggcgaactg gtgcaacaaa caaatggcac aaccacgcca
1021 ccaaccggtg aaataagtgg cccgtgcgat ttacattttg tacctgccaa agccgaggct
1081 gaaagcttct gtaccgccaa aggcattcaa tttgaaacca ccaccgacac gggcggcggc
1141 caaaacatgg gctggctaga tgccggcgac tgggtaactt ttgatgtaga tgtacctgct
1201 agcggccaat atttaataga ttaccgcgta gcatcagagc taggtgatgg tcggttccgc
1261 accgaagccg ccaacggcac tgcccttggc acaatatctg tacccaatac cggcggctgg
1321 cagaattggc aaacgcacac acacacagtg caactctcgc aaggcacaca aaccgttaaa
1381 ctagttgccg aaactggtgg ctggaactta aattggtttg aagtgcgcgc aggtgaggtg
1441 tgcgaaggcg ctgactgccc atgtgaagga gccgaatgcc cttgcccaga ttgcaacggc
1501 acaccggtta agtttgaggc agaaacgttt gtggctatgc aaggcgtgca gctagaaaac
1561 acatccgatg tgggcggcgg ccaaaacgtt ggctacattg atagcggcga ctggataact
1621 tacaacgggg ccttgcccgc aagtgcagac aaccgctatg tagtgtctta tagagtagcg
1681 cgtcaaccta gcggcaatgc caaatttaaa atagaacagc caggtggagc agcggtatat
1741 ggcgaaattt cggtgcccag caccggcggc tggcaaacat ggacaaccat tagccacacc
1801 ataacaattc ccgctaacgc aaacggcttt gcactagcag caatagatgg cggttggaat
1861 ataaactgga tagaaataaa accggcgacc actcaaccac ccgagccaat caacccgtta
1921 aaacttcaag ctgaagatta catcaacttt aacgacacca cccccggtaa cgaaggcggt
1981 gcacacagaa gcgatgatgt agatattcaa gcaactaccg ataccggtgg cggttttaat
2041 gttggctggg tagacgctgg cgaatggcta gagtatgagt tctttttaga gtctcctgat
2101 ttttatgcag ctgatgtacg ggttgcttca gaccaaactg gcggcgcact gcaactacaa
2161 atagatggcc aaaacgttgg ccaagccatt accgttggca acaccggtgg ctggcaagcg
2221 tggacaacca aaaacacact cattggcgac ctaagtgcag gcacccacac gttgcgtgta
2281 tacgcgcaaa gcggcccatt aaatttaaac tgggtagagc taaagcgtac aacgcccgca
2341 ccagccactt cgtgttttaa tattgccgaa gaccgcttaa acgttcacct agatgcgcac
2401 tgtactgcag gcagcaacct gcaatacaat tgggattttg gtgacggcaa cagcgcaacc
2461 ggcgtagcca ctagccacag ctactacact agcggcactt acaccattac cttaaccgtt
 
2521 agtgataccc gcaccacaga cacctctagc caacaggtaa cggtagattt ttctgcccct
2581 gcaggccctg tggattttta cggcgaacta atggtgaatg gcaaccgcat tcacggcgaa
2641 aaaaccggcg aacccgcaca agtacgcggc atgagctttt tttggagcaa caccggttgg
2701 ggccaagaaa aatggtggaa cgccagcacc gtggaccgca tggttgatga gttcaaagta
2761 gaacttgtgc gcggcgcaat gggcactgat gaaggcggcg gttatttaca cgacgcgtct
2821 aataaggctc gcttacaagc agttgttgaa caagccattg cacgcaatgt gtatgtaatt
2881 atcgactggc acacccacca tgccgaagat aacattgccg aagccattac attctttagc
2941 gaaatggcgc agctttatgg ccaccacgac aacgtgattt tcgagattta caacgagcca
3001 ttaaacacca caagctgggg cactattaag cactacgctg aacaagttat tcctgctatt
3061 cgcgctcatt ccgataattt aattgttgtg ggcacgcgca cctggtcgca aaacgtagac
3121 gaagccgcgt tcgataaaat taacgacagc aacaccgcct acgccctgca cttttatgtt
3181 ggctcgcacg gcaaccacgt tcgcaaccta gcacaaaccg cactaaacaa cggcgcggct
3241 atttttgcta gcgaatgggg aatttggcca aacaacaact acgatggcat gaacgccgac
3301 gattggatga actttttaga ccaaaacaaa atatcttggg ctaactgggc catatccgac
3361 aaagtagacc ccaacacagg ccaactagaa ccacccagca tgttcaaccc agacggcagc
3421 ctaagcagta atggtcaata tgtagtgaac aaactaaatg aatacgcagc acaagcaccg
3481 tggagggagg caatcgctaa ttga
LOCUS NZ_AABl02000001 1701 bp DNA linear BCT 23-SEP-2003
DEFINITION Microbulbifer degradans 2-40 Mdeg_1, whole genome shotgun sequence.
ACCESSION NZ AABl02000001 REGION: complement(279610..281310)
VERSION NZ_AABl02000001.1 Gl:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1701)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1701
      /organism="Microbulbifer degradans 2-40"
      /mol_type="genomic DNA"
      /strain="2-40"
      /db_xref="taxon:203122"
   gene complement(1..>341)
      /gene="Mdeg0215"
   CDS complement(1..>341)
      /gene="Mdeg0215"
      /codon_start=1
      /transl_tab!e=11
      /product="hypothetical protein"
      /protein_id="ZP 00064854.1"
      /db_xref="Gl:23026368" 
/translation="MVVSLADNSAGAISCWHAKASPPEELEELLDEELELDELELEEE
LEELVEELLEELLDELLLDELEDEPLAAPPSLPPPQAVSPAKQLISSADFKKVSFRVQ
LNAVRVKSKRNINHSRIFLFWLFSHFRSRRC"
   gene 1..1701
      /gene="Mdeg0214"
   CDS 1..1701
      /gene="Mdeg0214"
      /codon_start=1
      /transl_table=11
      /product="COG2730: Endoglucanase"
      /protein_id="ZP 00064853.1"
      /db_xref="Gl:23026367"
      /db_xref="COG:COG2730" 
/translation="MFLLDFTRTAFSCTRKLTFLKSALLISCFAGLTACGGGSDGGAA
SGSSSSSSSSSSSSSSSSSSTSSSSSSSSSSSSSSSSSSNSSSSSSSGGDALACQHEM
APALLSASDTTMVQAEYYDTCASSALDNTTGNSGGELRTDDVDIVAIADGYAITDMQS
 
GEYVEYSLTVQTSGLFDISFAVQPHAANTAGLALSVDGAVLGTVDIAANDSTAFGEYT
LNGVYISDGAQVIRVTMAGEGAAIGLDSIAFNYTDNTVYTPENAVLGMGIGINLGNTL
DAFPNEGDWAPAAQEYYFKAYKDAGFRHVRIPATWDDHTADTAPYAVNAARMDRTEQI
VDWALAQGYFVILNAHHEHWLKENYGNQTYRDRFDAIWQQIAERFKNKSARLMFEILN
EPNGMTVADVDDLNPRILDIIRETNPTRLVVFSGNGYTPVDALLAAAIPNDDYLIGNF
HSYDPWQFGGQCVRSWGTEQDYTDLENIYKRANTWSEQHDIPVMVNEFGAAHYDFTAP
QNVCNQQARLAYLGAHATFAIQYGFGASVWDDGGSFEVYKRGENSWREAKDVLVAPNP
"
ORIGIN 
1 atgtttcttt tagactttac ccgcactgcg tttagctgta cacgaaagct taccttttg
61 aaatccgcgc tacttataag ctgctttgcc gggcttactg cctgtggtgg cgggagtgat
121 ggcggtgctg caagtggctc atcctctagc tcgtctagca gcagttcgtc tagtagctct
181 tcgagcagtt cttcaactag ttcctcaagc tcctcttcaa gctctagttc gtccagttcc
241 agctcttcgt ctaatagttc ctctagctcc tctggtggcg atgctttagc gtgccagcat
301 gaaatggcac cagcgctatt atctgcaagt gatactacca tggtgcaagc ggagtattac
361 gatacctgtg cttcttcggc attagataac accactggta acagtggcgg tgagttgcga
421 actgacgatg tagatatagt ggccattgcg gacggctatg ctattacgga tatgcagtca
481 ggcgagtacg tagaatattc actaacagtg caaacttccg gtttgtttga cattagtttt
541 gcggtacagc cgcacgcagc taatactgcc ggtttggcgc tgagtgtaga tggcgcagtg
601 ttaggcacag ttgatattgc cgctaatgac agcaccgcat ttggcgaata tacgcttaac
661 ggcgtgtaca taagcgatgg cgcgcaagta ataagggtaa ccatggccgg cgaaggcgct
721 gctattgggt tagattccat tgcctttaat tacaccgata ataccgttta caccccagaa
781 aacgccgtgt tgggtatggg aataggtatt aacctaggca ataccttaga tgccttcccc
841 aacgaaggtg actgggcacc ggctgcgcag gaatactatt ttaaagccta caaggatgca
901 ggtttccgcc atgtacgcat cccagcaact tgggatgatc acacggctga tacagccccc
961 tacgctgtaa atgcagcacg tatggatcgc actgagcaga ttgtagattg ggccttggcg
1021 cagggctatt tcgtaattct taatgcccac cacgaacact ggctaaaaga aaactacggc
1081 aatcaaacat accgcgatcg ctttgatgca atttggcagc aaattgccga acgctttaag
1141 aataagtcgg ctcgcttaat gtttgagata ctcaatgagc caaacggcat gacagtggcc
1201 gatgtggatg acctcaaccc acgtattctc gatattattc gcgaaaccaa tcccacgcga
1261 ttggtagtgt tctctggtaa tgggtatacc cctgtggatg ccttacttgc ggctgcaatc
1321 cctaatgatg attaccttat tggtaacttt cactcctacg acccttggca gtttggcggt
1381 cagtgcgtac gatcgtgggg tacagagcaa gattacaccg acctagagaa catatataag
1441 cgcgcaaata cttggtctga gcagcacgac atacccgtta tggtgaacga atttggcgct
1501 gcccattacg attttactgc accgcagaat gtatgtaacc agcaggctcg tttggcttat
1561 ttaggtgccc atgccacatt tgctattcag tacggctttg gcgcaagtgt atgggacgac
1621 ggtggatcat ttgaggtgta caagcgcggt gaaaatagct ggcgcgaagc taaagatgta
1681 ttagtggcgc caaacccgta g
LOCUS NZ_AABI02000019 1356 bp DNA linear BCT 23-SEP-2003
DEFINITION Microbulbifer degradans 2-40 Mdeg_19, whole genome shotgun sequence.
ACCESSION NZ AABI02000019 REGION: complement(10740..12095)
VERSION NZ_AABI02000019.1 Gl:28878283
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1356)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029085. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL - http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1356
      /organism=" Microbulbifer degradans 2-40"
      /mol_type="genomic DNA"
      /strain="2-40"
      /db_xref="taxon:203122"
   gene 1..1356
      /gene="Mdeg2867"
   CDS 1..1356
      /gene="Mdeg2867"
      /codon_start=1
      /transl_table=11
      /product="COG2730: Endoglucanase"
      /protein_id="ZP 00067454.1"
      /db_xref="G1:23029093"
      /db_xref="COG:COG2730" 
/translation="MRIITAFAVMLLCITGCSGSGASDSPQASNSSSGSSSSSSSSSS
SSSSSSSSSSSSTSSSSSSSSSSSSSSSSSSGGEALYPSYNTNPPAPDMTGMTSTATQ
LADRITVGWNIGNTLEAIGGETNWGNPLVTNELIQAVKASGFDSIRIPAAWDQYANQE
TAAIDINWLNRVKQVVQYSIDNDMVVVLNIHWDGGWLERNVEPSEQVAVNAKQKAYWE
QIATHLRDFDERLIFASANEPHVETEAQMAVLNVYHQTFVDTVRATGGKNAYRVLVLQ
GPKTDIETTSLLWTQMPQDSAVNKLMAELHFYTPYNFTLMNVDESWGNQFYYWGEGNH
STTDTGRNPTWGEEATVDSLLAITKQQFVDQGIPVIIGEYGAQRRDNLTGDELALHLQ
SRNYYLKYVTQKCVELGLKPFYWDTGGLDNNQSGLFNRSTYQVFDQNALDAIMEGARG
E"
ORIGIN 
1 atgagaataa taacggcgtt tgcagttatg ctgctatgca taacaggctg tagcggatcg
61 ggcgcgagtg atagcccgca agcatccaat tcgtcttcgg gcagttcttc tagctctagc
 
121 agttcgtcaa gttcgagcag ttcctctagt tcgtcgtcta gctcttcaac aagctctagc
181 agctcatcta gctccagctc atcatcaagc tctagcagtt cttcgggcgg cgaagcgctt
241 tacccaagct acaatacaaa cccgccagcg ccagatatga ccggcatgac aagtactgcc
301 acacaactag cagatcgtat aaccgtgggc tggaatattg gtaacacgct agaggcaata
361 ggcggcgaaa ccaactgggg taacccgctg gttactaacg aattaattca agcggtaaaa
421 gccagtggct ttgattccat tcgtataccc gccgcgtggg atcaatacgc caaccaagaa
481 acggccgcaa tagatataaa ctggctaaac cgcgttaaac aagttgtgca atacagcata
541 gataacgaca tggtggtagt gctaaacatc cactgggatg gcggttggct agagcgcaat
601 gtagagccca gcgagcaagt agcagtaaat gcaaaacaaa aagcctattg ggaacaaatt
661 gccactcacc tgcgcgactt tgacgagcgc ctaatatttg ccagcgccaa cgaaccccat
721 gtagaaaccg aagcacaaat ggccgtacta aacgtatacc atcaaacgtt tgtagataca
781 gtgcgtgcaa ctggcggtaa aaatgcttac cgcgtactgg tattgcaggg gccaaaaaca
841 gatatagaaa ccacctcgct attgtggacc caaatgccgc aagatagcgc cgtaaataaa
901 cttatggcag agctacactt ctataccccg tacaacttta cgttaatgaa tgtagatgaa
961 agctggggca accagttcta ctactggggc gaaggtaatc attccactac cgacacaggc
1021 cgcaacccaa cctggggcga agaagcaaca gtagattcac tgctggcaat taccaaacaa
1081 cagtttgtgg accaaggtat acccgtaatt attggcgaat acggtgcaca acgccgcgat
1141 aaccttaccg gcgatgaatt ggccctgcac ttacaatcgc gcaactacta cttaaaatac
1201 gttactcaaa aatgtgtaga gctaggctta aaaccttttt attgggatac cggcggctta
1261 gacaacaatc aatctggcct gtttaatcgc agtacctacc aagtattgga tcaaaatgcc
1321 ctagatgcca ttatggaagg ggccagaggg gaataa
LOCUS NZ_AABI02000001 1866 bp DNA linear BCT 23-SEP-2003
DEFINITION Microbulbifer degradans 2-40 Mdeg_1, whole genome shotgun sequence.
ACCESSION NZ AABI02000001 REGION: complement(472006..473871)
VERSION NZ_AABI02000001.1 Gl:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1866)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1866
      /organism="Microbulbifer degradans 2-40"
      /mol_type="genomic DNA"
      /strain="2-40"
      /db_xref="taxon:203122"
   gene 1..1866
      /gene="Mdeg0348"
   CDS 1..1866
      /gene="Mdeg0348"
      /codon_start=1
      /transl_table=11
      /product="COG2730: Endoglucanase"
      /protein_id="ZP_00064986.1"
      /db_xref="Gl:23026500"
      /db_xref="COG:COG2730" 
/translation="MLKHQFSKALRALGFGGAVFAASLMASQASALECEHSISNDWGA
GFTGAMKVTNNDSSPITGWRVEWAYSGNVNIVNSWNASVTKGSNYVAVDAGWNGNLQP
SQSTEFGLQGDGADRNVTIISCVAEGGSSSSSSSSSSSSSSSSSSSSSSTSSSSSSSSST
SSSSSSTSSSSSSTSSSSSSSSSSSSSSGGNCVAMCNWYGENRPVCANQNTGWGWENNQ
SCIGANTCNDQWGDGGVVSSCGTSSS SSSSSSSSSTSSSSSSSSSTSSTSSSSSSSSS
SGGLSAVEFSQQMGLGWNLGNSLEAIGGETAWGNPMVTQQLINSIKAAGFDTIRIPVA
WSQFSDEANFVINSNWIARVEEVVNYALSADMYVVMNQHWDGGWMQPTYAQQEYVNNR
LQIMWTQIANHFKDYDSRLLFAGTNEVMVEGDYGTPTFEYYTVQNSFNQTFVDAVRAT
GGANASRYLVVQGFNTNIDHTVNFAVVPTDPATNRLMMEVHYYDPYNFTLNTNSNITQ
WGVIATDPSVTETWANESYVDATFQKMKTNFVDQGIAVILGEYGVVSRANVAGHETYR
EYWNQYITQSAVDHGMVPIYWDNGYSGDGGMALFDRASGNQLYPNIINAIINAGN"
   gene complement(264..521)
      /gene="Mdeg0350"
   CDS complement(264..521)
      /gene="Mdeg0350"
      /codon_start=1
      /transl_table=11
      /product="hypothetical protein"
      /protein-id="ZP 00064987.1"
      /db_xref="Gl:23026501" 
/translation="MLEEELEVELEEELVEELELLDEEVLELDEELLEELEELDELED
DPPSATQLIMVTFLSAPSPCKPNSVDWLGCKLPFHPASTAT"
ORIGIN 
1 atgttgaaac atcaattcag caaagcgctg cgtgcgctag gctttggtgg ggctgtgttt
61 gcggcatcgc taatggctag ccaagcaagt gcccttgagt gtgagcattc aatcagtaat
121 gattggggcg ccggctttac cggtgcaatg aaagttacca ataatgactc tagccccatt
181 accggttggc gggtcgaatg ggcgtatagc ggcaatgtaa atattgttaa ttcgtggaac
241 gcctcagtaa caaaaggcag taattatgtt gccgtagatg ccggatggaa tggtaattta
301 cagccgagcc aatctaccga atttggctta cagggtgatg gcgccgatag aaatgtaacc
361 attattagtt gtgttgccga aggcggatca tcttctagtt catcaagttc ttccagctcc
421 tcaagtagtt cttcatctag ctcaagtact tcttcatcga gtagctcaag ttcctcgacg
481 agctcttctt ctagttcgac ttctagctct tcttcaagca cctcttctag ctcatcgtcc
541 agttcatcaa gctcttcttc gggcggcaac tgtgttgcaa tgtgtaattg gtacggtgaa
601 aaccgccctg tttgtgccaa tcaaaatact ggttgggggt gggaaaacaa ccaaagctgt
661 ataggtgcaa acacctgtaa cgatcaatgg ggcgacgggg gcgtggtgtc cagctgtggt
721 acgtctagct cttcatccag ttcttcgtcc agttcgtcta ccagttcatc ctcgtcttct
781 agctcgagca ccagctctac aagcagctca tcaagctcta gttcgtcgtc tggtgggtta
841 agcgcggtag agttttcgca gcaaatgggc ttggggtgga atcttggaaa ctccctagaa
901 gcgattggtg gcgaaaccgc gtggggcaac ccaatggtta cgcagcaatt aattaactcc
961 ataaaagctg ctgggttcga cactattcgc attccggttg cgtggagcca attctcggac
1021 gaagctaatt ttgttatcaa tagcaattgg attgcacgcg tagaagaagt agtgaactac
1081 gcattgagcg ccgatatgta cgtggtaatg aaccaacatt gggacggcgg ttggatgcag
1141 cccacatatg cacagcaaga atatgttaac aatcgcttgc aaattatgtg gacgcaaata
1201 gctaatcact ttaaagatta cgatagtcgc ttactgtttg caggcaccaa cgaagtgatg
1261 gtggaaggcg attacggtac gcccaccttc gaatactaca cagtacaaaa tagctttaac
1321 caaacgtttg tggatgctgt acgtgcaacc ggtggcgcta atgctagccg ttacttagtg
1381 gtacaggggt ttaataccaa catagatcac acggtgaact tcgcggtagt gccaaccgac
1441 ccggcaacaa acaggttaat gatggaagta cactattacg acccctataa ctttacgtta
1501 aataccaaca gcaacattac tcagtggggc gtaattgcaa ctgaccctag cgttaccgaa
1561 acatgggcga atgaatctta tgtggatgcg actttccaaa aaatgaaaac taacttcgtt
1621 gatcaaggta tagcggtaat tttaggtgag tacggggttg tatcgcgcgc gaatgtggcc
1681 gggcacgaaa cttaccgaga gtattggaac caatacatta ctcaatctgc ggtagatcat
1741 ggaatggtgc ctatttatgg ggataacggt tattccggtg atggtggtat ggcattgttt
1801 gatcgcgcca gtggcaatca actttacccc aatattatta acgcaattat caatgccggt
1861 aactaa
LOCUS NZ_AABI02000006 2022 bp DNA linear BCT 23-SEP-2003
DEFINITION Microbulbifer degradans 2-40 Mdeg_6, whole genome shotgun sequence.
ACCESSION NZ_AABI02000006 REGION: 82200..84221
VERSION NZ_AABI02000006.1 Gl:28878296
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2022)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027577. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2022
      /organism="Microbulbifer degradans 2-40"
      /mol_type="genomic DNA"
      /strain="2-40"
      /db_xref="taxon:203122"
   gene 1..2022
      /gene="Mdeg1459"
   CDS 1..2022
      /gene="Mdeg1459"
      /codon_start=1
      /transl_table=11
      /product="COG2730: Endoglucanase" /protein_id="ZP_00066079.1"
      /db_xref="Gl:23027641"
      /db_xref="COG:COG2730" 
/translation="MLIGTVTASALVGRGRGTPKKIINKGSIMWQINKSALAAVVLVC
SSSSFAQSACDTQRIEAENYVAMSGIQTESTADTGGGLNVGWIDAGDWLSYQVNLPAA
GQYEVRYRVASRNGGGVLRLEGNAGQTLYGTMNVPNTGGWQNWQTLSHSVTLAAGEQS
IGIGVPSGGFNINWLEFVPLDCSGPIDPPINPPSNCASIVFEAENYDQMSGIRTQTTS
DTGGGLNVGWIDAGDWLSYATVNIPSTQVYNFEYRVASPNGGSFNLQGSAGAENFDTA
TLPNTGGWQNWTTVTGSALLPAGNVNFGISAITGGWNINWFKATPESCDDINPPSTGI
TAKQAAAAMGKGFNLGQMFESTQHPRTFNAAKSKIDAYYNMGYRNVRIPITWTEAVGG
NRLVADANVGAVNRNHSRLAVITQVVDYALSLPGMYVVINAHHEGGLKTNNRWWVLET
LWADIADIFKDRDHRLLFEILNEPHLSDANKSPMPPANLRFMTGKAYNKIRAIDAQRI
VIIGGNQWFGAGEMANVWPNLNDVGGGSDAYVMATFHHYDPWSFSGDNQGDYADAWTL
 
SNVGNPMDIMQSWANGVGQGMPVYIGEWGVGWGSRYSAMQCNNIRYWYQLFDASYASA
KGQPTAVWDDGGWFKIFDHGTNSFNNNLAQCIGGNCAWDGADRFNSGCN"
ORIGIN 1 atgttgattg gtactgttac ggcttcagca ctggttggtc gaggccgtgg cacccctaaa 
61 aaaataatca acaagggttc tattatgtgg caaatcaaca aatcggcttt agcggccgtg
121 gtattagtgt gttcctcatc tagctttgcg caatctgcat gtgacactca acgcattgaa
181 gccgaaaatt acgtggcaat gagtggtatt caaaccgaaa gcacggcaga cactggtggc
241 ggtttaaatg tgggctggat agacgccggc gactggctta gttaccaagt taacctacct
301 gctgcagggc agtacgaggt gcgctatcgc gttgccagta gaaatggcgg cggtgtactt
361 cggttagagg gcaatgccgg tcaaaccttg tatggaacta tgaatgtacc caacacgggt
421 ggctggcaaa attggcaaac cctttctcat tcagtgacat tagcggcagg agagcagtct
481 attggtattg gtgtgccaag cggcgggttt aatattaatt ggctggagtt cgtaccttta
541 gattgcagtg ggccaatcga cccgcccatt aacccacctt cgaactgcgc gagcattgta
601 ttcgaggccg aaaattacga tcaaatgagc ggcattagaa cgcaaaccac aagtgatacc
661 ggaggcggct taaatgtggg gtggatagat gctggcgact ggcttagcta tgccactgtg
721 aatatcccca gcacgcaggt gtacaatttt gaataccgtg tggctagccc taatggcggc
781 agttttaatt tgcagggttc ggctggcgca gagaattttg ataccgctac tttgcccaat
841 acgggtggtt ggcaaaattg gacaacggta acaggctcgg cgcttttacc tgctggcaat
901 gtgaatttcg gtattagtgc gattactggt ggctggaata taaactggtt taaagctaca
961 ccagagagct gtgatgatat aaaccctcca agtaccggta ttactgctaa gcaagcagcg
1021 gcagccatgg gcaaggggtt taatttgggg caaatgttcg aaagtacgca acacccaaga
1081 acatttaatg ctgcaaaaag taaaatagat gcttactaca atatgggcta cagaaatgtg
1141 cgcatcccta ttacttggac tgaagccgta ggcggaaaca ggcttgttgc agatgcaaat
1201 gtaggcgcag tcaatcgcaa ccactctcgc ttagctgtaa ttactcaagt agtagattac
1261 gcgctttcgc tacccggcat gtacgtggtt attaatgcgc atcacgaagg tggattaaaa
1321 accaataatc gctggtgggt gttagaaact ctgtgggcag atattgccga tatatttaaa
1381 gacagagatc accgtttgct atttgaaata ttaaacgagc cacacctaag cgatgccaat
1441 aagtcgccta tgccccccgc caatttgcgt tttatgacgg gcaaagccta taacaaaatt
1501 cgcgcgatag atgcgcagcg aatcgttatt attggtggca accagtggtt tggtgcaggt
1561 gaaatggcaa acgtatggcc aaaccttaat gatgttggcg gcggttccga tgcatatgta
1621 atggctactt ttcaccatta cgacccgtgg tcgtttagtg gcgataacca aggcgattac
1681 gccgatgctt ggacgctatc taacgtgggt aacccaatgg atataatgca aagctgggca
1741 aacggcgtag gccaaggtat gcctgtgtat attggcgagt ggggcgtagg ttggggcagc
1801 cgctacagcg ccatgcagtg caataatatt cgctattggt accagctgtt cgacgcgagc
1861 tatgcctcgg caaaaggcca gcctacggca gtgtgggatg acggcggttg gtttaaaata
1921 ttcgaccacg gtaccaacag cttcaataat aatttagccc aatgtattgg tggaaactgc
1981 gcttgggatg gcgccgatag atttaattct ggctgtaatt aa
LOCUS NZ_AABI02000011 1098 bp DNA linear BCT 23-SEP-2003
DEFINITION Microbulbifer degradans 2-40 Mdeg_9, whole genome shotgun sequence.
ACCESSION NZ_AABI02000011 REGION: 115594..116691
VERSION NZ_AABI02000011.1 Gl:28878291
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1098)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028020. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi,nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1098
      /organism="Microbulbifer degradans 2-40"
      /mol_type="genomic DNA"
      /strain="2-40"
      /db_xref="taxon:203122"
   gene 1..1098
      /gene="Mdeg1925"
   CDS 1..1098
      /gene="Mdeg1925"
      /codon_start=1
      /transl_table=11
      /product="COG2730: Endoglucanase"
      /protein_id="ZP_00066536.1"
      /db_xref="Gl:23028118"
      /db_xref="COG:COG2730" 
/translation="MRTTKFLALALCLLASASALSANNSAPSNDWWDIPYPSQFDVKS
LKTQSFISVKGNKFIDDKGKTFTFRGVNIADTGKLLSQNQWQKSLFEELANNWGVNTI
RLPIHPVSWRKLGPDVYLGHIDEAVRWANDLGIYLILDWHSIGYLPTEQYQHPMYDTT
IKETRDFWRRITFRYKNVPTVAVYELFNEPTTMGNTLGERNWAEWKTLNESLIDMIYA
SDKTVIPLVAGFNWAYDLSPIKKAPIEREGIAYAAHPYPQKAKPEVKNDKNFFKLWDE
KWGFAADTYPVIATELGWVQPDGYGAHIPVKDDGSYGPRIVKYMQKKGVSYTVWVFDP
DWSPTMINDWDFTPSEQGAFFKQVMLEAKKR"
ORIGIN 
1 atgcgcacaa ccaaatttct tgcgcttgca ctctgcttgc tggcctcagc cagtgcactg
61 agtgcaaata acagcgcccc atcaaacgac tggtgggata taccctaccc gagccaattc
121 gatgtaaaaa gccttaaaac gcaaagtttt atatcggtaa aaggtaacaa gttcattgat
181 gataagggca aaaccttcac ttttagaggg gtaaacattg ccgatacagg taagctactt
241 agccaaaatc aatggcaaaa atcgctgttt gaagagctgg ctaataactg gggggtaaat
301 actattcgcc tgcctattca ccctgtaagt tggcgtaaac ttgggccaga cgtttattta
361 ggccacatcg atgaggcggt acgctgggcg aatgatttag gtatttacct tattcttgat
 
421 tggcactcca ttggctattt gcccaccgag caataccaac accccatgta cgacaccacc
481 attaaagaaa cccgcgactt ttggcgcaga attacgttcc gctacaaaaa cgtgcccacc
541 gtagcggtat acgaattatt taatgagcca accaccatgg gtaacaccct aggcgaacgc
601 aactgggccg agtggaaaac cttaaatgaa agcctaattg atatgatata tgccagtgac
661 aaaaccgtca ttccgctggt tgcaggcttc aactgggcct atgatttatc gccaatcaaa
721 aaggcaccta tcgagcgtga aggcattgct tacgccgcac acccctaccc gcaaaaggcg
781 aaaccagagg ttaagaacga taaaaacttc ttcaaactgt gggacgaaaa gtggggcttt
841 gctgcagaca cctaccctgt aatagcaaca gagctaggct gggtacaacc cgatggttat
901 ggtgcccaca tacccgttaa agacgacggc agttacggcc cccgcatagt gaagtatatg
961 cagaaaaaag gcgtttctta cacggtatgg gtattcgacc ccgactggag cccaacaatg
1021 attaacgact gggattttac ccccagcgag caaggcgcgt tttttaaaca ggttatgcta
1081 gaagctaaaa aacgctaa
LOCUS NZ_AABI02000008 1917 bp DNA linear BCT 23-SEP-2003
DEFINITION Microbulbifer degradans 2-40 Mdeg_7, whole genome shotgun sequence.
ACCESSION NZ AABI02000008 REGION: complement(17943..19859)
VERSION NZ_AABI02000008.1 GI:28878294
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1917)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027735. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1917
      /organism="Microbulbifer degradans 2-40"
      /mol_type="genomic DNA"
      /strain="2-40"
      /db_xref="taxon:203122"
   gene 1..1917
      /gene="Mdeg1560"
   CDS 1..1917
      /gene="Mdeg1560"
      /codon_start=1
      /transl_table=11
      /product="COG2730: Endoglucanase"
      /protein_id="ZP 00066178.1"
      /db_xref="GI:23027746"
      /db_xref="COG:COG2730" 
/translation="MTFTRMKSSHQGACRPRSSTLQRLIASSLTTACLLAASTFADVA
PLTVDGNRILSGGQEASFAGNSLFWSNNYWGGEKYYTAETVNWLKQDWGATLVRAAMG
VEDNGGYLDDKEGNKQKVKTVVDAAIANDMYVIIDWHSHHAEDHKSEAIAFFEDMART
YGNKKHVIYEIYNEPLQISWSNTIKPYAEDVIRAIRAIDPDNLIIVGTPTWSQDVDVA
SQDPITGYANIAYTLHFYAGTHKQSLRDKAQTALNNGIALFATEWGTVNANGDGAVNT
TETDKWMTFFKTNHISHANWALNDKSEGASALNPGASPNGNWSNADLTTSGKYVKNII
KNWNDGTPGGSSSSSSGGSTSSSSSSSSSNSSSGAGKVNLPARIEAENYNSAPVETTA
GNSGGSVSQCTYRGLNVDVQDASEGTCNIGWTAAGEKVTYNIGTANNTYNIALRTASL
DAGKRVSVYVGNTLADTISTQGGGWQNWKTQTIPNVYIPSNSVITVEFYDGRTNLNYL
NISAASGSSSSSSSSSSSTSSSSSSSSSSSSGGGSCSSYIDIPWNTRTEVTLTSGACV
RFNQNLSGKTLQVWDSDANSSCDFRGTVTTVGGTGSLNVSSNYVSSKSLTGTKLTFNS
ASNNNCKYVKVRAY"
ORIGIN 
1 atgactttca caagaatgaa atcatcacac caaggcgcgt gtcgaccaag gtcttccacc
61 ctacagcgac taatcgcctc atcacttacc accgcatgtt tgctagcagc gtctactttt
 
121 gccgacgtag cgccgttaac cgtagatggc aaccgcattc tcagcggtgg ccaagaggct
181 agctttgccg gtaacagttt gttttggagc aacaattatt ggggcggtga gaaatactac
241 acagccgaaa ctgttaactg gttaaaacaa gactggggcg caacactagt gcgcgcggcc
301 atgggtgtag aagataacgg cggctaccta gatgacaaag aaggcaacaa acaaaaggta
361 aaaaccgttg tagatgctgc tattgccaac gacatgtatg taattatcga ttggcacagc
421 caccacgccg aagaccacaa aagtgaagcc attgcttttt ttgaggatat ggcgcgcacc
481 tacggcaata aaaaacacgt tatttacgaa atttataacg agcctttaca aatttcgtgg
541 agcaacacaa ttaaacccta cgccgaagat gtaattagag ctattcgcgc gatagacccc
601 gacaacttaa ttattgttgg tacgccaacg tggtcgcaag atgtagacgt agcatcgcaa
661 gaccccatta ccggctacgc caatattgcc tacacattgc acttttacgc aggcacccac
721 aaacaatctt tacgagacaa agcgcaaacc gcacttaaca acggcatagc gcttttcgca
781 acagagtggg gaacagtaaa tgcaaacggt gatggcgctg taaacaccac cgaaacagac
841 aagtggatga cgttctttaa aaccaaccac ataagccacg caaactgggc gctaaacgac
901 aaatcagaag gcgcttctgc attaaacccc ggagccagcc ccaatggcaa ctggagcaac
961 gccgacttaa ccacatcggg taagtacgta aaaaacatta tcaaaaactg gaacgacggc
1021 acgccgggag gcagctcttc aagctcgtcc ggcggctcaa ccagttcctc ctcaagctca
1081 tctagctcta attccagctc tggtgctggc aaagtaaatt tacccgcacg cattgaagcc
1141 gaaaactata acagtgcacc ggtagaaaca actgcaggca atagtggcgg cagcgtttca
1201 caatgtacat acagagggct aaatgtagac gtacaagacg caagcgaagg cacttgtaat
1261 attggctgga cagcagcagg cgaaaaagtt acctacaaca taggcacagc aaataatact
1321 tacaatattg cacttcgcac cgcatcgctt gatgcaggca agcgcgtatc ggtatatgta
1381 ggcaacaccc tcgccgacac aataagcacc caaggtggcg gctggcaaaa ttggaagacg
1441 caaaccatcc ccaatgtata tattccatca aactcagtta ttaccgtgga attctacgat
1501 ggccgcacca accttaacta cttaaacatt agtgcagctt cggggtcttc ctcttcaagc
1561 tcctcatcta gctcgtcaac gtctagctct tcttcgagct catcttctag ctcttcaggt
1621 ggtggcagtt gtagcagcta tatagatata ccttggaata ctcgcaccga agttacccta
1681 acaagtggcg cctgcgttcg ctttaaccaa aacctttcgg gcaaaaccct acaagtgtgg
1741 gatagcgatg caaactcatc gtgcgatttc cggggcacag ttacaacagt aggcggcact
1801 ggcagtttaa atgtaagcag caactatgtt tcgtctaaga gcctaacagg aaccaaactt
1861 acatttaatt cagcaagtaa taacaattgt aagtacgtta aagttcgtgc ttattag
LOCUS NZ_AABI02000045 1893 bp DNA linear BCT 23-SEP-2003
DEFINITION Microbulbifer degradans 2-40 Mdeg_45, whole genome shotgun sequence.
ACCESSION NZ AABI02000045 REGION: complement(268..2160)
VERSION NZ_AABI02000045.1 GI:28878257
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1893)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029974. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi_{.}nlm.nih.gov/sutils/genom table.cgi.
   URL -- http://www.igi.doe.gov
   Contact: Paul Richardson
   microbes@cuba.jgi-psf.org.
   COG assignment was automatically updated on Sep 23, 2003.
FEATURES Location/Qualifiers
   source 1..1893
      /organism="Microbulbifer degradans 2-40"
      /mol_type="genomic DNA"
      /strain="2-40"
      /db_xref="taxon:203122"
   gene 1..1893
      /gene="Mdeg3677"
   CDS 1..1893
      /gene="Mdeg3677"
      /codon_start=1
      /transl_table=11
      /product="COG2730: Endoglucanase"
      /protein_id="ZP 00068260,1"
      /db_xref="GI:23029975"
      /db_xref="COG:COG2730" 
/translation="MKSATTNQSRARSSAFKNMLAASLAGLGLLSASAFADVAPLTVD
GNKILSGGQQASFAGNSLFWSNNGWGGEKYYTAGTVEWLKQDWGSNLVRAAMGVDENG
GYLEDPAGNKAKVTTVVDAAIANDMYVIIDWHSHHAEDYQNQAISFFQDMARTYGNNN
NVIYEIYNEPLQVSWSGTIKPYAEAVIGAIRAIDPDNLIIVGTPTWSQDVDVASRDPI
TQYSNIAYTIHFYAGTHKQSLRDKAQTALNNGIALFATEWGTVNANGDGGVDAAETDR
WMQFFKANHISHANWALNDKAEGSSALKPGSNANGGWSNSDLTASGTYVKNLIKTWND
GSPSSSSSSTSSSSSSSSSSSSSSSSSSSSGGTNLPARIEAENYDSAPVETTAGN
SGSPTNCSYKGMGVDVENSTEGACNIGWTAAGEKVTYNIGNADGTYDIALRVASMDAG
KRISVHVNNSLADTVTTQGGGWQAWTTETISNVYIPSNSVITVEFYDSGSNLNFLNIT
ESSGTEPPVEPPVEPPVEPPVDNGNFPCNDGNSTLANNGASINLNQGACVKYNHGWGD
IRLGTWSGNGTIRYDVLDCNNNVMSDIAQKLNDFTAVDTATMNCAHYIYVKQAPSSYT
LQFGSW"
ORIGIN 1 atgaaatcag caaccacaaa tcaatcgagg gcacgcagta gcgcctttaa aaatatgttg 
61 gcggcatcgc tcgcaggttt agggctacta tcagcttctg catttgccga tgtagccccg
121 ctaaccgtag acggcaataa aattcttagc ggtggccagc aagccagttt tgccggtaat
181 agcttatttt ggtctaacaa tggctggggc ggtgagaagt attacacggc cggtaccgtt
241 gaatggctaa agcaagactg gggcagtaat ttagttcgcg ccgcaatggg tgtcgatgaa
301 aacggcggct acttagaaga cccagcagga aacaaagcga aagtaacaac cgttgtagat
361 gcagccatcg ctaacgatat gtatgtaatt atcgattggc acagccacca cgccgaagac
421 taccaaaacc aagccattag ctttttccaa gatatggctc gcacctacgg taacaacaac
481 aacgttatat acgaaattta taacgagcca ttacaggttt cttggagcgg caccatcaag
541 ccttacgcag aagcggtaat tggcgcaatt cgcgcaatcg acccagataa ccttattatt
601 gtgggcacgc ctacttggtc gcaggatgta gacgtagcct cgcgcgaccc catcacgcag
661 tacagcaaca ttgcctacac tattcacttt tatgcgggca cccacaaaca atccctacgc
721 gataaagcac aaaccgcatt aaataatggt attgctttgt ttgctaccga atggggtaca
781 gtaaatgcca acggtgacgg cggtgtagac gcagccgaaa ctgatcgttg gatgcagttt
841 tttaaagcga atcatataag ccatgccaac tgggccttaa acgataaagc cgaaggctct
901 tctgcattaa agcctggctc taacgcaaac ggcggctgga gcaattccga cttaaccgcc
961 tctggtacct atgttaaaaa cttaattaaa acatggaacg acggctcacc gagcagcagc
1021 tcatctagca gcaccagttc ttcttcaagc agctcctcgt ctagtagctc atcatctagc
1081 agctcttcat ctagtagttc tggcggtacc aatttacccg cgcgcattga agcagaaaac
1141 tacgatagcg caccggtaga aaccactgca ggtaatagcg gctcacccac caattgttcg
1201 tataaaggta tgggcgtaga tgtagaaaac tctactgaag gtgcttgtaa tattggctgg
1261 actgcggcag gcgaaaaagt aacttacaac attggcaatg ccgatggcac ttacgatatt
1321 gcattgcgcg tagcctctat ggatgcgggc aaacgtatct ctgtgcatgt aaacaacagc
1381 ctagcagata ccgtaaccac acaaggtggc ggctggcagg catggactac cgaaaccatt
1441 tctaacgtgt atatcccatc aaactcggta attaccgttg agttttacga tagtggctct
1501 aacctaaact ttttaaacat taccgaaagc tcgggtaccg aaccacctgt agaaccaccc
1561 gttgagccgc cagtagaacc acccgtagac aacggtaact tcccatgtaa cgacggtaac
1621 tctacgcttg ccaacaacgg cgcctccatt aaccttaacc aaggagcgtg tgttaaatac
1681 aatcacggct ggggcgatat tcgtttaggc acctggagcg gcaacggtac cattcgatac
1741 gacgtactag actgcaataa caacgtaatg agtgatattg cacaaaaact taatgacttt
1801 actgctgtag acaccgcaac aatgaactgc gcacactaca tttatgtaaa acaagcccct
1861 agcagctaca ccctgcaatt tggtagctgg tag
LOCUS NZ_AABI02000017 2178 bp DNA linear BCT 23-SEP-2003
DEFINITION Microbulbifer degradans 2-40 Mdeg_17, whole genome shotgun sequence.
ACCESSION NZ AABI02000017 REGION: complement(88284..90461)
VERSION NZ_AABI02000017.1 GI:28878285
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2178)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028916. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2178
      /organism="Microbulbifer degradans 2-40"
      /mol_type="genomic DNA"
      /strain="2-40"
      /db_xref="taxon:203122"
   gene 1..2178
      /gene="Mdeg2776"
   CDS 1..2178
      /gene="Mdeg2776"
      /codon_start=1
      /transl_table=11
      /product="COG2730: Endoglucanase"
      /protein_id="ZP 00067367.1"
      /db_xref="GI:23028996"
      /db_xref="COG:COG2730" 
/translation="MKINTLFTPLRTVGAAVAIALSPVAFADVTCEVTNFNQWNSGYQ
ADVRVTNSGSAVSGWTVNLNFASAPQMTNGWNAALSTSGNTISASNISWNGNLGNGQS
TSFGFQGNSNGNLATPTCVGSGTGSSSSSSSSSTSSTSSSSTSSSSTSSTSSSSSSSG
GECVEMCKWYQDAPRPLCNNQDSGWGWENNQSCIGRTTCNSQSGNGGVINSCPSSSSS
SSSSSTSSTSSSSTSSTSSSSTSSTSSTSSSSTSSTSSSSTSSTSSSSSSGGGVFRVD
ATGNITKNGEVLPVRCGNWFGLEGQHEPSDAQNNPGGAPLELYVGNMWWVDSGRTIQQ
TMSEITAQGINMVRLPIAPQTLNPNDPQGVGDVRNGGVLKNHESVQQTNARQALEDFI
VQANENDIQVLIDIHSCSNYVGWRAGRLDAEPPYVDATRVGYDFTREDYSCGTNVGPG
VTVHEYNEEIWLNNLREIAGLSESLGVDNIIGIDIFNEPWDYTWEEWKALSESAYQAI
SEVNPDILIFVEGVAGGTGAGVDVPHGDESSNPNWGENFYPAQTAPLNIPKDRLVISP
HTYGPSVFVQRQFMDPNDPECVGLEGDEAAEAGCQIVIDYATLAAGWDEHFGFLREQG
FAMVVGEFGGNMDWPNGTRQAEKDMWSHITPGIDRQWQEAFVDYMVEKNIQACYWSIN
PESGDTGGWYGHEYDPVSNDAGWGRWLDFDSRKTNLLKELWGI"
   gene complement(570..785)
      /gene="Mdeg2777"
   CDS complement(570..785)
      /gene="Mdeg2777"
      /codon_start=1
      /transl_table=11
      /product="hypothetical protein"
      /protein_id="ZP 00067368.1"
      /db_xref="GI:23028997" 
/translation="MLEVELLLVELVELDEVLEVLLVELDEVLEVLDELVDEVLEELE
ELEELGQLLITPPLPDWLLQVVRPIQL"
ORIGIN 
1 atgaaaatca acactctctt tacgcctttg cgtactgtgg gtgctgcagt tgcgatagct
61 ttatcgcctg tagcctttgc agacgtaacg tgcgaagtaa cgaactttaa ccagtggaat
121 agtggctacc aagccgatgt tcgtgttaca aacagcggta gcgctgttag tggctggacc
181 gtaaatttaa attttgcctc agccccgcaa atgacaaatg gctggaacgc agctttgagt
241 actagcggca atacaattag tgcatctaat attagttgga atggcaattt gggtaatggt
301 cagtccacca gctttggttt tcagggcaat tcaaatggta acttggcaac gccaacgtgt
361 gtaggtagcg gtacggggtc ttctagcagc tcttcatcca gctctacttc tagcacaagc
421 tcatcatcta caagttcttc tagcacgtct tctactagct ctagcagttc atcctctggt
481 ggtgaatgtg tagaaatgtg taagtggtat caagatgcac cgcgcccatt atgtaataat
541 caagacagtg gttggggttg ggaaaacaat caaagctgta ttggtcgcac tacttgtaac
601 agccaatctg gcaatggtgg tgtaattaat agttgcccaa gttcttcaag ttcttcaagt
661 tcttctagca cttcgtctac cagctcatct agtacttcaa gtacttcatc gagctcaaca
721 agtagtactt caagcacttc atcaagttcc acaagctcta ctagcagcag ctcaacctct
781 agcactagct cgtcgtcttc aagtggtggt ggagtattcc gcgtagatgc taccggtaat
841 attactaaaa atggtgaagt actgcctgtt cgttgtggta actggtttgg tctagagggc
901 cagcacgagc cttcagatgc gcaaaataac ccaggcggtg cgccgcttga attatatgtt
961 ggcaacatgt ggtgggtaga tagtggccgc actattcagc aaaccatgag cgaaattacc
1021 gcccaaggta tcaacatggt tcgcttgcct attgcaccgc aaacattaaa ccctaacgac
1081 cctcaaggtg tgggtgatgt gcgcaacggc ggcgtgctta aaaatcacga atctgtgcag
1141 caaaccaatg cacgtcaagc gttagaagac ttcattgttc aagctaacga aaatgacatt
1201 caagtgctaa ttgatattca ctcttgtagt aactacgtgg gttggcgtgc aggccgttta
1261 gatgcagagc ctccttatgt ggatgcaacg cgagtgggtt atgactttac ccgtgaagat
1321 tattcttgtg gcaccaatgt gggcccaggt gtaactgtgc acgagtacaa cgaggaaatt
1381 tggttaaaca acttgcgtga gattgctggt ttatctgaat ccttgggcgt tgataatatt
1441 atcggtatcg atatttttaa cgaaccatgg gattacactt gggaagagtg gaaagcactt
1501 tctgaaagcg cttatcaagc cattagcgaa gttaacccag atattctaat ctttgttgag
1561 ggtgttgcag gcggcacggg tgctggtgtt gatgtgccac atggagacga gtcttctaac
1621 cctaactggg gcgaaaactt ttatcctgcg caaactgctc cgcttaatat tccaaaagat
1681 cgtctagtta tttcaccgca tacctatggc ccatctgtat ttgttcagcg tcaatttatg
1741 gacccgaatg atccagagtg tgttggttta gaaggtgatg aggcggctga agctggctgt
1801 caaattgtta tcgattatgc aaccttagca gctggttggg atgagcattt cggcttctta
1861 cgtgagcaag gctttgccat ggtagtgggt gagtttggtg gcaacatgga ttggccaaat
1921 ggcacgcgcc aagcagaaaa agatatgtgg agccacatca cccctggaat cgacagacag
1981 tggcaagaag cgtttgttga ctacatggtt gagaaaaaca tccaagcttg ttactggtca
2041 attaacccag agtctggcga cactggcggt tggtatggtc acgagtacga ccctgtttct
2101 aacgatgcag gttgggggcg ttggttagac ttcgattctc gcaaaactaa cttacttaaa
2161 gagctttggg gtatttaa
LOCUS NZ_AABI02000001 1833 bp DNA linear BCT 23-SEP-2003
DEFINITION Microbulbifer degradans 2-40 Mdeg_1, whole genome shotgun sequence.
ACCESSION NZ AABI02000001 REGION: 285915..287747
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1833)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL - http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1833
      /organism="Microbulbifer degradans 2-40"
      /mol_type="genomic DNA"
      /strain="2-40"
      /db_xref="taxon:203122"
   gene 1..1833
      /gene="Mdeg0218"
   CDS 1..1833
      /gene="Mdeg0218"
      /codon_start=1
      /transl_able=11
      /product="COG2730: Endoglucanase"
      /protein_id="ZP 00064857.1"
      /db_xref="GI:23026371"
      /db_xref="COG:COG2730" 
/translation="MMYTNLFNLKKHLFQTSLKLLACATLIGGTLNAAADVPAMSVQG
NKVLVGGEVKSLGGMSYFWSNNGWGGEKYYNASTVSYFKQDWKASIVRAAMGVEDAGG
YFDDPQGSKQKVRTIVDAAIANDMYVIIDWHSHYANTHDWAAAVQFFQEMARDYGQYN
NVIYEVYNEPLDIPWGHIKSYAETVIDAIRAIDPDNVIVVGTPRWSQGVKEASWDPIN
RNNIAYTLHFYSGSHGQWLRNDAAEAMSNGIALFVTEWGSVNANGDGAVNEGETAAWM
NFMRDNGIHHANWSVNDKAEGASALNPGASATGGWGDGDLTWSGHVVRGYLRDWNQIG
SGNGNGNGTGCTEVSLPGTIEAEAYCAMDGIQTENTNDTNGGSNVGYIDAGDWMSYSV
NVANAGTYTVSYRVASLGGGGVLSIENAGGSPVYGTLNVPQTGGWQEWTTVSHDISLQ
AGQQNIGIAAIEGGFNINWIALTPAGTNPNPVQSITLQAEDYSFMSGVQVENTSDNGG
GMNVGWLDAGDWLAYHGVNIPTSGQYTITYRVASQSGGGSLQLEQAGGGVVYGNLNVP
STGGWQNWVDVSHTVTLNAGVQDFGLGITSGGFNINWIKVEAIH"
ORIGIN 
1 atgatgtaca caaacctctt taatttaaaa aagcacctct ttcaaacctc acttaaacta
61 ctggcctgcg ccacattaat tggcggcacc ctaaacgcag ccgctgacgt gccagcaatg
121 tccgtacaag gcaataaagt actggtgggc ggtgaagtta aaagccttgg aggtatgagc
 
181 tatttttggt ctaacaacgg ctggggcggc gagaaatact acaacgcttc taccgttagt
241 tacttcaagc aagactggaa ggcatccatt gttcgagctg caatgggggt agaagatgcc
301 ggcggctact tcgatgaccc gcagggctct aagcaaaaag ttcgtacaat agtagatgcc
361 gccattgcga atgatatgta cgtcattatc gattggcact cacattacgc caacacccac
421 gactgggcag ccgctgtgca atttttccaa gaaatggcac gtgactatgg ccaatacaat
481 aatgtgattt atgaggtata caacgaacca ctggatatcc cttggggcca cataaaaagc
541 tacgccgaaa cggtaattga tgccattcgc gcaattgacc cagataacgt gatcgtagta
601 ggcactcctc gctggtcgca gggggtaaaa gaagcgtcat gggacccaat caaccgcaat
661 aatattgcct acacgctgca cttctattca ggtagtcatg gccaatggct gcgcaacgac
721 gcagcagaag ctatgagtaa tggtattgcc ttgtttgtta ctgaatgggg cagcgtaaat
781 gccaatggcg atggcgcagt caacgaaggc gaaaccgcag cgtggatgaa cttcatgcgc
841 gataacggta tccatcacgc aaactggtct gtaaacgaca aagcagaggg tgcatctgca
901 cttaaccctg gcgccagtgc cacaggtggt tggggcgacg gcgatttgac ttggtctggc
961 catgttgtgc gcggctacct gcgcgactgg aaccaaattg gttctggcaa tggtaacggc
1021 aacggcacag gctgcaccga ggttagccta ccaggcacga tagaagcgga agcctactgc
1081 gcaatggatg gtatccaaac cgaaaacacc aacgacacca acggcggcag taacgtgggc
1141 tacatagatg ctggcgactg gatgagctac agcgtaaacg ttgctaacgc aggcacttat
1201 accgtgtctt accgcgtggc tagccttggc ggcggcggtg ttctaagcat tgaaaatgcc
1261 ggcggctcgc ccgtttatgg cacgctgaat gtaccgcaaa ctggcggctg gcaagaatgg
1321 accactgtat ctcacgatat tagcttgcaa gccggccaac aaaacattgg catagcggca
1381 atagaaggtg gttttaacat caactggata gccctaaccc ctgctggcac caaccccaac
1441 ccagtgcaaa gtattacctt acaagcagaa gactactcct ttatgagtgg cgtgcaggta
1501 gaaaatacta gcgacaatgg cggcggtatg aacgtaggct ggttagatgc tggcgactgg
1561 cttgcctacc acggcgtaaa cattccaacc tctggccaat acaccataac ttaccgagta
1621 gccagccaaa gcggtggtgg aagcctgcag ctagaacaag caggtggcgg cgttgtttac
1681 ggtaacctga acgtaccaag cactggcggc tggcagaact gggtagacgt aagccatacc
1741 gttaccctta acgctggtgt acaagatttt gggttaggta ttactagtgg tggcttcaat
1801 attaactgga taaaagtcga ggcaattcac taa
LOCUS NZ_AABI02000001 2376 bp DNA linear BCT 23-SEP-2003
DEFINITION Microbulbifer degradans 2-40 Mdeg_1, whole genome shotgun sequence.
ACCESSION NZ AABI02000001 REGION: 28883..31258
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2376)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2376
      /organism="Microbulbifer degradans 2-40"
      /mol_type="genomic DNA"
      /strain="2-40"
      /db_xref="taxon:203122"
   gene 1..2376
      /gene="Mdeg0020"
   CDS 1..2376
      /gene="Mdeg0020"
      /codon_start=1
      /transl_table=11
      /product="COG5297: Cellobiohydrolase A
      (1,4-beta-cellobiosidase A)"
      /protein_id="ZP 00064659.1"
      /db_xref="GI:23026173"
      /db_xref="COG:COG5297" 
/translation="MLASNKNSKLANSEQHRPYKTRTARWLTGSGVIASSLLFSAQSF
AAQCEYIISNEWNSGFTGAVRITNNGTTPINGWDVSWQYAGDAVTSSWNANVSGSNPV
SATPLSWNANIQPGQSVEFGFQGSKAGSNAEIPTVTGAVCDSGSSSSSSSSSSSSSSS
SSSSSSTSSSSSSSSSTSSSSSSSGSSGTGGIACTVGNANIWGSGYQLDMQVVNNGTA
AVSSWDVTMAFGEAPQRTGGWNANFVESGNTIVASNISWNGNLAPGQSASFGIQGNHD
GSFGGVTCNGASSSGSSSSGSSTSSSSSSSSSSGSSTSSSSSSSSTGSTSSSSSSSTS
STSSSSSSSTSSTSSSSSSSSSSTSSSSSSSSSSSSSSSTSGSGAGFDNPFIGGKWYVDP
VWSAKAAAEPNGSLIANYNTAVWMDRIGAIEGPEDGDGMGLEEHLDEALAQGADIFMF
VVYDLPNRDCAALASSGELLIAENGFERYQNEYIGPIVDILSKPAYSSLRIIAIIEVD
SLPNLVTNLNIQKCVEANGPGGYVDGIQHALNELNTLDNVYPYVDIAHSGWLGWSDNF
AGATKLIGDAIKGTNKGVNSIAGFVSNSSNYTPVTEPYLPNPTLQIGSNQVRSADFYE
WTMYFEELSFVQDWRQAMIQQGFPESIGMLIDTARNGWGGPDRPTGESTSTDLNTYVN
ESRIDRRQHRGNWCNQPGGVGFRPQAAPEPGVDAYVWVKPQGESDGISDPNFPIDPND
PAKQHDPMCDPNAPNRDNNAVGTGALDNAPHAGRWFPEAFQILIENAYPPL"
   gene complement(369..566)
      /gene="Mdeg0021"
   CDS complement(369..566)
      /gene="Mdeg0021"
      /codon_start=1
      /transl_table=11
      /product="hypothetical protein"
      /protein_id="ZP 00064660.1"
      /db_xref="GI:23026174" 
/translation="MPLEPDDELDEEVLEELDEELLVLLELLEELDELDDELELEELE
PLSHTAPVTVGISALEPALLP"
   gene complement(813..1151)
      /gene="Mdeg0022"
   CDS complement(813..1151)
      /gene="Mdeg0022"
      /codon_start=1
      /transl_table=11
      /product="hypothetical protein"
      /protein-id="ZP 00064661.1"
      /db_xref="GI:23026175" 
/translation="MNGLSKPAPLPEVEELELEEDELELDDVLELELELELVELVLEL
ELEEVLEVEELLELEVEPVEELELELEVLEPDELDELLDELVLEPELDEPELEAPLQV
TPPKEPSWFP"
ORIGIN 
1 atgttggctt ctaataaaaa tagtaagctg gcaaactctg agcaacaccg cccttataaa
61 acccgcacag cgcgctggtt aaccgggtct ggggttattg cttcaagttt gctttttct
121 gcgcagagtt ttgcggcgca atgtgaatac atcattagca atgaatggaa cagcggcttt
181 actggcgcag ttcgcattac taataatggc actactccca tcaatggctg ggatgttagc
241 tggcagtatg ccggcgatgc agtcaccagc agctggaacg cgaatgtttc tggctcgaac
301 cccgtttctg ctacaccatt aagctggaat gccaacattc aacccggtca aagcgttgag
361 tttggttttc agggcagcaa agccggctcc aatgcagaaa ttccaaccgt taccggcgcg
421 gtatgtgata gcggctctag ctcttccagc tccagctcat catctagttc atcaagctct
481 tctagtagct caagcagcac tagcagctcc tcgtccagct cttcaagcac ctcttcgtct
541 agctcatcat ctggctccag tggcacaggt ggtattgcgt gtactgtagg caatgcgaat
601 atttggggct cgggctacca gctggacatg caagttgtta acaacggcac cgctgcagta
661 agcagttggg acgtaaccat ggcattcggc gaggcaccac agcgcaccgg tggctggaac
721 gcaaactttg tagagtcagg caataccatt gttgcgagca acattagctg gaacggcaac
781 ctcgcaccgg ggcaatcagc ttcgtttggt attcaaggga accacgacgg ctcttttggc
841 ggcgtaacct gtaacggcgc ttcaagctct ggctcgtcta gttctggctc tagcaccagc
901 tcatcaagta gctcatccag ttcgtctggc tctagcactt ctagctctag ctcaagctcc
961 tctactggtt ctacctctag ctctagtagc tcttcaactt ctagcacttc ttcaagttct
1021 agctctagca ccagctccac gagttctagc tccagttcta gctcgagtac atcgtctagt
1081 tccagctcat cttcctcaag ctctagctct tctacttcag gcagtggcgc aggttttgac
1141 aacccgttca ttggcggcaa gtggtatgta gacccagtat ggtcagcaaa agctgcagca
1201 gagccaaacg gttcacttat tgccaactac aacacggcag tttggatgga tcgcattggt
1261 gcgattgaag gcccagaaga tggcgatggt atgggcttag aagaacactt agatgaagct
1321 ttagcacaag gtgcagacat ctttatgttc gtggtatacg acctaccaaa ccgcgactgt
1381 gcagctttgg cctcaagtgg cgaactactc attgccgaga acggttttga gcgctatcaa
1441 aatgagtaca ttggcccaat cgtagatata ctcagcaagc ccgcgtattc tagcttgcgt
1501 attatcgcga ttattgaagt ggattctcta cccaacctcg ttaccaacct caacattcaa
1561 aaatgtgttg aagcgaatgg cccgggtggg tacgtagacg gtatccaaca tgcacttaac
1621 gagctaaaca cgcttgataa tgtgtaccca tacgtcgata ttgctcactc aggctggcta
1681 ggctggagcg acaacttcgc cggcgccacc aagcttattg gtgatgcaat taaggcaca
1741 aacaaaggtg taaacagtat tgcaggcttt gtaagtaact cttctaacta cacacctgtg
1801 actgaaccat acctacctaa ccctaccttg caaattggta gcaaccaagt tcgatctgcg
1861 gatttctacg agtggaccat gtacttcgaa gaacttagct ttgtacaaga ttggcgccaa
 
1921 gccatgattc agcaaggctt cccagaatca attggtatgc ttattgatac cgcacgtaat
1981 ggctggggtg gacctgaccg tccaactggt gagtctacat ctaccgacct caacacctat
2041 gtgaatgaat cgcgtataga ccgccgtcag catcgcggaa actggtgtaa ccagcccggt
2101 ggtgttggct tccgtccgca agcggcacca gaaccaggtg tagacgctta cgtttgggtt
2161 aagccacaag gtgagtcgga tggtattagt gatcctaact tccctatcga ccctaacgac
2221 ccagctaaac agcacgaccc aatgtgtgat ccaaacgcac ctaaccgcga taacaatgcg
2281 gttggcacag gcgcgctaga taacgctcca catgctggtc gctggttccc agaagcattc
2341 caaatactta tagaaaacgc ctacccaccg ctatag
LOCUS NZ_AABI02000004 1737 bp DNA linear BCT 23-SEP-2003
DEFINITION Microbulbifer degradans 2-40 Mdeg_4, whole genome shotgun sequence.
ACCESSION NZ AABI02000004 REGION: 239646..241382
VERSION NZ_AA6102000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1737)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.igi.doe.gov
   Contact: Paul Richardson
   microbes@cuba.jgi-psf.org.
   COG assignment was automatically updated on Sep 23, 2003.
FEATURES Location/Qualifiers
   source 1..1737
      /organism="Microbulbifer degradans 2-40"
      /mol_type="genomic DNA"
      /strain="2-40"
      /db_xref="taxon:203122"
   gene 1..1737
      /gene="Mdeg1139"
   CDS 1..1737
      /gene="Mdeg1139"
      /codon_start=1
      /transl_table=11
      /product="hypothetical protein"
      /protein_id="ZP 00065765.1"
      /db_xref="GI:23027310" 
/translation="MNKVKVLALCASVAVMIGCSDADTKLANSAKAEVGFTKVNQLGY
LPAAKKLAVVPAVAAAKFDIIDVTSGKVAFTGSLSDVKSWSAMGDESFKLADFSALQA
EGSYRLVVQGVSDSYTFDISPSVYSQAHDGALKAYYYNRASTELTEQYAGVYARPAGH
PDTDVRIFDNAASAARPADTSFAAPKGWYDAGDYGKYIVNSGISTYTLMAAYEHFPSF
YKQRDIDIPESGDAVPDILDEVMWNLEWMQVMQDPNDGGVYHKLTTLNFSGAVMPHEA
TAQRYFIKKSTAATLDFAAVMATASRVYAPFEGAFPGKSAAYRQAAIAAWEWAQANPS
ETYSQTPLSKVQTGAYGDKKLNDEFAWAAAELFILTGEQKYWQAFNKQKVQAGESSWA
NVAGLGFISLANNARSLLNEAQYKTVTDSIVRAADSLLVTYKENAYQVPIGNKDFFWG
GNSGTLNRAWVLLEANKIKPQQEYIDAALAAVDYIYGRNPTNYSFVTGFGDNPAVGIH
HRPSYADGIKAPVPGWLAGGAHNGKQDGCEYPSDAPAKSYLDDWCSYSTNEIAINWNA
PLVYILAAVNNL"
ORIGIN 
1 atgaacaaag ttaaagtttt agcgctgtgt gccagtgtgg ctgtaatgat aggttgcagt
61 gatgccgaca ctaaattagc taactcggcc aaggccgagg tgggctttac caaagtgaat
121 cagctgggtt atttgcccgc ggccaaaaag ctggcggtgg tacccgccgt tgcagctgca
181 aaattcgaca taatcgatgt aactagcggt aaagtagcgt ttacggggag tttaagcgac
 
241 gtaaaaagct ggagcgcgat gggggacgaa tctttcaagt tggcagactt tagcgccctg
301 caagccgaag ggagttaccg cttagttgtt cagggtgtga gtgattctta caccttcgat
361 attagcccaa gtgtatatag ccaagcgcac gatggagccc ttaaagccta ttactataat
421 cgagcgagca cagagttaac agaacagtac gccggggtgt atgcgcgacc tgcggggcac
481 ccagataccg acgtacgcat attcgataac gccgcctcag ccgcgcgccc agcagataca
541 agctttgctg caccaaaggg ttggtacgat gctggcgatt acggcaagta cattgttaac
601 agtggtattt ccacttacac cctaatggct gcgtacgagc atttcccgtc gttttacaag
661 caacgcgata tagatattcc cgaatctggc gatgccgtac cggatattct cgacgaggta
721 atgtggaacc ttgaatggat gcaggtcatg caagacccga acgacggcgg tgtgtaccac
781 aagcttacca ccctgaattt ttctggcgca gtcatgccgc acgaagcgac tgcgcagcgc
841 tattttatta aaaaatctac cgctgcaacg ctagattttg ccgcggttat ggccactgca
901 agccgagtat acgcaccgtt cgaaggtgct tttcctggta aatcagctgc ttatcgacag
961 gcggccattg ctgcgtggga gtgggcacaa gcaaacccta gtgagacata ttcgcagaca
1021 ccgctgagca aagttcaaac cggcgcctat ggtgataaaa agttaaacga tgaatttgcg
1081 tgggcggccg cagagttgtt tatattgacc ggcgagcaaa aatactggca ggcgtttaac
1141 aagcaaaaag tgcaggcggg tgagtctagc tgggcgaatg ttgcggggtt ggggtttatt
1201 tccttggcca ataatgcgcg cagcctgtta aacgaagctc aatacaaaac cgttaccgat
1261 tcaattgttc gcgctgcaga tagcttgctt gttacttaca aagagaatgc ctaccaagta
1321 cccattggca acaaagattt tttctggggt ggcaattccg gcacgttaaa tcgcgcttgg
1381 gttttgcttg aggccaataa aattaaaccg cagcaagaat acatcgatgc tgcacttgcc
1441 gcggtggatt atatttatgg tcgcaaccct accaactact cttttgtcac tgggtttggc
1501 gataaccctg cggtgggtat ccatcatcgt ccatcctatg ccgatggcat taaagcccct
1561 gtgcctggtt ggcttgcggg cggtgcgcac aatggcaagc aagatggttg tgagtaccct
1621 tccgatgcac cggcaaaatc ctatctagac gactggtgca gttactccac caacgaaatt
1681 gctattaatt ggaatgcgcc gttagtttac atactggctg cggtaaataa tttgtag
LOCUS NZ_AABI02000004 2604 bp DNA linear BCT 23-SEP-2003
DEFINITION Microbulbifer degradans 2-40 Mdeg_4, whole genome shotgun sequence.
ACCESSION NZ AABI02000004 REGION: 254535..257138
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2604)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2604
      /organism="Microbulbifer degradans 2-40"
      /mol_type="genomic DNA"
      /strain="2-40"
      /db_xref="taxon:203122"
   gene 1..2604
      /gene="Mdeg1150"
   CDS 1..2604
      /gene="Mdeg1150"
      /codon_start=1
      /transl_table=11
      /product="hypothetical protein"
      /protein_id="ZP 00065776.1"
      /db_xref="GI:23027321" 
/translation="MNLTSIMFEQSVKKVAKSAIAVAVASAVTLSAAQAEVGNPRVNQ
VGYIPNGAKVASYVAPSNTAQTWQLLRNGSVVASGTTTPKGTDAASGDNIHHIDFSAV
SATGEGFSLLVGGDESYPFEISADAFTPVLYDSIRYFYHNRSGIAIETQYTGGGNGSY
AANAQWARPAGHINQNANQGDNAVPCWSGSGCNYALDVTKGWYDAGDHGKYVVNGGIS
VWKLLNMYERALHISGSQNKYADGTLNIPESGNGVADILDEARWQMEFLLAMQVPEGE
AKAGMVHHKMHDVGWTGLPLAPHEDNRERALVPPSVTATLNVAATGAQCARLFDEIDA
SFAASCLTAAERAWDAALQNPNDVYTGGYDNGGGYGDEVADDEFFWAAAELYITTGD
SKYLSTINNYNVTRIDWGWPDTELPALMSLAVVPANHTANLRATARAKIVEIADTHVA
TSNAAGYLTPSSALDYYWGSNNGVANKIALLGLAYDFTGDDVYAKTVSKAVNYLFGNN
TLSFSYISGHGENALQQPHHRFWAGALNGSYPWLPPGALSGGPNAGLEDGVAAAALSA
CVSTPAKCYMDDIESWSTNEITINWNGALVWAMAFYDDYADSGSGSSSSSSSSSSSSS
SSSSSSTSSSSSSSSSSSSSGSSSSSSSSTSSSSSSSSSGGECVEMCKWYQDAPRPLC
NNQNSGWGWENQQSCIGRTTCESQSGNGGVINSCGTSSSSSSSSSSSSSSSSSSSSSS
SSTSSSSSSSSSSSSSSSSTSSSSSSSSSGGVAGVACAVTKMNHWGSGYQLDVTVSNNGAA
AVSGWSIELDFGESPQLTGSWNAAVSASGNTVSATNISWNGNLSAGQSTSFGMQGNSD
GSLSTPSCLVK"
   gene complement(1812..1961)
      /gene="Mdeg1151"
   CDS complement(1812..1961)
      /gene="Mdeg1151"
      /codon_start=1
      /transl_table=11
      /product="hypothetical protein"
      /protein_id="ZP 00131521.1"
      /db_xref="GI:24762929" 
/translation="MEELLEELEPLDEELLLEDDELEVELEELDEELDELEELDELEP
LPESA"
ORIGIN 
1 atgaatctta cttcaatcat gtttgaacaa tcagtaaaaa aagtcgctaa gtcagccatt
61 gccgtggcag ttgcttcggc ggttacctta agtgcggcgc aggccgaggt gggtaaccca
121 cgtgttaacc aagtaggcta tatacccaat ggtgccaaag ttgccagtta tgttgcgcca
181 tcaaatacgg cacaaacgtg gcagttactg cgtaatggca gtgtggttgc aagtggcact
241 acaaccccaa agggtacaga tgcagcctcg ggtgacaata ttcaccatat cgatttttct
301 gcggtgagtg caaccggcga aggttttagt ttgcttgtgg gcggcgatga aagttacccc
361 tttgaaattt ctgccgacgc atttacaccg gttttatacg attccatccg ttacttttat
421 cacaaccgtt cgggtatcgc gattgaaacg cagtacaccg gtggcggtaa cggtagctac
481 gcggcgaatg ctcagtgggc taggcccgca ggtcacatta atcaaaatgc taaccaaggc
541 gataatgcgg tgccgtgttg gtcgggcagt ggttgcaact acgccttaga cgtaactaaa
601 ggttggtacg atgccggtga ccacggtaaa tatgttgtaa acggtggcat ttccgtatgg
661 aagctattaa acatgtacga gcgtgccttg cacattagtg gcagccaaaa taaatacgcc
721 gacggtacat taaatattcc tgaaagcggc aatggcgtgg cggatatttt ggatgaagct
781 cgctggcaaa tggagttttt attagccatg caagtgccag agggcgaagc gaaagctggc
841 atggtgcacc acaaaatgca cgatgtgggt tggacaggct tgccactagc accccatgaa
901 gataatcgcg agcgcgcgct tgtgccgcct tcggttactg caacccttaa cgttgcggcc
961 acaggcgcgc agtgtgcgcg tttatttgac gaaatagatg cgagttttgc agcaagttgt
1021 ttaactgccg cagagcgcgc atgggatgca gccctgcaaa accctaacga tgtttacact
1081 ggcggctacg ataatggcgg cggtggttac ggcgatgaag tggcggacga cgagtttttc
1141 tgggctgctg ctgagttata cattaccact ggcgatagca aatatctttc aaccattaac
1201 aactacaatg taacgcgcat tgattggggc tggccagata ccgagttgcc tgcgttgatg
1261 tcgttagcgg ttgtgcctgc taatcacacc gcaaatttgc gtgcgactgc tcgtgcaaaa
1321 attgtagaaa ttgcagatac ccatgtcgct accagtaatg ctgccggcta tttaacacca
1381 tcgtccgcgc tggattacta ctggggttct aacaatggcg tagccaataa aattgcgtta
1441 cttggtttgg catacgattt tactggcgat gacgtttacg cgaaaacggt gtcgaaagca
1501 gttaactatt tatttggtaa taatacctta tcgttttctt atatttctgg gcatggcgaa
1561 aatgctttgc aacagccgca tcaccgcttt tgggctgggg cattaaatgg aagttaccca
1621 tggttgccgc ctggtgcgct ttctggtggc cctaacgcag ggttagaaga tggcgttgcc
1681 gccgccgcgc taagtgcttg tgtttcaacg cctgccaaat gctatatgga tgatattgaa
1741 tcttggtcga ccaacgaaat tactattaac tggaatggtg cattggtttg ggcaatggcg
1801 ttttatgatg actacgccga ttcgggtagc ggttctagct cgtcaagttc ttctagctca
1861 tctagctctt cgtcaagttc ttccagttcg acttctagct cgtcgtcttc tagtagtagc
1921 tcttcgtcga gcggctcgag ttcttctagc agctcttcca catccagttc cagctcttcg
1981 agttcatcgg gtggggagtg tgtagaaatg tgtaagtggt atcaagatgc accgcgccct
2041 ctatgcaata accaaaacag cggttgggga tgggagaacc agcagagttg tattggtaga
2101 acaacttgcg aaagtcaaag tggcaatggt ggagtgatta attcgtgcgg cacgtctagc
2161 tcgagctctt catctagctc tagcagtagc tcttcgagtt catccagctc ttctagcagt
2221 tcttccacat caagctcgtc gagtagttcg tcttctagct cttctagttc gacttcaagt
2281 tcttcgtcga gcagttcagg gggcgttgca ggtgtggctt gtgcggtaac caaaatgaac
2341 cattggggca gcggatatca attagatgta acagtttcta ataatggtgc tgcagcggta
2401 agtggttgga gtattgaact cgattttggt gaatcgccac agcttactgg tagttggaat
2461 gctgctgtat cggcatctgg taatactgta tcggctacta acattagttg gaacggtaat
2521 ttaagcgctg ggcaatctac ctcttttggt atgcagggta attcagatgg ttcgctgagc
2581 acgccaagct gtttagttaa gtaa
LOCUS NZ_AABI02000001 3219 bp DNA linear BCT 23-SEP-2003
DEFINITION Microbulbifer degradans 2-40 Mdeg_1, whole genome shotgun sequence.
ACCESSION NZ AABI02000001 REGION: complement(289745..292963)
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3219)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review.
   DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..3219
      /organism="Microbulbifer degradans 2-40"
      /mol_type="genomic DNA"
      /strain="2-40"
      /db_xref="taxon:203122"
   gene 1..3219
      /gene="Mdeg0221"
   CDS 1..3219
      /gene="Mdeg0221"
      /codon_start=1
      /transl_table= 11
      /product="COG1472: Beta-glucosidase-related glycosidases"
      /protein_id="ZP 00064860.1"
      /db_xref="GI:23026374"
      /db_xref="COG:COG1472"

/translation="MKNTLSFKTSLLAGLVASSLLVAACQGVKQQTEATQTKHNITLW
PQASSPVIKSPDYEAEVEAKVEALLGQMTLEQKVGQILQPEIQSIKPHEVKEYHIGSV
LNGGGSMPNRIENAPPIEWVKLADAFYDASMDDSDGGIAIPIIWGTDAVHGHGNVTGA
TIFPHNIGLGAARNPALIEKIGEITAKEVRATGIEWIFGPTLAVAQNDLWGRTYESYS
EDPAIVADYASAMVVGMQGKVDDSDFLSTNRVVATAKHFLADGGTLGGNDQGDARISE
EELVQIHNAGYVPAIESGVQTVMASFSLWNGVKMHGNNYLLTQALKERMGFDGFIVGD
WNGHGQVPGCTNESCPQSLNAGLDMYMVPYDWKKLYRNLISQVQSGEIAPSRLDDAVR
RILRVKIRANLWAAKPSERINLATIDEVVGHANHREVARQAVRESLVLLKNKNSVLPI
AANKTVLVAGDGADNIGKQSGGWSVSWQGTGNTNASFPGGTSIYKGIADAVTQGGGKA
TLSVDGSYKTKPDVAIVVIGEDPYAEGQGDRNSLEFEPVNKKSLELLKKLKADGIPVV
TVFISGRPMWANPEINASDAFVAAWLPGSEGQGVADVLIGNANGKPRFDFKGTLSFSW
PKLPTQGLLNPTHPNYDPLFKLGYGLTYASSETGPEQLAEDVEGVDKGSTGDINFYVG
RTLEPWEVFVRTPESSQRLSGPFADLGNASVRTSDMQVQEDALTFTWGGSWMSILGIE
GGRGYDLSSQYKEGGVISFNFNSIDMAKGDLKVQMACGEGCTREVDITTIARDLEGKG
WQSLTVPLACFAHEGDDFTHITAPFNLFAGGKGQVAVANIRILRAGTQTVPCVLPKDV

SVTPEPLNASWAIDWWMPRHKEKLARIQQGNVDLLMIGDSITHGWEDAGKDVWAQYYA
HRNAVDLGFSGDRTENVLWRLQHGEADGIKPKVAVVMIGTNNAGHRHEPSHYTAKGVA
AVVAELQKRLPETKILLLGIFPRGETSEDPLRVLNAKTNTLLAKMADGEKVVYLNINK
TFLDENGVLPKDIMPDLLHPNEKGYALWAKAMEPTLKKMLGE"

ORIGIN 
1 atgaaaaata ctttatcctt taaaacatcc ttgcttgcgg gcttggtggc atccagttta
61 ctggttgcgg cctgtcaggg tgttaaacag caaacggaag ctactcagac aaagcacaat
121 attaccttat ggccgcaggc gtctagccct gtaataaagt cgccagatta cgaagcggaa
181 gtggaagcca aggtagaagc gttgttagga caaatgacgc tagagcaaaa agtagggcaa
241 atcctacagc cagaaattca atctattaag ccgcatgaag taaaagaata ccacattggc
301 tctgtactaa atggtggtgg ctctatgcct aaccgcatag aaaatgcgcc gcccattgaa
361 tgggtaaaat tggccgatgc cttttacgat gcctctatgg acgattctga cggtggaatc
421 gcaattccca ttatttgggg taccgatgcc gtacacggtc acggcaatgt aactggcgca
481 accatattcc cgcataacat aggccttggt gctgcacgca acccagcgct tatcgaaaaa
541 attggcgaaa taacggcaaa agaagtacgc gcaaccggca ttgaatggat atttggccca
601 actttggccg tagcgcaaaa cgatttatgg ggccgcactt acgaaagcta ctcggaagac
661 ccagccatag tggccgacta cgccagtgcc atggtggtag gtatgcaggg caaagtggac
721 gacagcgatt ttctgtccac taatcgcgta gttgccacag caaagcactt tttagctgac
781 ggcggtacct taggaggcaa cgatcaaggt gatgcgcgca taagcgaaga agagttggtg
841 caaattcata atgcgggcta tgtgcctgcc attgaatcgg gcgtgcaaac ggttatggcc
901 agtttctctt tgtggaatgg cgtaaaaatg catggtaaca actacctact tacccaagca
961 cttaaagagc gtatggggtt tgatggtttt atagtagggg attggaatgg ccacgggcag
1021 gtacctgggt gcaccaacga atcttgccct caatcgctaa acgccggttt agatatgtac
1081 atggtgcctt acgattggaa aaaactgtac agaaacttaa ttagccaagt gcaatcgggt
1141 gaaattgccc caagccgttt agatgacgct gtacgccgta ttcttcgggt aaaaattcgc
1201 gctaatttgt gggctgcgaa accttcagag cgaattaatc tagccactat tgacgaggtg
1261 gttggccacg caaaccaccg tgaggtagcg cggcaggcgg tgcgagaaag tttagtattg
1321 ttaaaaaata aaaatagcgt actgcctatt gctgccaata aaaccgtgct ggttgcaggt
1381 gacggcgccg ataatattgg caaacaatct ggcggttgga gtgtaagctg gcagggcact
1441 ggtaacacca atgcatcctt ccccggtggt acatctattt ataaaggtat tgccgatgca
1501 gtcactcagg gcggcggtaa agctacgctt tctgtggatg gcagctacaa aactaaaccc
1561 gatgttgcca ttgtggtaat aggcgaagac ccttacgccg aaggccaagg cgaccgcaat
1621 agtttagagt tcgagccggt gaataaaaaa tcgcttgagc tattaaaaaa attaaaagca
1681 gatggcatac ccgttgtaac agtatttatt tctggccgac ctatgtgggc taacccagaa
1741 attaacgcgt ctgatgcatt tgttgccgcg tggttacctg gctctgaagg gcagggcgta
1801 gcagatgtac ttataggcaa cgccaacggc aagcctcgtt ttgatttcaa gggcaccttg
1861 tcgttctctt ggcctaagct gccgacccaa ggcttgctca acccaacgca ccccaactac
1921 gacccgttat ttaaattggg atacgggcta acttatgcct cgagtgaaac tggcccagag
1981 caattggcgg aagatgttga aggtgtagat aaaggctcaa ccggcgacat taatttttat
2041 gttggccgca cattagagcc gtgggaagtg tttgttcgaa ctcctgaaag ttcgcagcgt
2101 ttaagtggcc catttgcaga cttaggcaat gccagtgtgc gtaccagtga tatgcaggta
2161 caagaagatg cccttacttt tacttggggc ggtagctgga tgtctattct gggaatagaa
2221 ggagggcgcg gttacgacct ttcttcgcaa tataaagaag gcggagtaat aagctttaac
2281 ttcaattcaa tagatatggc taaaggcgat ttaaaagtac aaatggcctg tggtgaaggt
2341 tgcacgcgtg aagtagatat cacaactatc gcacgcgact tggaaggcaa aggctggcag
2401 tcgttaacag tgcccttagc gtgctttgca cacgaaggcg acgatttcac ccatattact
2461 gcgccgttta acttatttgc cggtggaaaa ggtcaagttg ctgtagccaa cattcgcata
2521 ctgcgcgccg gtacacaaac cgtgccgtgt gtattgccta aagatgtttc cgtaacgcca
2581 gagccgctga atgctagctg ggcgatagat tggtggatgc cgcgccacaa agaaaaactg
2641 gcgcgtatcc agcaaggtaa tgtggattta ctaatgattg gcgattccat tacccacggc
2701 tgggaagatg caggtaaaga cgtgtgggcg caatattacg cgcaccgcaa tgcagtggac
2761 ttaggcttta gtggcgaccg aaccgaaaac gtattgtggc gcttacagca cggcgaagca
2821 gacggtatta agcctaaagt ggcagtggtt atgattggta ccaacaatgc cggccatcgt
2881 cacgagcctt cgcactacac agccaagggt gttgcggctg tcgttgctga attgcaaaaa
2941 cgattgcctg aaacaaagat attattactg ggtatattcc ctcgcggcga aaccagtgaa
3001 gaccctttgc gggtattaaa tgccaaaacc aatactcttt tggcgaaaat ggccgacgga
 
3061 gagaaggtgg tgtatttgaa tatcaataaa acgtttttag atgaaaacgg cgtattgcct
3121 aaagatataa tgcccgacct attgcacccc aatgaaaagg ggtacgcatt gtgggcgaaa
3181 gcgatggaac ccacccttaa aaaaatgctg ggcgaatag
LOCUS NZ_AABI02000040 2589 bp DNA linear BCT 23-SEP-2003
DEFINITION Microbulbifer degradans 2-40 Mdeg_40, whole genome shotgun sequence.
ACCESSION NZ AABI02000040 REGION: complement(12943..15531)
VERSION NZ_AABI02000040.1 GI:28878262
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2589)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029901. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2589
      /organism="Microbulbifer degradans 2-40"
      /mol_type="genomic DNA"
      /strain="2-40"
      /db_xref="taxon:203122"
   gene 1..2589
      /gene="Mdeg3627"
   CDS 1..2589
      /gene="Mdeg3627"
      /codon_start=1 1
      /transl_table=11
      /product="COG1472: Beta-glucosidase-related glycosidases"
      /protein_id="ZP 00068210.1"
      /db_xref="GI:23029914"
      /db_xref="COG:COG1472" 
/translation="MLKKINKKGLALSLAIAAMLSGCNEGDSNKTKPSAETLSATQAS
NTVANPSIWPKVTSKVAKDAKMEADISAILSGMTLEQKVAQMIQPEIRAFSKEDMKKY
GFGSYLNGGGAFPNDNKHSTMADWVALADDMYEASIDDSIDGSTIPTMWGTDAVHGHN
NVVKATIFPHNIGLGAMHNPKLMQQIGAATAKVVQVTGIDWVFAPTVAVVRDDRWGRT
YEGYSEDPAIVKEYARAMVIGMQGEANSEAFMGDGTVIATAKHFLGDGGTDKGDDQGN
NLSTEQELIDIHAQGYISAIEEGVQTIMASFNSWNGEKMHGNKSLLTDVLKKQMGFDG
LVVGDWDGHGQVKGCSNASCAQAINAGVDIIMVPNEWKPMFENTVAQVKSGEISEARI
NDAVTRILRVKMRAGIFDGVKPSDRAFAAEEKYLGSAENRAIARQAVRESLVLLKNQN
KLLPLDRKMNVLMAGSGADNIGKQSGGWTLSWQGTGNVNSDFPGATSIYDGVNQVVSS
AGGKVELSENGNYQAKPDVAIVVFGENPYAEGVGDIEGIEYQLNNKRDINLLQKLKAD
GIPVVSVFLTGRPLWVNKELNASDAFVAAWLPGSEGVGVSDVLFKKADGSINYDFKGK
LTYSWPKYDDQVVINKGDKDYAPLYPYGYGLTYSDVDTQGDDLPEETKVKIGRADDEP
MAIFDSLPQSDLGFFLGDKANWVVPIATSVVTTHNSDNLTMRTYNWKVQEDARQLIWK
GDSKANAFFAWPDPHNMQGMLEHKAAYSFSIKVDKAPAGDLTLGIHCMEECGKKLVLN
 
EALSKIPAGEWGELTIDLACIADAEALAEVRSPFMLSTDAPASIVFGDVKLVPGGADS
AAIKCD"
ORIGIN 
1 atgctcaaaa agataaacaa gaaaggtctt gctttaagct tagcaattgc agcaatgcta
61 agcggctgca acgaaggcga cagcaacaaa accaaaccaa gtgcggaaac cctctccgct
121 actcaagcca gtaacactgt agccaacccc agcatttggc ccaaggtaac tagcaaggtt
181 gccaaagacg ccaaaatgga agcagatata agcgcaatac tcagcggtat gacccttgag
241 caaaaagtag cccaaatgat ccaacccgaa attcgtgcct tcagcaaaga agacatgaaa
301 aagtatggtt ttggctccta ccttaacggt ggcggcgcat tccctaacga caacaaacat
361 tccaccatgg ccgactgggt tgccctagcc gacgacatgt atgaagcctc tatagacgac
421 agcatagacg gcagcactat tccaaccatg tggggtaccg atgcagtaca cggccacaac
481 aacgtggtta aagcgactat tttcccacac aacattggcc ttggcgccat gcataacccc
541 aagctcatgc agcaaatagg cgctgccacg gctaaagtgg tacaagttac tggtatcgac
601 tgggtatttg cgcccactgt tgcggtagtg cgcgacgacc gctggggccg tacttacgag
661 ggctactctg aagaccccgc catagtaaaa gaatacgctc gcgccatggt tattggcatg
721 cagggcgaag ccaatagcga agcgtttatg ggtgacggca ctgttatagc caccgccaaa
781 cactttttgg gcgatggcgg caccgacaaa ggcgacgacc aaggcaacaa cttatccacc
841 gaacaagaat taattgatat tcacgcccaa ggctatataa gcgccattga agaaggtgtg
901 caaactatca tggcatcttt caatagctgg aatggcgaaa agatgcacgg caataaatct
961 ctgcttaccg atgtccttaa aaagcaaatg ggctttgacg gtttggtggt tggcgattgg
1021 gatggccacg gccaagtaaa aggttgctct aatgcaagct gtgcccaagc catcaacgcc
1081 ggtgtcgata tcatcatggt acccaatgag tggaaaccca tgttcgaaaa caccgttgca
1141 caagttaaaa gcggcgaaat ctctgaagcg cgaattaacg atgcagttac ccgtatttta
1201 cgtgtaaaaa tgcgcgctgg tattttcgac ggtgttaaac catcggatcg cgccttcgca
1261 gcagaagaaa aatacctagg ctctgccgaa aaccgcgcta tcgctcgtca agctgtacgc
1321 gaatcgttag tgttgcttaa aaaccaaaac aaactgctgc cattagaccg caaaatgaac
1381 gttttaatgg cgggttctgg cgcagacaac atcggcaagc aaagtggtgg ttggacatta
1441 agctggcagg gtactggcaa cgtgaacagc gacttccctg gcgcaacatc tatttacgac
1501 ggcgttaacc aagtagtgag cagcgctggc ggtaaagtag agctaagcga aaacggcaac
1561 taccaagcca aaccagatgt agcgattgta gtatttggtg aaaaccctta cgcagaaggc
1621 gtaggcgata ttgaaggtat tgaataccaa ctaaacaata agcgcgatat caatttgtta
1681 caaaaactca aagccgatgg cattcctgtt gtatcggtat tcttaaccgg tcgtccactt
1741 tgggtaaaca aagagcttaa tgcctccgat gcttttgttg cagcttggct gccaggctct
1801 gaaggtgtag gcgtttctga tgtgctattc aaaaaagccg acggtagtat taactacgac
1861 tttaaaggca agctaactta ctcttggcca aagtatgatg accaagtagt aataaacaaa
1921 ggcgacaaag attacgcccc gctttaccct tatggttacg gcttaaccta cagcgatgtt
1981 gacacccaag gtgacgactt acctgaagaa accaaagtta aaattggccg cgctgacgac
2041 gagccaatgg ccatcttcga cagcctaccc caaagcgacc tcggcttctt ccttggcgac
2101 aaagccaact gggtagtacc tattgcaaca agtgtagtta caacgcacaa cagcgataac
2161 ctaaccatgc gcacctacaa ctggaaagta caagaagatg ctcgccagtt aatttggaaa
2221 ggcgacagca aagccaatgc cttctttgca tggccagacc cacacaatat gcaaggcatg
2281 ttagaacaca aagcggctta cagctttagc attaaagtag ataaagcacc cgctggcgac
2341 ctaacactag gcatacactg catggaagaa tgcggtaaaa aacttgtgct taacgaagcg
2401 cttagcaaaa ttcctgctgg tgagtgggga gagctaacaa tagatctagc ttgcatagca
2461 gatgccgaag ccttggccga agttcgctca cccttcatgc taagcaccga tgcacccgca
2521 tctatcgtgt ttggcgatgt
LOCUS NZ_AABI02000040 2589 bp DNA linear BCT 23-SEP-2003
DEFINITION Microbulbifer degradans 2-40 Mdeg_40, whole genome shotgun sequence.
ACCESSION NZ AABI02000040 REGION: complement(12943..15531)
VERSION NZ_AABI02000040.1 GI:28878262
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2589)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029901. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2589
      /organism="Microbulbifer degradans 2-40"
      /mol_type="genomic DNA"
      /strain="2-40"
      /db_xref="taxon:203122"
   gene 1..2589
      /gene="Mdeg3627"
   CDS 1..2589
      /gene="Mdeg3627"
      /codon_start=1
      /transl_table=11
      /product="COG1472: Beta-glucosidase-related glycosidases"
      /protein_id="ZP 00068210.1"
      /db_xref="GI:23029914"
      /db_xref="COG:COG1472" 
/translation="MLKKINKKGLALSLAIAAMLSGCNEGDSNKTKPSAETLSATQAS
NTVANPSIWPKVTSKVAKDAKMEADISAILSGMTLEQKVAQMIQPEIRAFSKEDMKKY
GFGSYLNGGGAFPNDNKHSTMADWVALADDMYEASIDDSIDGSTIPTMWGTDAVHGHN
NVVKATIFPHNIGLGAMHNPKLMQQIGAATAKVVQVTGIDWVFAPTVAVVRDDRWGRT
YEGYSEDPAIVKEYARAMVIGMQGEANSEAFMGDGTVIATAKHFLGDGGTDKGDDQGN
NLSTEQELIDIHAQGYISAIEEGVQTIMASFNSWNGEKMHGNKSLLTDVLKKQMGFDG
LVVGDWDGHGQVKGCSNASCAQAINAGVDIIMVPNEWKPMFENTVAQVKSGEISEARI
NDAVTRILRVKMRAGIFDGVKPSDRAFAAEEKYLGSAENRAIARQAVRESLVLLKNQN
KLLPLDRKMNVLMAGSGADNIGKQSGGWTLSWQGTGNVNSDFPGATSIYDGVNQVVSS
AGGKVELSENGNYQAKPDVAIVVFGENPYAEGVGDIEGIEYQLNNKRDINLLQKLKAD
GIPVVSVFLTGRPLWVNKELNASDAFVAAWLPGSEGVGVSDVLFKKADGSINYDFKGK
LTYSWPKYDDQVVINKGDKDYAPLYPYGYGLTYSDVDTQGDDLPEETKVKIGRADDEP
MAIFDSLPQSDLGFFLGDKANWVVPIATSVVTTHNSDNLTMRTYNWKVQEDARQLIWK
GDSKANAFFAWPDPHNMQGMLEHKAAYSFSIKVDKAPAGDLTLGIHCMEECGKKLVLN
 
EALSKIPAGEWGELTIDLACIADAEALAEVRSPFMLSTDAPASIVFGDVKLVPGGADS
AAIKCD"
ORIGIN 
1 atgctcaaaa agataaacaa gaaaggtctt gctttaagct tagcaattgc agcaatgcta
61 agcggctgca acgaaggcga cagcaacaaa accaaaccaa gtgcggaaac cctctccgct
121 actcaagcca gtaacactgt agccaacccc agcatttggc ccaaggtaac tagcaaggtt
181 gccaaagacg ccaaaatgga agcagatata agcgcaatac tcagcggtat gacccttgag
241 caaaaagtag cccaaatgat ccaacccgaa attcgtgcct tcagcaaaga agacatgaaa
301 aagtatggtt ttggctccta ccttaacggt ggcggcgcat tccctaacga caacaaacat
361 tccaccatgg ccgactgggt tgccctagcc gacgacatgt atgaagcctc tatagacgac
421 agcatagacg gcagcactat tccaaccatg tggggtaccg atgcagtaca cggccacaac
481 aacgtggtta aagcgactat tttcccacac aacattggcc ttggcgccat gcataacccc
541 aagctcatgc agcaaatagg cgctgccacg gctaaagtgg tacaagttac tggtatcgac
601 tgggtatttg cgcccactgt tgcggtagtg cgcgacgacc gctggggccg tacttacgag
661 ggctactctg aagaccccgc catagtaaaa gaatacgctc gcgccatggt tattggcatg
721 cagggcgaag ccaatagcga agcgtttatg ggtgacggca ctgttatagc caccgccaaa
781 cacttttggg gcgatggcgg caccgacaaa ggcgacgacc aaggcaacaa cttatccacc
841 gaacaagaat taattgatat tcacgcccaa ggctatataa gcgccattga agaaggtgtg
901 caaactatca tggcatcttt caatagctgg aatggcgaaa agatgcacgg caataaatct
961 ctgcttaccg atgtccttaa aaagcaaatg ggctttgacg gtttggtggt tggcgattgg
1021 gatggccacg gccaagtaaa aggttgctct aatgcaagct gtgcccaagc catcaacgcc
1081 ggtgtcgata tcatcatggt acccaatgag tggaaaccca tgttcgaaaa caccgttgca
1141 caagttaaaa gcggcgaaat ctctgaagcg cgaattaacg atgcagttac ccgtatttta
1201 cgtgtaaaaa tgcgcgctgg tattttcgac ggtgttaaac catcggatcg cgccttcgca
1261 gcagaagaaa aatacctagg ctctgccgaa aaccgcgcta tcgctcgtca agctgtacgc
1321 gaatcgttag tgttgcttaa aaaccaaaac aaactgctgc cattagaccg caaaatgaac
1381 gttttaatgg cgggttctgg cgcagacaac atcggcaagc aaagtggtgg ttggacatta
1441 agctggcagg gtactggcaa cgtgaacagc gacttccctg gcgcaacatc tatttacgac
1501 ggcgttaacc aagtagtgag cagcgctggc ggtaaagtag agctaagcga aaacggcaac
1561 taccaagcca aaccagatgt agcgattgta gtatttggtg aaaaccctta cgcagaaggc
1621 gtaggcgata ttgaaggtat tgaataccaa ctaaacaata agcgcgatat caatttgtta
1681 caaaaactca aagccgatgg cattcctgtt gtatcggtat tcttaaccgg tcgtccactt
1741 tgggtaaaca aagagcttaa tgcctccgat gcttttgttg cagcttggct gccaggctct
1801 gaaggtgtag gcgtttctga tgtgctattc aaaaaagccg acggtagtat taactacgac
1861 tttaaaggca agctaactta ctcttggcca aagtatgatg accaagtagt aataaacaaa
1921 ggcgacaaag attacgcccc gctttaccct tatggttacg gcttaaccta cagcgatgtt
1981 gacacccaag gtgacgactt acctgaagaa accaaagtta aaattggccg cgctgacgac
2041 gagccaatgg ccatcttcga cagcctaccc caaagcgacc tcggcttctt ccttggcgac
2101 aaagccaact gggtagtacc tattgcaaca agtgtagtta caacgcacaa cagcgataac
2161 ctaaccatgc gcacctacaa ctggaaagta caagaagatg ctcgccagtt aatttggaaa
2221 ggcgacagca aagccaatgc cttctttgca tggccagacc cacacaatat gcaaggcatg
2281 ttagaacaca aagcggctta cagctttagc attaaagtag ataaagcacc cgctggcgac
2341 ctaacactag gcatacactg catggaagaa tgcggtaaaa aacttgtgct taacgaagcg
2401 cttagcaaaa ttcctgctgg tgagtgggga gagctaacaa tagatctagc ttgcatagca
2461 gatgccgaag ccttggccga agttcgctca cccttcatgc taagcaccga tgcacccgca
2521 tctatcgtgt ttggcgatgt gaagttagta cctggcggtg cagatagcgc agctattaag
2581 tgtgactaa
LOCUS NZ_AABI02000016 1386 bp DNA linear BCT 23-SEP-2003
DEFINITION Microbulbifer degradans 2-40 Mdeg_16, whole genome shotgun sequence.
ACCESSION NZ AABI02000016 REGION: complement(53198..54583)
VERSION NZ_AABI02000016.1 GI:28878286
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1386)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028824. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.igi.doe.gov
   Contact: Paul Richardson
   microbes@cuba.jgi-psf.org.
   COG assignment was automatically updated on Sep 23, 2003.
FEATURES Location/Qualifiers
   source 1..1386
      /organism="Microbulbifer degradans 2-40"
      /mol_type="genomic DNA"
      /strain="2-40"
      /db_xref="taxon:203122"
   gene 1..1386
      /gene="Mdeg2646"
   CDS 1..1386
      /gene="Mdeg2646"
      /codon_start=1
      /transl_table=11
      /product="COG2723:
      Beta-glucosidase/6-phospho-beta-glucosidase/beta-
      galactosidase"
      /protein_id="ZP 00067238.1"
      /db_xref="GI:23028862"
      /db_xref="COG:COG2723" 
/translation="MKTFNPDFVWGAASSAYQVEGATTTDGRGPSIWDAFSSIPGKTY
HNQNADIACDHYNRWQEDVAIMKEMGLKAYRFSISWSRIFPTGRGEVNEKGVAFYNNL
IDELIKNDITPWVTLFHWDFPLALQMEMDGLLNPAIADEFANYAKLCFARFGDRVTHW
ITLNEPWCSAMLGHGMGSKAPGRVSKDEPYIAAHNLLRAHGKMVDIYRREFQPTQKGM
IGIANNCDWREPKTDSELDKKAAERALEFFVSWFADPIYLGDYPASMRERLGERLPTF
SDEDIALIKNSSDFFGLNHYTTMLAEQTHEGDVVEDTIRGNGGISEDQMVTLSKDPSW
EQTDMEWSIVPWGCKKLLIWLSERYNYPDIYITENGCALPDEDDVNIAINDTRRVDFY
RGYIDACHQAIEAGVKLKGYFAWTLMDNYEWEEGYTKRFGLNHVDFTTGKRTPKQSAI
WYSTLIKDGGF"
ORIGIN 
1 atgaaaacct ttaacccaga tttcgtatgg ggagcagcca gttccgccta tcaggtagaa
61 ggcgccacca ccaccgatgg cagaggcccc agtatttggg atgcgttcag ttccattccc
 
121 ggtaaaacct accacaacca aaacgccgac atagcctgcg accactacaa ccgctggcaa
181 gaagacgtgg ccataatgaa agagatgggg ctaaaggctt accgcttttc tatttcttgg
241 tcgcgcatat tccctactgg gcgcggcgaa gttaacgaaa aaggcgtagc cttttacaac
301 aaccttatcg acgaattaat aaaaaacgac attacccctt gggtaaccct atttcactgg
361 gactttcctc tggcactgca aatggaaatg gacggcctac ttaaccccgc catcgccgac
421 gaattcgcca actacgccaa gctgtgtttc gcgcgctttg gcgaccgcgt tacccactgg
481 attaccctaa acgaaccttg gtgcagtgcc atgcttggcc acggcatggg cagcaaagcc
541 cctggccgcg tatctaagga tgaaccctat atagccgccc acaacttgct gcgtgcacac
601 ggcaaaatgg tagatattta ccggcgcgaa tttcagccca cacaaaaagg catgataggc
661 atagccaaca attgcgactg gcgcgaaccc aaaaccgatt ctgaattaga taaaaaagca
721 gccgagcgcg ccctagaatt ttttgtaagc tggtttgccg accccattta tttgggcgac
781 tacccagcca gcatgcgcga gcgcttgggt gagcgtttac ccacctttag cgacgaagac
841 attgcgctaa taaaaaactc tagcgacttt tttggtttga atcactacac caccatgctt
901 gccgaacaaa cccacgaagg tgacgttgtt gaagatacta ttcgcggcaa cggcggcata
961 tcggaagacc aaatggtcac cctctccaaa gacccaagct gggaacaaac cgacatggag
1021 tggagcattg tgccctgggg ctgtaaaaaa ttattaatct ggttaagcga gcgctacaac
1081 taccccgaca tttacattac cgaaaacggc tgcgccctac ccgacgaaga cgacgtaaac
1141 atagccatta acgatacacg ccgcgtagat ttttaccgcg gttatatcga tgcgtgtcac
1201 caagcaatag aggccggcgt aaaactaaaa ggctattttg catggacact tatggataac
1261 tacgaatggg aagaaggcta caccaaacgc tttggcttaa accatgtaga tttcaccaca
1321 ggcaaacgca cacctaaaca gtctgcaatt tggtatagca cgttaattaa agatggtggg
1381 ttctag
LOCUS NZ_AABI02000013 1335 bp DNA linear BCT 23-SEP-2003
DEFINITION Microbulbifer degradans 2-40 Mdeg_12, whole genome shotgun sequence.
ACCESSION NZ AABI02000013 REGION: 65269..66603
VERSION NZ_AABI02000013.1 GI:28878289
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1335)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028387. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1335
      /organism="Microbulbifer degradans 2-40"
      /mol_type="genomic DNA"
      /strain="2-40"
      /db_xref="taxon:203122"
   gene 1..1335
      /gene="Mdeg2234"
   CDS 1..1335
      /gene="Mdeg2234"
      /codon_start=1
      /transl_table=11
      /product="COG2723:
      Beta-glucosidase/6-phospho-beta-glucosidase/beta-
      galactosidase"
      /protein_id="ZP 00066838.1"
      /db_xref="GI:23028439"
      /db_xref="COG:COG2723" 
/translation="MNRLTLPPSSRLRSKEFTFGVATSSYQIEGGIDSRLPCNWDTFC
EQPNTIIDNTNGAIACDHINRWQDDIELIANLGVDAYRFSIAWGRVINLDGSLNNEGV
TFYKNILTKLREKNLKAYITLYHWDLPQHLEDAGGWLNRDTAYKFRDYVNLITQALDD
DVFCYTTLNEPFCSAYLGYEIGVHAPGIKDLASGRKAAHHLLLAHGLAMQVLRKNCPN
SLSGIVLNMSPCYAGSNAQADIDAAKRADDLLFQWYAQPLLTGCYPDAINSLPDNAKP
PICEGDMALISQPLDYLGLNYYTRAVFFADGNGGFTEQVPEGVELTDMGWEVYPQGLT
DLLIDLNQRYTLPPLLITENGAAMVDELVNGEVNDIARINYFQTHLQAVHNAIEQGVD
VRGYFAWSLMDNFEWALGYSKRFGITYVDYQTQKRTLKASGHAFAEFVSSRS"
ORIGIN 
1 atgaatagac ttacactacc gccttcttct cgtttgcgca gcaaagagtt tacctttggt
61 gttgcaacgt cgtcttacca aattgaaggc ggcatagatt ctcgcctgcc ctgtaattgg
121 gatacgttct gtgagcagcc caataccatt attgataaca ccaacggcgc cattgcttgc
 
181 gaccacataa atagatggca agacgatata gaacttattg ccaacctagg ggtagatgcc
241 taccgctttt ctattgcgtg gggccgtgtt attaatttag acggcagcct caataatgaa
301 ggcgttacat tttacaaaaa tattttaact aagcttcgcg aaaagaattt aaaagcttat
361 ataacgctat accactggga cttgccacaa catttagaag atgctggcgg ctggcttaac
421 cgcgataccg cctacaagtt tcgcgactat gtaaacctta taacccaagc gcttgatgac
481 gatgtatttt gctacacaac gttaaacgag cccttttgca gtgcctacct tggctatgaa
541 attggtgtac acgcaccggg tataaaagac ttagccagtg ggcgcaaagc cgcacaccat
601 ttattacttg cccatggctt agctatgcaa gtgctgcgaa aaaactgccc caatagttta
661 agcggcatag tgttaaacat gagcccttgt tacgccggca gcaacgcaca agcagatata
721 gatgcagcaa aacgcgcgga cgatttatta tttcagtggt atgcacaacc gctacttact
781 ggctgctacc ctgatgcaat aaacagcctg ccagacaatg ccaaaccacc tatttgtgaa
841 ggcgacatgg cgttaataag ccaaccttta gattatttag gccttaacta ctatacccgc
901 gcagtatttt ttgccgacgg taatggcggt tttaccgaac aagtacctga gggtgtagag
961 ctaaccgata tgggctggga agtttacccg caaggcttaa ccgatttact aatagaccta
1021 aaccaacgct ataccctacc cccgttactt attaccgaaa acggcgcagc aatggtggac
1081 gaacttgtta acggcgaagt taacgatatt gcccgaataa attattttca aacccattta
1141 caagcggtac acaacgccat tgaacaaggt gttgatgtac gcggttattt tgcttggagc
1201 ctaatggata attttgagtg ggcactgggt tacagcaaac gattcggtat tacctatgta
1261 gattaccaaa cacaaaagcg aacgctaaaa gccagcggcc acgcatttgc tgagtttgtc
1321 tcgagtagga gctaa
LOCUS NZ_AABI02000009 2601 bp DNA linear BCT 23-SEP-2003
DEFINITION Microbulbifer degradans 2-40 Mdeg_10, whole genome shotgun sequence.
ACCESSION NZ AABI02000009 REGION: 594..3194
VERSION NZ_AABI02000009.1 GI:28878293
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2601)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028157. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL - http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2601
      /organism="Microbulbifer degradans 2-40"
      /mol_type="genomic DNA"
      /strain="2-40"
      /db_xref="taxon:203122"
   gene 1..2601
      /gene="Mdeg1959"
   CDS 1..2601
      /gene="Mdeg1959"
      /codon_start=1
      /transl_table=11
      /product="COG1472: Beta-glucosidase-related glycosidases"
      /protein_id="ZP 00066570.1"
      /db_xref="GI:23028159"
      /db_xref="COG:COG1472" 
/translation="MLLSLKNTQLKRSMNMNLKHLFLVALALNIAACNVKEPAATNDN
HISYQAAREARLAKVEAEVERLLPLLTLEEKASLVHANSKFSIASIERLGIHEMWMSD
GPHGVRYQIERHGWAPAGWTDDNSTYLPPLTTVAASWNPEIAALHGDVLGAEARHRRK
DVILGPGVNLARLPLYGRNFEYMGEDPFLASRLAVAEIKAIQENDVAACIKHFALNNQ
ELNRTGVNAKPDERTLREVYLPAFEAAVKEAGVHTIMGAYNEFRGTNANQSKHLVMDI
LKGEWGYKGVLLTDWNVDINTYDAAVNGLDIEMGTNVDSYDDYMLAQPMIDMIKAGSI
PESVLDDKVRRILRVQLSIGMMDKYRLSGERNTAKHHEAARKIASEGIVLLKNENILP
LNKNKIKNVLVLGPNADKVHGLGGGSSEVPALYEITPLQGLKQKLGDNVNITVMRARY
DGVLMPIASDYVTSRHWTGTPAWNMVRYSDAARTQAIGDSAIVDSAYSSPAGTTKEYV
TMTATIKPLKSGEHTLKTSVMGDFELKINGKTTVKHSSTSGDVVTQKIALNGGETYSF
EILYSGNKNFTLGWDAPGDLFTAEKEYIAAAKKADVVFYFGGLTHGDDREAIDRPHMK
LPNHQDPVISKVLAANPNTVVFLIAGSAVEMPWADKAKAIVWGWYGGMEAGNAYADML
FGDTNPSGKMPITLPKALEDTAPIALNDYNPVESLYTEGVFIGYRWFEKQNIEPLFPF
GHGLSYTQFKYNNIKLSSANIKGDQTVTVSATITNTGKVAGAEVVQLYLHDEQASVER
 
PAKELKGFQKVFLKPGESKAVNITLNKRALSFWDENSNDWLAETGKFNVLLGASVSDI
RLQTSFQYQQ"
ORIGIN 
1 atgctgctaa gcttaaaaaa cactcaactc aaaagaagta tgaacatgaa ccttaaacac
61 ctctttctgg ttgctttggc gctaaatatt gctgcgtgca atgtaaaaga gcccgcggcg
121 acaaatgata accacattag ctaccaagcc gctcgcgaag cgcgcttggc aaaagttgaa
181 gccgaagttg aacgcctgct gccactatta acactagaag aaaaagcctc tttggttcat
241 gcgaacagca aattctctat cgcctctatc gagcggctag gcattcacga aatgtggatg
301 tctgatggcc cccacggcgt gcgctatcaa atcgaacgcc acggctgggc accagcaggc
361 tggacagatg acaactccac ttacttacca ccgcttacta ccgtagccgc cagctggaac
421 cccgaaatag ctgcccttca cggcgatgta ctcggcgcag aagctcgcca ccgccgtaaa
481 gatgtaatat taggcccagg cgtaaactta gctcgcctgc cactttatgg tcgtaacttt
541 gaatatatgg gtgaagaccc cttcttggca tcacgtcttg ctgtggcaga aattaaagcc
601 attcaagaaa atgacgtggc cgcctgtatc aaacatttcg cgcttaacaa tcaagagctg
661 aatcgcaccg gcgtaaacgc caaacccgat gaacgcacat tacgcgaagt gtatttaccc
721 gccttcgaag ccgccgttaa agaagcgggc gtgcacacca taatgggggc ctacaatgaa
781 tttcgcggta ccaacgccaa ccaaagcaaa catttagtaa tggatattct aaaaggcgaa
841 tggggctaca aaggcgtgtt actcacagac tggaacgtag atatcaacac ttacgatgcc
901 gctgttaacg gcctcgatat cgaaatgggt acaaatgtag atagctacga cgactacatg
961 cttgcccaac caatgatcga catgattaaa gcgggcagca ttccagagtc agtacttgat
1021 gataaagttc gtcgcatact gcgcgtgcaa ctcagcatag gcatgatgga caaataccgc
1081 ttatctggtg agcgcaatac tgccaagcat cacgaagctg cacgcaaaat tgcatctgaa
1141 ggtattgtgc tactaaaaaa tgaaaacatt ctgccgctaa ataaaaacaa aattaaaaac
1201 gtattggtgc ttggccccaa cgcagacaaa gtgcacggtt taggcggtgg ctcgtcagaa
1261 gtgccagcac tttatgaaat aaccccgtta caagggttaa aacagaagct gggagataat
1321 gtaaacatta ccgttatgcg cgcacgctat gacggtgtgt taatgcctat cgccagtgat
1381 tatgttactt ctcgtcactg gaccggcaca cctgcatgga acatggtgcg ttactcggat
1441 gctgcgcgca cccaagctat tggcgactcc gccattgttg attcggctta ttcttcgcct
1501 gcaggcacga ctaaagaata cgtcaccatg accgccacaa ttaaaccgtt aaaatcgggc
1561 gagcacacac tcaaaacatc ggtgatgggc gatttcgaat taaaaattaa cggtaaaacc
1621 acagtaaaac atagcagcac tagcggcgat gtagtaaccc aaaaaatcgc cctcaacggc
1681 ggtgaaacat acagcttcga aattttatac agcggcaata aaaactttac cttgggctgg
1741 gatgcaccgg gagatttatt taccgcagaa aaagaataca tagccgccgc gaaaaaagcg
1801 gatgtagtgt tttactttgg cggcctaacc cacggcgacg accgcgaagc aattgaccgc
1861 cctcacatga agctgcctaa ccatcaagac ccagttatta gcaaagtatt agctgcaaac
1921 ccgaacacgg ttgtattttt aattgcaggc tctgctgtag aaatgccgtg ggccgataaa
1981 gctaaagcta ttgtgtgggg ctggtatggc ggtatggagg ccggtaacgc ctacgccgat
2041 atgctatttg gcgataccaa ccccagcggc aaaatgccaa taactttacc aaaggcactg
2101 gaagatactg ctccaatcgc actgaatgat tacaaccctg ttgaatcact ctacaccgag
2161 ggcgtgttta ttggttaccg ctggttcgaa aaacaaaaca tcgagccgct attcccgttc
2221 ggtcatggtt tgtcttatac ccagtttaag tacaacaata taaagctctc tagcgcgaac
2281 attaaaggcg accaaaccgt caccgtaagc gcaaccatta ccaatactgg caaagtggcc
2341 ggcgctgaag ttgtacaact gtatttgcat gacgagcaag caagcgtaga acgcccagca
2401 aaagaactta aaggtttcca aaaagtgttt ttaaagccgg gtgaaagcaa agcggtaaat
2461 attacgctta ataaacgcgc cctttcattt tgggatgaaa acagcaacga ctggcttgca
2521 gaaacaggta aatttaatgt gctattgggc gcatcagtaa gcgatatacg cttacaaact
2581 agcttccaat accagcagta a
LOCUS NZ_AABI02000028 2436 bp DNA linear BCT 23-SEP-2003
DEFINITION Microbulbifer degradans 2-40 Mdeg_28, whole genome shotgun sequence.
ACCESSION NZ AABI02000028 REGION: 14441..16876
VERSION NZ_AABI02000028.1 GI:28878274
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2436)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029569. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2436
      /organism="Microbulbifer degradans 2-40"
      /mol_type="genomic DNA"
      /strain="2-40"
      /db_xref="taxon:203122"
   gene 1..2436
      /gene="Mdeg3326"
   CDS 1..2436
      /gene="Mdeg3326"
      /codon_start=1
      /transl-table=11
      /product="COG3459: Cellobiose phosphorylase"
      /protein_id="ZP 00067911.1"
      /db_xref"GI:23029584"
      /db_xref="COG:COG3459" 
/translation="MKFGHFDDKAREYVITDPKTPYPWINYLGNEDFFSLVSNTGGGY
SFYKDAKFRRLTRYRYNNVPVDNGGKYFYINDSGDVWSPGWKPVKAELDAYSCAHGLS
YTRITGERNGIQAEVLSFIPLGTWAEIQKVSLKNTSGATKKFKLFSFAEWCLWNAEDD
MTNFQRNFSTGEVEVEDSVIYHKTEFKERRNHYAFYSVNAPIQGFDTDRDKWKGLYND
FDKPDAVFEGEPRNSEAHGWSPIASHYLEVELAPGESKDLIFVLGYIEVAPENKWESK
GVINKSPAKELIARFDSVEKVDAELTKLADYWANLLSTYSVESGDEKLDRMVNIWNQY
QCMVTFNMSRSASFFESGIGRGMGFRDSNQDLIGFVHQVPERARERIIDIASTQFEDG
SAYHQYQPLTKRGNNAIGGNFNDDPLWLILSTTDYIKETGDFSILEEQVPYDNDASKA
TSHFEHLKRSFYHTVNNLGPHGLPLIGRADWNDCLNLNCFSEDPNESFQTTGNKTGRT
AESLMIAGLFVLYGNEFVKLCREIGQDGEAAEAQAHIDQMVEAVKKHGWDGEWFLRAY
DYYGKKVGSKENEEGKIFIESQGFCGMAGIGLEDGLVEKSMDSVKEWLDCDYGIVLQQ
PAFTKYYIEYGEISTYPAGYKENAGIFCHNNPWIMITETLLGRGDKAFEYYRKIAPAY
LEEISDLHKVEPYAYCQMIAGKDAYLPGEGKNSWLTGTASWNFAAITQYILGVKPDYS
GLAINPCIPSSWDGFKVTRKYRGATYNIIVTNPTHVSKGVKSLTLNGNAIDGYIVPPQ
 QAGTVCNVEVTLG"
ORIGIN 
1 atgaaatttg ggcactttga cgacaaagca cgcgagtatg taattaccga cccgaaaact
61 ccctacccgt ggataaacta cttaggcaac gaagacttct tcagcctagt atctaacact
121 gggggtggct acagttttta caaagatgca aagttccgtc gtttaacacg ctatagatac
181 aacaacgtac ccgtagacaa cggcggtaaa tatttttaca tcaatgatag tggcgatgta
241 tggagccccg gttggaagcc ggtaaaagca gagctagacg catacagctg cgctcacggc
301 cttagctaca cccgcattac cggcgaaaga aacggcattc aagcggaagt acttagcttt
361 atccctctcg gcacttgggc cgaaattcaa aaagttagcc ttaagaatac ctctggcgct
421 accaaaaaat ttaaactgtt ttctttcgcc gaatggtgcc tatggaacgc agaagatgac
481 atgaccaact tccaacgcaa cttctccacc ggtgaagtag aggtggaaga ctctgttatt
541 tatcacaaga cagaatttaa agagcgccgc aatcattacg cattctactc tgtaaacgca
601 ccaattcagg gcttcgacac cgacagagac aaatggaaag gcttgtacaa cgattttgat
661 aaacccgatg ccgtttttga aggcgagcct cgcaactccg aagcgcacgg ctggtcgcca
721 attgcatctc actatctaga agtggagctc gcaccaggcg aaagcaaaga cttaattttt
781 gtgcttggct atatagaagt tgccccagaa aacaaatggg aatcaaaggg cgttatcaac
841 aagtctccag ccaaagaact tattgcgcgt ttcgatagcg tagaaaaagt agatgccgag
901 ttaaccaagc tagccgatta ttgggcaaat ttgctttcta cttacagcgt agaaagtggc
961 gacgaaaagc tagaccgcat ggtaaatatt tggaaccaat accagtgtat ggtgacattt
1021 aatatgagtc gctctgcgtc tttcttcgaa tctggcattg gccgtggtat gggcttccgc
1081 gattccaatc aggatttgat aggctttgta caccaagtac ccgagcgcgc ccgcgaacgc
1141 ataattgata ttgcttctac tcagtttgaa gacggttcgg cctaccacca gtatcagcct
1201 ttaaccaaac gcggcaacaa cgcaattggc ggcaacttta acgatgaccc tctttggcta
1261 atcctttcta ccaccgatta cataaaagag actggcgatt tctctatttt agaagagcaa
1321 gtgccttacg ataatgatgc gagcaaagcc acaagtcatt ttgaacattt aaagcgctcg
1381 ttttatcaca cggttaataa tttaggccca catggcttgc cacttattgg tcgcgccgac
1441 tggaacgact gcctaaacct aaactgcttt agtgaagacc ctaacgaatc attccaaacc
1501 acgggcaaca aaaccggcag aacggctgag tcgttaatga ttgcaggttt atttgtttta
1561 tacggcaacg agtttgtaaa actgtgccgt gaaataggcc aagacggaga agcggcagaa
1621 gcccaagccc atattgacca aatggtagaa gctgtgaaaa agcacggctg ggatggcgag
1681 tggtttttgc gtgcttacga ctactacggt aaaaaagtag gcagtaaaga aaacgaagaa
1741 ggcaaaatat ttatcgaatc gcaaggtttc tgcggcatgg caggaatcgg cctagaagac
1801 ggccttgtcg aaaaatcgat ggattctgtt aaagaatggt tagattgcga ttacggtatt
1861 gtgttgcagc aaccggcgtt taccaagtac tacatagagt atggtgaaat ctccacctac
1921 cctgctggct acaaagagaa cgcaggtatc ttctgccaca acaacccgtg gattatgatc
1981 accgaaactt tgcttggccg cggtgacaaa gcctttgaat actaccgcaa aattgcacct
2041 gcatacctag aggaaattag cgatcttcac aaagtagagc cttacgccta ctgccagatg
2101 attgcaggta aagatgccta cttacctggc gagggtaaaa actcatggct aacaggaacc
2161 gcttcgtgga acttcgctgc aattactcag tacattttag gcgtaaaacc agactatagc
2221 ggtttagcaa ttaacccttg cataccgtct agctgggatg gctttaaagt tacccgtaag
2281 tatcgcggcg caacctataa catcatcgta accaacccaa cccatgtaag caaaggcgta
2341 aaatcgctca ccctaaatgg caacgctatt gatggctaca tagtgccacc gcaacaagct
2401 ggcaccgtat gtaacgtaga agttacattg ggctaa
LOCUS NZ_AABI02000049 2367 bp DNA linear BCT 23-SEP-2003
DEFINITION Microbulbifer degradans 2-40 Mdeg_48, whole genome shotgun sequence.
ACCESSION NZ AABI02000049 REGION: complement(7846..10212)
VERSION NZ_AABI02000049.1 GI:28878253
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2367)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029998. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2367
      /organism="Microbulbifer degradans 2-40"
      /mol_type="genomic DNA"
      /strain="2-40"
      /db xref="taxon:203122"
   gene 1..2367
      /gene="Mdeg3704"
   CDS 1..2367
      /gene="Mdeg3704"
      /codon_start=1
      /transl_table=11
      /product="COG3459: Cellobiose phosphorylase"
      /protein_id="ZP 00068287.1"
      /db_ xref="GI:23030008"
      /db_xref="COG:COG3459" 
/translation="MLKAINNGERYQLTSPTAMPQSASFLWNKKMMIQVNCRGYAVAQ
FMQPEPAKYAYAPNLEAKTFMQPEQPYYAHHPGRFFYIKDEETGEIFSAPYEPVRSQL
NNFSFNAGKSDISWHIAALGIEVELCLSLPVDDVVELWELKIKNGGAQPRKLSIYPYF
PVGYMSWMNQSGDYSQTAGGIIASCVTPYQKVADYFKNKDFKDKTFFLHETAPAAWEV
NQKNFEGEGGLHNPNAIQQETLGCGNALYETPTAVLQYRRELAAQEQQTFRFIFGPAF
DESEAIALRNKYLSAEGFAKAKSEYQTYITSGKGCLQINTPDPELNNFVNHWLPRQVF
YHGDVNRLTTDPQTRNYIQDNMGMSYIKPNITRQAFLHALSQQEESGAMPDGILLLEG
AELKYINQIPHTDHCVWLPVCMQAYLDETNDYALLDEIVPYASGEKRETVEQHMHHAM
RWLLQARDERGLSFIAQGDWCDPMNMVGYKGKGVSGWLSVATAYALNLWADVCEQRQQ
NSCANEFRQGAKDINAAVNKHIWDGEWFGRGITDDGVLFGTSKDKEGRIFLNPQSWAI
LGGAADEQKIPCLLDAVEQQLETPYGVMMLAPAFTAMRDDVGRVTQKFPGSAENGSVY
NHAAVFYIFSLLSIGESERAYKLLRQMLPGPDEADLLQRGQLPVFIPNYYRGAYYQHP
RTAGRSSQLFNTGTVSWVYRCLIEGVFGLKGSPQGLVVQPQLPVAWQTAEAVREFRGA
TFNVSYRKSSDIKEMEIQLNESVISGNTISDITAGATYQLTVLLPATH"
ORIGIN 
1 atgttaaaag ccattaacaa cggcgaacgc tatcaactca ctagccctac cgctatgccg
61 caaagcgcat cgtttttatg gaataaaaaa atgatgatac aagtaaattg ccgcggctac
121 gccgttgcgc aatttatgca gccagaacca gccaaatacg cttacgcacc caatctggaa
181 gcaaaaacat ttatgcaacc agagcaaccc tattacgcgc atcaccccgg gcgctttttc
241 tatataaaag atgaagagac aggcgagatt ttttcggcac cctacgagcc tgtgcgcagc
301 cagctgaaca actttagctt taacgcaggc aagagcgata taagctggca tattgccgct
361 ttaggcattg aagtagagct atgtcttagc ctgccggtgg acgatgtagt agaattgtgg
421 gaactaaaaa taaaaaacgg cggcgcgcaa cctcgtaaac tcagtattta cccgtacttt
481 cctgtgggtt acatgtcgtg gatgaatcaa tctggtgact acagccaaac cgccggcggc
541 attattgcca gctgcgtaac gccttatcaa aaagtcgccg actactttaa gaataaagac
601 tttaaagata aaacgttctt tcttcacgaa accgccccag cagcatggga agtaaaccag
661 aaaaacttcg aaggcgaagg cgggttgcac aaccccaacg ccatacaaca agaaacgctg
721 ggctgcggca acgcattgta cgaaacgccc acagcggtat tgcaataccg ccgcgaactt
781 gcagcgcaag agcagcaaac ctttcgcttt atttttggcc cagcatttga cgagagcgaa
841 gccattgcac tgcgcaataa gtatttatct gccgaaggtt ttgccaaagc aaaaagcgaa
901 taccaaacct atataacgag cggcaaaggc tgcttgcaaa ttaacacccc agacccagaa
961 ctaaacaact ttgtaaacca ctggctaccg cgccaagtgt tttatcacgg cgatgtaaac
1021 cggttaacca ccgacccgca aacgcgcaat tatattcaag acaatatggg catgagctac
1081 attaagccca acattacgcg gcaggcgttt ttacatgcct taagccagca ggaagaaagc
1141 ggtgcaatgc ccgacggcat ttttattgct gaaggcgccg agcttaaata cataaaccaa
1201 ataccccata ccgatcactg cgtttggctg ccggtgtgta tgcaagccta tttggatgaa
1261 accaatgact acgccctatt agacgaaata gtaccctatg cgagtggcga gaagcgcgaa
1321 actgttgagc aacatatgca tcacgctatg cgctggcttt tgcaagcacg cgacgaacgc
1381 ggcctaagct ttatcgcaca gggcgactgg tgcgacccca tgaacatggt gggctacaag
1441 ggcaaagggg tatccggctg gctttcagtc gctaccgctt atgcattaaa cctgtgggca
1501 gatgtttgcg aacaacggca gcaaaacagt tgcgccaacg aatttagaca gggcgctaaa
1561 gatataaacg cggcggtaaa caagcatatt tgggatggcg aatggtttgg ccgcggcatt
1621 acagatgacg gcgtactgtt tggcaccagc aaagataaag aaggcagaat tttctaaac
1681 ccacaaagct gggcaatact tggcggcgcc gccgacgaac aaaaaatccc atgcctgcta
1741 gacgcagtag agcaacaact ggaaacccct tacggcgtaa tgatgctggc ccccgcgttt
1801 accgccatgc gcgatgacgt aggccgagtt acccaaaaat tcccaggctc tgcagaaaac
1861 ggctctgttt ataatcacgc ggcggtgttt tatatattta gcttgttatc cattggcgag
1921 agcgaacgcg catataaact gctacgccaa atgctgcctg ggccagatga agccgatctt
1981 ttacagcgcg gccaactgcc agtattcata cctaactatt atcgcggcgc atactaccag
2041 cacccccgca ccgccggtcg ctctagccag ctctttaata cgggtacagt ctcgtgggtt
2101 taccgctgct taattgaagg ggtattcggc ttgaaaggct cgccacaagg cttagttgta
2161 caaccgcaac tgcctgtcgc ctggcaaaca gcagaagccg ttagggaatt tagaggcgca
2221 acgtttaacg tgagctaccg caaaagcagc gatataaaag aaatggaaat acagctaaat
2281 gaatcggtaa taagtggcaa caccatctcc gacatcaccg ccggcgcgac ctatcaatta
2341 accgttctat tacctgccac acactaa
LOCUS NZ_AABI02000007 1725 bp DNA linear BCT 23-SEP-2003
DEFINITION Microbulbifer degradans 2-40 Mdeg_8, whole genome shotgun sequence.
ACCESSION NZ AABI02000007 REGION: 110169..111893
VERSION NZ8AABI02000007.1 GI:28878295
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1725)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027895. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   htto://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1725
      /organism="Microbulbifer degradans 2-40"
      /mol_type="genomic DNA"
      /strain="2-40"
      /db_xref="taxon:203122"
   gene 1..1725
      /gene="Mdeg 1780"
   CDS 1..1725
      /gene="Mdeg 1780"
      /codon_start=1
      /transl_table=11
      /product="COG3693: Beta-1,4-xylanase"
      /protein_id="ZP 00066395.1"
      /db_xref="GI:23027970"
      /db_xref="COG:COG3693" 
/translation="MKKLIKPTLSWVAGVALSLGIAQGAGAQNVQFVGNITTNGSVRN
DFMDYWDQITPENEGKWGSVERSRDNYSWSGQDAAYNFARANGIPFKAHTLVWGSQYP
SWINNLSNAEKAAEIEEWIRDYCNRYPATDIIDVVNEATPGHAPANYARDAFGDNWII
KSFQLARQYCPNATLVLNDYNVLIWNTNDFIAMAQPVINAGVVDALGLQAHGLESLSA
SQLKSTLDRIANLGLPIYISEYDVRSTNDQEQLRIMRDQFPVFYNHPSVRGITLWGYM
VGATWREGTGLIRADGSHRPAMTWLMNYLENNRGGSTSSSSSSSSSSSSSSSSSSSGSS
SGGPSSLTVELESLSDSSNFSPFSVQSDSSAAGGQYVVWPNNGNQIVSSPSDSASGQI
QVHFTLSQSADVQFQIRADLANGNDDSFYYKLDSGSWNTQNNASTSGWGTLTPATFSN
VSTGSHTLHILRREDGAKLDKVTLNASVGQVSASTGSSSSSSSSSSSSSSSSSSSSSG
 
AAVASCDGVNEYPSWTAKDWSGGDYNHANSGDYMSYQGVLYRANWYTATVPGSDSSWT
RVGDCNFV"
ORIGIN 
1 atgaagaagt taattaagcc tacgctatcg tgggttgcag gagttgcttt gtcgctgggt
61 attgcccagg gagctggtgc tcaaaatgtg cagtttgttg gtaatatcac taccaatggt
 
121 agtgtgcgca acgacttcat ggactactgg gatcagatta ccccagagaa tgaaggcaag
181 tggggctcgg tggagcgcag tcgcgacaac tattcatgga gcggccaaga tgccgcctac
241 aattttgccc gtgccaacgg catcccattt aaagcacata ctttagtatg ggcagtcaa
301 tatcccagct ggataaacaa tttaagtaac gcggaaaaag ccgctgagat tgaagagtgg
361 attcgcgatt actgtaaccg ttacccagcc accgatatta tcgatgttgt caatgaagca
421 acgccgggcc acgcgccagc aaattatgct cgcgatgcat ttggcgacaa ctggataatc
481 aagtccttcc agctggcacg tcagtactgc cccaatgcca cgttagtgtt gaacgactac
541 aacgtactta tttggaacac caatgatttt atagcgatgg cccagccggt aattaacgcc
601 ggagtagtag atgctttggg tttgcaggcc cacggtctgg agagcctttc tgcgtcgcaa
661 ttaaaatcga ctctggatcg tatcgccaat ttgggtttgc caatttatat ctctgaatac
721 gatgttcgca gcaccaatga tcaggagcag ctgcgtatta tgcgtgatca attccctgta
781 ttttacaacc acccaagtgt acgtggcata actttgtggg gttatatggt gggggccacc
841 tggcgagaag gcacaggttt gattcgtgct gatggctccc atcgtccagc gatgacctgg
901 ttgatgaact atctggagaa caatcgtggc ggctcaacct cttcaagtag ttcatcctcc
961 tctagcagtt cgtcttccag tagttcttct tcgggaagtt cctctggtgg cccaagtagt
1021 ttgacggtag agctagaatc tttgtcggat agcagtaact tttcgccatt ctcggtacag
1081 agtgacagca gcgcagcggg cggccagtac gtggtatggc ctaacaacgg caatcagatt
1141 gtaagctcac cctccgatag cgccagcggg caaattcagg tgcactttac cctgtcgcaa
1201 tcggcggatg tgcaatttca gattcgtgca gacctagcta acggcaatga cgactctttt
1261 tattacaagc tggactcagg ctcttggaat actcagaaca acgcttccac gtctggttgg
1321 ggcaccttaa ccccagcaac tttctctaat gtatccacag gatcccatac cttacacatt
1381 ctccgcagag aagatggggc gaaactcgat aaggtaactc tgaatgcttc agttggtcag
1441 gtttccgcta gtacaggcag tagctccagc tcttccagca gctccagttc atccagcagt
1501 tctagttctt caagcagcag cggcgcggca gtcgcaagtt gtgacggtgt taatgaatac
1561 cccagctgga cagcaaaaga ttggtctggg ggtgactata accacgccaa tagcggtgac
1621 tacatgagct atcagggtgt tctatatcga gcaaactggt acaccgcaac tgttcctgga
1681 agtgattctt cctggactcg agttggcgat tgcaattttg tgtaa
LOCUS NZ_AABI02000006 1860 bp DNA linear BCT 23-SEP-2003
DEFINITION Microbulbifer degradans 2-40 Mdeg_6, whole genome shotgun sequence.
ACCESSION NZ AABI02000006 REGION: 76803..78662
VERSION NZ_AABI02000006.1 GI:28878296
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1860)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027577. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1860
      /organism="Microbulbifer degradans 2-40"
      /mol_type="genomic DNA"
      /strain="2-40"
      /db_xref="taxon:203122"
   gene 1..1860
      /gene="Mdeg1453"
   CDS 1..1860
      /gene="Mdeg1453"
      /codon_start=1
      /transl_table=11
      /product="COG3693: Beta-1,4-xylanase"
      /protein_id=ZP 00066073.1"
      /db_xref="GI:23027635"
      /db_xref="COG:COG3693" 
/translation="MIKLRQSIHGALARTVGIISISTGLVLAAQTASAACEYTVTNSW
GSGFTASIRITNDTGSAVNGWAVNWQYANGNRVTNSWNATLSGNNPYSASNIGWNGGI
QPGQSVEFGFQGTANGAAETPAVTGAVCATGSSSSSSSSSSSSSSSSSSSSSSSSSSSSSS
SSTSSSSSSSSSSSSSGANCVEMCKWYQDAPRPLCNNQNSGWGWENNQSCIGRATCESQ
PSNAGGVVNSCPSSSSSSSSSSSSSSSSSSSSSSSSSSSSSTSSSSSSSTSSTSSSSSSSSS
GSAANLYTLADFPIGVAVTAGNESRSFLSIAAKEATVKKHFDQITAGNIMKMSYLHPS
ENSYTFSQADAMVNWANSNGVSVHGHTFIWHSDYQVPNWMNNYSGNFASMMDTHVTTI
ADHFEGRVVSWDVVNEAIDESQSSCYRNSLFYQRLGKAYIANAFRAARAADPSVELYY
NDYDTEGGNANKLNCLLQLVDDLQANNVPIDGVGFQMHVQIDWPSTSNIAAAFQAIVD
RGLKVKITELDVPINNPYGSGSFPQYSTYTSQAAALQKARYKSIVKTYLTVVPAHLRG
GLTVWGIWDGDSWLLDFDNRQGADDWPLLFSGPANGPYVEKEAFYGVAEALTE"
   gene complement(336..488)
      /gene="Mdeg1454"
   CDS complement(336..488)
      /gene="Mdeg1454"
      /codon_start=1
      /transl_table=11
      /product="hypothetical protein"
      /protein_id="ZP 00066074.1"
      /db_xref="GI:23027636" 
/translation="MLDELLDELELLLELELLDEELELELEELDPVAHTAPVTAGVSA
APLAVP"
   gene complement(558..869)
      /gene="Mdeg1455"
   CDS complement(558..869)
      /gene="Mdeg1455"
      /codon_start=1
      /transl_table=11
      /product="hypothetical protein"
      /protein_id="ZP 00066075.1"
      /db_xref="GI:23027637" 
/translation="MGKSAKVYKFAAEPLELELLEEVELVLDELELVDELELLDELEL
LDELELEELEELLELDGQLFTTPPALDGCDSHVARPIQLWLFSQPQPLFWLLHKGRGA
S"
ORIGIN 
1 atgatcaagc tacgtcaatc tatccacggc gccttggcgc gtaccgtggg cataataagt
61 ataagcaccg gacttgtact cgcagcgcaa actgcaagtg cagcctgtga atacaccgta
121 accaattcgt ggggttcggg ttttaccgcg agtattcgca taacaaacga taccggtagc
181 gcagtaaacg gttgggcggt taactggcaa tacgctaatg gcaaccgtgt aacaaattca
241 tggaacgcta cgctgtctgg caataaccct tatagcgcca gcaatattgg ttggaacggc
301 ggtattcaac ctgggcagtc ggtggaattt ggttttcaag gcacggctaa tggcgcggca
361 gaaacaccag cggtaacggg ggctgtatgt gctacagggt ctagctcttc cagctcaagc
421 tctagttctt cgtctagcag ctctagttct agcagcagtt cgagttcatc gagtagctcg
481 tctagcactt caagctcgtc atctagcagc tcttccagtt catcgggcgc aaactgtgta
541 gaaatgtgta agtggtatca agatgcgcct cgccctttat gcaataacca aaatagtggt
601 tggggttggg aaaacaacca aagttgtatc ggccgagcaa cgtgcgaatc gcaaccgtct
661 aatgcgggtg gggtggtaaa tagttgtccg tctagttcta gcagctcttc aagttcctct
721 agttcgagct cgtctagcag ttcaagctcg tctagcagtt caagctcgtc tactagttct
781 agttcatcaa gcacaagttc tacttcttca agtagttcta gctctagcgg ttctgctgca
841 aacttatata ccttggcaga tttccccatt ggcgttgctg taactgcggg taatgagagc
901 cgtagctttt tatctattgc tgcgaaagag gcaactgtta aaaaacactt cgaccaaatt
961 acagccggta acattatgaa gatgagttac ttgcacccat ccgaaaatag ctacaccttt
1021 agtcaagcgg atgccatggt taactgggca aatagcaacg gcgtaagtgt gcacggccat
1081 acttttattt ggcattccga ttaccaagta ccaaattgga tgaataatta cagcggtaat
1141 tttgcgtcta tgatggatac ccacgtaacc actattgccg atcattttga aggccgagta
1201 gtaagctggg atgtggtaaa cgaagctatc gatgagagcc aatctagttg ttatcgcaac
1261 tctttgtttt accagcgttt aggtaaagct tatattgcca atgcgttccg cgcggcccga
1321 gcagcagacc ctagcgtaga gttgtattac aacgattacg ataccgaagg tggcaatgcc
1381 aataagttaa attgcttgtt gcaattagtc gatgacttgc aagcgaacaa tgtgcctatc
1441 gatggtgtgg gctttcaaat gcacgtgcaa attgattggc ccagcaccag caatattgct
1501 gcggctttcc aagctattgt ggatcgcggc ttaaaggtaa aaattactga gctggatgtg
1561 cctattaata acccttatgg cagtggttca ttcccgcaat attcaactta cacgtcacaa
1621 gccgctgcgt tgcaaaaggc gcgttataaa tccattgtaa aaacctactt gactgttgtg
1681 ccagcgcatt tgcgcggggg cttaaccgta tggggtatat gggatggtga tagctggttg
1741 ttagattttg ataatcgtca aggcgctgat gattggccgc tattatttag tggcccagct
1801 aatggcccct atgtagaaaa agaagcattc tatggcgtgg cagaggcgct tacagaatag
LOCUS NZ_AABI02000001 1413 bp DNA linear BCT 23-SEP-2003
DEFINITION Microbulbifer degradans 2-40 Mdeg_1, whole genome shotgun sequence.
ACCESSION NZ AABI02000001 REGION: complement(468091..469503)
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1413)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   ttp://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1413
      /organism="Microbulbifer degradans 2-40"
      /mol_type="genomic DNA"
      /strain="2-40"
      /db_xref="taxon:203122"
   gene 1..1413
      gene="Mdeg0345"
   CDS 1..1413
      /gene="Mdeg0345"
      /codon_start=1
      transl_table=11
      product="COG3693: Beta-1,4-xylanase"
      /protein_id="ZP 00064983.1"
      db_xref="GI:23026497"
      db_xref="COG:COG3693" 
/translation="MNCTRRNIVKAGLLGSAFVALPAVARALPGLATKFRDQFYVGTA
VSARSLNTPSGAFAATVAHQFNALTAENAMKPALLQPQMGEWRWQDADAIVRFAEQHQ
MLMHGHTLVWHSQTPDWFFQNKQGEPADKATLYRRQEEYINAVVGRYKGRVHSWDVVN
EAEDEGKGWRKSHWYNICGPEFMERAFRLAHAADPKAHLCYNDYNMHLPQKREFLVKL
FKDYIKRGVPIHGVGLQGHVGLDYPSLDELEKTIVAMADLGKVHITELDVDVLPAPW
QLASADISTKFEYDKSLNPYVDGLXXXXXXXXXXXXXXXXXXXXXXXXXXXXXXXXX
XXXXXXXXXXXXXXXXXXXXXXXXXXXXXXXXXXXXXXXXXXXXXXXXXXXXXXXX
XXXXXXXXXXXXXXXXXXXXXXXXXXXXXXXXXXXXXXXXXXXXXXXXXXXXXXXX
XXXXXXXXXXXXXXXXXXXXXXXXXXXXXXXXXXXXXXXXXXXXXXXXXXXXXXXX
XXXXXXXTMYKMRLPAKILA"
ORIGIN

1 gtgaattgta cgcgtaggaa tatagtaaaa gcaggccttc ttggctcggc attcgtcgcc
61 ctgcctgccg tggcgcgcgc gctgcctgga ttggccacga aatttcgcga tcagttttac
121 gtgggcactg cggttagtgc gcgctcactt aatacgccca gcggcgcgtt tgcagccact
181 gtcgcgcatc aattcaatgc actaaccgct gaaaacgcca tgaagcccgc cttacttcaa
241 ccacaaatgg gggagtggcg ctggcaggat gccgatgcca ttgtgagatt tgccgagcag

301 catcagatgc taatgcatgg tcacaccctt gtgtggcatt cgcaaacgcc agattggttc
361 ttccaaaaca agcagggcga accggcagac aaagcaaccc tataccgcag gcaagaggag
421 tatatcaatg ccgtagttgg gcgctataaa gggcgggtac actcgtggga tgtggtgaat
481 gaagcagaag atgagggtaa aggctggcgc aagagccact ggtataacat ttgtgggcca
541 gagtttatgg aacgagcctt tcgcttagct cacgcagcgg acccaaaagc acacttatgt
601 tacaacgatt acaatatgca cttgccgcaa aagcgcgaat ttttggttaa gttattcaaa
661 gactacatta agcgcggcgt gcctattcac ggcgtagggt tgcaggggca tgtgggctta
721 gactacccct cgctggacga gttggaaaaa accatcgtgg ccatggccga tttaggtcta
781 aaagtacaca ttacagaatt ggatgtagat gtattacccg cgccatggca actagctagc
841 gcagatataa gtactaaatt cgagtacgac aaaagcttaa acccgtacgt tgatggtttg
901 cnnnnnnnnn nnnnnnnnnn nnnnnnnnnn nnnnnnnnnn nnnnnnnnnn nnnnnnnnnn
961 nnnnnnnnnn nnnnnnnnnn nnnnnnnnnn nnnnnnnnnn nnnnnnnnnn nnnnnnnnnn
1021 nnnnnnnnnn nnnnnnnnnn nnnnnnnnnn nnnnnnnnnn nnnnnnnnnn nnnnnnnnnn
1081 nnnnnnnnnn nnnnnnnnnn nnnnnnnnnn nnnnnnnnnn nnnnnnnnnn nnnnnnnnnn
1141 nnnnnnnnnn nnnnnnnnnn nnnnnnnnnn nnnnnnnnnn nnnnnnnnnn nnnnnnnnnn
1201 nnnnnnnnnn nnnnnnnnnn nnnnnnnnnn nnnnnnnnnn nnnnnnnnnn nnnnnnnnnn
1261 nnnnnnnnnn nnnnnnnnnn nnnnnnnnnn nnnnnnnnnn nnnnnnnnnn nnnnnnnnnn
1321 nnnnnnnnnn nnnnnnnnnn nnnnnnnnnn nnnnnnnnnn nnnnnnnnnt gactatgtac
1381 aaaatgcgct tgcccgcgaa aatattagct taa

LOCUS NZ_AABI02000016 3561 bp DNA linear BCT 23-SEP-2003
DEFINITION Microbulbifer degradans 2-40 Mdeg_16, whole genome shotgun sequence.
ACCESSION NZ AABI02000016 REGION: 34542..38102
VERSION NZ_AABI02000016.1 GI:28878286
KEYWORDS WGS.
SOURCE Microbulbifer degradans 2-40
ORGANISM Microbulbifer degradans_2-40
   Bacteria; Proteobacteria; gamma subdivision; Alteromonadaceae;
   Microbulbifer.
REFERENCE 1 (bases 1 to 3561)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028824. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.ntm.nih.gov/sutils/genom table. cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..3561
      /organism="Microbulbifer degradans 2-40"
      /mol_type="genomic DNA"
      /strain="2-40"
      /db_xref="taxon:203122"
   gene 1..3561
      /gene="Mdeg2637"
   CDS 1..3561
      /gene="Mdeg2637"
      /codon_start=1
      /transl_table=11
      /product="COG3693: Beta-1,4-xylanase"
      /protein id="ZP 00067229.1"
      /db_xref="GI:23028853"
      /db_xref="COG:COG3693" 
/translation="MLRSTQSTPIVKRKISAYVGWGLCVLLSVCTASISWAGNPIVSH
VYTADPAARVINGRAYVMVTHDQDNQNDYGGLIDYYLFSSDDMVNWQDHGIVWNSRTD
SSWASLAYAPDFIERNGKYYLYFPNGANSIGVAVADSPEGPYTDPLGRPLVDRNTPNA
NVDWLFDPGVFIDDDGQAFLYFGGGADGTARVIRLNNDMISTSGAAISIDVPNFFEAL
YMHKRNGIYYLSYSTNPSAGMSIDYMTSNNPTSGFTHRGTILPNPWENNSNNNHQSII
EFNNEWYIFYHNRAVANTRGDSTFSRSINVDRLYYNSDGSIREVNASSIGVPAVRNVN
AFSINQAETFDQEGGIETEPSSEGTLNIQMGPGDWVKVANVDFGNGATQFNARVASAI
DNSKLEIILGSLSNTPHASLEITNTGGWQNWQTQSTSFNAITGVHDVYLRGTSGHNLN
WFEFEGENNGGSSQLTVELEDLASQSLFAPLSVRSDNMANNGAYIEWSNDGSNQILSV
ASEQSQGQISVPFTLSQASDVEFNVRVNLANGNDDSFYYKLNSNSWQTFNNQATTGWQ
VLTPNTFTGLSPGNHILTLLRREDGAKLDTLTLVASAGSIQTNNSSSSSTSSSSSTTS
SSSTSSSSSSSGAAPTGNVTYSINVTNDWQSGYCAELTVTNNTNNALQWQASVSMSDSV
DSMWNASWSQSGNILNVSGVEWNNTLQAGQSQSGIGFCATRASSSSSSSTTSSTSGST
SSSSSSGGYTVPSNNFAVNGGVENNLQSWGATAGSVTRSTEQRYSGNASARITNRAEN
 
WHGLTFSVGELTQGNLYEVAVWVKLAAGSADTPITLTAKRQNDSDDSTYNEYTGIVTT
IANDSEWVLLHGQYTQTGTAFEHFIIESESDSVSFYADEFSIGGEVTPKNEVGFFVGN
ITTNGNVRNDFTQYWDQLTPENEGKWGSVERTRDVYDWSGLDRAYNYAKQNNIPFKQH
TMVWGSQQPNWIDSLSPAEQAAEIEEWIRDYCARYPDTEMIDVVNEATLGHAPANYAA
SAFGNNWIIRSFELTRQYCPNSILILNDYNVLSWNTQEFIQMATPAVNAGVVDAIGLQ
AHGLADWSLSDLETKLNQVAALGLPIYISEYDIEKTNDQEQLRVMQTQFPLFYNHPSV
KGITIWGYVVGATWRDGTGLLHSNGTPRPALTWLMDYLNR"
ORIGIN 
1 atgttgcgaa gcacccaatc aacacccatt gttaagcgaa agatttctgc ctatgtaggt
61 tggggtctgt gcgtgttact tagcgtctgc acggcctcga tctcttgggc aggtaaccct
121 attgtgtctc atgtatatac cgcagaccct gctgcacggg taataaacgg aagagcctat
181 gtaatggtta cccacgatca ggataaccaa aatgattacg gtggtttgat tgattactac
241 ctgttctcat cggacgatat ggttaattgg caagatcacg gtattgtgtg gaattctcga
301 acagacagta gttgggccag tcttgcttac gccccagatt ttatcgagcg caatggaaag
361 tactacctgt actttcccaa cggcgcaaac tctattggtg tcgctgtggc cgatagccct
421 gagggcccct atactgatcc actcggtagg ccgctggttg accgcaatac ccccaatgcc
481 aatgttgact ggctgttcga tcccggtgta tttattgatg acgacggaca agcctttttg
541 tactttggtg gaggcgctga tggaaccgcg cgcgttattc gtttaaataa cgacatgata
601 agtaccagtg gtgcagccat aagtattgac gtacctaact tctttgaagc gctatacatg
661 cataagcgca acggcattta ctacttatcc tactcgacca accccagcgc ggggatgagc
721 atagattaca tgacgagtaa taaccctacc tcagggttca cccatcgcgg caccattttg
781 cccaacccct gggaaaataa ttccaataac aaccaccagt caattattga atttaataac
841 gaatggtaca tttttaccca caatagagct gtcgcaaata cgcggggcga tagtaccttt
901 tcccgctcta ttaacgtgga tcgtctttac tacaattccg acggcagtat tcgagaagta
961 aatgccagtt caataggtgt acccgcggta cgtaatgtta atgctttttc cataaaccaa
1021 gcagaaacat tcgatcaaga aggtggcata gaaactgagc cgtcttctga aggtaccttg
1081 aatattcaga tgggcccagg agattgggta aaagttgcta acgtcgattt tggtaacggc
1141 gccacacaat ttaacgctcg agttgctagc gcaatcgata attcaaagct ggaaattatt
1201 ttaggcagtc tcagtaatac cccgcatgcc tcgctcgaaa ttaccaacac aggcgggtgg
1261 caaaattggc aaacacaaag cacaagtttt aatgcaataa ctggtgttca cgatgtatac
1321 ctgcgcggta cttctgggca caacctaaat tggtttgaat ttgaaggcga aaataatgga
1381 ggaagcagtc agctaacggt tgagttggaa gacttggctt cgcaatctct tttgctcccc
1441 cttagcgtac gctccgataa catggctaat aacggcgctt acattgaatg gagtaatgat
1501 gggagcaatc agattctcag tgtggccagc gagcaatcgc aaggccaaat cagtgtccca
1561 tttactctat cgcaagcttc cgatgtcgaa tttaacgtac gcgtgaatct tgctaatggc
1621 aatgatgatt cgttttatta caagctaaac agtaatagct ggcagacttt taataatcaa
1681 gctaccactg gttggcaggt gctcacgccc aacaccttca ctggtcttag ccctgggaat
1741 cacattctta ctctacttcg gcgtgaagat ggcgccaaat tagataccct cacgttggta
1801 gcctccgcgg gcagtattca aaccaataac agctcatcaa gttctacctc cagcagtagt
1861 tcaacgacta gctcaagttc aaccagctcg agtagttcct ctggcgccgc gccaactggt
1921 aacgttactt actctataaa tgttactaac gactggcaaa gtggttattg tgcggagctt
1981 accgttacca ataacacgaa caacgctctg cagtggcaag ctagtgtttc tatgagcgat
2041 agtgtcgaca gtatgtggaa tgctagctgg tcgcagagcg ggaacatact taacgtaagc
2101 ggggtagagt ggaataatac gttgcaagca gggcaaagcc agagtggcat aggattttgt
2161 gctacacgtg ccagctcgtc ttcctccagc tctacaacaa gttctacttc cggttccaca
2221 tcaagctcta gttcatcggg aggctatacc gttccgagta ataatttcgc agtcaatggt
2281 ggtgtagaaa acaacctgca gagctggggc gcaacggcgg gttcagtaac acgttctact
2341 gaacaacgtt atagcggaaa cgcaagcgcg cgtataacaa atcgagcaga aaactggcac
2401 ggtttgacgt tcagtgttgg tgagcttacg caaggcaacc tgtacgaagt tgcggtgtgg
2461 gtaaaacttg cggcaggcag tgcggacaca cctattacgc ttaccgccaa acgacaaaat
2521 gatagcgacg attccactta taacgaatat accggcatag tcacgaccat tgctaacgat
2581 tctgaatggg tgctgctgca cgggcaatac actcaaactg gcacagcgtt tgagcatttt
2641 attatcgagt cagaaagcga tagcgtaagt ttttatgccg atgagttttc tattggtgga
2701 gaggtcacgc ccaaaaacga agtgggattt tttgtgggta acattaccac taatggcaat
2761 gtgcgcaatg attttactca gtactgggat caactaacac cagaaaatga aggaaagtgg
2821 ggttcggtag aacgcactcg tgatgtgtat gattggagtg gactagacag agcctataac
 
2881 tacgccaaac aaaataatat tccgtttaaa cagcatacta tggtgtgggg tagccaacag
2941 cccaactgga ttgattcgct cagcccagca gaacaggctg cagagataga ggagtggata
3001 agagattatt gtgcgcgcta tcctgatact gaaatgattg atgtggtaaa cgaagcaacg
3061 ctgggccatg ctcctgctaa ctacgcggcg agtgcgtttg gcaataattg gatcattcgt
3121 tcgttcgagc ttactcgtca atattgtcct aacagcattt taatattgaa cgattacaat
3181 gttttaagtt ggaacactca agagtttatc cagatggcta ctccggctgt caatgcaggc
3241 gttgtagatg caattggatt acaagcacac ggcttagcgg attggtcttt aagtgattta
3301 gaaaccaaac taaaccaggt tgcggcattg ggtttaccca tttatatatc cgaatacgat
3361 atagaaaaaa ctaacgacca agaacagctg cgcgtaatgc aaactcagtt cccgctgttt
3421 tataaccatc catcggtgaa aggcattact atttgggggt atgttgttgg ggctacttgg
3481 cgcgatggga cgggattgtt gcacagtaac ggaacaccca gaccggcact tacttggtta
3541 atggattact tgaatagata g
LOCUS NZ_AAB102000019 2013 bp DNA linear BCT 23-SEP-2003
DEFINITION Microbulbifer degradans 2-40 Mdeg_19, whole genome shotgun sequence.
ACCESSION NZ AABI02000019 REGION: complement(13709..15721)
VERSION NZ_AABI02000019.1 GI:28878283
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2013)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029085. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   htty://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2013
      /organism="Microbulbifer degradans 2-40"
      /mol_type="genomic DNA"
      /strain="2-40"
      /db_xref="taxon:203122"
   gene 1..2013
      /gene="Mdeg2869"
   CDS 1..2013
      /gene="Mdeg2869"
      /codon_start=1
      /transl_table=11
      /product="COG3693: Beta-1,4-xylanase"
      /protein_id="ZP 00067456.1"
      /db_xref="GI:23029095"
      /db_xref="COG:COG3693" 
/translation="MVIITMKAGLLLRILLTVLALNMLAACGGSSSNTKEPVTQPEPE
PEQQPEPEPEPEPEPEPEPEPEPEPEPEPEPEPEPEPEMEPQPEPQAPPAGGVSIIDTNPNNAS
FWAGSNNGDVGSRAVIDVDHPEFSQATRITVSNPASDYWNGQLSFPLNASVAAGDVVL
VRLYMRSVENTYESGASFTTVFIEDNIDFTKFLNREITAAQDWVEYYLPAEITDNHAT
GEVGLRIGFGAGPRAQVFDIGGVELLHYTNTDISAMPSTRPSYEGREPDAAWRTAAAE
RIEQHRKGDFELTVVDDGNPIANATIDVDFQKHAYHFGSVTVGHLLMGTSEDSAIYRE
 
KVLELFNQSGPENDLKWGPWEGEWGNNFNQTQTLNGLQWLRDNGLYTRGHVMVWPSKR
NLPNLMQQYLPEGDPASANPEAKQVVLDHIDDIATATANYLDEWDVLNEPYDNHYLMD
AFGDSVMVDWFNRARTNLPAHGLYINDYSILSAGGRNFAHQEHYTNTIQYLVDNNAPI
TGIGLQSHFGDSPTAIT·RIYEIIDQYSTAFPQLDIRATEFDVSTTDEDLQADFTRDFL
TIFFSHPKTVGVQLWGFWANAHWYPNAALYDADWREKPNALAWKEQIFNEWWNDFDGT
TNAQGKFDERGFYGDYQVTVTGEEQQIFTFSLVKGGEQNFSFEWQ"
ORIGIN 
1 atggtcataa taactatgaa agccggttta cttctacgca tcctattaac tgtactcgcg
61 ctcaatatgc ttgccgcatg tggcggtagt tctagcaata ccaaagaacc cgttacccag
121 ccggaaccag agccagagca gcagccagaa ccagaaccag agccagagcc agagccagag
181 ccagaaccag agccagaacc agagccagaa ccagagccag agcctgaaat ggaaccgcag
241 ccagagccac aagcgccgcc tgcaggtggt gtatctatca ttgataccaa ccccaacaat
301 gcatcgtttt gggcaggctc aaacaatggt gatgtgggca gtagggctgt tatagatgtc
361 gatcaccccg aatttagcca agcgacgcgc ataaccgtaa gcaaccccgc tagcgactat
421 tggaatggtc agctctcctt cccgcttaat gcgagtgtgg cggcggggga tgtagtatta
481 gtgcgtttgt acatgcgctc ggtggagaat acttacgaat cgggtgctag ttttactacc
541 gtatttattg aagacaacat cgactttact aaatttttaa accgcgaaat aaccgccgcg
601 caagattggg tagagtatta cctacccgca gaaattaccg ataaccatgc aaccggtgaa
661 gtgggcttgc gcattggctt tggcgctggc cctagggcgc aggtgtttga tattggcggt
721 gtagagctat tgcattacac caatactgat ataagcgcta tgcctagtac acgcccaagt
781 tacgaaggcc gcgagccaga tgccgcatgg cgtacagcgg cggcagagcg aattgagcag
841 caccgcaaag gcgactttga gctaacagta gtggacgatg gcaaccctat cgccaatgcc
901 accatagatg tagattttca aaaacacgcc tatcattttg gctcggtaac tgttggccat
961 ctattgatgg gcaccagtga agatagcgcc atttaccgcg aaaaagtgct cgagctattt
1021 aaccaaagtg gcccagaaaa cgatttaaag tggggcccat gggaaggcga gtggggcaac
1081 aattttaacc aaactcaaac cctaaacggc ttgcagtggc tgcgcgataa cggcctgtac
1141 acacgtggcc atgtaatggt ttggccttct aagcgcaact tgccaaactt aatgcagcaa
1201 tatttaccag aaggcgaccc cgccagcgcc aacccagaag caaaacaagt ggtgctggat
1261 cacatcgatg atatagcaac cgcaacagct aattatttag atgagtggga tgtactaaac
1321 gagccttacg acaaccacta tttaatggat gcctttggcg atagtgtaat ggtggattgg
1381 tttaatcgcg cgcgtactaa cctgcctgcg cacggtttgt acataaacga ttacagtatt
1441 ttatctgcgg gcgggcgcaa ttttgctcac caagaacact acaccaacac gattcaatat
1501 ttggtcgata acaacgcacc catcaccggt ataggtttgc aaagtcactt tggcgactcg
1561 cctacagcca ttacgcgtat ttacgaaatt attgatcaat acagtaccgc gtttccgcag
1621 ttagatattc gcgcaacgga atttgacgta agtacaacag atgaagacct gcaggcagat
1681 tttacccgcg acttcttaac gatattcttt agccacccta aaacagtggg tgtgcagttg
1741 tggggttttt gggcaaatgc acattggtac cctaatgcag cgctttatga tgccgattgg
1801 cgagaaaagc ccaatgcact agcttggaaa gagcaaattt ttaacgagtg gtggaacgac
1861 tttgacggca cgaccaacgc acagggtaaa tttgatgaac gcggttttta cggcgattac
1921 caagtaactg taaccgtagg tgaagagcag caaattttta cctttagcct agttaaaggc
1981 ggcgaacaaa actttagttt tgagtggcaa tag
LOCUS NZ_AAB102000014 828 bp DNA linear BCT 23-SEP-2003
DEFINITION Microbulbifer degradans 2-40 Mdeg_13, whole genome shotgun sequence.
ACCESSION NZ AABI02000014 REGION: complement(14092..14919)
VERSION NZ_AABI02000014.1 GI:28878288
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 828)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028504. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.igi.doe.gov
   Contact: Paul Richardson
   microbes@cuba.jgi-psf.org.
   COG assignment was automatically updated on Sep 23, 2003.
FEATURES Location/Qualifiers
   source 1..828
      /organism="Microbulbifer degradans 2-40"
      /mol_type="genomic DNA"
      /strain="2-40"
      /db_xref="taxon:203122"
   gene 1..828
      /gene="Mdeg2305"
   CDS 1..828
      /gene="Mdeg2305"
      /codon_start=1
      /transl_table=11
      /product="hypothetical protein"
      /protein_id="ZP00066908.1"
      /db_xref="GI:23028515" 
/translation="MNIKTFFPALIASVFLLINASTGYAASITKTLCNPADSDNGYGA
GTFNGKFYSWFELSQEDITDCDTKIGFYNETNRHFRVEWNVAQSWGEDAIGGMGWSSG
SRDRKIGYNVGQLTTNSSIQKALVAMYGWSCSTSGGNQISQEYYVVDTWDGGKFVPWD
ENANNGNGAPAQSVGTVSANGATYDVYKVRRNGAQYCFNGSSRSFDQFWSVRRTPRAI
NGNRNMDFRPHANRWDNSDLGFKVDGLSSGYQILAVEIFGDANLRHKGAADITLWPR"
ORIGIN 
1 atgaacataa aaacattctt ccccgcactt attgcaagtg tatttttatt aattaacgcc
61 agcactggct atgcagcaag cattaccaaa acgctttgca acccagccga ttccgataac
121 ggctacggtg caggaacctt caatggcaaa ttttattctt ggtttgagtt aagccaagaa
181 gacattaccg attgcgatac aaaaattggt ttttacaacg aaaccaatcg acactttagg
241 gtggagtgga atgttgctca atcttgggga gaagatgcaa ttggtggaat gggttggagc
301 tctggctcga gagatagaaa aataggttac aacgttggcc aacttacaac taattcttct
361 attcaaaaag cattggttgc tatgtatggc tggtcttgct ctaccagtgg tggcaaccaa
421 atatcacaag aatattatgt agtggataca tgggacggcg gcaagtttgt gccttgggat
481 gaaaacgcaa ataatggcaa cggtgctcca gcacagagtg taggaacagt tagcgctaat
 
541 ggtgcaacat acgatgttta taaggttcgc cgcaacggtg cgcaatattg ttttaatggc
601 agcagccgct cgtttgatca gttttggagt gtgcgtagaa cgcctagagc gattaacggc
661 aaccgtaata tggattttcg cccgcacgcc aaccgctggg acaacagtga cctaggtttt
721 aaagttgacg ggttaagcag cggttaccaa attttagcgg ttgaaatatt tggtgatgcg
781 aacctaagac ataaaggtgc agcagatatt actttatggc cacgctaa
LOCUS NZ_AABI0200001 02304 bp DNA linear BCT 23-SEP-2003
DEFINITION Microbulbifer degradans 2-40 Mdeg_14, whole genome shotgun sequence.
ACCESSION NZ AABI02000010 REGION: 110928..113231
VERSION NZ_AAB102000010.1 GI:28878292
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2304)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028613. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2304
      /organism="Microbulbifer degradans 2-40"
      /mol_type="genomic DNA"
      /strain="2-40"
      /db_xref="taxon:203122"
   gene 1..2304
      /gene="Mdeg2474"
   CDS 1..2304
      /gene="Mdeg2474"
      /codon_start=1
      /transl_table=_11
      /product="COG0726: Predicted xylanase/chitin deacetylase"
      /protein_id="ZP 00067071.1"
      /db_xref="GI:28683"
      /db_xref="COG:COG0726"
      /translation="MKSINVCGRRLKQALAAIATAAATLWFTPVDAQTLTSNQTGTHG 
GYYYSFWTDSAGTVSMTLGNGGNYSSSWSNTGNWVGGKGWQTGGRKTVNYSGTFNPSG
NGYLTLYGWTQNPLIEYYIIESWGTYRPGESGTYYGTVNTDGGTYDIYRTQRVNQPSI
EGTATFYQYWSVRQQKRVGGTITTGNHFDAWASHGLNLGTHNYMVMATEGYQSSGNSN
ITVSEGSGSSSTSSSSSSTGGPSGTNIVVRAQGVSGQEHINLIIGGNVVADWTLSTSM
QDYTYTGNAAGDLQVEYDNDASGRDVELDYVYVNGEIRQAEDMEYNTATYSGECGGGS
YSQTMHCSGVIGFGDTSDCFSGNCNGASSTSSSSSSSTSSSTSSGGNNNSGITVRAR
GTNGDEHINLIVGGNIVGNWTLTTSNQNYVYNGNASGDVEVQFDNDANGRDVILDYVI
VNGETRQAEDMEYNTATYSGSCGGGSYSETMHCSGEIGFGHTDDCFSGNCTSSSGTTG
SSGGTSSNNGTSSCNGYVGITFDDGPGNNTATLINLLQQNNLTPVTWFNTGQNIAANT
GQFAQQKSVGEIQNHSYTHSHMLNWSYQQVRDELASTNQAIVNAGGATPTLFRPPYGE
TNSTINQAAQDLGLRVITWDVDSRDWDGASASAIANSANQLQNGQVILMHDASYNNTN
GAISQFAANLRARGLCAGKIDPSTGRAVAPSTNTGGNTGSNTGNGGNGGMCNWYGTSI
 PLCQTTNDGWGWENSQSCVSQNTCNSQ"
ORIGIN 
1 atgaagtcaa tcaatgtatg cggcagacgc ctcaagcaag ccctcgcagc aatagcaacc
61 gctgcagcaa ctctctggtt tacgccagtg gatgcacaaa ccttaacctc aaaccaaact
121 ggtactcatg gtggttacta ctattccttc tggaccgaca gtgctggcac tgtttctatg
181 acactcggca atggcggcaa ttacagttca tcgtggagca ataccggtaa ctgggtggga
241 ggtaaaggct ggcaaacggg gggacgcaaa accgtaaact attccggtac gtttaacccc
301 tcgggcaatg gttatttaac cctctacggt tggacccaaa acccactcat tgaatactac
361 atcattgaaa gctggggcac ctatcgccca ggtgaaagcg gaacctacta cggcaccgtc
421 aacaccgatg gcggcactta cgatatttat cgcacccaac gcgttaacca accgtcaatt
481 gaaggcactg caacgtttta tcagtactgg agtgttaggc aacaaaaacg cgtaggcggc
541 accataacaa ccggcaacca ttttgatgcg tgggcgagtc atggccttaa cctaggcaca
601 cacaattaca tggtaatggc caccgaaggt tatcaaagta gcggcaactc caatattacc
661 gttagcgaag gcagcggttc gagcagtact agttcgagta gctctagcac cggtggccca
721 agtggtacca atattgttgt gcgcgcacaa ggtgtaagcg gccaagaaca tatcaattta
781 attattggcg gtaacgtagt ggcagactgg acgctttcaa ccagcatgca agattacacc
841 tacaccggta atgccgcagg cgacctgcaa gtagaatacg acaacgatgc tagtggtcgc
901 gatgtagagc tagactatgt gtatgtgaat ggcgaaattc gtcaagcaga agacatggaa
961 tacaacaccg caacttacag tggtgaatgt ggtggcggtt cctattcgca aaccatgcac
1021 tgcagcggtg taattggctt tggcgatacc agtgattgtt ttagcggcaa ctgtaatggt
1081 gcatcttcta caagttctag ttcgtctagt agctcaacca gctctagcac aagctctggc
1141 ggtaacaata acagcggcat tactgttcgc gcacgcggta ccaatggcga tgaacatatc
1201 aaccttattg ttggcggcaa tatagtaggc aattggacgc tcaccaccag caaccaaaat
1261 tatgtttaca acggcaatgc atctggtgat gtagaagtac aattcgacaa cgatgccaac
1321 ggtcgcgatg ttattctcga ttacgtaata gtaaatggcg aaactcgcca agcggaagat
1381 atggaataca acacggcgac ctacagcggt tcctgtggtg gtggctccta ttcggaaaca
1441 atgcactgca gcggcgaaat tggttttggt cacaccgacg attgctttag tggaaattgc
1501 actagcagca gcggcacaac cggtagctct ggaggaacat caagcaataa cggtacaagt
1561 agctgtaacg gttatgtagg tattaccttc gatgatggcc caggcaataa caccgctaca
1621 ttaataaact tactacaaca aaataactta accccagtaa cttggtttaa cacaggccaa
1681 aatattgctg ccaatacagg tcagtttgcc cagcaaaaaa gtgttggtga aattcaaaac
1741 cacagctaca cccattccca tatgcttaat tggagctatc aacaagttcg cgacgaactc
1801 gccagcacca atcaagctat tgtgaatgct gggggcgcaa cgccaactct attccgtccg
1861 ccttatggcg aaacaaactc caccattaat caagcggcac aagatttagg cctgcgcgta
1921 ataacctggg atgtagattc gcgcgattgg gatggcgcaa gcgcttcagc tattgccaac
1981 tcggctaatc agttgcaaaa cggccaagta attttgatgc acgatgccag ctacaacaat
2041 accaacggag ccatatcaca atttgcagcc aatctaagag caagagggct atgtgcaggt
2101 aaaatagacc caagcactgg ccgcgcagtt gcaccaagca caaataccgg cggcaacact
2161 ggcagcaata caggaaatgg cggtaatggc ggcatgtgta actggtacgg caccagcatt
2221 ccattatgcc aaactaccaa cgacggttgg ggctgggaaa actcacaaag ctgcgtttcg
2281 caaaatacct gtaactcaca ataa
LOCUS NZ_AABI02000001 1083 bp DNA linear BCT 23-SEP-2003
DEFINITION Microbulbifer degradans 2-40 Mdeg_1, whole genome shotgun sequence.
ACCESSION NZ AABI02000001 REGION: complement(131880..132962)
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1083)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1083
      /organism="Microbulbifer degradans 2-40"
      /mol_type="genomic DNA"
      /stain="2-40"
      /db_xref="taxon:203122"
   gene 1..1083
      /gene="Mdeg0096"
   CDS 1..1083
      /gene="Mdeg0096"
      /codon_start=1
      /transl_table=_11
      /product="hypothetical protein"
      /protein_id="ZP 00064735.1"
      /db_xref="GI:23026249" 
/translation="MCLKINRCWVFVWLCICATTAHSETYVPADNDQYLYTGRIDFSD
IKAPSLSWPGTSIKANFTGEHLEVVLDDQNGKNFFNVIIDGNDRFPYVLEAKQGEHRY
LISSALSKGKHSVEIYKRTEGEEGATLFKGLWLADDSYLLKPPKRPKRRIEIYGDSIT
SGMGNEGADNGADHLGSEKNNYLAYGAITARNLNAELHTISQSGIGVMVSWFPFIMPQ
FYNQLSAVGNNDSIWDFKQWTPHVVVINLMQNDSWLIDREKRLTPIPADAQRIAHYQA
FVQSIRAEYPKAQIICALGSMDATANEKWPNYVREAVKNMQDNGDNKIDTIFFEYIGY
GQHPRVAQHNANADKLTKFIKKKMKW"
ORIGIN 
1 atgtgcctaa aaataaaccg gtgctgggtg tttgtttggt tgtgtatttg cgcaactact
61 gcccatagtg aaacctacgt acccgcagat aacgaccaat acctttatac cggccgtata
121 gattttagcg atataaaagc accctcgcta agctggcccg gcacaagtat aaaagccaac
181 tttaccggcg aacatttaga ggtagtgtta gacgatcaaa acggtaagaa ttttttaat
241 gtgattatcg acggtaacga tcgatttcct tatgtgctag aagctaaaca aggtgagcat
301 cgatatttaa tttcttctgc gctaagcaag ggcaagcaca gcgtagaaat ttataaacgt
361 acagaaggcg aagagggcgc aacgctattt aaagggcttt ggttagccga tgatagttat
421 ttattaaaac cccctaaacg cccaaaacgc agaatagaaa tttatggtga ctcaattaca
 
481 agcggtatgg gtaacgaagg cgcagataac ggcgccgacc atttgggctc cgaaaaaaat
541 aattaccttg cctatggggc tattaccgca cgcaatttaa acgccgagct acataccatt
601 tcgcaaagcg gtattggggt aatggtaagt tggtttccgt ttattatgcc gcagttttac
661 aaccagctaa gtgctgttgg taataatgat tccatatggg actttaaaca atggacgccc
721 catgtagttg taataaacct aatgcaaaac gatagctggc taatagatag agaaaagcgc
781 cttacgccaa ttcctgcaga tgcacaacgc atagcccatt atcaagcgtt tgtgcaaagc
841 attcgtgccg aataccccaa ggcgcaaata atatgcgcac tgggcagtat ggatgcaacc
901 gcaaacgaaa aatggccaaa ctacgtgcgc gaagctgtaa aaaatatgca agataatggc
961 gataataaaa tcgatactat tttctttgaa tacatcggct acggccaaca cccgcgcgta
1021 gcgcaacaca atgcgaatgc agataagtta actaaattta ttaagaaaaa aatgaaatgg
1081 tag
LOCUS NZ_AABI02000001 2922 bp DNA linear BCT 23-SEP-2003
DEFINITION Microbulbifer degradans 2-40 Mdeg_1, whole genome shotgun sequence.
ACCESSION NZ AABI02000001 REGION: complement(296895..299816)
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2922)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www .jgi.doe.gov
   Contact: Paul Richardson
   microbes@cuba.jgi-psf.org.
   COG assignment was automatically updated on Sep 23, 2003.
FEATURES Location/Qualifiers
   source 1..2922
      /organism="Microbulbifer degradans 2-40"
      /mol_type="genomic DNA"
      /strain="2-40"
      /db_xref="taxon:203122"
   gene 1..2922
      /gene="Mdeg0224"
   CDS 1..2922
      /gene="Mdeg0224"
      /codon_start=1
      ltransl_table=_11
      /product="COG1501: Alpha-glucosidases, family 31 of
      glycosyl hydrolases"
      /protein_id="ZP00064863.1"
      /db_xref="GI:23026377"
      /db_xref="COG:COG1501" 
/translation="MNYYLNKKRLGQLLTGAAIIPVLYACGSQEKNVEPATVNWHKTS
DGVVVSLQDSEAKKVRLQVINDRIVRVTATPQQDFNNLPNTLMVVAKPEQTAFEVKQN
DASVVLSTADLSAEVSLVTGVVSFKDEHGKVLTTEVDRGNFGAVTRDPGVVDADSFAI
RQQFTSDENEGYYGLGQQQDGEVNYAGDNVELTTYNLEISIPYVVSSKDYALLWNNTS
ISRLGDPNPPEPLKEGFKLFDANGNPGGLTARYFDGDKLLLERVEADLDYQFLAQGSN
RTTPMPDETADAKNLRIEWEGSIESDTNGVHELKMYSSGYAKLYLNGELVLDRWRMNW
NPWYHNTKLEMQAGKKVALKLDWQVDGGYMRIKQHKPLPVAEQGRLSIASDTAKAIDY
YFVVGDNKDELVSGYRTLTGKAVMLPKWVFGFWQSRERYKTQDEIIDALQEYRDRKIP
IDNIVLDWSYWPQDAWGSHDFDEQFFPDPSALVDKVHELNGNIMISVWPKFYPTTDNY
KALNAKGCMFNKNIEQKNLDWIGEGYLNGFYDAYNPECREMFWAQIRDKINVHGFDAW
WLDAVEPDIHSNLSFEHRKDLMTPNALGTGAEVFNAYALPHAETVYQGERRDDGDKRA
FILTRSGFAGIQRTGSAIWSGDVVSRWSDLKEQIAAGVGVGISGMPYWTFDIGGFTPE
DRYRYSAKGSVGHFSMMNESEVPEWQEINLRWFQFGTFVPLFRSHGQNPYREIYNIAD
KGTEVYDSMVWYTKTRYRLMPYIYSLVGDAHHKDGTFMRALVMDFPSDLNVRDINDQY
 
MFGPALLVNPVSEFKARSRDVYLPAGADWYDFYTGVKHTGGKTIKADAPLAKMPIFVK
AGSIIPTGVEIQHVYDKPDAPYTLNVYTGANGSFEIYEDDGKTYAYEQGAWARIPVSY
NDKTGELTIGDRVGSFEGMTKEREFRVRWISAKRDDAANFDTGVAKAVTYTGKAITIK
R"
ORIGIN 
1 gtgaattatt atttaaacaa aaagcgactg gggcaattgc tcaccggcgc ggccattatt
61 cccgtgctat atgcatgtgg ctcacaggaa aaaaacgtag agcctgcaac ggttaattgg
121 cataaaacaa gcgacggcgt cgttgtaagc ttgcaagata gcgaagcaaa aaaagtgcgc
181 ttgcaagtca ttaacgatcg gatagtacgt gttaccgcta cgccacagca ggatttcaac
241 aacctgccaa atacgcttat ggtggtggcc aagcccgagc aaacggcgtt tgaagttaaa
301 caaaacgatg catctgttgt gttatcaacg gcagatctat ctgccgaagt gtcattagta
361 actggtgttg taagttttaa agatgagcac ggcaaggtgc ttacaacaga agttgatcgc
421 ggcaattttg gggcggtaac ccgcgaccca ggtgtggtgg acgccgattc atttgctatt
481 cgccaacagt ttacaagcga cgaaaatgaa ggctactacg gtttaggtca gcagcaggat
541 ggcgaagtaa actacgctgg cgataacgta gagttaacaa cttacaactt agaaatttct
601 ataccttatg ttgtatcaag caaagattac gcgctgctat ggaacaatac ctcaatttct
661 cgtttgggcg accccaatcc acccgagcca ctaaaagagg gctttaaact ctttgacgct
721 aatggtaacc ccggcgggct aaccgcacgt tattttgatg gcgataaatt actgctcgag
781 cgtgtagagg ccgatttaga ttatcaattt ttagcgcaag gtagtaatcg cactacgccc
841 atgcctgatg aaaccgctga tgcaaaaaat ctgcgtattg aatgggaagg tagtatcgaa
901 tccgatacca acggtgtgca cgagttaaaa atgtattcca gtggctacgc taaattgtat
961 ttgaatggcg agttagtgtt agatcgctgg cgtatgaact ggaacccttg gtatcacaac
1021 accaagttag aaatgcaggc cggtaaaaaa gttgcattaa agttagattg gcaagtagat
1081 ggtggttata tgcgcataaa acagcataaa ccactgccgg tagcagagca gggacgtttg
1141 tctattgctt ccgataccgc gaaagccatt gattactact ttgtagttgg cgataacaag
1201 gatgagttgg tgtctggcta ccgtacgctc acaggtaaag cagtgatgct acctaagtgg
1261 gtgtttggtt tttggcaaag ccgcgagcgc tataaaacac aagatgaaat tatcgacgcc
1321 ttgcaagaat accgcgatcg taaaattcct atcgataaca ttgtattaga ttggagttat
1381 tggcctcagg atgcatgggg tagtcatgat ttcgacgagc aatttttccc cgacccatct
1441 gcactagtag ataaagtaca cgagctaaac ggcaatatta tgatttccgt atggcctaag
1501 ttttacccta caaccgacaa ctacaaagcg ctaaacgcta aaggttgtat gtttaataaa
1561 aacatcgagc agaaaaacct cgattggatt ggcgagggtt acctaaatgg cttttacgat
1621 gcctataacc cagagtgccg tgaaatgttt tgggcgcaaa ttcgcgataa gatcaatgtg
1681 cacggtttcg atgcttggtg gttagatgcg gtagagccag atatccattc caacctttct
1741 tttgagcacc gcaaagattt aatgacaccc aatgcactcg gcaccggtgc cgaagtgttt
1801 aacgcttacg ctttgccgca cgcagaaact gtttaccaag gcgagcgtag agatgacggt
1861 gacaagcgcg catttattct aacgcgttct gggtttgccg gtattcagcg caccggttcg
1921 gctatttgga gtggcgatgt ggtatcgcgc tggtccgact taaaagaaca aattgcagca
1981 ggtgtgggcg tgggcatttc tggtatgccg tattggacgt tcgatatcgg tggctttact
2041 ccagaagatc gctaccgtta tagcgccaaa ggttctgttg gtcatttctc tatgatgaac
2101 gaatcggaag tgcctgaatg gcaagaaatc aatctgcgtt ggttccaatt tggtaccttt
2161 gtgccgctgt ttaggtccca cggccaaaac ccatatcgcg aaatatataa catcgccgat
2221 aaaggcaccg aggtatacga cagcatggtg tggtacacca aaactcgcta tcgcttaatg
2281 ccttatattt attcgttagt tggcgatgct caccacaaag acggcacctt tatgcgcgct
2341 ctggtgatgg atttccctag cgaccttaat gtgcgcgata ttaacgacca gtatatgttt
2401 ggccccgcgc tactcgtaaa ccctgtgtcg gaatttaaag cgcgttcacg ggatgtgtat
2461 ctacctgcgg gcgcagattg gtacgatttc tatacaggtg tgaagcacac aggtggtaaa
2521 accattaagg ccgatgcacc gcttgccaaa atgcctattt ttgttaaggc cggctctatt
2581 attccaacag gtgtagaaat ccagcatgtg tacgataagc ccgatgctcc ttacaccctt
2641 aacgtgtata ccggtgcgaa tggcagcttc gaaatttatg aagatgacgg caaaacctac
2701 gcttacgagc aaggggcttg ggcgcgcatt cccgtttcgt acaacgataa aaccggtgag
2761 ctaaccattg gcgatcgcgt aggtagcttt gagggaatga ccaaagagcg cgaattccgc
2821 gtgcgctgga tatctgccaa gcgagacgat gccgccaatt tcgatacagg tgtggccaaa
2881 gccgttacct atacgggtaa ggcaataacc attaagcgct aa
LOCUS NZ_AABI02000024 2682 bp DNA linear BCT 23-SEP-2003
DEFINITION Microbulbifer degradans 2-40 Mdeg_22, whole genome shotgun sequence.
ACCESSION NZ AABI02000024 REGION: complement(16977..19658)
VERSION NZ_AABI02000024.1 GI:28878278
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2682)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029266. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2682
      /organism="Microbulbifer degradans 2-40"
      /mol_type="genomic DNA"
      /strain="2-40"
      /db_xref="taxon:203122"
   gene 1..2682
      /gene="Mdeg3040"
   CDS 1..2682
      /gene="Mdeg3040"
      /codon start=1
      /transl_table=11
      /product="COG14472: Beta-glucosidase-related glycosidases"
      /protein_id="ZP00067626.1"
      /db_xref="GI:23029277"
      /db_xref="COG:COG1472" 
/translation="MNKHFLVGVITLGVILQGLTACSKSAAPNANQPQDTAASTATYP
FRDASLSVDARVDDLVSRLTTTEKIAQMFNDTPAIERLGIPAYNWWNESLHGVARAGK
ATVYPQAIGLASTFDEDLMLRVATSISDEGRAKYHDFLSKDVRTIYGGLTFWSPNINI
FRDPRWGRGQETYGEDPFLTGRMAINFVKGIQGENDNSDYLKAVATIKHYAVHSGPEK
TRHSDDYHPTRKDLFETYLPAFRMAIAETNVQSLMCAYNRVDGAPACGNNELMQEILR
GDMGFNGYVVSDCGAIADFYESRSHHVVDSPAEAAAWAVKSGTDLNCGDSHGNTYTNL
HYALQQGLITEDYIDIAVKRLFKARIKLGMFDEQDRVPYSEIGMDVVGSPKHLALTQE
AAEKSIVLLKNNGVLPLKAGVKVAVIGPNAVDEDVLVGNYHGVPVKPVLPLEGIVNRV
GEANVFYAPGSAQIADIYSHYEPISAENFYHKDANGNLAAGLKAEYYADYYNAAEIND
DTFSATPALNRIDADINFSWPVSPIDNSLDDEFSAVWTGILKPKKSGSYRFSGTVALA
INGKPVNGAVNLKAGESYNIKAIFGVQKWWPVNAIHPYGKLTWLDESRDLEEEALAAA
RKADVIIFMGGIDAHLEGEEMPLELDGFTHGDRTHINLPKVQTNLLKQLKATGKPVVM
VNFSGSAMALNWESEKLDAILQAFYPGEATGTALANILWGDVSPSGRLPVTFYKGVDD
LPAFNDYHMENRTYKFYRGEPLYAFGHGLGYVDFAYNNLVVANTAEAGKALPIAVSVT
 
NTGKMQAEDVAQVYISLLDAPANTPIRDLKAFKRTKLAAGESTELEFNLPARVLTYID
DNGKTQTYTGRVEVTVGSGQKGYVKENAIAVATINVQ"
ORIGIN 
1 atgaataaac actttttagt aggtgtaatt acgttagggg taattctgca ggggctaact
61 gcatgtagca aaagcgctgc acctaatgcc aatcaaccgc aagataccgc agctagtacg
121 gctacctacc cgtttaggga tgcaagctta agtgtagatg cccgcgtaga cgacttggta
181 tcgcgtttaa ccacaaccga aaaaattgcc caaatgttta acgatacgcc cgcaatcgag
241 cgattgggta ttcccgccta caattggtgg aacgaatcgt tgcacggtgt ggcccgtgcg
301 ggtaaagcaa cggtataccc gcaggcaata ggcttagcgt ctacatttga tgaagactta
361 atgttgcgcg tggctacttc tatttctgat gaggggcgcg ctaagtatca cgacttccta
421 tcgaaagacg tgcgcaccat atacggcggg cttacctttt ggtcgccaaa tattaatatc
481 ttccgcgacc cgcgttgggg cagggggcaa gaaacctacg gtgaagaccc gttcttaacg
541 gggcgtatgg ccattaattt tgttaagggt attcaaggcg aaaacgacaa cagcgattac
601 ctaaaagccg tagcgacaat taagcactat gccgtacaca gcggccccga aaaaacgcgt
661 cattcggatg actaccatcc aacccgtaaa gatttattcg aaacctattt gcctgcattt
721 cgcatggcaa tagcagagac taacgtgcaa tcgttaatgt gtgcctacaa ccgtgtagat
781 ggggcacctg cctgtggcaa taatgaatta atgcaagaaa ttttgcgtgg cgatatgggc
841 tttaacggtt atgtcgtgtc tgactgtggc gccattgccg atttttacga gagtagatcg
901 caccacgtgg ttgactcacc tgcagaggct gcagcgtggg ccgttaaatc gggtaccgat
961 ttaaactgtg gcgattcaca tggcaatacc tacaccaacc tgcattacgc gttacagcaa
1021 ggtttaatta cagaagatta tattgatata gcggtaaagc gtttgtttaa agcgcgtatt
1081 aagcttggca tgtttgacga gcaagaccgc gtgccttaca gcgaaattgg tatggatgtt
1141 gtaggttcac ctaagcacct agcgctaacc caagaagcgg cagaaaaatc tattgtgctg
1201 ctaaaaaaca atggtgtatt gccattaaaa gcaggggtaa aggtagccgt aatagggcca
1261 aatgcagttg atgaagatgt attggtaggc aactaccacg gcgtaccagt gaaacctgtg
1321 ttgccgctag aggggattgt taatcgtgtt ggcgaggcca acgtatttta tgccccaggc
1381 agtgcacaaa tagccgatat atacagccac tacgaaccga taagtgcaga aaatttttat
1441 cataaagatg caaatggtaa tttagctgca ggcttaaaag cagagtatta cgccgattat
1501 tacaacgcag ctgaaattaa cgacgatacc tttagcgcaa ccccagcgtt aaatagaatt
1561 gatgcagata ttaatttctc ttggcctgta tcgcctattg ataattcgtt agatgatgaa
1621 tttagtgcag tatggacagg catacttaaa ccgaaaaagt cgggtagcta ccgtttctcg
1681 ggcacggttg cattagccat taacggcaaa cctgttaatg gggctgttaa cctaaaggca
1741 ggtgaaagct ataacataaa agctattttt ggcgtgcaaa aatggtggcc cgttaatgca
1801 atacacccgt acggaaaact tacttggcta gatgagtcgc gcgatttaga agaagaggca
1861 ttagctgctg cccgaaaagc cgatgtgatt atttttatgg gcggtataga tgcgcacctt
1921 gaaggcgaag aaatgccgct agagctagat ggctttactc acggtgatcg tacgcacatt
1981 aatttaccta aagtacaaac caatttgctt aaacaattaa aagcaacggg taaacctgtt
2041 gtaatggtta actttagtgg tagtgccatg gctttaaatt gggaaagcga aaagctagac
2101 gcaatactgc aagcgtttta cccaggtgaa gcaaccggta cagcgttagc taatattttg
2161 tggggcgatg taagcccgag tggccgctta cctgtaacct tttacaaagg cgtagacgat
2221 ctaccagcat ttaatgatta ccacatggaa aaccgcacct ataaatttta ccgcggtgag
2281 cctttgtatg catttggcca cggtttaggt tacgttgatt ttgcttataa caatttagtc
2341 gtagcaaata ctgcagaagc gggcaaagcg ctacctatag ctgtaagcgt aaccaatacc
2401 ggtaaaatgc aagcagaaga cgttgcccaa gtttatataa gtttgctaga tgcccccgca
2461 aacacgccca tccgcgattt aaaagcgttt aaacgtacca agcttgcggc aggcgaaagc
2521 accgagcttg aatttaactt gccggcgaga gtgcttacct atatagacga taatggtaaa
2581 acccaaacct atactggcag ggtagaagtt actgttggct ctgggcaaaa gggatacgta
2641 aaagaaaatg cgatagctgt agcgactatt aacgttcagt ag
LOCUS NZ_AABI02000026 954 bp DNA linear BCT 23-SEP-2003
DEFINITION Microbulbifer degradans 2-40 Mdeg_26, whole genome shotgun sequence.
ACCESSION NZ AABI02000026 REGION: complement(16626..17579)
VERSION NZ_AABI02000026.1 GI:28878276
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 954)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029482. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..954
      /organism="Microbulbifer degradans 2-40"
      /mol_type="genomic DNA"
      /strain="2-40"
      /db_xref="taxon:203122"
   gene 1..954
      /gene="Mdeg3244"
   CDS 1..954
      /gene="Mdeg3244"
      /codon_start=1
      /transl_table=11
      /product="COG3507: Beta-xylosidase"
      /protein_id="ZP 00067829.1"
      /db_xref="GI:23029495"
      /db_xref="COG:COG3507" 
/translation="MYTYVSAIALFIFSIASSCCVAQNPLDFGSNIKTADPSGHIWAD
GRMYLYTSHDQECQEDFYMKDWHTFSSSDLINWTAHGPSLSVADITWADNYAWAPDAA
YKNGKYYLFFPAGTGVKDRVNPEKSTKWMGIGVAVSDSPTGPFKDAIGAPLWTDPYAN
DPSIFIDDDGKGYLYFHGKGADYLVAEMADDLLSVKGEFHKMDMGGYEPKMEGPWVFK
REGMYYFTMPENNRSLAYYMAKSPFGPWEYKGIFMQEEGGNNHHSIVQFKGKWILFYH
RWLMGEGECKKKQRHTAAEYLHFNADGTIKEVKRTREGLTK"
ORIGIN 
1 atgtacacat atgtatccgc catagcacta tttatatttt caattgcctc gtcgtgttgt
61 gttgcccaaa acccgctcga ctttggcagt aatattaaaa ccgcagatcc gtctggccat
121 atatgggctg atggcagaat gtacctttac acctcgcacg accaagaatg ccaagaagat
181 ttttatatga aggattggca taccttttcg tccagcgact taataaattg gactgcccac
241 ggcccaagtt tatctgtagc ggatattacg tgggcagata actacgcatg ggcgcccgac
301 gcggcctata aaaatgggaa gtactatttg ttctttccgg cgggaaccgg tgttaaagat
361 agagtaaacc ccgaaaaaag cactaagtgg atgggcattg gtgttgcagt aagcgatagc
 
421 cctacaggcc cctttaaaga tgcgattggc gcccccttgt ggaccgaccc ctatgccaac
481 gacccaagta tttttataga tgatgacggc aagggctact tatattttca cggtaaaggt
541 gcagactacc tagtagccga aatggcagac gatttactga gtgtaaaagg tgagtttcac
601 aaaatggata tgggcggtta cgagccaaaa atggagggcc cttgggtttt taagcgcgag
661 ggaatgtatt actttaccat gccagaaaac aatcgttcac ttgcttacta tatggcgaaa
721 tctccctttg ggccgtggga atacaagggc atttttatgc aagaagaagg cggtaacaac
781 caccattcta ttgtgcaatt taaaggcaag tggatattgt tttatcaccg ctggttaatg
841 ggcgaaggcg agtgtaaaaa gaagcaacgc cacaccgcag cggaatacct tcactttaat
901 gccgacggca caattaaaga agtaaaaaga acgcgcgagg ggttaactaa gtag
LOCUS NZ_AABI02000004 1734 bp DNA linear BCT 23-SEP-2003
DEFINITION Microbulbifer degradans 2-40 Mdeg_4, whole genome shotgun sequence.
ACCESSION NZ AABI02000004 REGION: complement(182992..184725)
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1734)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jqi.doe.gov
   Contact: Paul Richardson
   microbes@cuba.jgi-psf.org.
   COG assignment was automatically updated on Sep 23, 2003.
FEATURES Location/Qualifiers
   source 1..1734
      /organism="Microbulbifer degradans 2-40"
      /mol_type="genomic DNA"
      /strain="2-40"
      /db_xref="taxon:203122"
   gene 1..1734
      /gene="Mdeg1098"
   CDS 1..1734
      /gene="Mdeg1098"
      /codon_start=1
      /transl_table=11
      /product="COG3507: Beta-xylosidase"
      /protein_id="ZP00065724.1"
      /db_xref="GI:23027269"
      /db_xref="COG:COG3507" 
/translation="MKIKCLLLAVYAGLLAACALDAPLKTSSKPLAHFSWFEYQGNDE
IFKAPLASNQYQNPILAGYHPDPSIVRVGEDYYLVNSTFGFYPGIPVFHSRDLVNWTQ
LGNAIHRPEQLSFDGIHLGYNGVYAPAIEYRDGTFYVINTCVACGGNFIVTATNPAGP
WSDPIWLPEVIGIDPSLFFDEDGKTYIVHHRNPPVQKYPAHTALWVMEVDSKTFAPVS
DDVMLVDGGDEAPWHTEYIEGPHIYKIDGTYYLYAPGGGTGYFHGQLVYRSDNVFGPY
EANPNNPVLTQVGLPDDREHPVTATGHADLFQDTNGDWWTVFLGTRVYDLAKPPQDPG
NFATGRETFMLPVTWQNGWPHVLEKGEAVPYRVTKPKLPAGKPAPRAMTGNFTVREEF
TNASLAPHWLFVRTPRSKWWQTGNGELILEARADTIGAVNQPSFIGRRLAHMTASFAT
QLTFNPHTVGDEAGLLAVQNDEHFYAFGLGLNSKGQTVLRVRKKAGKNESIRGDTVAE
QVVKLKHGHPIYLRVNIGKAELNFAYSTNGKRYTTLLNQADANLLTTAKAGGFTGAVV
GMYAESTAQQN"
ORIGIN

1 atgaaaatta agtgcttact ccttgctgtt tacgcgggtc tacttgcggc ttgcgcgctg
61 gacgcgccgc tcaaaacctc aagtaaaccg ctagcgcatt tttcgtggtt tgaatatcaa
121 ggtaacgacg agatatttaa ggctccactc gcctcaaatc aataccaaaa ccccatactc

181 gccggctacc acccagaccc aagtattgtg cgagtaggcg aagattatta tttggtgaac
241 tccacctttg gcttctaccc tggcattcca gtatttcaca gccgtgactt agtgaattgg
301 acccaactgg gtaacgctat tcaccgccca gagcaacttt catttgatgg tattcactta
361 ggctacaacg gcgtttatgc accggcaatc gaataccgcg acggaccttt ttacgtaata
421 aatacctgcg tagcctgcgg aggaaatttt atcgttaccg ccaccaatcc cgcgggcccc
481 tggtcagacc caatatggct accagaggta attggcatag acccctcgct atttttcgac
541 gaggacggca aaacctatat cgtgcatcat cgtaatccac ctgtgcagaa ataccctgcc
601 cacacagccc tgtgggtaat ggaagttgac tccaaaacat ttgcgccggt atctgacgat
661 gtaatgcttg tggacggtgg cgacgaagcg ccatggcaca cagaatatat tgaaggccgg
721 catatatata aaattgatgg cacctactac ctctatgccc ctggtggcgg cacgggatac
781 ttccacggcc aattggtgta tagatctgac aatgtatttg gaccctacga agccaacccc
841 aataaccctg tgttgactca agttggttta cccgacgaca gagaacaccc tgtaacggca
901 acgggtcatg cagatttatt tcaagatacc aacggcgact ggtggacggt atttctgggt
961 actcgcgttt acgatttagc taagccacca caagaccccg gcaattttgc caccggacgc
1021 gaaacattta tgttgccagt aacatggcaa aacggctggc cacacgtgct cgaaaaaggc
1081 gaggctgtgc cctaccgagt aaccaaaccc aaattacctg caggcaaacc cgccccgcgc
1141 gcaatgactg gaaactttac tgtgcgcgag gaatttacca acgcttcgct tgccccccac
1201 tggctatttg ttcgcacacc gcgttccaaa tggtggcaaa caggtaatgg cgaacttatt
1261 ttagaagcgc gcgccgatac cattggggca gttaaccagc cgtcgtttat tggccgacgg
1321 ctcgctcata tgacggcctc cttcgccacc caactaacct ttaacccaca caccgttggc
1381 gacgaagcag ggttactcgc cgtacaaaac gacgaacact tttacgcctt tggcctaggg
1441 ttaaacagta aagggcaaac cgttttgcgc gtgcgtaaaa aagcgggtaa aaatgaatcg
1501 ataaggggag atacggttgc cgagcaggtt gttaagctta agcacggcca ccctatttac
1561 ctgcgtgtaa atataggtaa agccgaatta aatttcgcgt atagcaccaa cggcaaacgc
1621 tacaccacct tgttgaacca agccgatgcc aacctactta ccacagctaa agcgggcggg
1681 tttactggcg cagtagtggg tatgtacgcc gaatccaccg cacaacaaaa ctaa

LOCUS NZ_AAB102000011 1701 bp DNA linear BCT 23-SEP-2003
DEFINITION Microbulbifer degradans 2-40 Mdeg_9, whole genome shotgun sequence.
ACCESSION NZ AABI02000011 REGION: 31082..32782
VERSION NZ_AABI02000011.1 GI:28878291
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1701)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028020. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1701
      /organism="Microbulbifer degradans 2-40"
      /mol_type="genomic DNA"
      /strain="2-40"
      /db_xref="taxon:203122"
   gene 1..1701
      /gene="Mdeg 1856"
   CDS 1..1701
      /gene="Mdeg 1856"
      /codon_start=1
      /transl_table=11
      /product="COG3507: Beta-xylosidase"
      /protein_id="ZP00066469.1"
      /db_xref="GI:23028051"
      /db_xref="COG:COG3507" 
/translation="MRLLPILLVSLLPLLSSCTSAINGQQNSQTSPVFDWFEYAGSDA
LYNTVAPSKNAYTNPVIKGFYPDPSIVRVGADYYLVNSSFGYFPGVPIFHSTDLVNWV
QIGNILERPSQLQIPSGMGVSRGIFAPTLRHHNGIFYMITTMVDGGGNFIVTAKNPAG
PWSDPVWLPEVGGIDPDLFFDDNGKAYILNNDAPIGEPLYDGHRAIWIREFDLATLKT
VGDAKLIVNGGVDITTKPVWIEGPHLFKNKGAYYLINAEGGTSVNHSQVVFKAQSPWG
PYIPWENNPILTQRHLPADRANPVTSVGHVDLVQTQHGDWWAVFLGCRPYKDNYYNTG
RETFLLPVDWSGEYPVILRGDAEVPYHHQRPQLGASQQPAIALSGNFIERDEFDSALK
LYWRKVRTPTNNFTDLTSQKGKLVLTANNTDLSDFGSPAFIARAQQHLTGSATTKLVY
TPPHVGDKAGIAAFQNDEYFYALTVTKNNSGLAIQLEKQLGKNKEIVAQYPLQEKTLR
NGLYLKIEFNNDKYDFSYSTNNTKWQSVGETQDGTILSTQSAGGFVGATLGIFAYTAH
"
ORIGIN 
1 atgcgacttt tacctatctt actcgttagc ttacttccac tgctctcaag ctgcacaagc
61 gccataaacg ggcaacaaaa tagccaaacc tcgcctgtat ttgattggtt tgaatacgcg
121 ggaagcgatg ctttatacaa cacggttgcg ccaagtaaaa atgcctatac caacccagta
 
181 ataaaagggt tttatccaga tccaagcatt gtaagagtgg gagcagatta ctacctcgtg
241 aactcttcat ttggctactt ccctggcgtg ccgatatttc atagcacaga tttagtgaat
301 tgggttcaaa taggtaatat tctcgagcgc ccatcacaat tacaaatacc cagcggcatg
361 ggtgtgtcgc gaggtatatt cgccccaaca ctgcgccacc acaacggtat tttttacatg
421 attactacaa tggtagacgg tggcggcaat tttattgtta ctgcaaaaaa ccccgcaggc
481 ccttggtcgg acccagtatg gttacctgaa gtgggcggta tagacccaga tttatttttt
541 gatgacaacg gcaaagccta catacttaac aacgacgccc ccattggcga gccgctttac
601 gatggccacc gagccatttg gattcgcgaa ttcgacttag ccacattaaa aaccgttggc
661 gacgccaagt taatagtaaa cggcggtgta gatataacta ccaaacccgt ttggatagaa
721 ggcccacacc ttttcaaaaa taaaggcgct tactatttaa ttaatgcaga aggtggcacc
781 agcgtgaatc acagccaagt tgtatttaaa gcgcaaagcc cttgggggcc gtatattcct
841 tgggaaaaca atccaatttt aacacagcgc catttaccgg ctgatcgcgc caaccccgtc
901 acatccgttg gccatgtcga tttagtacaa actcaacatg gcgactggtg ggcggtattt
961 ttaggctgca ggccctataa agataactac tacaataccg gccgcgaaac atttttatta
1021 ccggtagatt ggtctggcga ataccccgtc attcttcgcg gcgatgccga ggtgccctat
1081 catcaccaac gcccccaatt gggagcatcc caacaaccag ccattgccct tagcggtaac
1141 tttattgagc gcgatgaatt tgactcagca cttaaacttt attggcgcaa ggttcgcacc
1201 cccacaaaca actttacaga tttaacctct caaaaaggca agcttgtttt aactgcaaac
1261 aatacagatt taagcgactt tggatcacca gcatttattg cgcgcgcaca gcagcaccta
1321 acaggcagcg caacaaccaa actggtttac acacccccac acgtgggcga caaagcgggt
1381 attgctgcct ttcaaaacga tgagtatttt tatgcgctta ccgttacaaa aaataatagc
1441 ggccttgcca tacaactaga aaaacaactt ggcaagaaca aagaaattgt tgcgcaatat
1501 ccactacaag aaaaaacgct tcgcaatggc ttatatttga aaatagaatt taataacgac
1561 aaatatgatt tcagctattc cacaaataac accaagtggc aatcggtagg cgaaacacaa
1621 gatggaacta tattaagcac gcaaagtgca ggcgggtttg taggtgccac gctaggtata
1681 tttgcatata ccgcgcacta a
LOCUS NZ_AABI0200001 4 960 bp DNA linear BCT 23-SEP-2003
DEFINITION Microbulbifer degradans 2-40 Mdeg_13, whole genome shotgun sequence.
ACCESSION NZ AABI02000014 REGION: 125907..126866
VERSION NZ_AABI02000014.1 GI:28878288
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 960)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028504. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..960
      /organism="Microbulbifer degradans 2-40"
      /mol_type="genomic DNA"
      /strain="2-40"
      /db_xref="taxon:203122"
   gene 1..960
      /gene="Mdeg2391"
   CDS 1..960
      /gene="Mdeg2391"
      /codon_start=1
      /transl_table=11
      /product="COG3507: Beta-xylosidase"
      /protein_id="ZP00066992.1"
      /db_xref="GI:23028599"
      /db_xref="COG:COG3507" 
/translation="MSMFNKKTLAAGIVAACLTNVSASYAANPAITDTHTADPAALVH
GDTVYLYVGNDEAKDNRVFYDLKKWLVYSSKDMVNWTNHGSPLAATDFKWASGDAWAA
HTVEKDGKFYWYTTVRHATINGFAIGVAVSDSPTGPFKDALGKALISNDMTTDTDIDW
DDIDPAVFIDDDGQAYIFWGNTKPRWAKLKPNMIELDGPIHAIDIPHFTEALYVHKHG
EYYYLSYATGFPEKTAYAMSKSIEGPWEYKGILNELAGNSNTNHQSVIDFKGKSYFIY
HNGGLGQDGGSFRRSVCIDYLNYNADGTIKRIVMTSEGVDPVK"
ORIGIN 
1 gtgagtatgt ttaataaaaa aacactagca gccggtattg tagctgcatg tttaactaac
61 gtaagtgcaa gctatgctgc caaccccgca attaccgata ctcacacggc cgatcccgct
121 gcgttagtgc acggcgatac cgtttatttg tacgtgggta acgatgaagc gaaggataac
181 cgcgtatttt acgatcttaa aaaatggttg gtgtattcat caaaagatat ggtgaactgg
241 accaatcacg gttcgccgtt agctgcaacg gattttaagt gggccagcgg cgatgcgtgg
301 gcggcgcaca cggtagaaaa agatggcaag ttttattggt ataccacggt gcgtcacgca
361 accattaatg gttttgccat tggcgttgca gtaagtgata gccctacagg gccattcaaa
 
421 gatgctttgg gtaaagcact aataagtaat gacatgacca ccgataccga tattgattgg
481 gacgatatag acccagcagt atttattgac gacgatggcc aagcgtatat tttttggggc
541 aacaccaaac cgcgctgggc caagttaaaa cccaatatga ttgaactaga tggacctatt
601 cacgcaatcg atattccaca ctttaccgaa gcgctatacg tgcacaaaca cggtgaatat
661 tactacttaa gctatgcgac aggctttcca gaaaaaacag cttacgctat gagcaaatct
721 atagaagggc cgtgggaata caaaggcatt cttaatgaat tggctggtaa ctcaaatact
781 aatcaccaat ctgtcatcga ttttaagggc aagtcatact ttatttatca caatggtggc
841 ttgggtcaag atggcggtag cttccgtcgc agtgtatgta tcgattattt gaactacaac
901 gcggatggta ctatcaagcg aattgtaatg acatcagaag gtgtagaccc agttaaataa
LOCUS NZ_AABI03000012 1158 bp DNA linear BCT 17-JUN-2004
DEFINITION Microbulbifer degradans 2-40 Mdeg03_12, whole genome shotgun sequence.
ACCESSION NZ AABI03000012 REGION: complement(71021..72178)
VERSION NZ_AABI03000012.1 GI:48861146
KEYWORDS WGS.
SOURCE Microbulbifer degradans 2-40
ORGANISM Microbulbifer degradans 2-40
   Bacteria; Proteobacteria; Gammaproteobacteria; Alteromonadales;
   Alteromonadaceae; Microbulbifer.
REFERENCE 1 (bases 1 to 1158)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbl.nlm.nih.gov/sutils/genom table.cgi.
   URL -- http://www.jgi.doe.gov
   Contact: Paul Richardson
   microbes@cuba.jgi-psf.org.
FEATURES Location/Qualifiers
source 1..1158
   /organism="Microbulbifer degradans 2-40"
   /mol_type="genomic DNA"
   /strain="2-40"
   /db_xref="taxon:203122"
gene 1..1158
   /locus_tag="Mdeg02003585"
CDS 1..1158
   /locus_tag="Mdeg02003585"
   /codon_start=1
   /product="COG3507: Beta-xylosidase"
   /protein-id="ZP 00315109.1"
   /db_xref="GI:48861205"
   /db_xref="COG:COG3507" 
/translation="MPEHTRKRLLSTLGLALSGTAITLTLVGCGKDNPATQTEGSHSA
GHTEVAAEQTHDIGGPGPEGKPINDPLVTHIYTADPSAHVFDGKLYIYPSHDVEAGIP
QNDNGDHFDMRDYHVLSMEEPGGKVTDHGVALAREDVAWAGRQLWAPDAAEKDGTYYL
YFPMKDKDDIFRIGVASGSTPYGPFKAEPEPMPGSYSIDPSVFQDGDDYYMYIGGIWG
GQLQRWTTGEYNPEDVYPADDEPALLPKMAKLSADMKSFAEPLRDIQILDENGELIKA
GDNDRRFFEAAWVHKYNGKYYLSYSTGDTHYIVYAIGDNPYGPFTYQGVVLNPVIGWT
NHHSIAEFKGKWYLFYHDSSLSGGVTHLRSVKMTELTHNPDGTIQTINAYK"
ORIGIN 
1 atgccagaac atacgcgtaa gcgcttacta tcaaccttag gcctagcttt atcgggcaca
61 gctataaccc taacgcttgt ggggtgcggt aaagacaacc ccgcaactca aacagaaggc
121 agccacagcg ctggccatac agaagttgcc gcagaacaaa cacacgacat aggcggccca
181 ggccctgagg gcaagccaat taacgacccg cttgttaccc acatatacac cgcagaccct
241 tctgcccatg tgtttgacgg caaactttat atttacccat cgcacgatgt ggaagcgggt
301 attccgcaaa acgataacgg cgatcacttc gatatgcgcg attatcacgt gctttccatg
361 gaagagcctg gtggcaaagt caccgatcac ggcgtagccc ttgcgcgcga agatgtagct
421 tgggctggtc gccaactgtg ggcgcccgat gcggctgaaa aagacggcac ttactacctg
481 tatttcccca tgaaagataa ggatgacatc ttccgcattg gtgtcgccag tggcagtacc
 
541 ccttatggcc catttaaagc cgagccagag ccaatgcccg gcagctatag catagaccca
601 agcgtatttc aggatggcga cgactactac atgtacatag gtggtatttg ggcggccag
661 ttgcagcgtt ggacaaccgg tgagtacaac ccagaagatg tatacccagc ggatgacgag
721 cctgcgctat tacctaaaat ggccaagcta agtgcagata tgaaaagctt tgccgagcca
781 ttaagagaca ttcaaatttt ggatgaaaat ggcgagctaa ttaaagctgg cgataacgac
841 cgacgtttct tcgaagccgc gtgggtacac aaatataacg gcaagtatta cttgagctat
901 tcaaccggtg acacccacta tattgtgtat gccattggcg ataacccata cggcccgttt
961 acttaccagg gtgtagtgct caaccccgtt attggttgga ctaaccatca ctcaattgct
1021 gaatttaaag gtaagtggta tttgttctac cacgatagtt cgctttccgg tggtgtaaca
1081 catttgcgca gcgtgaaaat gacagagcta actcacaacc cagatggcac tatccaaacc
1141 attaatgcct ataagtaa
LOCUS NZ_AABI03000013 2217 bp DNA linear BCT 17-JUN-2004
DEFINITION Microbulbifer degradans 2-40 Mdeg03_13, whole genome shotgun sequence.
ACCESSION NZ AABI03000013 REGION: complement(100192..102408)
VERSION NZ_AABI03000013.1 GI:48861059
KEYWORDS WGS.
SOURCE Microbulbifer degradans 2-40
ORGANISM Microbulbifer degradans 2-40
   Bacteria; Proteobacteria; Gammaproteobacteria; Alteromonadales;
   Alteromonadaceae; Microbulbifer.
REFERENCE 1 (bases 1 to 2217)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/lgenom table.cgi.
   URL-- http://www.jgi.doe.gov
   Contact: Paul Richardson
   microbes@cuba.jgi-psf.org.
FEATURES Location/Qualifiers
   source 1..2217
      /organism="Microbulbifer degradans 2-40"
      /mol_type="genomic DNA"
      /strain="2-40"
      /db_xref="taxon:203122"
   gene 1..2217
      /locus_tag="Mdeg02003703"
   CDS 1..2217
      /locus_tag="Mdeg02003703"
      /codon_start=1
      /product="COG3661: Alpha-glucuronidase"
      /protein_id="ZP 00315039.1"
      /db_xref="GI:48861134"
      /db_xref="COG:COG3661" 
/translation="MATLGVNAAKFAMFAAICLQFSVAEAAKSRDGYGLWLDYQPITN
TREREGYIKALSPWQVEGEAATADFIRQELTAALGAMLGVEAGPVGDYTHNSLAHPVA
RLLVATPEESAVIRSLALGDALTRVGQEGYLIKTTRYRDKPITIVTANTHAGLLYGTF
KLLQLLQTGQAVSNLAIESAPATKLRVLNHWDNLDRYVERGYAGESIWNWHKLPHYKS
QRYYDYARANASIGINGVVLNNVNADPLILTPQYLVKVKALADIFRPYGIKVYLSVKF
SSPNLIGGLPTSDPLDKNVQAWWQAKANEIYSLIPDFGGFLVKANSEGQPGPGDFGRS
HAQGANMLADALAPHGGNVMWRAFVYNVEANVERSKQAYNEFKPLDGTFRQNVLVQVK
NGPIDFQPREPFSPLFGAMPKTPLMMEFQITQEYLGFSTHLVYLGPLYEEVLKADTYA
KGAGSTVAKVVDGSLYGHGITGMAGVANIGSDRNWTGHIFGQANWYVFGQLAWNPEVS
TKQIADDWIRMTLTRDDKAVNTIRAMMMASRETAVNYMTPLGLHHIMGWGHHYGPAPW
IGEQKPDWMREDWTSVYYHSANATGLGKDRTASGSNVIAQYHAPLRQAYSDPKTTPTE
LLLWFHHLPWHYELANGNSLWHELVARYYLGAQAVAEMAKTWDGLEANIPPQLFKQVQ
MALAIQTQEAAWWRDACVLYFQSYSKQSLPEGFAKPKHSLEYYKGLSFPHAPGDGR"
ORIGIN 
1 atggctactt tgggggtaaa tgccgctaag tttgccatgt ttgcagctat ttgcttgcag
61 tttagtgtcg ccgaagcggc taaaagccgc gatggatatg ggctgtggtt agattaccag
121 ccaattacca atacccgcga acgcgagggc tatataaaag cattaagccc atggcaggta
 
181 gaaggcgaag ctgcaactgc cgattttatt cggcaagagc ttactgcagc gttgggcgct
241 atgcttggcg ttgaggctgg tccagtgggt gattacaccc ataactccct cgctcaccct
301 gtggcgcggc tattggttgc aactccagaa gaaagcgctg ttattcgctc tttggcttta
361 ggcgatgctt taactcgagt agggcaagag gggtacctta ttaaaaccac gcgttaccgt
421 gacaagccta tcaccattgt taccgcgaac acgcatgcag gcctgctgta tggcacattc
481 aaactactgc agctgctgca aacagggcag gccgtttcta atttagctat tgagtccgcc
541 ccagcaacca aactgcgtgt gcttaaccac tgggataacc tcgatcgcta tgtggagcgc
601 ggctatgccg gtgagtctat ttggaactgg cacaagctgc cgcactacaa atcgcagcgc
661 tactacgatt acgctcgcgc taacgcgtcc attggtatta acggtgtggt actaaacaat
721 gttaacgccg accccttaat tcttaccccg cagtaccttg taaaagtaaa agcactggca
781 gatattttta ggccctacgg cattaaagtt tatctttcgg tgaagtttag ctcgccgaat
841 cttattggcg ggctgccaac atccgacccg ttagataaaa atgtgcaagc ttggtggcaa
901 gcgaaagcga atgaaattta ctcgctcatt cccgactttg gtggcttttt agtaaaagcg
961 aattcggaag ggcagcccgg cccaggggac tttggccgca gccatgcaca aggggcaaat
1021 atgttggccg atgcactggc accccatggc ggcaatgtaa tgtggcgcgc gtttgtatat
1081 aacgtagaag ccaatgtgga gcgatccaag caggcataca acgaatttaa gccattagac
1141 ggtaccttta ggcaaaacgt attggtgcaa gtaaaaaatg ggccaattga ttttcagcca
1201 cgtgaaccgt ttagcccgct gtttggtgct atgcccaaaa cgccgttaat gatggagttt
1261 caaattactc aggagtactt ggggtttagt actcaccttg tttacttggg gccgctgtac
1321 gaagaagtac ttaaggccga tacctatgcg aagggggcag ggtctactgt tgcgaaggtg
1381 gtcgatggct cgctctacgg gcacggtata acgggtatgg ctggggtagc taatattggc
1441 agcgatcgca attggaccgg ccatattttc ggccaagcca actggtatgt atttggccaa
1501 ttggcgtgga accccgaggt aagcactaag caaatagccg atgattggat tcgcatgaca
1561 ctcacccgcg acgataaagc ggtaaacacc attcgcgcaa tgatgatggc cagccgcgaa
1621 acggcggtta actacatgac gcccctgggg ctgcatcaca ttatggggtg gggcaccac
1681 tacggcccag cgccgtggat aggcgagcaa aaacccgatt ggatgcgtga agattggaca
1741 tctgtttact atcatagcgc aaacgccaca gggctaggca aagatagaac agcttctggc
1801 agcaatgtca tagcgcaata ccacgcccct ttacggcagg cctatagcga cccgaaaacc
1861 acgcccaccg agttgctatt gtggtttcat catttgcctt ggcattatga attagcgaat
1921 ggcaatagcc tgtggcatga actggtagcg cgttactatt taggcgcgca ggctgtggca
1981 gaaatggcca aaacgtggga tggcctagaa gctaatatcc ccccgcagct attcaaacaa
2041 gtacaaatgg cgctggctat tcaaacccaa gaagccgcgt ggtggcgcga tgcctgcgtg
2101 ctgtattttc aaagctattc taagcagtcg ctacccgagg gctttgcaaa acctaagcac
2161 tcgctcgaat actataaagg gttaagcttc ccgcatgcgc cgggtgacgg gcgttaa
LOCUS NZ_AABI02000026 3951 bp DNA linear BCT 23-SEP-2003
DEFINITION Microbulbifer degradans 2-40 Mdeg_26, whole genome shotgun sequence.
ACCESSION NZ AABI02000026 REGION: complement(19875..23825)
VERSION NZ_AABI02000026.1 GI:28878276
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3951)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029482. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jqi.doe.gov
   Contact: Paul Richardson
   microbes@cuba.jgi-psf.org.
   COG assignment was automatically updated on Sep 23, 2003.
FEATURES Location/Qualifiers
   source 1..3951
      /organism="Microbulbifer degradans 2-40"
      /mol_type="genomic DNA"
      /strain="2-40"
      /*db*_xref="taxon:203122"
   gene 1..3951
      /gene="Mdeg3247"
   CDS 1..3951
      /gene="Mdeg3247"
      /codon_start=1
      /transl_table=11
      /product="COG3866: Pectate lyase"
      /protein_id="ZP00067832.1"
      /db_xref="GI:23029498"
      /db_xref="COG:COG3866" 
/translation="MRNKLGSMLKMSAAIGGLVAAGSAVAGPVGFASLNGGTTGGAGG
QVVYASTGAEINQAMCNRASDDTPLIIYVTGTINHGNTAKYSGSCDTTADEIQFKGVK
NISLIGTGSGAVFDQIGIHLRDTSNIILQNLHIKNVKKSGSPTSNGGDAIGMESGVYN
VWVDHCELEASGGESDGYDSLLDMKATTQYVTVSYTYYHDSGRGGLMGSSDSDDTNTF
VTFHHNYYENMDSRLPLLRHGTAHAFNNYYNGIAKSGMNPRIGGQIKAENNYFENAHN
PIGTFYTDDMGYWDLRGNIFGSNVTWASADDETPAGPNPTSTTSIHISYPYDLDDAAC
VPDIVKSTAGVGTGLAVSDGSCTITTPPSTSSSSSSSSSTSSTGSSSSSSSSSSSSSS
SNGGSLVLGNNLSIGAGSDGSSKGAGSYGNVRDGDVSSYWAPSGSTGRVSIKWSGSQT
VNAIVIKEAAGYEGNISGWQVTDNDTGAVLAAGSSVGTITFDAVTTSKINFEITSSNG
TPTVAEFETYNATGSSSSSSSSSSSSSSSSSSSSSSSSSSSSSTGGTATLSTTVSGDQ
VTLNWSVNNATVTGQQIYRDVDSDPAGRVRIASGVTGNTYTDTGLANGTYYYWVKVTD
SNSATINSNYSEAQVNVYTTSTTTFEEDAGYCSVDGSVDSNNSGFAGSGFANTDNASG
NGVNYAVSVPVAGVYTLQVRFANGSSARPADVLVNYGNAGVFDLPSTGSWTSWSNSNE
ISVNLVAGNNIIRLEATTSGGLANIDSLSVTGVEPSAGDCNGSVGSSSSSSSSSSTSS
 
TSSSSTSSGGSSTSSSSTSSSSTSSTSSSSTSSSSTSSTSSSSGGGTASCEQLINDPS
VNWDESALASEQEIVACLAQSLGSPVGFGEGTTGGYDPSGGSNLVVIKKNIGISVEQQ
ILDAISTENHNWIVFDKDDFAARTAVAMYRLDCDNADVRSALGGASAAQCRDHIAWCS
ANGISDEHDCENEFFNNRLNDSDLPIRNQMIQSNTTIDGRGANAYFFFNGFSIGKDSS
GASLYAAQNVIVTNNEFIGAGHTEDHDLDPDMIRSTGESNKIWIHQNTFDHTGDSAFD
VKVGAYDITISFNKLVNVKRAALHGSSDSRAINSQITTTMHNNLFYTSDDQYALSTYD
TLRRVPLMRRGQSHMFNNVFYGYRKDILSVRVGGRIAFEDNIILNKESSSTPGDGLKK
GDDMEYYVETLLRDFREGGLEISGSYVSFADSACNSYGASGDLTASHGATPDMFDDYS
SASKNTISANRFVAGDDLTDYVFATAGKGGKAPYVSTFTAGQNSLISQANPVCQ"
   gene complement(1383..1664)
      /gene="Mdeg3248"
   CDS complement(1383..1664)
      /gene="Mdeg3248"
      /codon_start=1
      /transl_table=11
      /product="hypothetical protein"
      /protein_id="ZP00067833.1"
      /db_xref="GI:23029499" 
/translation="MPPVELLDELLDEELDELLDEELEELELLEELEPVALYVSNSAT
VGVPLEEVISKLILLVVTASNVIVPTLEPAANTAPVSLSVTCQPLILPS"
ORIGIN 
1 atgagaaata aattaggctc aatgttaaaa atgagcgcag ccattggcgg tttagttgca
61 gcgggttccg ctgttgcagg cccagttggt ttcgcaagtt taaacggcgg cactaccggc
121 ggcgcgggcg ggcaagttgt atatgctagc accggtgctg aaattaacca ggctatgtgt
181 aatcgcgcaa gcgacgatac accgctaatt atttatgtga cgggtaccat taaccacggt
241 aacaccgcca agtattctgg tagctgcgat accactgcag atgaaattca gtttaaaggt
301 gtaaaaaata tatcgttgat aggaacgggc agcggtgctg tgttcgatca aatcggtatt
361 cacctacgcg atacctcgaa tattattttg caaaatttgc atattaaaaa cgttaagaag
421 tctggttcgc ctacttcgaa tggcggtgac gctattggta tggaatctgg cgtatacaat
481 gtgtgggtag accactgtga gctagaagct tcaggcggtg aaagtgatgg atatgattca
541 ttgctagata tgaaagccac cacgcagtat gtaacggttt cttacactta ctatcacgat
601 tctggtcgcg gtggtttaat ggggtctagt gatagcgacg ataccaatac cttcgtcacc
661 ttccaccaca actactacga aaatatggat tcgcgcttgc cgttactgcg tcacggtaca
721 gctcatgcat ttaacaacta ctataatggt attgctaaat ctggcatgaa cccacgtata
781 ggtgggcaaa taaaagcgga aaacaattac ttcgaaaatg cgcacaaccc aattggtact
841 ttttatacag acgatatggg ttactgggac ttacgcggca atatatttgg cagtaacgta
901 acgtgggcgt ctgcggatga tgaaacccct gcaggcccga acccaacatc cactacgtct
961 attcatattt cttaccccta tgatctagat gacgctgctt gtgtgcctga tattgtaaaa
1021 tccacagcag gtgtgggtac tggcctagcg gtttcagacg gaagctgcac cataacaacg
1081 ccaccttcaa cgagttcgtc tagctccagt tctagctcaa cctcgtcgac tggttcgagt
1141 tcgtcttcaa gctcttcctc ttcaagcagc tctagctcca atggcggcag cttagtatta
1201 ggtaacaacc tttcaattgg tgctggctct gatggtagta gcaagggagc aggttcgtac
1261 ggcaatgtgc gcgatggcga tgtaagtagc tattgggcgc cgagtggcag tactggtcgt
1321 gtttcaatta aatggagcgg cagccaaact gttaacgcta ttgttattaa agaagcggca
1381 ggctatgaag gtaatattag tggttggcaa gtaactgata acgataccgg tgcagtattg
1441 gctgctggct caagcgtagg cacaattacg tttgatgcgg taacgactag caagatcaat
1501 ttcgaaatta cttcttctaa cggtacacca acggtagcgg aattcgaaac atataatgct
1561 acaggttcta gctcttcaag cagctcgagt tcttctagct cttcatcaag tagctcgtct
1621 agttcttcat cgagcagttc gtctagtagt tctacaggcg gcaccgctac tttaagtaca
1681 acggtttctg gcgatcaagt aacgttgaat tggagcgtaa ataatgcaac cgtaactggt
1741 cagcaaattt atcgcgatgt ggattcagac ccagctggcc gtgtgcgcat tgcatccggt
1801 gtaactggaa atacttacac agataccggt ttggctaacg gaacttatta ctactgggta
1861 aaagtaaccg attcaaactc agctacaatt aattccaact actcagaagc gcaagtgaat
1921 gtttatacaa catctactac aacgtttgaa gaggatgcgg gttattgctc ggtagacggt
1981 tcagtagata gcaataacag tggctttgct ggcagtggtt ttgctaatac cgataatgct
2041 tcgggtaatg gcgtaaacta cgcggtaagc gtacccgttg ctggtgtgta cacgctgcaa
 
2101 gtgcgttttg ctaatggctc aagtgcacgt cctgctgatg tgttagtgaa ctatggtaac
2161 gccggtgtat ttgatctgcc tagcacaggt tcttggacca gctggagcaa ctcaaacgaa
2221 attagcgtta acttagttgc tggcaataat attattcgtt tagaggctac cacgtctggc
2281 ggcttggcga atattgatag cctatctgta acgggtgtag agccttctgc aggtgactgt
2341 aacggtagtg ttggttctag cagttctagt tcttccagct cttctacttc tagcaccagt
2401 tcatctagca ctagctctgg tggcagctct actagctcaa gctcaacgtc tagctcatct
2461 acaagctcta catctagtag ttcaaccagc tctagcagca cgtcttctac ctcaagctct
2521 tcgggcggtg gtacggcaag ttgtgagcag ttgattaacg atccaagtgt taactgggat
2581 gagtctgcac tggcttcaga gcaagaaatt gtagcctgtt tggctcagtc tctaggtagc
2641 cctgttggct ttggggaagg tactaccggt ggttacgatc caagtggcgg cagcaacctt
2701 gttgttatta aaaagaacat aggtatttct gttgagcaac aaattttgga tgctataagc
2761 accgaaaacc acaactggat tgtgttcgac aaagatgatt ttgctgcgcg cactgcggta
2821 gcgatgtatc gcttagattg tgacaatgcc gatgtgcgtt cagcattggg tggcgcaagt
2881 gctgcacaat gtcgcgatca tatagcttgg tgttctgcta atggtatttc tgacgagcat
2941 gactgtgaaa atgaattctt taacaaccgt ttaaatgatt cagatttgcc aatccgcaat
3001 caaatgattc agtcaaacac taccattgat ggtcgtggtg caaacgcata cttcttcttt
3061 aatggtttct ccattggtaa agatagcagt ggtgcaagct tgtacgcagc gcaaaatgtg
3121 attgtaacga ataacgagtt tattggtgcc ggtcacactg aagatcacga tctagaccca
3181 gatatgattc gatctactgg cgaatcgaac aaaatttgga ttcaccaaaa cacgttcgac
3241 catactggtg attctgcgtt tgacgtaaag gtgggtgctt acgatataac aatatcattc
3301 aataagttgg tgaacgtgaa gcgtgctgcg ctacatggtt caagtgatag ccgagcaatt
3361 aactcgcaaa tcacaaccac tatgcacaac aacctgttct atacttcaga tgatcaatac
3421 gcgctaagta cctacgacac tttgcgtcgt gtaccgctaa tgcgtcgcgg tcaatcacac
3481 atgtttaaca acgttttcta cggttaccgt aaagatattc taagcgtgcg tgttggcggt
3541 cgtatcgcct ttgaagataa cattattttg aataaagaaa gcagctctac cccaggtgat
3601 ggcctgaaga aaggcgacga catggaatac tatgttgaaa ccttgttgcg cgacttccgt
3661 gagggtgggt tagaaattag cggtagctat gtatcgtttg cagatagcgc ttgtaattcc
3721 tatggcgcat cgggtgactt aaccgcatcg catggtgcta cgccagatat gtttgatgat
3781 tacagctctg catctaaaaa tactatatca gccaatcgct ttgttgctgg cgatgactta
3841 actgactatg tatttgctac tgcaggtaag ggcggtaaag cgccttatgt ttccaccttt
3901 actgctgggc aaaatagcct tatttcacag gctaacccag tttgtcagta g
LOCUS NZ_AABI02000026 1284 bp DNA linear BCT 23-SEP-2003
DEFINITION Microbulbifer degradans 2-40 Mdeg_26, whole genome shotgun sequence.
ACCESSION NZ AABI02000026 REGION: complement(32631..33914)
VERSION NZ_AAB102000026.1 GI:28878276
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1284)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029482. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1284
      /organism="Microbulbifer degradans 2-40"
      /mol_type="genomic DNA"
      /strains="2-40"
      /db_xref="taxon:203122"
   gene 1..1284
      /gene="Mdeg3255"
   CDS 1..1284
      /gene="Mdeg3255"
      /codon_start=1
      /transl table= 11
      /product="hypothetical protein"
      /protein_id="ZP 00067840.1"
      /db_xref="GI:23029506" 
/translation="MNKNNVIAYLLISTFLLFSATVFAVKPSNAETRYSAMGADTPAG
LGGTLPDGQSRIVRVTNLNASGEGSLAWALGLARPRVVVFEVGGVIDLAGQSITVTQP
FLTVAGQSAPAPGITLIRGGLNIRTHDVRVQHIRVRPGDNLQPKRSGWESDGISVAGE
NAKDVHIDHVSVSWAVDENLSASGNRYKGYGQTAERVTFSNNLIAEALDYASHKKGKH
SKGLLVHDYVRDVAVVRNLFVSNDRRNPYFKAHTIGFVANNIIYNAGNAAIQVNYIER
EWEGQSTGPANARVAVVNNQLVYGRDTYSDLALVSVRGDAYLTGNSVTNLMGEPMPIT
EGAVNSLASAPSWLTGYELWDADEMRELLVASVGATPWARDAIDTRIINGVATGKARI
IDSQQDVGGYPSYKQTNKKFDIPDDKIAEWLLGYL"
ORIGIN 
1 atgaataaaa ataatgtaat tgcttatctg ctaatttcaa cttttctatt attttctgcg
61 actgtgttcg cggttaaacc cagcaacgct gaaacccgat attccgcaat gggtgcagat
121 accccagcag gtttgggagg cactttgcca gatggtcagt ctcgtatcgt tagggtgact
181 aatttaaatg caagtgggga gggctcgctc gcatgggcgc tgggtttagc tcgaccacgc
241 gtagtggtgt tcgaagttgg cggtgttata gatcttgctg ggcaaagtat taccgtcacg
301 cagccattcc ttactgttgc cggtcagtcg gcgccagcac cgggtattac attaattcgc
 
361 ggcggtttaa atatacgaac ccacgatgta agagtgcagc atattcgcgt gcggccggga
421 gataacttac aaccaaagcg ctccggctgg gaaagtgacg gtatatctgt ggccggtgaa
481 aatgccaaag atgtacatat agatcatgta tcggtaagtt gggcggtaga tgaaaacctc
541 tccgcttcgg ggaatcgtta caaaggttac ggtcaaaccg ctgagcgggt aacgtttagt
601 aataatctca ttgccgaagc gttagattat gccagccata aaaaaggcaa acactctaag
661 ggattattgg tacacgatta tgtgcgagat gttgccgtag ttagaaattt gtttgtgtct
721 aatgatcgtc gcaacccgta ctttaaagcg cacaccatag gttttgtagc aaataatatt
781 atttacaatg cgggtaatgc cgctatacag gttaactata ttgagcgtga gtgggagggc
841 cagagtacag gcccagctaa tgctagagta gcggtggtaa ataaccagtt agtttacggc
901 cgcgatacat actcagactt ggcgctagtg tctgtgcgtg gggatgctta tttgacgggt
961 aatagcgtta caaatttaat gggcgagccc atgcctatta cggaaggggc ggttaattct
1021 ttagcctctg caccttcatg gttaacaggt tatgagttgt gggatgctga cgagatgcgt
1081 gagctgctag tagccagtgt tggtgcaaca ccctgggcca gggatgcgat agatacccga
1141 ataattaatg gggtggcaac ggggaaggcg cgaataatag atagccagca agatgtgggt
1201 ggctacccga gctataagca aacaaataaa aaatttgata taccagacga caaaattgcc
1261 gaatggttac tgggttacct gtaa
LOCUS NZ_AABI02000026 2310 bp DNA linear BCT 23-SEP-2003
DEFINITION Microbulbifer degradans 2-40 Mdeg_26, whole genome shotgun sequence.
ACCESSION NZ AABI02000026 REGION: complement(24098..26407)
VERSION NZ_AABI02000026.1 GI:28878276
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2310)
AUTHORS NCBI Microbial Genomes Annotation Project
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029482. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL-- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2310
      /organism="Microbulbifer degradans 2-40"
      /mol_type="genomic DNA"
      /strain="2-40"
      /db_xref="taxon:203122"
   gene 1..2310
      /gene="Mdeg3249"
   CDS 1..2310
      /gene="Mdeg3249"
      /codon_start=1
      /transl_table=_11
      /product="COG3866: Pectate lyase"
      /protein_id="ZP00067834.1"
      /db_xref="GI:23029500"
      /db_xref="COG:COG3866" 
/translation="MRNTKHLLNSGAVLLASSIATAAMAGPVGFASLNGGTTGGQGGQ
VVYANTGTQINEAMCNRPSHDTPLIIYVSGTINHGNTEKVSGNCDTTGDEIQFKKVKN
LSLIGTGNGAVFDQIGIHLRETSNIILQNLHIKNVKKSGSPTSNGGDAIGMESGVYNV
WVDHCELEASGGEKDGYDSLLDMKATTQYVTVSYTYYHDSGRGGLMGSSDSDDTNTYV
TFHHNYYKNMDSRLPLLRHGTAHAFNNYYDGITKSGMNPRIGGQIKAENNYFENAHNP
IGTFYTNDMGYWDLSGNIFGNNVTWASADDETPAGPNPQSTTSIHISYPYSLDDATCV
PKIVKATAGVGNGLAVSTGGSNCGTSSSSSSSSSSSTSSTSSTSSSSSSSNSSSGGS
GVNLSIGAGSDGSSKGAGSYGDVRDGNMSTYWAPSGSTGRVSIKWSSATTVSSIVIKE
AAGFEGNITGWQVVNNENGAVLKSGSNAGVISFSPVSTTKLNFEITSSNGMPTVAEFE
TYSGTVGGTSSSSSSSSSSSSSSSSSSSTSSSGGSANLGTSVSGDQVSLNWSTSNIDV
GSQQVYRDTDSNPSGRVRISAGVSGNSYTDYGLASGTYYYWIKITDQNGVVYNTNAAE
AVVGSQAPTTFTAQESAGFCSVNGSVDSNNAGYTGDGFVNTDNASGNAAVYAFNAPSA
GMYSLQVRYANGSSARPGDVLVNAGNIGTFDFSSTGSWTSWANSNELSAYFSAGNNTV
RIQATNSGGLPNLDSVSVTGNAPAAGNCN"
ORIGIN 
1 atgagaaaca ctaaacacct actcaatagc ggtgccgtat tgctagctag cagtattgca
61 acagctgcga tggcggggcc tgtgggcttt gcatcgctta atggtggcac aacggcggt
121 caaggcggtc aagttgtata cgccaacacc ggtacacaaa ttaacgaagc catgtgtaac
181 cgcccatctc acgatacgcc gttaattatt tatgtatccg gtaccattaa ccacggcaac
241 accgaaaagg tgtcgggtaa ttgcgataca accggcgacg agattcagtt taaaaaagtt
301 aaaaacctat cgttaattgg tactggtaac ggtgcggtgt ttgatcaaat aggtattcat
361 ttacgcgaaa cctccaatat tattctgcaa aaccttcata ttaaaaatgt taaaaaatcg
421 ggttctccaa cctccaatgg cggcgatgca attggaatgg agtctggcgt atacaatgtg
481 tgggtggatc actgtgagct agaagcatcc ggtggtgaaa aagatggtta cgattcattg
541 ctagatatga aagcaaccac gcagtatgta accgtttctt acacctatta ccacgattca
601 ggccgcggtg gtttaatggg gtcgagcgat agtgacgata ccaacaccta cgtgactttc
661 caccacaatt actacaaaaa tatggattca cgcttaccgc ttttacgcca cggtactgcg
721 catgccttta acaactatta cgatggcatt accaaatctg gtatgaaccc ccgtataggc
781 ggtcaaataa aagcagaaaa taactatttc gaaaacgcac acaacccaat aggtacgttt
841 tacacaaacg atatgggtta ctgggactta agcggcaata tatttggcaa caacgtaacg
901 tgggcgtctg cggatgatga aacccctgca gggccgaatc cacaatccac aacgtccatt
961 catatttctt acccctacag cttggatgac gcaacgtgcg tgccgaagat tgtaaaagct
1021 actgcgggtg tgggtaacgg tttggctgtg tctaccggtg gtagcaattg cggtacttct
1081 agctcttcgt ctagcagctc ttcgtccagc tctacctcgt ctaccagttc tacatctagc
1141 agttcttcat catccaatag ttcttctggt ggctcaggtg taaacctttc aattggcgca
1201 ggttctgatg gtagcagtaa aggtgcgggc tcttatggcg atgtgcgcga tggcaatatg
1261 agcacctact gggcaccgag tggcagcact ggtcgcgtat ctattaagtg gagttcggca
1321 acgacggtaa gtagcattgt tattaaagaa gcggctggct ttgaaggtaa cattactggc
1381 tggcaagttg taaacaacga gaatggcgca gtattaaaaa gcggctctaa cgctggcgta
1441 atttcttttt ctccggtttc tactacgaag ttaaatttcg aaattacctc ttcgaacggc
1501 atgcccacgg ttgcagaatt tgaaacctat agcggcacgg ttggtggtac ttcgtcatcc
1561 agttcttcaa gtagttcgtc aagcagttct tcaagcagct caagttcaac gagcagctct
1621 ggtggttccg cgaatctagg tacctcggta agtggcgatc aggtttcact taattggtct
1681 acctcaaaca ttgatgtggg ttcgcagcaa gtttatcgcg ataccgattc aaacccatct
1741 ggtcgtgtgc gtatctctgc aggcgtttct ggtaattcgt ataccgatta cggcttagct
1801 agcggtactt attactactg gataaaaatt accgatcaaa acggtgttgt ttacaacaca
1861 aatgccgcag aagcggttgt aggcagccaa gcgccaacga cgtttactgc gcaagagtct
1921 gcgggtttct gctctgttaa cggttctgtt gattccaata acgctggcta cactggcgat
1981 ggttttgtaa ataccgataa cgccagtggc aatgcagcag tttatgcctt caatgcacct
2041 agtgcgggta tgtatagctt gcaggttcgc tacgcaaacg gttctagtgc acgcccaggt
2101 gatgtgctgg tcaacgctgg caatattgga acatttgatt tttccagtac cggttcttgg
2161 acatcttggg caaacagtaa tgagttaagt gcgtacttct ctgcgggtaa caatactgtt
2221 cgtattcaag ctactaactc tggcggctta cctaacttag atagtgtttc tgtaacgggt
2281 aatgcaccag cggcaggcaa ctgtaattaa
LOCUS NZ_AABI02000015 1785 bp DNA linear BCT 23-SEP-2003
DEFINITION Microbulbifer degradans 2-40 Mdeg_15, whole genome shotgun sequence.
ACCESSION NZ AABI02000015 REGION: complement(111927..113711)
VERSION NZ_AAB102000015.1 GI:28878287
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1785)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028706. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nim.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1785
      /organism="Microbulbifer degradans 2-40"
      /mol_type="genomic DNA"
      /strain="2-40"
      /db_xref="taxon:203122"
   gene 1..1785
      /gene="Mdeg2589"
   CDS 1..1785
      /gene="Mdeg2589"
      /codon_start= 1
      /transl_tabte=11
      /product="COG3866: Pectate lyase"
      /protein_id="ZP00067182.1"
      /db_xref="GI:23028800"
      /db_xref="COG:COG3866" 
/translation="MKIFKLLLMFVLAHNLVACGGSNDGGEIELNFGEENTPEPETEP
EAEPEGEPEGEPEGEPEGEPEGEPEGETADATADAGFAGHNFNLTGGEGGTAYTVNNG
KDLQTVLDNAKSSNSPVIIYVDGTINSFNSANGNQPIQIKDMDNVSIIGYGAEATFDG
VGIAIRRANNIIIRNLTFKSVLTEGKDAISIEGDDDGSTTSNIWVDHNEFYSAPTADK
DFYDGLIDSKSGASNITISYNYLHDHWKASLHGHTENDEGAHNTDRKITFHHNRFENI
ESRLPLFRRGVGHLYNNYYKDVGSTAINSRIGAELLIENNVFEDSQNPIVSFYSDVIG
YWNTSGNLFTNVTWTTPGTGEVSAGATQTPTSDYVVPYSYTLMPAADVKAHVIASAGV
GKIDQTGLTIPDPVTPEGDLGEPEAPVQGDVSLPYTENFAATDAANFFSAAYRDITGS
AGTSTPMYHRVTGTVEINAQQLDMTGARVSIGNTTPSVSTTGADTTTTGVLDLSAPYT
VSFKVVSVGGTLTKKFQIYVDNNTSASGDSIHGGSSRFYSETLDSLVAGQTYTVTGFT
ATNSSFITLRTESSGQIVLDDLSIQAAE"
ORIGIN 
1 atgaaaattt ttaaattgtt attaatgttt gtactcgccc acaacttagt tgcttgtggt
61 ggcagtaacg acggtggtga aattgaatta aactttggcg aagaaaacac accagagcca
 
121 gaaaccgaac cagaagctga gcctgaagga gaaccagagg gcgagccgga gggagaacct
181 gaaggagagc ctgaagggga accagagggc gaaacagccg acgcaaccgc agatgctggc
241 ttcgccggcc acaattttaa tcttaccggt ggcgaaggcg gcacagccta taccgttaat
301 aacggcaaag atttgcaaac agttttagac aacgccaaat cgagtaattc accggtcatt
361 atttacgtag acggcaccat aaattcgttt aactctgcca acggcaacca gcctattcaa
421 attaaagata tggataacgt atctataatt ggttacggcg ccgaagcaac atttgacggt
481 gttggtatag caatacgccg cgccaacaac attattattc gcaaccttac ttttaaaagc
541 gtccttaccg aaggtaaaga tgcaattagt atagaaggtg atgacgacgg cagcaccacg
601 tcaaacattt gggttgatca caacgaattc tacagcgccc caacggcaga caaagatttt
661 tacgacggtt taatcgatag taaaagcggc gcgagcaaca ttactatttc ttacaactac
721 ctgcacgacc attggaaagc atcgttacac ggccataccg aaaatgacga aggtgcacac
781 aacaccgacc gcaaaattac tttccaccac aaccgttttg agaatattga atcgcgttta
841 ccgctgttcc gtcgcggtgt aggccatttg tacaataact actacaaaga cgtaggctca
901 acggctatca actcacgtat tggtgccgag ttattaattg agaataacgt ttttgaagat
961 tcacaaaacc cgattgtctc tttttactct gacgtaattg ggtactggaa cacctcaggc
1021 aacctcttca ccaatgtaac ttggacaacc ccaggtactg gcgaagtatc tgcaggcgca
1081 acacaaacgc caacctcaga ttacgtagtg ccatacagct acacgcttat gccggcagcc
1141 gatgtaaaag cccacgtcat tgcgagtgca ggcgttggca aaatagacca gacagggctt
1201 accattccag accccgttac ccctgaaggc gacctaggtg aaccagaagc cccagtgcaa
1261 ggtgatgtaa gcctacctta cactgaaaat tttgccgcca ctgacgccgc caatttcttt
1321 agcgccgcgt accgcgatat tactggctct gctggcacca gcacacccat gtaccaccgc
1381 gtaaccggca cggtggaaat taacgcacag caattggata tgactggcgc acgcgtatca
1441 attggcaaca caacgccaag tgtaagcaca accggtgcag acaccactac aacgggcgta
1501 ttagatttaa gcgcgcccta caccgtaagc tttaaagtgg taagcgtagg cggcacccta
1561 actaagaaat ttcaaatata tgtagacaac aatacctctg ccagcggcga ctctattcac
1621 ggcggctcat cgcgctttta cagtgaaact ttagactcgc tagttgcagg ccaaacctac
1681 acagtaaccg gctttaccgc caccaacagc tctttcataa cattacgtac cgaaagtagc
1741 ggccaaattg tattagatga cctaagtatt caagccgcag aataa
LOCUS NZ_AABI02000001 1278 bp DNA linear BCT 23-SEP-2003
DEFINITION Microbulbifer degradans 2-40 Mdeg_1, whole genome shotgun sequence.
ACCESSION NZ AABI02000001 REGION: complement(71234..72511)
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1278)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nim.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1278
      /organism="Microbulbifer degradans 2-40"
      /mol_type="genomic DNA"
      /strain="2-40"
      /db_xref="taxon:203122"
   gene 1..1278
      /gene="Mdeg0054"
   CDS 1..1278
      /gene="Mdeg0054"
      /codon_start=1
      /transl_table=11
      /product="hypothetical protein"
      /protein_id="ZP00064693.1"
      /db_xref="GI:23026207" 
/translation="MFKYALYVVALVAGVVVSLAACSKRATQQVETEFYEINERGGDD
GRLLRVVNLNNQGVGSLRWALAQTGARKIIFDVGGVIDLEEKSLKIREAHVTIAGETA
PSPGITLIKGGLRIETHNVKVSHLMIRPGDAGYSKGQGWKPDGITIYGSKARHVVIDH
CSVTWAVDENIAVSGPADKGAEATAGKVLIRNSIIAEALSNASHPEGEHSKGILIHNN
VQHVSLVNNLLAHNRRRNPYFKAGTTGIVIGNIIYNPGKRAIHMSSGRADASLPTLSI
TGNLFIPAANTSPNLSLISNYGKIYSSGNLVQGESRPITDGKSISLTAPPLQQVGINL
TDTGTQNDFCQTLSNAGARPWDPDPIDIRIKTQLLAGEGRIIDSQSEVGGYPIHNVNN
KETAEAGSTQGDGMLQFDTELLRKIPNLCSGMM"
ORIGIN 
1 atgtttaagt atgcgctata tgttgtggcc ttggtggctg gcgtagtggt tagcttagca
61 gcctgcagca agagagctac gcagcaagta gagactgaat tctacgagat taatgagcgg
121 ggcggagatg atggtcgcct gctacgcgtt gtgaatttaa ataatcaggg ggttggctct
181 ttgcgctggg cgttggcgca gacaggtgct agaaaaataa ttttcgatgt aggggggtg
241 atagatctag aagaaaaatc gctcaagatt cgtgaagccc atgtgacaat cgctggagag
301 acggccccat caccgggtat cacccttatc aagggcggac taagaataga aacccacaat
361 gtcaaagttt cgcaccttat gattaggcct ggtgacgcag ggtactccaa aggtcaaggt
 
421 tggaaacccg acggcataac tatatatggc agcaaagcga ggcatgttgt tattgatcat
481 tgctcggtta catgggctgt cgacgaaaat atcgcagtat ctggcccagc agataagggg
541 gcagaggcta ccgcgggtaa ggttcttatt cgcaattcaa ttattgccga agcgctaagc
601 aatgcatccc acccagaggg ggagcattct aagggcatac tcatacacaa caatgtgcag
661 catgtaagct tggttaacaa tttgttggct cacaataggc gaagaaaccc ttattttaag
721 gcgggtacaa cgggaattgt aattggcaat ataatttata acccagggaa acgtgctatt
781 catatgtctt cgggccgtgc tgatgctagc ctgccaacac tgtcaattac agggaatttg
841 tttattccag cagctaatac ttcccccaac cttagtttga taagcaacta tggaaaaatt
901 tattcgagtg gaaacttggt gcagggggag agcaggccga taactgatgg taaaagtatt
961 tcattgactg cgccgccgct acagcaagta ggcataaatt taaccgatac aggtacacaa
1021 aacgactttt gccaaaccct aagcaacgca ggtgcccgcc cgtgggaccc cgacccgatt
1081 gatattagaa taaaaacaca acttttggct ggcgaaggac gaattattga tagccagagc
1141 gaggttggag gctacccaat acataacgtc aataacaaag aaaccgctga agcaggctct
1201 acgcaggggg atggtatgtt gcagtttgat actgagttat tgagaaaaat accaaacctg
1261 tgtagtggca tgatgtaa
LOCUS NZ_AABI02000001 2319 bp DNA linear BCT 23-SEP-2003
DEFINITION Microbulbifer degradans 2-40 Mdeg_1, whole genome shotgun sequence.
ACCESSION NZ AABI02000001 REGION: 76773..79091
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2319)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http:/lwww.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2319
      /organism="Microbulbifer degradans 2-40"
      /mol_type="genomic DNA"
      /strain="2-40"
      /db_xre="taxon:203122"
   gene 1..2319
      /gene="Mdeg0058"
   CDS 1..2319
      /gene="Mdeg0058"
      /codon_start=1
      /transl_table=11
      /product="COG3210: Large exoproteins involved in heme
      utilization or adhesion"
      /protein_id="ZP 00064697.1"
      /db_xref="GI:23-026211"
      /db_xref="COG:COG3210" 
/translation="MRDITMKNNKFRSSFTLKKLTPFFVAGTMLGGSNAWAGCDYTVT
NQWGSGFTGNVRITNSGNTPTNGWAVNWQYAGDNRISNSWGAQLSGSNPYSATAESWN
AVIQPSQSIEIGFQGTGDGNEIPTINGDVCQTSSGSTSSSSSSSTSSSSSSSSSTSSS
SNSSSSSSSSSSSSSSSSGSTTGYIHIEENELGFCYVQGSIDSNNGGFTGTGFANTDNV
NGSQINWKVNVDFDGYYALEWRYANGSGTARTASVSANGAQSEISFPTTGSWDSWLLD
STTLFLKAGVNDVILSANTSSGLANIDSLTVHGDGVAAADCNTDGSSSSSSSSSSSS
STSSSSSSSSSSSTSSSSGGPQILKAFPTAEGYGKITAGGRGGDVYIVTNLNDSGAGS
LRQAVEASGPRTVVFEVSGTITLNKPLTIKNNNITIAGQTAPGDGITLRKHNFSIQAD
DVIVRYIRVRFGDETLTDSDAISMRYQKNIILDHVSASWGDDETLSLYHGENITVQWS
MITETLNRGGEHAFAAIWGSPFSTFHHNLIAHNVARNVRFASGSGYTDYRNNVVYNWG
YSSTHGGEAQQVGNANFNFTTVNMVGNYYKPGPRTESGVRSRLLTPNTRNGDADLGSF
YVSGNHMVGSPNVTADNSIGVSNKNALISSPWNSMKIEGEQTAEQAYESVLAYAGASK
VRDSVDTRIIEEVRTGTATYGGNGIIESQNEVGGWPQLRSETPPQDSDRDGMPDDWER
ANNLNPFNAADRNTKDSIGYTMLERYINGLVD"
ORIGIN

1 atgagagata tcacgatgaa gaataataaa ttcaggtcgt cttttacatt aaaaaaactc
61 acaccgtttt ttgttgcggg caccatgctt ggcggttcca acgcctgggc tggctgcgac
121 tatacggtca ctaatcagtg gggctcaggc tttactggca acgttcgtat aactaatagc
181 ggtaacacgc caacaaatgg ttgggctgtt aactggcagt acgctggcga taatcgtatt
241 agtaatagct ggggagcaca gctttcaggg tcgaacccat actctgccac ggcagaaagc
301 tggaatgctg ttattcagcc tagtcagtcc atagaaattg ggtttcaagg taccggcgac
361 ggaaatgaaa taccaactat aaatggcgat gtttgccaga ctagcagcgg aagtacttca
421 tccagctcat cttcaagtac gtcttctagc agctcttcaa gctcgtccac tagcagctct
481 tcaaacagct cttctagctc tagctcgtcc agctcgtcta gctcctcctc tggctctaca
541 actggatata ttcatataga agagaatgaa cttggttttt gttatgtaca aggttccatt
601 gactccaaca acggtggctt taccggcaca ggctttgcca ataccgataa cgttaatggc
661 tcacagatta actggaaagt aaatgtcgac tttgatggat attatgcgct cgaatggcgc
721 tatgcgaatg gctccggcac cgcgcgcact gcaagcgtta gcgctaatgg agcacaaagc
781 gaaatttcct tccctacaac aggttcgtgg gatagctggt tattagacag cactacccta
841 tttttaaaag ctggcgtaaa cgacgtaata ttgagcgcaa atacaagcag tggcctagcg
901 aacatagatt cacttacagt gcacggtgat ggcgtagctg cggcagactg taatactgat
961 ggaagctcaa gcagcagctc tagttcaagc tctagttcca gctcaacttc tagtagctcc
1021 tcaagttcca gctcgtctag cacgtccagc tcttctggtg gcccgcaaat attaaaagca
1081 ttccccaccg cagaaggcta cggaaaaata accgcaggtg gtcgtggtgg cgatgtctat
1141 atagttacaa acctgaatga ctcaggcgcg ggtagtttgc gtcaggccgt agaggcatct
1201 ggccctagaa ccgttgtgtt cgaagtgtct ggaaccatca ctctaaataa accactcaca
1261 atcaaaaata ataacatcac aatagcagga caaactgcac caggcgatgg cattacactt
1321 agaaagcaca acttttctat ccaagctgat gatgtcatcg tacgttacat acgtgttcgc
1381 tttggtgatg aaaccctaac cgattctgat gcgatttcca tgaggtacca aaaaaatatt
1441 attttggatc atgtgagtgc tagctgggga gatgatgaaa ccttatctct ttatcacggc
1501 gaaaatatca ctgtgcaatg gagcatgatt acagagaccc tcaatcgtgg cggcgaacat
1561 gcattcgcag ctatatgggg ttcgcctttt agtaccttcc accacaattt aattgctcac
1621 aatgttgcga gaaacgttcg ctttgcgtcg ggttccggtt atacggatta tcgtaacaat
1681 gtcgtatata actggggcta tagcagcaca cacggaggcg aagctcaaca agttggcaac
1741 gctaatttta atttcaccac cgtcaatatg gtcggcaact attacaaacc tgggccgaga
1801 actgaatctg gcgttcgtag tcgactactt acacctaaca cgcgtaacgg cgatgcggac
1861 ttaggtagtt tttacgtttc tggtaaccac atggttggca gcccaaatgt aactgcagac
1921 aactcgattg gcgtatcgaa taaaaatgcc ttaataagta gcccttggaa ttcaatgaaa
1981 atagaaggcg aacaaacagc tgagcaagca tatgagtcag ttcttgctta cgcaggtgca
2041 tctaaagtac gcgactcggt agatactcgt attattgaag aagtacgtac aggcacagct
2101 acttatggtg gaaacggcat aattgaatcg cagaatgaag tgggtggttg gccacaactt
2161 agaagtgaaa cgcccccgca agacagtgat cgcgacggaa tgccagatga ctgggaacgc
2221 gcgaacaacc taaatccatt caacgcagcc gatagaaaca ctaaagacag tattggctac
2281 acaatgttag agcgatatat taacggcctt gttgattaa

LOCUS NZ_AAB102000001 1536 bp DNA linear BCT23-SEP-2003
DEFINITION Microbulbifer degradans 2-40 Mdeg_1, whole genome shotgun sequence.
ACCESSION NZ AABI02000001 REGION: 73615..75150
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1536)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http:/Iwww.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1536
      /organism="Microbulbifer degradans 2-40"
      /mol_type="genomic DNA"
      /strain="2-40"
      /db_xref="taxon:203122"
   gene 1..1536
      /gene="Mdeg0055"
   CDS 1..1536
      /gene="Mdeg0055"
      Icodon_start=1
      /transl_table=11
      /product="COG3210: Large exoproteins involved in heme
      utilization or adhesion"
      /protein_id="ZP 00064694.1"
      /db_xref="GI:23026208"
      /db_xref="COG:COG3210" 
/translation="MLRIPKAWLALPLVLGSTNLYAQVTCSISNTNVWNNGYTVNVNV
TNTGSSQVGSWQVPINFSEPPQVSSGWNAILSTNGNTVTAGNIGWNGNLNPGQSASFG
FQGGHDGSFVEPTCSGGGSSTSSSSSSSSSTSSTSSSSTSSSSSSSSGGSELLIQEN
ASGFCRVDGSIDNNNSGYTGSGFANTENQNGSAVEYALNVPSNGNYLLDARYASATTR
SASVVVNGSSVGSFSFPSTGSWTSWTVDSANVPLKGGNNIVRIVATNSSGLPNIDSLK
VIGTNPSAGSCSSNSSSTSSSSSSSSSSSSNSGGKGSSCRSTGSQSVSSTIKVTSGTFD
GNCKTYNPTSALGDGSQSESQKPAFRVENGATLKNVILGNNGVDGIHVYNGGTLDNIR
WTNVGEDAMTVKSEGNVTVSNIEGYDGSDKFIQVNAVTNLKVSNCIVDKMGKFLRQNG
GKTFAMSVTVDNCDISNMGEGVFRSDSPNATARITNSRLKNAGDICIGKWKSCTSSNI
TSF"
ORIGIN 
1 atgttgcgaa tccccaaggc ttggctggca cttccacttg tactgggaag taccaatcta
61 tacgctcaag taacttgcag tatctctaac accaatgttt ggaataacgg atacaccgtt
121 aatgttaatg taaccaacac aggctcttca caggttggtt cttggcaggt tcctattaat
 
181 ttttctgagc cacctcaagt aagcagcggc tggaatgcaa tattaagcac aaacggaaac
241 accgtaactg ccggcaatat tggttggaat ggtaatttaa atcccggcca aagcgcctcc
301 tttggttttc aaggtggcca cgatggcagc tttgtggagc ccacctgctc gggcggaggc
361 tctagcacta gctcaagcag ctctagtagt tctagctcaa caagttctac cagttcttca
421 tccacaagtt caagtagctc ttctagctcc ggcggctctg aacttttaat ccaagaaaat
481 gcatccggct tctgccgtgt ggacggatcg atagataaca ataactcagg ctataccggt
541 agtggctttg ccaacaccga gaaccaaaac ggttccgcag ttgaatacgc acttaacgtt
601 ccctctaatg ggaattatct cctcgacgct cgatatgcaa gcgctactac acgatcggct
661 agcgtggtag ttaatggatc ttcagtaggc agctttagtt ttccatctac gggttcgtgg
721 acaagctgga cagttgactc cgccaacgtt ccgttaaaag gcgggaataa tattgttcga
781 attgttgcaa ctaacagcag cggattacct aatattgatt cattaaaggt aataggcacc
841 aacccgtcag ccggcagttg ttcaagcaac tcgtcatcca ctagttcatc gtctagctca
901 agttcatcaa gcagtaactc cggtggcaaa ggctctagct gccgttctac aggcagtcaa
961 tctgtttcct ctactattaa agttactagc gggactttcg atgggaactg taaaacgtat
1021 aaccctacaa gtgcccttgg cgatggcagt caatcagaaa gccagaaacc ggcattccga
1081 gtggagaacg gcgcaacact caaaaacgtg attctaggca acaatggcgt agacggtatt
1141 catgtttata acggcggcac cttggataac atccgctgga ccaatgtggg tgaagatgca
1201 atgaccgtta aatctgaagg aaacgttacc gtttcaaata ttgagggtta tgacggttca
1261 gataaattta tacaagtaaa cgcagttacc aacctaaagg tttctaattg cattgtagat
1321 aaaatgggta aatttttacg tcagaatggc ggtaaaactt tcgctatgtc tgtaaccgta
1381 gataattgtg atatctcaaa tatgggtgaa ggtgttttcc gctcagacag cccaaatgca
1441 acagcgagaa tcacaaatag ccgattaaaa aatgcaggcg acatttgtat tggtaagtgg
1501 aaaagctgca catcttccaa cattaccagc ttctaa
LOCUS NZ_AAB102000004 1368 bp DNA linear BCT 23-SEP-2003
DEFINITION Microbulbifer degradans 2-40 Mdeg_4, whole genome shotgun sequence.
ACCESSION NZ AABI02000004 REGION: 203603..204970
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1368)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1368
      /organism="Microbulbifer degradans 2-40"
      /mol type="genomic DNA"
      /strain="2-40"
      /db_xref="taxon:203122"
   gene 1..1368
      /gene="Mdeg 1109"
   CDS 1..1368
      /gene="Mdeg 1109"
      Icodon_start=1
      /transl_table=_11
      /product="hypothetical protein"
      /protein id="ZP 00065735.1"
      /db_xref="GI:23027280" 
/translation="MIMMRNKILLALVLCGASASAFAASNRPSGYTTICKTDQTCSVS
SSTNVAFGAAGKFVYKVINGTFTCNTSTFGSDPNPAKSVKECSVPTNGSSSTSSTSSS
SSSSSSSSSGSSSSCGTGGGATVCLSAGGGSNDIDLTWTVSGSISSAQVYRDTDSNPS
GRTRIAQLGGDARSYSDTNVSAGKQYYYWIKFGANGSNYNSNAASATYSGSSSSSSSS
SSSSSSSGGSAECKAGATISGKTVDCGGKEIGLSCSGDSETQPPVLTLKNATIKNLVI
SAKGGSDGIHCTGNCTMENVVWKDICEDAATNKTDGITMTIIGGSAYNSTSGYGGKPD
KVFQHNSKNSTTVIKGGFTLTGEHGKLWRSCGNCTNNGGPRNVTIDNVKVDAKIGSIV
GVNRNYGDKATIKNLKIKDYKSGSPKVCEEYKGVQKGSGESSKYGEYWDTANCDVSKS
DVSAL"
   gene complement(174..356)
      /gene="Mdeg1110"
   CDS complement(174..356)
      /gene="Mdeg1110"
      /codon_start=1
      /transl_table=11
      /product="hypothetical protein"
      /protein_id="ZP 00065736.1"
      /db_xref="GI:23027281" 
/translation="MPHELLEPDELLELELEELLVLLVLEEPLVGTEHSFTDLAGLGS
LPKVDVLQVKVPLITL"
ORIGIN 
1 gtgataatga tgcgtaataa aatcctattg gcgcttgtat tgtgtggagc ttctgcctct
61 gcctttgcgg ctagtaatcg tcctagtggt tacacaacta tctgtaaaac cgatcaaact
121 tgttctgtaa gctcgtctac caacgttgcg ttcggcgctg ctggtaagtt tgtttacaaa
181 gtaattaacg gtacctttac ttgtaataca tctacttttg gcagcgatcc taaccctgct
241 aaatctgtaa aagaatgttc tgtacctact aatggttctt ctagcactag cagcaccagt
301 agttcttcta gctctagttc aagtagctca tcgggttcaa gcagctcatg tggcactggt
361 ggcggcgcaa ctgtatgttt aagtgcaggt ggcggcagta acgatatcga tttaacttgg
421 acagtatctg gttctatttc tagcgctcag gtttaccgcg acacagattc taaccctagt
481 ggtcgcacac gtattgctca attaggtggc gatgcaagaa gctatagcga tacgaatgtt
541 agtgctggta agcagtacta ctactggatt aagtttggcg ccaacggctc taactacaat
601 tcgaatgcgg cttctgctac ctatagtggt tcaagtagct catcgagttc ttcaagctct
661 tctagttcct catctggcgg ctcggctgaa tgtaaagctg gcgctactat ttctggtaaa
721 accgtagatt gcggtggtaa agaaattggc ttgtcgtgct cgggtgatag tgaaactcaa
781 ccaccagtat taacgcttaa aaatgccacc attaaaaact tggtaatttc tgctaaaggt
841 gggtccgacg gtattcactg tactggcaac tgcaccatgg aaaatgttgt ttggaaagat
901 atctgtgaag atgctgctac caacaaaacc gacggtatta ccatgaccat tattggtggt
961 agtgcgtata actctacaag cggttacggc ggcaagccag ataaggtttt ccaacataac
1021 tctaaaaaca gtactactgt aattaaaggc ggctttacat taacaggtga gcacggcaaa
1081 ttgtggcgtt catgtggtaa ctgtactaat aacggcggcc cacgtaatgt gactatcgac
1141 aacgttaaag tagacgcgaa aataggcagt attgttggcg ttaaccgcaa ctatggcgat
1201 aaggcaacaa tcaaaaactt aaagattaaa gactacaaat ctggtagccc caaagtgtgt
1261 gaagaataca agggtgtaca aaagggtagt ggcgagtctt ctaagtatgg cgaatactgg
1321 gatactgcaa actgcgatgt aagtaaatca gatgtgtctg ctctttaa
LOCUS NZ_AAB102000010 1275 bp DNA linear BCT 23-SEP-2003
DEFINITION Microbulbifer degradans 2-40 Mdeg_14, whole genome shotgun sequence.
ACCESSION NZ AAB102000010 REGION: complement(30476..31750)
VERSION NZ_AABI02000010.1 GI:28878292
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1275)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028613. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1275
      /organism="Microbulbifer degradans 2-40"
      /mol_type="genomic DNA"
      /strain="2-40"
      /db_xref="taxon:203122"
   gene 1..1275
      /gene="Mdeg2416"
   CDS 1..1275
      /gene="Mdeg2416"
      /codon_start=1
      /transl_table=11
      /product="COG5295: Autotransporter adhesin"
      /protein_id="ZP 00067017.1"
      /db_xref="GI:23028629"
      /db xref="COG:COG5295" 
/translation="MFNKILVAVGLLAASLSVHAATNRPSGYTTICKVGETCSVSQST
NVAFGASGQFVYKVLNGSFSCSVSTFGSDPIPSKSVKECSIPSNGSSSSGSSSSSSSS
SSGSSSGGGCGSGGGSTVCLSASGSSNGINLSWSVSGSISSVQLYRDTDSNPSGRTRI
ASVSSSTTSFSDTGAASGTTYYYWVKYYVNGTAYNSGVASAVRGSSSSSSSSSSSTSS
SSGGKGSSCSSTGSQSVSSTIKVTSGTYDGGCKTFNPTSALGDGSQSESQKPAFRVEN
GATLKNVIIGNNGVDGIHVYNGGTLNNILWTNVGEDAMTVKSEGNVTVTNVEGYDGED
KFIQVNAVTNLKVSNCIVNKMGKFLRQNGGKTFAMSVSVDNCDISNMGEGIFRSDSPN
ATAVITNSRLRNAGDICIGAWKSCKSSNISSF"
ORIGIN 
1 atgtttaaca agatactcgt tgcagtagga ttacttgcgg ctagcctttc tgtgcacgcc
61 gcaacaaacc gcccaagtgg ttatacaaca atttgtaagg ttggtgaaac atgctcggta
121 agtcagtcta cgaatgtagc ctttggcgcg tctgggcagt ttgtgtataa agtattaaac
181 ggtagctttt cttgtagtgt ttctacgttt ggtagtgacc ctattccttc taaatctgta
241 aaagaatgtt caatcccatc aaacggctct agctcttctg gctcgtcttc atcttcgtct
 
301 agcagctctt ccggtagctc ttctggtggt ggctgtggca gcggtggtgg ttctacggtg
361 tgcttatcgg cctcgggttc tagcaatggt atcaatttaa gttggtctgt atctggttct
421 atatcttccg tgcagcttta tcgcgatacc gattcaaacc caagcggtcg cacgcgtatt
481 gctagtgtat ctagctctac tactagcttt agtgataccg gcgcggcatc gggcaccact
541 tattactact gggttaaata ttatgtaaat ggtactgctt acaactcggg tgttgcttct
601 gcggtgcgcg gttcttctag ctctagtagt tcaagttctt ccagcacttc tagcagttct
661 ggtggaaaag gttctagttg tagctctact ggtagccaat ctgtgtcttc tactattaag
721 gtaaccagcg gtacttacga tggtggttgt aaaacattta accctaccag tgctttgggt
781 gatggtagcc aatctgaaag ccaaaaacct gctttccgtg tagaaaacgg tgcaacgtta
841 aagaatgtaa ttattggcaa taacggtgtg gatggtattc acgtttacaa cggcggtacg
901 ttaaataata ttctttggac taacgtaggt gaagatgcca tgaccgttaa gtctgaaggt
961 aacgtgacgg taaccaatgt tgaaggctat gacggcgaag ataagtttat tcaggtaaac
1021 gcagtgacta acttaaaagt ttctaactgt attgtgaata aaatgggtaa gtttcttcgt
1081 cagaatggtg gtaaaacatt tgccatgtcg gtaagtgtag ataactgcga tatatctaat
1141 atgggtgaag gtatcttccg ttcagacagc ccgaacgcta cagcggttat tactaacagc
1201 cgtttacgca acgctgggga tatttgtatt ggggcttgga aaagttgtaa atcttccaat
1261 atcagcagct tttaa
LOCUS NZ_AABI02000027 1179 bp DNA linear BCT 23-SEP-2003
DEFINITION Microbulbifer degradans 2-40 Mdeg_27, whole genome shotgun sequence.
ACCESSION NZ AABI02000027 REGION: complement(32448..33626)
VERSION NZ_AABI02000027.1 GI:28878275
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1179)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029524. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.igi.doe.gov
   Contact: Paul Richardson
   microbes@cuba.jgi-psf.org.
   COG assignment was automatically updated on Sep 23, 2003.
FEATURES Location/Qualifiers
   source 1..1179
      /organism="Microbulbifer degradans 2-40"
      /mol_type="genomic DNA"
      /strain="2-40"
      /db_xref="taxon:203122"
   gene 1..1179
      /gene="Mdeg3302"
   CDS 1..1179
      /gene="Mdeg3302"
      /codon_start=1
      /transl_table=11
      /product="hypothetical protein"
      /protein id="ZP 00067887.1"
      /db_xreg="GI:23029557" 
/translation="MKKLILMVALLAFSVSSFAALSSGRYIIVSKLNGNALDVDSFST
ADGANVMQWFALGGVNQQFDVAVLSDGSYSIRPVHSGKSLDVYAWNADDGAELRQWAY
TGADNQRWYIDNQSGDYYSITSKFSGRALDVWGMSMYTGADVRLYSYWGGAGQLWTFQ
KVGSSSECYAGATLTNRFVDCGGKTIGLSCVGDSETQGAVLTLKNSSIRNVKLAANGG
ADGIHCTSGNCTLADVVWNDICEDAATNKSEGGTLTIVGGSAYNSTGGYGGTPDKIFQ
HNSKNSTTIVAGGFTAYGTHGKLWRSCGNCTNNGGPRNLLVYSVNIDASIGAIAGVNR
NYGDRATIRDLKIKNYSSGSPHVCDEYQGVQKGNSSTKYGEYWNTASCDVSRSDVSGL
"
ORIGIN 
1 atgaaaaaac ttatccttat ggtggcgctg ttggctttta gtgttagttc ttttgctgca
61 ctgtcttcag gccgctacat tattgtttct aaacttaatg gcaacgcgtt agatgtagat
121 agctttagca ccgcagatgg cgccaatgtt atgcagtggt ttgctttggg tggtgtgaac
181 cagcagtttg acgtggcagt gcttagcgat ggcagttact ccatacgacc agtgcacagc
241 ggtaagtcat tagatgtata tgcgtggaac gcagacgatg gtgcggaact tcgtcagtgg
301 gcatacacag gcgcagataa ccaacgttgg tatatcgata atcaaagtgg cgattactat
 
361 tcaattacgt ctaaatttag cgggcgcgca ttggatgtat ggggtatgag tatgtacacc
421 ggcgcagatg tccgccttta ttcatattgg ggcggcgcgg ggcagctgtg gaccttccaa
481 aaggtaggta gctcaagtga gtgttacgca ggtgctacgt taacaaaccg ctttgtggat
541 tgtggcggca aaacaatagg ccttagttgt gtaggcgata gtgaaactca aggcgcggtg
601 ctaaccctta aaaactcgtc cattcgcaat gttaagttgg ctgcaaacgg tggtgcggat
661 ggcattcact gcactagtgg caactgcaca ttagccgacg ttgtttggaa cgatatttgt
721 gaagatgctg ccacgaataa gtctgaaggt ggcaccctga ctattgtggg tggttcggcg
781 tataactcta ctggcgggta tggtggtaca ccggataaaa tttttcagca caactcgaaa
841 aacagcacaa caattgttgc cggcggcttc actgcatatg gtacccacgg taagttgtgg
901 cgctcgtgtg gtaactgtac aaacaacggc ggtccgcgta atttactggt ttatagcgtg
961 aatattgacg caagtattgg cgcaattgct ggtgttaacc gcaattacgg cgatagagcg
1021 accattcgcg acctaaaaat aaagaattat tcttctggca gcccgcatgt gtgtgacgaa
1081 tatcaaggcg tacagaaggg caattcttct acaaaatatg gcgagtactg gaataccgca
1141 agttgtgatg tttcgcggtc agatgtaagt gggctttaa
LOCUS NZ_AABI02000006 2202 bp DNA linear BCT 23-SEP-2003
DEFINITION Microbulbifer degradans 2-40 Mdeg_6, whole genome shotgun sequence.
ACCESSION NZ AABI02000006 REGION: 58091..60292
VERSION NZ_AAB102000006.1 GI:28878296
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2202)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027577. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2202
      /organism="Microbulbifer degradans 2-40"
      /mol_type="genomic DNA"
      /strain="2-40"
      /db_xref="taxon:203122"
   gene 1..2202
      /gene="Mdeg1441"
   CDS 1..2202
      /gene="Mdeg 1441"
      /codon_start=1
      /transl_table=11
      /product="hypothetical protein"
      /protein_id="ZP 00066061.1"
      /db_xref="GI:23027623" 
/translation="MFRYILTAFALVAAASCAQAATNRPSGYTTICKTNQTCSVSSPT
NVAFGASGKFTFKVLNGSFVCSVATFGSDPNPAKSAKECSIPSDGSSSTSSTSSTSSS
SSSSSSSTSSSSSSSSSSSSSSSSSSSSSSGSSQAGCGSGGGATVCLSATDTASAIN
LNWTVSGSLSSVQVYRDTDPNPSGRTRLTSLSPSVTSYTDNNAQAGTTYYYWIKFGAN
GSNYNSGAASAVIANTGNDDEGCGSDVCLTATANIGSIGLSWGSSAALTSVQIYRDTD
SNPSGRTRIASLSTSATSFTDSTTAVGTTYYYWVKYGLNGSQLNSNVASATALQNNTG
NASCPGETSGETAATVYYVTPNGSASASGNSFASAMDIDTALSIVGAGQMILMQPGTY
TVAYSAGNKNTKVLSRSGAAGAPIKMVAANCGRAVFDFSFPEREWVQDSYGFFLTGDY
WYFKGIEITRAGYQGVYVTGAHNTFENCAFYYNRNTGLEINKGGSYTTVINSDAYRNY
DPKKNGSMADGFGPKQTQGPGNKFIGCRAWENSDDGFDLYDSPEEVTIENSWAFRNGV
DVWGYGGFAGNGNGFKLGGNHVAANNRITNSVAFGNPVKGFDQNNNAGGITVLNCTAY
ANGTNYGFGNNLNSGEQHYFRNNVSVSGAVNISNADNKYNSWNGGVTASTADFENVDL
SKATAARNIDGSLPNNGLFRLKSGSDLIDAGVEVGLPSNGSAPDMGAFEAN"
ORIGIN 
1 atgtttcgat acatccttac tgctttcgca ttggtggcag cggcttcctg cgcgcaagca
61 gctaccaatc gccctagcgg ttacaccacc atatgtaaaa ccaatcaaac ctgctctgtt
 
121 tcaagcccta ccaatgtggc gtttggcgca tcgggtaaat ttacctttaa agtgcttaat
181 ggctcttttg tttgtagcgt agccactttt ggctccgacc ctaacccggc taaaagtgct
241 aaagagtgct ctattccgtc ggatggctct tccagcacct ctagtacttc gagcacatcg
301 tctagttcta gtagttcatc tagcagcaca agttcaagca gcagctctag cagttcttct
361 agctcttcgt cttccagtag ctcaagttct agttcaagtg gctcttcgca agctggctgc
421 ggtagtggcg ggggagcaac ggtttgttta tcggcaaccg atacagctag tgctatcaat
481 ttaaattgga cagtgagtgg ctcgctgtcg agcgtgcagg tgtatcgcga taccgatccc
541 aacccaagtg gacgtacgcg gttaacgtcg ttaagccctt cggtaacaag ctacaccgac
601 aacaacgcac aggccggtac aacctactat tactggatta aattcggcgc aaacggcagc
661 aactataatt ccggtgctgc gtcggctgta atagccaata ccggtaacga tgatgaaggt
721 tgtggtagtg atgtgtgctt gacggcaacc gctaatattg gctctatagg cttaagctgg
781 ggctcttctg ccgctttaac cagcgtacaa atttatcgcg atacagattc aaacccaagt
841 gggcgtacac gtattgcatc gcttagcact tctgcaacca gctttaccga ttcaaccact
901 gcagtaggca caacgtatta ttactgggtt aaatacggct taaacggcag ccagctaaac
961 tctaatgttg catctgccac tgctttgcaa aataacactg gcaacgcaag ttgccccggt
1021 gaaacaagtg gcgaaactgc agcaaccgtg tattacgtaa caccaaacgg ttcggctagt
1081 gcaagcggca atagctttgc atctgcaatg gatatagaca cagcactttc aattgtaggc
1141 gcggggcaaa tgatattaat gcagcctggt acctacaccg ttgcctatag tgcgggtaat
1201 aaaaatacca aagtgctttc gcgctcgggt gccgctggtg cacctatcaa aatggtggca
1261 gccaattgcg gtcgtgcagt gtttgatttt tcgttcccag aacgtgagtg ggtgcaagat
1321 tcttacggct ttttcttaac tggcgattac tggtatttta aaggaataga aattacccgt
1381 gcaggctacc aaggtgtgta tgtaacgggt gcgcacaaca catttgaaaa ttgcgccttt
1441 tattacaacc gcaatacagg tttagaaatt aacaaaggcg ggtcttacac caccgtcatt
1501 aattcagatg cctatcgcaa ttacgatccc aagaaaaacg gcagcatggc cgatggcttt
1561 ggccctaaac aaacccaagg cccaggcaat aaatttattg gttgccgcgc gtgggaaaac
1621 tccgacgatg gatttgacct gtacgatagc ccagaagaag taactattga aaatagctgg
1681 gcatttcgca acggtgtaga tgtatggggt tacggtggtt ttgcgggtaa tggcaacggc
1741 tttaaattgg gcggtaacca cgtggctgca aacaatcgta ttaccaactc ggttgcgttc
1801 ggcaaccccg taaaaggttt tgatcaaaac aataatgccg gcggtattac agtgcttaat
1861 tgcacagcct acgccaacgg cactaactac ggctttggca acaacttaaa ctcgggtgag
1921 caacactact tccgcaataa tgtttctgta tctggcgctg tgaatattag caatgccgac
1981 aacaaataca attcgtggaa cggcggagta acagcatcca cggcagattt tgaaaacgta
2041 gatttatcca aagccaccgc tgcacgtaac atagatggca gcctgccaaa caacggccta
2101 ttccgcttaa aaagcggcag cgatttaata gacgccggtg tagaggtagg tttaccaagt
2161 aacggcagcg cgcccgatat gggagcgttc gaagctaact ag
LOCUS NZ_AAB102000042 2103 bp DNA linear BCT 23-SEP-2003
DEFINITION Microbulbifer degradans 2-40 Mdeg_42, whole genome shotgun sequence.
ACCESSION NZ AABI02000042 REGION: 7251..9353
VERSION NZ_AABI02000042.1 GI:28878260
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2103)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029937. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.ggi.doe.gov
   Contact: Paul Richardson
   microbes@cuba.jgi-psf.org.
   COG assignment was automatically updated on Sep 23, 2003.
FEATURES Location/Qualifiers
   source 1..2103
      /organism="Microbulbifer degradans 2-40"
      /mol type="genomic DNA"
      /strain="2-40"
      /db xref="taxon:203122"
   gene 1..2103
      /gene="Mdeg3649"
   CDS 1..2103
      /gene="Mdeg3649"
      /codon_start=1
      /transl_table=11
      /product="hypothetical protein"
      /protein_id="ZP 00068232.1"
      /db_xref="GI:23029941" 
/translation="MKNVFNTQKTKRHCNYAYNAKAAKPFSQKALVQKCAAAALSVGL
LGAVGNAYAISCSATADTWGGGYVLNVTVTNDTNNAISNWALALNYDQAAAITNSWNA
SVSANGNVVNATNIGWNGNLAAGQSTSFGLQGTYTGNFSLPVCVGQGQSSSSSSSTSS
SSSSSSSSSSSSSSSSSSSSSSSSSSSSSSSSSSSSSSTSSTGGSSELTIQEDNSGFCGV
DGSIDSNNSGFTGSGFANTDNATGKSVDWSVSVPYSGNYLLEWRYANGSGNNRAGAIE
VNGNARGNQSFPTTGAWTSWTTASANVSLDAGTNLISLVASTGEGLGNIDSLTVIGND
IQTGACDSTGSSSSSSSSSSSSTSSSSSSSSSSTSSSSSGAPMLPQAGNPINGKFGKYK
SWQKGSLSADKQFADILLSHQYTNGGFPKNQAYDSMGSGGNSAGTIDNDATTTELLFL
ADVYQRTGETKYRDGARKALDFLLDMQYSSGGWPQYYPVRSGYYEHVTFNDDAMARVL
IVLDKAKQGVAPLNGDLLTSNQRARLSSAVNKGVDYILKSQWRQNGTLTVWCAQHGKD
DYLPKKARAYELESLSGSESVLVVAFLMSQPQTPEIKTAVKAAINWFRSPNTYLAGYT
YDSSRKGDGNSPIVAKSGSKMWYRFYDLNTNRGFFSDRDSRKVYDILDISTERKDGYR
WGGDYGSGIISYAESVGY"
   gene complement(405..623)
      /gene="Mdeg3650"
   CDS complement(405..623)
      /gene="Mdeg3650"
      /codon_start=1
      /trans_table=11
      /product="hypothetical protein"
      /protein_id="ZP 00068233.1"
      /db_xref="GI:23029942" 
/translation="MVNSLLPPVELVDEDDELEDEELLELLEELLEELEDELLDELEE
LDELLLVLLLEEELWPWPTQTGKLKLPV"
ORIGIN 
1 atgaaaaacg tatttaatac acaaaaaacc aagcggcatt gcaactatgc ctataacgcg
61 aaagctgcca aacccttcag ccaaaaagca ctggtacaaa agtgtgcggc tgcggcattg
121 tctgttggct tactgggggc cgtgggtaat gcgtatgcaa tatcctgttc ggcaactgct
181 gatacctggg gtggtggcta tgtgctaaat gtgaccgtta ctaacgacac aaataatgca
241 attagcaatt gggcgctagc tttaaattac gatcaagctg cagccataac taattcgtgg
301 aacgcgagtg tgtctgcaaa tggcaatgtg gttaatgcta ccaacattgg ttggaatggc
361 aatttagcgg ctggccaaag tacaagtttt ggtttgcaag gcacctatac cggcaacttt
421 agtttacctg tctgtgttgg ccagggccaa agctcttcct ctagtagcag tactagtagt
481 agttcgtcca gctcttcgag ttcatccagc agttcatctt ccagctcttc aagtagttct
541 tcaagcagct ctagtagttc ttcatcttct agctcatcgt cttcgtccac cagttcaact
601 ggtggaagta gtgagttaac cattcaagaa gataatagcg gcttttgcgg ggttgatggt
661 tcaatcgatt ccaataattc aggctttacc ggaagcggct tcgccaatac cgataacgcg
721 acaggcaaaa gcgtagattg gagtgtgagc gttccttata gtggcaacta tttgcttgaa
781 tggcgttacg caaatggttc cggtaataac cgtgctggcg cgattgaagt aaatggtaac
841 gctcgtggta atcaaagctt tcctactaca ggggcctgga ctagctggac aacggcaagt
901 gctaacgtaa gtttggatgc aggtacaaac ttaattagct tggttgcatc tacgggcgaa
961 ggcttaggga atattgattc acttactgtc attggtaacg atattcagac cggcgcttgt
1021 gattctacag ggtctagctc ttccagtagc tctagttcct ccagtacttc tagctcaagt
1081 tccagctcca gttcaagtac cagtagttct agcagcggtg cgcccatgtt acctcaagct
1141 gggaacccca ttaatggcaa gtttggcaaa tataaatctt ggcaaaaagg aagcttgtct
1201 gccgataagc aatttgcaga catccttcta tcgcaccaat ataccaatgg cggatttccc
1261 aaaaaccaag cctacgacag tatgggtagc ggtggtaaca gtgcgggcac aatcgacaac
1321 gatgccacaa caacagagtt gttattctta gctgatgtgt accagcgtac tggtgaaacc
1381 aaataccgag acggtgcgcg taaagcgtta gatttccttt tggatatgca gtattcatcg
1441 ggcggctggc cgcaatacta ccctgtgcgc agtggctact acgagcatgt aacatttaac
1501 gacgatgcaa tggcgcgagt gctaattgtt ttagataaag cgaaacaagg tgtggcgccg
1561 ctaaatggcg atctattaac atctaaccag cgtgcgcgtt taagcagtgc ggttaataag
1621 ggcgtggatt acattcttaa atcgcagtgg cgtcaaaacg gaaccttaac tgtttggtgt
1681 gcgcaacatg gtaaagatga ttatctacct aaaaaggcgc gtgcttacga gctggaatcg
1741 ttgagtggta gcgaatcggt attggtagtt gcattcctta tgtctcaacc tcaaacccct
1801 gaaattaaaa cggcggttaa ggctgctatc aattggttta gaagccccaa tacttactta
1861 gctggttaca cttacgattc atctagaaaa ggcgacggca acagccccat cgtcgcgaaa
1921 agcggtagta aaatgtggta tcgcttctac gacctaaata ctaaccgtgg cttcttcagt
1981 gatagagata gcagaaaagt ttacgatatt ttagatattt ctacagagcg taaagatggc
2041 tatcgttggg gcggtgatta tggctccggc atcattagtt acgcggaaag cgttggttac
2101 taa
LOCUS NZ_AAB102000006 1725 bp DNA linear BCT 23-SEP-2003
DEFINITION Microbulbifer degradans 2-40 Mdeg_6, whole genome shotgun sequence.
ACCESSION NZ AABI02000006 REGION: complement(56047..57771)
VERSION NZ_AABI02000006.1 GI:28878296
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1725)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027577. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1725
      /organism="Microbulbifer degradans 2-40"
      /mol type="genomic DNA"
      /strain="2-40"
      /db_xref="taxon:203122"
   gene complement(1..>248)
      /gene="Mdeg1440"
   CDS complement(1..>248)
      /gene="Mdeg1440"
      /codon_start=
      /transl_table=11
      /product="hypothetical protein"
      /protein_id="ZP 00066060.1"
      /db_xref="GI:23027622" 
/translation="MSVPALPPEELLELEDELLELEDELLELDELLELDELLELVELL
ELEELVEAFISLAGPELPPPQALNSVAASASVIRRRKFIIVALYVFILRAHK"
   gene 1..1725
      /gene="Mdeg 1439"
   CDS 1..1725
      /gene="Mdeg1439"
      /codon_start=1
      /transl_table=11
      /product="hypothetical protein"
      /protein_id="ZP 00066059.1"
      /db_xref="GI:23027621" 
/translation="MNFLRLITLALAATLLSACGGGSSGPAKEINASTSSSSSSSSTS
SSSSSSSSSSSSSSSSSSSSSSSSSSSSSSSGGSAGTLTIQESEKGFCTVNGEIVNNH
EGYSGTGFVDTANAEGASITWKVDVDGGNYDVSVRFANGSTARGATLSSNEINTTYGF
ATTGDWATWADETHTVSLAAGENTIQLSALTAGGLPNVDAITIAGAGVLAADCATEHT
GPMLSQTGNPIYTELNNYKSWLTGSGTTAAKLAADKTIADNMITWQMPHGGFYKYGVS
 
KYSSAWNGSDARSGWTGANGVELGTIDNDATVSELLFLADVYKRSGETKYRDAARSAL
EFLLTMQYSTGGWPQVYPARTGTSYSNHVTFNDNAMARVLILLDKAARLEAPLDGDIF
TTDQHTRITTAINGGIDFILNAQIVQGDVKTVWCAQHDPYTYEAKAARSYELASKSGK
ESVLVVAFLMTRPQSEAIENAVKAALAWYRNPNVQVANTEYVKRTNNDDNYNPIQTKA
GSTMWYRFYDLDQDVGFFSGRSASDNPAGNGKQYDIMLIEPERRYGYEWGGNYGKKII
DYANSVGY"
ORIGIN 
1 atgaattttc tacgccttat tacactcgca ctcgccgcaa cactattaag tgcctgcggt
61 ggaggtagtt ctggcccagc caaagagata aacgcttcca ccagttcctc tagctctagc
121 agctccacga gttctagcag ctcatccagc tctagtagtt cgtctagctc aagcagctcg
181 tcttccagtt ccagcagctc atcctctagc tctagcagct cttctggtgg tagcgccggc
241 acactcacta ttcaggaaag cgaaaaaggc ttttgcactg ttaacggtga gattgtgaat
301 aatcacgagg ggtatagcgg tacaggtttt gtagacaccg ccaatgccga aggcgccagc
361 attacttgga aggtagatgt cgatggcggc aattatgatg taagtgtgcg attcgcaaac
421 ggttccactg cacgcggcgc aacgcttagc agtaacgaaa taaataccac ctatggtttt
481 gctaccactg gcgactgggc cacatgggca gacgaaaccc acaccgtttc gttagccgca
541 ggcgaaaaca ccatccagct aagtgcactt accgcgggtg gcttacccaa tgtagacgct
601 attactattg caggtgcagg tgtactcgca gcagactgcg ccacagagca tactgggcca
661 atgctttcgc aaacaggcaa ccctatttat accgagttaa ataattacaa gtcctggcta
721 acgggtagcg gcacaacagc agccaaatta gccgcagata aaacaattgc cgacaatatg
781 attacctggc aaatgcctca cggtggtttt tataaatacg gcgtatctaa atacagctcg
841 gcttggaacg gtagcgatgc ccgctctggc tggactgggg ccaacggcgt tgagcttggc
901 acaattgata atgatgcaac cgttagcgaa ttattatttt tagcggacgt atataaacgc
961 agcggtgaaa ctaaatatag agatgccgca agaagcgcgt tggaattttt acttaccatg
1021 caatattcca ctggcggttg gccacaggtt taccctgcgc gcactggcac cagttactcc
1081 aatcacgtta cgtttaacga taacgccatg gctcgtgtac ttattttatt ggataaagcc
1141 gcgcgattag aagcaccact cgatggcgac attttaccca cagaccagca cacgcgtatt
1201 actaccgcaa taaatggcgg catcgatttt attttgaatg cgcaaatagt acagggcgac
1261 gtgaaaaccg tttggtgtgc gcaacacgac ccttatacct acgaggcaaa agcagctcgc
1321 tcttatgagt tggcctctaa aagcggtaaa gaatctgtat tggttgtagc gtttttaatg
1381 acacgcccgc aaagcgaagc catagaaaat gccgtgaaag cagcccttgc ttggtaccgc
1441 aacccaaatg ttcaagtcgc caacaccgag tatgtaaaac gcacaaataa cgatgacaac
1501 tacaacccga tacaaacgaa agcaggtagc actatgtggt accgctttta cgatttagac
1561 caagacgttg gattctttag cggccgctct gcaagtgaca acccagcagg taacggtaag
1621 caatacgaca ttatgcttat tgaacccgag cgcaggtatg gctatgaatg gggtggcaat
1681 tacggcaaaa aaataatcga ttacgctaat tcggtagggt attaa
LOCUS NZ_AAB102000026 1392 bp DNA linear BCT 23-SEP-2003
DEFINITION Microbulbifer degradans 2-40 Mdeg_26, whole genome shotgun sequence.
ACCESSION NZ AABI02000026 REGION: 5430..6821
VERSION NZ_AABI02000026.1 GI:28878276
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1392)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029482. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1392
      /organism="Microbulbifer degradans 2-40"
      /mol type="genomic DNA"
      /strain="2-40"
      /db_xref="taxon:203122"
   gene 1..1392
      /gene="Mdeg3237"
   CDS 1..1392
      /gene="Mdeg3237"
      /codon_start=1
      /transl_table=11
      /product="hypothetical protein"
      /protein id="ZP 00067822.1"
      /db_xref="GI:23029488" 
/translation="MYKISRRTTLKGLGLTCLAGCTTSLPTLEQDPWAFAQNIADNTT
IPTFPNKEFNLLEFGGKEGSDNTLAFKKAIAACSKAGGGKVVVPAGRFETGAIHLESN
VNLHISEGATIAFFTDPKYYLPAVFTRWEGMECMGYSPLIYAYGKTNIAITGKGTLDG
 
QADPTHWWAWKGNKEWGVEGYPSQKESRNQLFAQAEAGDPVRERVYADGHYLRPSFVQ
PYKCENVLIEDITIINAPFWLLHPTLSQNVTVRGVHLESLGPNSDGCDPESCKNVVIE
NCFFNTGDDCIAIKSGRNNDGRRLATPTENVIIRNCKMEAGHGGVVIGSEISGGVRNV
FAENNVMSSPDLEKGIRIKTNSVRGGLLENIYVRNCTIGEVQQAIVINFQYEEGDAGK
FDPTVRNVEIRNLVCQHALQVFNIRGFERAPIQNFRIIDSTFVRGDNPGVIEHTTGLV
IDNVQVNGKAFNI"
ORIGIN 
1 atgtataaaa tttcacgccg cacaacactc aaaggcttag gcctaacttg cctagccggc
61 tgcaccacca gcctacccac actagagcaa gacccatggg cttttgcaca aaacatagcg
121 gacaacacca ccatccccac attcccaaac aaagaattta atttactcga attcggcggc
181 aaagaaggga gcgacaacac cctcgccttc aaaaaagcga ttgcagcatg cagcaaagca
 
241 ggtggcggca aggtggtagt acccgcagga cgatttgaga caggcgccat ccacttagag
301 tcgaacgtta accttcatat tagcgaaggc gctaccatcg ccttttttac cgaccccaaa
361 tattacctgc ctgcggtttt cactcgctgg gaaggcatgg agtgcatggg ctactcaccc
421 cttatatacg cctacggcaa aaccaacata gccattaccg gtaaaggcac cctcgacggt
481 caagccgacc caacgcactg gtgggcatgg aaaggcaaca aagaatgggg cgtagagggc
541 tacccaagcc aaaaggaaag ccgcaaccaa ctatttgccc aagcagaagc tggcgacccc
601 gttagagagc gcgtgtatgc agacggccac tacctgcgcc cctcgtttgt gcaaccctac
661 aagtgcgaaa acgtgctgat agaagacata actattatca acgctccctt ctggttgcta
721 caccccaccc tttcacaaaa cgtcactgta cgcggtgttc acctagaaag cctaggcccc
781 aactcggatg gctgcgatcc tgaaagctgt aagaatgtag ttatcgaaaa ctgcttttt
841 aataccggtg acgactgtat cgctattaaa tctggccgca acaacgatgg ccgcaggctt
901 gccacaccta ccgagaacgt gattattcgc aactgtaaaa tggaagcggg tcacggtggc
961 gtagttatag gctcagaaat ttctggcggc gtgcgcaatg tgtttgccga aaataacgta
1021 atgagcagcc ccgatttaga gaaaggcatt cgcattaaaa ccaactctgt gcgcggcgga
1081 ctgctagaga acatctatgt gcgcaactgc accataggcg aagtacaaca agccattgtt
1141 attaacttcc aatacgaaga aggcgatgcg ggtaaatttg accccaccgt gcgcaatgta
1201 gaaatacgca atttggtctg ccagcacgcc ttacaagtgt ttaacatccg cggttttgag
1261 cgcgccccca ttcaaaactt taggataatc gacagcacct ttgtgcgtgg tgacaaccca
1321 ggcgtaattg aacataccac agggttagtt atcgacaacg tccaagtcaa cggcaaagcg
1381 tttaacatct ag
LOCUS NZ_AABI02000015 3255 bp DNA linear BCT 23-SEP-2003
DEFINITION Microbulbifer degradans 2-40 Mdeg_15, whole genome shotgun sequence.
ACCESSION NZ AABI02000015 REGION: complement(113840..117094)
VERSION NZ_AABI02000015.1 GI:28878287
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3255)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028706. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..3255
      /organism="Microbulbifer degradans 2-40"
      /mol type="genomic DNA"
      /strain="2-40"
      /db_xref="taxon:203122"
   gene 1..3255
      /gene="Mdeg2590"
   CDS 1..3255
      /gene="Mdeg2590"
      /codon_start=1
      /transl_table=11
      /product="COG4677: Pectin methylesterase"
      /protein id="ZP 00067183.1"
      /db_xref="GI:23028801"
      /db_xref="COG:COG4677" 
/translation="MLDMTKRTLSALLALCATLTACGGGDITSGGDAIPAVNQPAPVQ
EPEPEPEPQPEPEPEPEPEPEPEPEGAWTCPETGFYFCDDFEDGTFDDKWDDLIATYDLP
SPGVFDILDEASGKSLRFTAGTRGGDLADGELIVVKDTAFENVTNADYSLEYRIRPRN
NGNTGNKYLHAMSRYEGPKEYYFGGLSMQGSTASTQVEAGFVLPENTTSISNRLVQAK
YPLELGTTGMSDGYWYEVRFDMIGNTGTIYLDGEPQGSFTDADGLYPLTGKIGFMTYN
RSFEIDWVRVGDPAIKPVQLSLDYASPLWEAAADQDPLNVTVTAIQSDGVTADTFTAV
SSDTNVVTTSIANNVVTITPVAQGSATVTFTAGSDANRVKTIDVEIARAFVMSTTDYG
DIASKVTPTVGMTDANPDAHLSITFDSAPTLSGVGSIRIYNAADDSEVDVIRLTDESD
ALGYAGQANKRELNTTPVYLDGNTLHVSPHSNALAYGQDYYVAIGDNVLTGATLNTIA
FDGLGKNAGWTFSTKASAPTGNTVTVDDDASADFSTVQGALNYAMANTTDDSITINIA
NGNYYEPLYLAERNNVTLKGESRDGVVIHYNNHEAMNGGSTGRANFYVANSDMLTLET
LTLKNGHQRTGGGDQAETIYFNSSSNTDRLIAKGAAFISEQDTLLLKGYNWFYNSLVV
GNVDFIWGYSAVTLFEETEIRSIADSKPGAGDSGGYILQARTPLETDLGFVFLNSELT
KATGVNGNEIGDGKTYLARSGGSTGYFDNISFINTKMGSHIADIGFAYADINGQPAPN
 
PAVATADAGWREFGSMDSAGTALDVSARCGDSGSCIQLTQAQVDAQYCNRAQIFASWN
DWTGWDPLPEDTSDDACADPVIPGAVTWTGIAMSLGGSTTSVSGNITEQTDSNITFTA
DGGKFESSKLSTYFAYQELTGDFVISAKAKTIGLLRENGSYQFPTGILMCVCDAAAAT
TGLMGHASLNDITVDTTVNLVATYGHIQTTAGSWNKTGTTDVTAGDNLYIQLERAGNS
YTARYSTDGGATYSNIGGSSFTDTLPDTLKVGFFATPNNTGEQTFVYEDIQITQ"
ORIGIN

1 atgctcgata tgacaaaacg aactctatct gcgttgttag ccctgtgcgc aacattaacg
61 gcttgcggtg gtggcgatat aaccagcggc ggcgatgcta taccggcagt aaaccaaccc
121 gccccagtac aagagcctga acctgaacct gaaccacaac cggaacccga acccgaaccc
181 gagcccgagc cagaaccaga gggcgcgtgg acctgcccag aaacaggctt ctacttctgt
241 gacgactttg aagacggcac gtttgatgac aagtgggacg atctcattgc cacatacgac
301 ctaccaagcc ctggtgtatt cgacatatta gacgaagcaa gcggcaaatc tttgcgcttt
361 acagcaggca cccgtggcgg tgacttagca gatggcgaac ttattgttgt aaaagataca
421 gcattcgaaa atgtaaccaa cgcagattac tccttagagt accgtattcg cccgcgcaac
481 aacggcaaca caggcaacaa gtacctgcac gctatgtcgc gctacgaagg ccctaaagaa
541 tattactttg gcggtttaag catgcaaggc tctactgcaa gtacgcaagt agaagcaggt
601 ttcgtattgc cagaaaacac cactagcatt agcaaccgct tggtgcaggc caagtacccg
661 ttagagctag gtacaacagg catgagcgac ggctactggt acgaagtacg cttcgatatg
721 ataggcaata caggcaccat ttacctagat ggcgaaccac aaggcagctt taccgatgcc
781 gatggccttt acccattaac aggtaaaatt ggctttatga cttacaaccg ctcattcgaa
841 attgattggg tgcgagtagg cgacccagct attaagcctg tacaactttc actggattac
901 gccagcccgc tatgggaagc agcggcagac caagacccgc taaacgtaac agttactgcc
961 atacaaagcg atggcgtaac agcagatacc tttaccgcag ttagcagcga taccaatgta
1021 gtaaccacaa gtattgcaaa taacgtagta accattaccc ctgtagctca aggtagtgcc
1081 accgtgacct ttaccgctgg ttcagatgct aatcgcgtta aaacaattga tgtagaaatt
1141 gcacgcgcgt ttgttatgtc tactaccgac tacggcgata tagcttctaa ggtaacacca
1201 actgttggta tgactgacgc caacccagac gcacatttaa gcattacatt cgatagcgca
1261 cctaccctaa gcggtgttgg ctctatacgt atatacaatg cagcagacga tagcgaagta
1321 gatgttattc gccttaccga cgaaagtgat gcattgggtt acgccggcca agccaacaag
1381 cgtgaattaa ataccacacc ggtttacttg gatggcaaca ccctacacgt tagcccacac
1441 agtaacgcac ttgcctacgg ccaagactac tacgttgcca ttggcgataa cgtacttacc
1501 ggcgcaacac taaacaccat tgcgtttgat ggtttaggta aaaacgcggg ttggactttc
1561 tctaccaaag cctctgcccc taccggcaac accgtaactg tagacgacga tgcaagtgca
1621 gatttcagca cagtacaagg tgcgttgaac tatgctatgg caaataccac ggacgattca
1681 atcaccatta acattgctaa cggcaactac tacgagccgc tatatctagc agagcgcaac
1741 aacgtaacgc taaaaggtga aagccgcgac ggcgttgtta ttcattacaa caaccacgaa
1801 gccatgaacg gtggcagcac tggccgcgca aacttctatg ttgccaactc agacatgcta
1861 accctagaaa cgctaaccct taaaaacggt catcagcgca ctggtggtgg cgaccaagca
1921 gaaactatct acttcaatag cagcagcaat accgatcgct taattgccaa aggcgctgct
1981 tttattagtg aacaagatac gctgttactt aaaggctaca actggttcta caactcgctt
2041 gtggtaggta acgtagactt tatttggggc tacagcgcag taaccttgtt tgaagaaaca
2101 gaaattcgat ctattgccga ctctaaacca ggtgcgggcg actcgggtgg ctatattctg
2161 caagcgcgta cgccactaga aacagacctt ggctttgttt tcttaaatag cgaattaaca
2221 aaagctaccg gcgtaaacgg taacgaaatt ggcgatggca aaacctacct tgcgcgcagc
2281 ggcggcagca cgggttactt cgataatatt tcgtttatta acaccaaaat gggtagccat
2341 attgccgaca taggcttcgc ctacgccgac attaacggtc aacctgcccc taacccagcg
2401 gtagctactg ctgacgcagg ctggcgtgaa tttggcagca tggattctgc aggcacggct
2461 ctagatgtat ctgcacgctg cggtgatagc ggcagctgta tccaacttac gcaagcacaa
2521 gtagatgcgc agtactgtaa ccgcgcgcaa atttttgcta gctggaacga ttggacaggc
2581 tgggacccgt tgccagaaga tacctctgac gatgcctgtg ctgaccccgt tatacctggt
2641 gcagtaacgt ggactggcat tgcaatgagc cttgggggtt ctacaacatc tgtttccggc
2701 aacattaccg agcaaacaga cagcaatatt acattcactg cagacggcgg taagtttgaa
2761 tcgagcaaac tttcaactta cttcgcttat caagaattga ctggcgactt tgtaattagc
2821 gccaaagcta aaaccattgg cttactgcgc gaaaacggca gctaccagtt ccctacaggc
2881 atattgatgt gtgtttgcga tgcggcagcg gcaacaactg gcttaatggg ccacgccagc
2941 ctcaatgaca ttacagttga tactactgtg aatttagttg ccacctacgg ccacattcaa

3001 accacagctg gtagctggaa taaaactgga acgactgacg taaccgctgg cgacaacctg
3061 tatatacagc tagagcgcgc aggtaatagt tataccgcac gctactcgac tgatggcggt
3121 gccacctata gcaacattgg tggcagctca tttacagaca cccttccaga cacacttaaa
3181 gtgggtttct tcgctacgcc taacaacacc ggtgagcaaa ctttcgttta cgaagatata
3241 caaatcactc agtaa

LOCUS NZ_AAB102000026 1176 bp DNA linear BCT 23-SEP-2003
DEFINITION Microbulbifer degradans 2-40 Mdeg_26, whole genome shotgun sequence.
ACCESSION NZ AABI02000026 REGION: complement(18555..19730)
VERSION NZ_AABI02000026.1 GI:28878276
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1176)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029482. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1176
      /organism="Microbulbifer degradans 2-40"
      /mol type="genomic DNA"
      /strain="2-40"
      /db_xref="taxon:203122"
   gene 1..1176
      /gene="Mdeg3246"
   CDS 1..1176
      /gene="Mdeg3246"
      /codon_start=
      /transl_table=11
      /product="COG4677: Pectin methylesterase"
      /protein_id="ZP 00067831.1"
      /db_xref="GI:23029497"
      /db_xref="COG:COG4677" 
/translation="MGMGTKINFLLLGFILSACSLSGCADKIKRNTPLTETALPSKKI
LYVQTEVCDPPSQLTGSCYNSLQRAIDVAHTVPSATHVTIEMAAGHYHERIVLSRGNI
DIVGAGKNKTYVQYNLNAEQGKAYHRDGWGTPGSATFTINASEVNVSDLTIENTFDFL
RNDSKDKTDPSKVRASQGVALLLDEHSDKVALYRVGLYGYQDTLFANGKRAFIYQSDI
AGNVDFIFGAGQVVIENSRVISRPRGKAIASNEIAGYITAPSTNITDAFGLVFINSRL
EREQGVADASVTLGRPWHPTTNFSDGRYADPNAIGHALFFNCFMDAHIHPARWSSMKG
TAKDGSKTLVFTPEQSRFFEVQSFGPSGNDEVTTSYHSLSADSLREQALGDWNVSIN"
ORIGIN 
1 gtgggtatgg gtacaaaaat taattttttg ctattaggtt ttattttgtc tgcatgttca
61 ttaagtggct gcgccgacaa aataaagcgc aatacgcctt taaccgaaac ggcattgccg
121 agtaaaaaaa tactctatgt acaaaccgaa gtatgtgacc cgccttcgca attaacgggt
181 agttgctaca acagcttgca gcgagctatt gacgtggcgc acacagtgcc gtctgctacc
241 catgtaacca ttgaaatggc tgcggggcat taccacgaac gcattgtgct tagccgtggc
301 aatatcgata ttgttggtgc aggtaaaaat aaaacctatg ttcaatacaa cctgaatgcc
 
361 gagcagggta aagcttatca ccgcgacggt tggggtactc ctggctcagc tacatttacc
421 attaatgcca gtgaagtaaa tgttagcgat ttaactatcg aaaatacttt cgactttta
481 agaaatgatt caaaagataa aaccgaccct tcaaaagtga gggcatcgca aggcgttgca
541 ttattattgg atgaacacag cgataaggtt gcgctgtatc gagtaggcct atacggatac
601 caagacaccc tatttgcaaa tggaaagcgt gcatttatct accaatcaga tattgcaggc
661 aatgttgatt ttatttttgg cgctggccaa gtggttatag aaaatagtcg tgttatttct
721 aggccgcgcg gcaaagccat tgcttccaat gaaattgccg gctatatcac agcgccatcc
781 accaatatta cggacgcctt tggtctggtt tttattaata gtcgattaga acgtgagcaa
841 ggcgtggcag atgcgtcggt caccttgggt cgcccttggc accctacaac caatttcagc
901 gatggccgat atgccgaccc aaacgcgatt ggccatgcgc tattttttaa ctgctttatg
961 gatgcgcata ttcaccccgc gagatggtct agcatgaaag gcaccgctaa agacggcagt
1021 aaaacgctag tgtttacgcc cgagcaatcg cgtttttttg aagttcagtc ctttggcccc
1081 agcggcaacg atgaagtaac cacctcgtat cattcgttaa gcgccgactc attacgcgaa
1141 caagcgctcg gcgattggaa tgtatcaatt aactaa
LOCUS NZ_AABI02000011 2745 bp DNA linear BCT 23-SEP-2003
DEFINITION Microbulbifer degradans 2-40 Mdeg_9, whole genome shotgun sequence.
ACCESSION NZ AABI02000011 REGION: 36905..39649
VERSION NZ_AABI02000011.1 GI:28878291
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2745)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028020. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2745
      /organism="Microbulbifer degradans 2-40"
      /mol type="genomic DNA"
      /strain="2-40"
      /db xref="taxon:203122"
   gene 1..2745
      /gene="Mdeg1862"
   CDS 1..2745
      /gene="Mdeg 1862"
      /codon_start=1
      /transl_table=11
      /product="hypothetical protein"
      /protein id="ZP 00066475.1"
      /db_xref="GI:23028057" 
/translation="MSALTRPKFGAHTKLFHAIKHALTPVIFLGAAAFPLAAHSQYNM
ENLDRGLVAIDRKDGSVLVSWRWLGQEPDNTSFNVYRNGTLLTSSPLTNKTNFVDTSG
NPNANYAVEAIVNGASQSLATTHVWSDIYRTIPLQRPPGGTTPDGVAYTYSPNDISAA
DLDGDGGYELIVKWDPSNAKDNSQSGYTGNVYLDAYEISGEFMWRIDLGRNIRAGAHY
TQFLAFDFDSDGKAEVAVKTADATKDSQGVVIGDSNADYRNSAGYVLSGPEYLTMFEG
QTGRALNTVNYVPARGSVSSWGDNYGNRVDRFLGGVAYLDGQNPSLIMSRGYYTRTVV
AAWDWRNGQLSQRWVFDSNTSGNSSYAGQGAHSLTIGDVDADGKQEIVFGAMTIDDNG
TGLNNTRLGHGDALHLSDMDPSNPGLEVFMVHECPSCYGEHGIEMHDAATGQILWSHP
GDYIDIGRGVAMDIDPRYAGYEAWASRGGLYSAKGETISSTRPSQINFAAWWDGDLLR
EILDNNYINKWNYTASSTTRLLSAGNYGAASNNGTKATPGLSADILGDWREEVVWRNS
NNQELMVFTTPHESEYRLRTLMHDPQYRTAIAWQNVGYNQPPHPSYFLGAGMTTPNQP
HITIVGEGTVQPPAPTGDAIQENATGFCGYEGTIDSNHSGYTGAGFTNTTNATGAGIN
WNLHASTAGTYRLSMRYANGSTARGAVLNVETTGNSYPMAFAPTSTWTNWQEEYVDAH
LNAGYNSIRLEANQAAGLPNLDAIYLADGLTAAACGQTSSSSSSSSSSSSSSSSSSS
SSSSSSSSSSSSSGGTTAGLACANGSTDTWGTGFVLNGFQVENEGQQATNNWQVTLQF
DQPVNITNAWGVNVETTGTTVVATSVGYNSHLNPGQSASFGMQGTSATAVSNPLCSAQ
 11
   gene complement(2292..2540)
      /gene="Mdeg1863"
   CDS complement(2292..2540)
      /gene="Mdeg1863"
      Icodon_start=1
      /transl_table=11
      /product="hypothetical protein"
      /protein_id="ZP 00066476.1"
      /db_xref="GI:2-3028058" 
/translation="MACWPSFSTWKPFNTNPVPHVSVLPLAQARPAVVPPDELLLELL
LELLLELEELLEELEELLEDELLLDVWPHAAAVRPSAR"
ORIGIN 
1 atgtcggcac tcactcgtcc caaatttggt gcacacacca aactgttcca cgctataaag
61 catgcgttaa cccccgttat atttttaggt gctgctgctt tcccacttgc cgctcacagc
121 caatacaaca tggaaaacct cgaccgcggg ttggtggcca tagatcgcaa agacggcagc
181 gtattagtaa gctggcgctg gttggggcaa gagccagata acaccagctt caacgtatat
241 agaaacggca cactattaac cagctccccc cttaccaata aaacaaactt tgtagatacc
301 agcggcaacc caaacgctaa ttacgcggta gaagccatag taaacggcgc gagccaatca
361 ctagccacca cacatgtatg gagcgatata taccgcacta ttccgctgca aagaccacca
421 ggtggcacca cccccgacgg agtggcatac acctatagcc ccaacgatat aagcgcagcc
481 gatttagacg gcgatggcgg ttacgagcta atcgtaaaat gggacccctc caacgcaaaa
541 gacaactcgc aaagtggcta caccggcaat gtgtatctcg acgcttacga aatatctggc
601 gagtttatgt ggcgcataga cctaggccgc aatattcgag caggcgccca ctacacgcaa
661 tttttagcat tcgattttga tagcgatggc aaagcagaag ttgcagttaa aaccgcagac
721 gccaccaaag acagccaagg agtagtaata ggagacagca atgccgatta ccgcaacagt
781 gcaggctatg ttttatctgg ccccgaatac ctcaccatgt ttgaaggcca aaccggtaga
841 gcgctaaata ccgttaacta cgtacccgcg cgcggcagtg tttctagctg gggcgacaac
901 tacggcaacc gtgttgatag atttttaggt ggcgtagcct acttagatgg ccaaaaccca
961 agtttgatta tgtcgcgcgg gtactacacc cgcaccgtgg tagcagcatg ggactggcgc
1021 aatgggcagc ttagccaacg ctgggtattt gattccaaca ccagtggcaa cagcagctat
1081 gcaggccaag gcgcgcacag ccttaccatt ggcgatgtag atgcagacgg aaaacaagaa
1141 attgtatttg gcgccatgac catagatgac aacggcaccg gcttgaacaa tacccgccta
1201 ggtcacggcg atgcactgca cctatccgac atggacccaa gcaacccagg cttagaagtg
1261 tttatggtgc acgaatgccc ctcttgctat ggcgagcacg gaatagaaat gcacgatgcc
1321 gccaccggcc aaatactatg gagccaccca ggcgactaca tagacatagg ccgaggtgta
1381 gccatggata tagacccacg ttatgccggc tacgaagctt gggcttcgcg cggtggttta
1441 tacagtgcaa aaggcgaaac aatatcgagc acgcgcccgt cgcaaataaa ctttgccgct
1501 tggtgggatg gcgacttact gcgcgaaata ctcgataaca attacataaa caaatggaac
1561 tacaccgcca gctctaccac gcgcttgcta agcgcaggta actacggtgc agcatcgaac
1621 aacggcacca aggctacacc agggctttcg gccgatattc ttggtgactg gcgcgaagaa
1681 gtggtatggc gcaacagcaa caaccaagaa cttatggtgt ttaccacccc acacgaaagt
1741 gaataccgct taagaaccct aatgcacgac ccgcaatacc gcacagccat agcttggcaa
1801 aacgtgggct acaaccaacc acctcacccc tcttactttt tgggtgcggg tatgactacc
1861 cccaaccaac cacacataac catagttggc gaaggcacag tgcagcctcc agcccctaca
1921 ggcgacgcaa tacaagaaaa cgccaccggc ttttgcggct acgaaggcac tatagatagc
1981 aaccactctg gctatacagg cgcgggcttt actaacacta ccaacgcaac aggtgcaggc
2041 attaactgga acctacatgc gtccaccgcc ggtacatacc gcctaagcat gcgttacgcg
2101 aatggcagta cggcacgagg tgctgtgcta aacgtagaaa caaccggtaa tagctacccc
2161 atggcatttg caccaacaag cacatggaca aactggcaag aagaatatgt agacgcccac
2221 ctaaacgccg gctacaacag cattcggctt gaagcaaacc aagcggcagg tttgcccaac
2281 ctcgatgcca tctacctagc cgatggtctt acggcggcag cgtgcggcca aacatctagc
2341 agcagctcgt cctccagtag ctcctctagt tcttccagca gctcttcaag ctctagtagc
2401 agttccagta gtagctcgag cagcagctca tccggcggta caacagcagg cctagcttgc
2461 gccaacggca gtacagatac atggggaacg gggtttgtgt taaacggctt ccaagtagaa
2521 aacgaaggcc agcaagccac caataactgg caagtgacac ttcaattcga ccagcccgta
 
2581 aacataacca acgcatgggg tgtaaacgta gaaacaacag gcacaaccgt tgtagcaaca
2641 agcgtaggct acaacagcca cctaaacccg gggcaaagtg ctagctttgg aatgcaagga
2701 acatcggcaa cggcggtaag caacccgcta tgcagtgccc agtaa
LOCUS NZ_AAB102000009 2370 bp DNA linear BCT 23-SEP-2003
DEFINITION Microbulbifer degradans 2-40 Mdeg_10, whole genome shotgun sequence.
ACCESSION NZ AABI02000009 REGION: 151580..153949
VERSION NZ_AABI02000009.1 GI:28878293
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2370)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028157. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2370
      /organism="Microbulbifer degradans 2-40"
      /mol type="genomic DNA"
      /strain="2-40"
      /db_xref="taxon:203122"
   gene 1..2370
      /gene="Mdeg2067"
   CDS 1..2370
      /gene="Mdeg2067"
      /codon_start=1
      /transl_table=11
      /product="COG3507: Beta-xylosidase"
      /protein id="ZP 00066674.1"
      /db_xref="GI:23028263"
      /db_xref="COG:COG3507" 
/translation="MRSLAPIKIREKIRETLMFNIRAWQLDLPLALLAFSSTSYAIDN
GTYTIQSKHSGKVVEVAAGSVDDAANVAQWPSNGHPTQQWIITQISGDDYSVINVNSG
KAMEVYDFGTTDGGNIVQYPYWGGAPQLWTITDQGGYYSLINKHSGKALDLLNWDTTD
GANIGQWAWWGGDAQLWALNTVQPSTVTFTLEENQAGFCSVDGSIDSNHTGYTGSGFA
NTTNANGQGVNWSVNVATAGTYTFTWRYAGTSNRPANLLIDGSTQVSGIALNSTGAWA
TWANSAEISVWLDTGVHSLRLQATTSAGLPNIDSLSITGQSAAAGNCSGAIEPITFAT
PSFTNIAVHDPSVIEANHQYYVFGSHLSVAKTPDLKNWSRVADGVTTNNPLFNDVTSE
LAEALAWAETTTLWAPDVTYVNGRYLMYYNACRGDSPLSAMGIASSNNIEGPYTNDGI
FLKSGMWGQTSEDGTVYDATVHPNAVDPVIFSDANNRMWMTYGSYSGGIFIMELNPST
GFPYAGQGYGKHLMGGNHARIEGAYTIYSPETGYYYMYVSYGGLGADGGYNVRVARAT
SPDGPYYDANGTNMANVKSNPSLPLFDDASIAPHGVKLMGNHVFSGTNNVLGYVSPGH
NSAYRDATTGQTFLLFHTRFPGRGEEHEVRVHEVFYNDAGWPVIAPLRYAQKVDANNP
NRSASELNAVYASELPGSYQLINHGKDISATIKNSVNITLNSNGSISGELSGSWTYNA
NTRNTVITVAGVAYRGVVSRQWNQARNRFEVTFSALSADGTAIWGVNSD"
ORIGIN 
1 gtgcgcagtc tagcccccat aaaaataaga gagaaaataa gagagacact catgtttaat
61 atacgcgctt ggcaacttga cttgcccttg gcgttattgg cgttttcgtc tacaagttac
121 gctatcgata acggcactta cacaattcaa tctaaacaca gtggcaaggt tgtagaagtg
181 gccgcaggca gtgtagatga tgctgcaaat gtggcccaat ggcccagtaa tggccatcct
241 acccagcagt ggataattac ccaaattagc ggcgatgatt actcggtaat aaatgtaaat
301 agcggcaaag ctatggaggt atacgacttc ggcaccacgg acggcggcaa catagtgcaa
361 tacccctact ggggaggcgc cccccagttg tggacaatta cagatcaagg cggttattac
421 agcttaataa acaaacacag tggtaaagct ttagatttgt taaattggga taccacagac
481 ggcgccaata taggccagtg ggcttggtgg ggcggcgatg cgcaactgtg ggcactgaac
541 acagtgcaac ccagcacagt caccttcaca cttgaagaaa accaagcggg tttttgcagc
601 gtagatggca gcatagatag caatcatacg ggctataccg gcagcgggtt tgccaataca
661 accaatgcaa atggccaagg agttaattgg tcggtaaatg tagccacagc tggtacctat
721 acgtttacat ggcgctatgc gggcactagc aaccgcccag ccaacttgct aatagatggc
781 agcacacagg tttcgggcat tgctttaaat tcaaccggcg catgggcaac ttgggctaat
841 agtgcagaaa taagtgtttg gcttgacaca ggtgtgcact cacttcggct gcaagccaca
901 accagcgcgg gcttacctaa tatagattcg cttagcataa ccggccaaag cgcagcagcg
961 ggcaactgca gcggcgcaat tgaacctatc acttttgcca caccaagctt taccaatata
1021 gcggtgcacg acccctcggt aatagaagca aaccatcaat actacgtatt tggctcccac
1081 ctttctgtgg ctaaaacgcc cgacctaaaa aactggtcgc gcgtggcgga tggtgtaacc
1141 accaataacc cattgtttaa cgatgtaacc agcgaacttg cagaagcatt agcttgggca
1201 gaaaccacta ccctgtgggc gccagatgtt acctatgtaa atggtcggta tttgatgtat
1261 tacaacgcct gccgtggcga ctcaccactg tcggctatgg gtattgcttc ttcgaacaac
1321 atagaaggcc cttacactaa cgatggtata ttccttaaat ctggaatgtg gggccaaacc
1381 agcgaagatg gcactgtgta cgacgcaacc gtgcacccca atgctgtgga ccccgttatt
1441 tttagcgacg caaataatcg catgtggatg acctacggtt cgtattcggg tggtattttt
1501 attatggagt taaacccatc cacggggttc ccttacgcgg ggcaaggtta tggtaaacat
1561 ttaatgggtg gcaaccacgc gcgcattgaa ggcgcttaca ccatctacag cccagaaacg
1621 ggctactact atatgtatgt aagctacggt ggcctaggcg cagatggcgg ctataacgtt
1681 cgtgtggccc gagcaactag cccagacggc ccctactatg atgccaacgg caccaatatg
1741 gccaacgtaa aaagcaaccc aagcttgcca ctgttcgacg acgccagcat agcaccccac
1801 ggtgtaaaac ttatgggtaa ccacgtgttt agcggcacta acaatgtact tggttacgta
1861 tcaccagggc acaactctgc ataccgtgac gccactaccg gccaaacatt tttactattc
1921 cacacacgct tccctgggcg cggcgaagag catgaagtgc gagtgcatga agtgttctac
1981 aacgatgcag gctggccggt aatagcacca ttgcgctatg cccaaaaagt agatgccaac
2041 aacccaaata gaagtgcgag cgagctaaat gcagtgtacg caagcgaact gccaggtagc
2101 tatcagctaa ttaaccacgg caaagacata agcgcgacaa ttaaaaattc cgttaacatc
2161 acgctaaaca gcaacggcag tatctctggc gagctatctg gcagctggac atacaacgcc
2221 aacacccgca ataccgtaat caccgttgcc ggtgtggcct accgcggcgt ggtatctcgc
2281 caatggaacc aagcacgcaa ccgcttcgaa gtcaccttca gcgccctatc tgcagacggc
2341 acagcaatat ggggggtgaa cagcgactaa
LOCUS NZ_AAB102000014 1089 bp DNA linear BCT 23-SEP-2003
DEFINITION Microbulbifer degradans 2-40 Mdeg_13, whole genome shotgun sequence.
ACCESSION NZ AABI02000014 REGION: 123595..124683
VERSION NZ_AABI02000014.1 GI:28878288
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1089)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028504. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1089
      /organism="Microbulbifer degradans 2-40"
      /mol type="genomic DNA"
      /strain="2-40"
      /db_xref="taxon:203122"
   gene 1..1089
      /gene="Mdeg2389"
   CDS 1..1089
      /gene="Mdeg2389"
      /codon_start=1
      /transl_table=11
      /product="COG3940: Predicted beta-xylosidase"
      /protein_id="ZP 00066990.1"
      /db_xref="GI:23028597"
      /db_xref="COG:COG3940" 
/translation="MSSFIMDKSQLQSGFAFKTSGFNVLIVVTFLALLAALVGCSSAK
LAPVASPSLPQPLVAQRADPWVHKHSDGYYYFIATVPAYDRLEMRRATTIAGLRSAPA
VVVWQRNTIGGMSANIWAPELHFIDGKWYIYVAAATDHNKPWTIRMHTLSNASANPMQ
GEWQEEGRFHTPLDTFSLDATTFEHRGKRYLVWAQQNEARTYNSALLIAQMDSPTSIT
GPIVTLSEPTLPWEIIGHKVNEGAAVIKHGKRIFISYSASATDHNYAMGLLWADENAD
LLDAASWTKSPEPVFYSNEQLKRFGPGHNCFVKAEDGVTDLMVYHARDYKEIDGEPLR
DPNRHTRVRKVYWDEQGMPDFRQHEADL"
ORIGIN 
1 atgagtagct tcataatgga taaaagccag ctacaaagtg gttttgcgtt taaaacaagt
61 ggttttaacg tgctgattgt tgtcacattt ttggcattgc ttgcagcgct tgttggctgc
121 agcagcgcca agctcgcacc cgtcgcctct cctagtttac cgcagccatt agtggcccaa
181 cgggcagacc cttgggtgca caagcacagc gatggttatt actactttat agcaacggta
241 ccagcatacg accgcttaga aatgcgtagg gccacaacca tagcaggctt acgtagcgcg
301 cccgctgtag tggtatggca gcgcaatact attggaggta tgagcgcgaa tatttgggcg
 
361 cccgagctgc attttattga tggtaaatgg tacatctatg tagcggctgc caccgatcac
421 aacaagccgt ggacaattcg tatgcacacg ctttccaatg catcggccaa ccctatgcaa
481 ggtgagtggc aagaagaggg gcgctttcat acaccgctag atactttctc gctagatgcc
541 acaacctttg agcacagggg taaacgctat ttagtatggg cgcaacagaa tgaagcccgt
601 acttataact cggcgttact tatagcgcaa atggatagcc ctacaagtat tactggcccc
661 attgttacct taagtgaacc gacattaccg tgggaaatta ttggccataa ggttaatgag
721 ggtgcggcag taattaaaca cggtaagcgt atttttataa gttattccgc cagtgcgacc
781 gatcataact atgcgatggg tttgttatgg gcagacgaaa acgctgattt gctcgacgca
841 gcaagctgga ccaagtcacc cgagcctgta ttttactcaa acgaacaatt aaagcgcttt
901 ggccctggcc ataattgttt tgttaaagct gaagatggtg ttaccgattt aatggtgtac
961 cacgcgcgtg attataaaga gatagatggt gagccattgc gagacccaaa ccgccacacg
1021 cgggtgcgca aagtgtattg ggatgaacag ggcatgccgg atttcgtcaa acatgaagca
1081 gacctatag
LOCUS NZ_AAB102000014 945 bp DNA linear BCT 23-SEP-2003
DEFINITION Microbulbifer degradans 2-40 Mdeg_13, whole genome shotgun sequence.
ACCESSION NZ AABI02000014 REGION: 109190..110134
VERSION NZ_AABI02000014.1 GI:28878288
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 945)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028504. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..945
      /organism="Microbulbifer degradans 2-40"
      /mol_type="genomic DNA"
      /strain="2-40"
      /db_xref="taxon:203122"
   gene 1..945
      /gene="Mdeg2380"
   CDS 1..945
      /gene="Mdeg2380"
      /codon_start=1
      /transl_table=11
      /product="COG3940: Predicted beta-xylosidase"
      /protein_id="ZP 00066981.1"
      /db_xref="GI:23028588"
      /db_ref="COG:COG3940" 
/translation="MKKLSPLIEQRADPYIYKHTDGYYYFTASVPAYDGIELRRAKTI
QALATAETVMVWRKPSEGDYSELIWAPEIHFNMGAWYVYFAAAPSREIKFDLFQHRMY
AISCSDANPLTGEWIFEGKIDSGIDAFCLDATTFTHSNELYYVWAQKELDVRGNSNLM
IAKMETPTKLATKPVRLSKPEYDWEIQGFWVNEGPSIVKHGSRIFISYSGSATDERYA
MGILWAEQSADLLDPASWTKSVEPVLVSEPSEKVFGPGHNSFTVDEEGNDMLVYHARN
YTEIEGDPLWDPNRHTYVKKLRWDETGMPIFGSPAFEE"
ORIGIN 
1 gtgaaaaaat tatcgcctct tatagagcaa cgtgcagacc cttatattta taaacacacc
61 gacggctact attattttac ggcttcggtg cccgcctatg atggcataga actgcgtcgc
121 gcaaaaacta tacaagcgtt agccaccgca gaaaccgtta tggtgtggcg caagccaagc
181 gaaggtgatt atagtgagct tatttgggcg ccagaaatac actttaatat gggggcttgg
241 tatgtatatt ttgctgcggc tccatcacgt gaaattaagt tcgatttatt tcaacaccgc
301 atgtatgcca ttagctgtag cgatgccaac ccgctaacag gtgaatggat atttgaaggt
361 aaaatagata gcggcataga tgcattctgt ttagatgcca ccacctttac tcacagcaat
 
421 gagctctact atgtttgggc gcaaaaagaa ttagatgttc gcggcaactc taatttgatg
481 atcgcaaaaa tggaaacgcc caccaagctt gccaccaagc ccgtgcgttt atctaaaccc
541 gaatacgact gggagattca gggtttttgg gttaacgaag gcccatccat tgttaagcac
601 ggctcacgta tttttatttc ttattctggc tctgctaccg atgagcgcta cgcaatgggt
661 attttgtggg cagaacaaag cgcagactta ctagacccag caagttggac caagtcggta
721 gagcctgtat tagtttctga accctctgaa aaagtatttg gcccaggcca caatagtttt
781 actgtggatg aagagggtaa cgatatgttg gtgtatcatg ctcgcaatta taccgaaatt
841 gaaggcgacc cgctgtggga cccaaatcgt catacttacg ttaaaaaatt gcgctgggat
901 gaaacaggca tgcctatttt tggcagccct gcgtttgaag agtag
LOCUS NZ_AAB102000038 1053 bp DNA linear BCT 23-SEP-2003
DEFINITION Microbulbifer degradans 2-40 Mdeg_38, whole genome shotgun sequence.
ACCESSION NZ AABI02000038 REGION: <3..1055
VERSION NZ_AABI02000038.1 GI:28878264
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1053)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029863. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1053
      /organism="Microbulbifer degradans 2-40"
      /mol_type="genomic DNA"
      /strain="2-40"
      /db_xref="taxon:203122"
   gene <1..1053
      /gene="Mdeg3581"
   CDS <1..1053
      /gene="Mdeg3581"
      /codon_start=1
      /transl_table=11
      /product="COG3507: Beta-xylosidase"
      /protein_id="ZP 00068164.1"
      /db_xref="GI:23029864"
      /db_xref="COG:COG3507" 
/translation="NGYYAVLNKHSGKALDLYGFDTSNGANIAQWAFWGGDPQQWQFT
KIANVGAPPVDTSTTNGATNHWSLTGNLVTHDPTMAYENGSWWLYQTGEGIYGKYSAN
GLAWDGLPSVFPNGLSWWKTYVPGQSNNDVWAPDVRTYNGRVYLYYSISTFGSRVSAI
GLASASSLAASDWQDHGLVINTTSSSDWNAIDPDLVVDEHGNPWLTMGSWNSGIKVMR
LNPITMKPIGTLYSIAQKGGGIEAPSIVYRRGYYYLFVSIGKCCAGVDSTYQIAYGRS
TSITGPYLDKNGNDMMSGGGSILDAGNNVWVGPGGQDIINTDVIVRHAYDATDAGTPK
MIISTLNWDANGWPKY"
ORIGIN 
1 aatggctatt atgccgtgct aaataaacac agcggcaaag cgttagattt gtatggtttt
61 gatacgtcta acggcgcgaa tattgcgcaa tgggcctttt ggggcgggga cccgcagcag
121 tggcaattta ccaaaatcgc caatgtaggt gcgccgccag tagatacatc taccaccaac
181 ggtgcaacca accactggtc cttaaccggt aatctagtga ctcacgaccc cacaatggcc
241 tacgaaaacg gctcatggtg gttgtatcaa accggcgagg gaatttacgg taagtattca
301 gccaatggtt tggcgtggga tggcttacct tctgtgtttc ccaatggttt aagttggtgg
 
361 aagacctatg tacccggcca gtcgaacaac gatgtatggg cgcctgatgt acgcacttat
421 aatgggcggg tttatttgta ctattccatc tctacttttg gctcgcgtgt atctgccatt
481 ggtttggcgt cggcatcgag tttggctgcg agtgattggc aggaccacgg cttagtaatt
541 aataccacct catctagcga ttggaatgcg atcgacccag atttagtggt cgatgagcat
601 ggcaaccctt ggttaacaat gggaagttgg aacagcggta ttaaagtgat gcgcttgaac
661 cccattacca tgaagccaat tggcacactt tattctattg cgcaaaaggg cggcggtatt
721 gaagcgcctt ctattgtgta tcgccgtggg tattactatt tatttgtttc tatcggcaaa
781 tgctgtgcgg gcgtagatag cacctatcaa attgcttacg ggcgctctac aagtattacc
841 ggcccttatt tggataagaa cggcaacgat atgatgagtg gtggtggcag tatttagat
901 gcgggcaaca acgtgtgggt tggccctggt gggcaagata ttattaacac cgatgtcatt
961 gtgcgccacg cgtacgatgc cacagatgca ggcacaccta agatgattat tagtaccttg
1021 aattgggatg ctaatggatg gccgaaatac tag
LOCUS NZ_AABI02000014 1041 bp DNA linear BCT 23-SEP-2003
DEFINITION Microbulbifer degradans 2-40 Mdeg_13, whole genome shotgun sequence.
ACCESSION NZ AABI02000014 REGION: complement(121237..122277)
VERSION NZ_AABI02000014.1 GI:28878288
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1041)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028504. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1041
      /organism="Microbulbifer degradans 2-40"
      /mol type="genomic DNA"
      /strain="2-40"
      /db xref="taxon:203122"
   gene 1..1041
      /gene="Mdeg2388"
   CDS 1..1041
      /gene="Mdeg2388"
      /codon_start=1
      /transl_table=11
      /product="COG3507: Beta-xylosidase"
      /protein id="ZP 00066989.1"
      /db_xref="GI:23028596"
      /db_xref="COG:COG3507" 
/translation="MLNKNKRPITFALVVSLLALLALAGCSEAKQVSIHDPVMIKEGD
TYYLFSTGPGITMYSSSDMKNWRREGEVFNQAPSWASNAVPYFKGHLWAPDIIEKDGL
FYLYYSVSAFGKNTSGIGVTVSPTLNPRAPNYGWQDKGMVLRSVPERDEWNAIDPNIV
VDNNGTAWMAFGSFWQSLKMVALDSSWTKIAEPQQWHTIAALPKGSMPTGDAVKDGEI
EAPFIFKKNDDYFLFVSWGKCCRKDESTYRLAMGRSKNTTGPFLDKNGKDLAQGGGTL
LISGNKNWPGLGHNSAYTFDGKDWLVLHAYESADNGLQKLKILEINWDKDGWPTVDTK
ELDEFVSIELTQ"
ORIGIN 
1 atgcttaaca aaaacaaacg cccaattaca ttcgctttag tcgttagcct cttagccctg
61 cttgcccttg caggctgcag cgaggcaaaa caagtaagca tccacgaccc agtaatgatt
121 aaagaaggtg acacctacta cttgtttagc actggccccg gcataacaat gtatagctct
181 agcgatatga aaaactggcg ccgcgaaggc gaagtattta atcaagcccc tagttgggcc
241 tccaacgccg taccctattt taaaggccac ctgtgggcac ccgacatcat tgaaaaagat
301 ggtctgtttt acctctacta ttctgtgtct gcttttggaa agaacacatc cggcattggc
 
361 gttaccgtat cgcccacgct taacccacgc gcgcccaatt acggttggca agataaaggc
421 atggtattgc gcagcgtgcc tgagcgcgac gagtggaacg ctatcgaccc caatattgtg
481 gtagataaca acggcaccgc atggatggct tttggctcct tttggcaaag cttaaaaatg
541 gtggcactag acagcagctg gacaaaaata gctgagcctc aacagtggca taccatagca
601 gccttaccca aaggcagtat gcccacaggc gacgcagtaa aggacggcga aatagaagct
661 ccttttattt ttaaaaagaa cgacgattac tttttgtttg taagttgggg taaatgctgc
721 cgcaaagatg aaagcaccta ccgcctagca atgggccgca gcaaaaatac taccggtcca
781 ttcttagata aaaacggcaa agacctcgcc caaggtggtg gcaccctatt aataagtggc
841 aacaaaaact ggcccggctt aggccacaac agcgcctaca ccttcgacgg caaagattgg
901 cttgtgctac acgcctatga atctgcagat aacggtttac aaaaactaaa aatattagaa
961 ataaactggg ataaagacgg ctggccaact gtagatacca aagaactgga tgagtttgtt
1021 agtattgaat taactcaata a
LOCUS NZ_AABI02000014 1860 bp DNA linear BCT 23-SEP-2003
DEFINITION Microbulbifer degradans 2-40 Mdeg_13, whole genome shotgun sequence.
ACCESSION NZ AABI02000014 REGION: 129042..130901
VERSION NZ_AABI02000014.1 GI:28878288
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1860)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028504. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1860
      /organism="Microbulbifer degradans 2-40"
      /mol_type="genomic DNA"
      /strain="2-40"
      /db_xref="taxon:203122"
   gene <1
      /gene="Mdeg2392"
   CDS <1
      /gene="Mdeg2392"
      /codon_start=1
      /transl_table=11
      /product="hypothetical protein"
      /protein_id="ZP 00066993.1"
      /db_xref="GI:23028600" 
/translation="MDNIMKMIIKLALAVTLAVWVAGCTNQAGLNAENKNIERQTINS
PDKSLKVRLSLDESGKVFYSISRNGEQVMLPSQLGVELNSQAFTDGLTITDVDAGKVN
DSYTLLHGKQRDITYNANEKIYSLKNKQGDKLIIAFRVSNDGVAFQYRFPNTAKQLLA
VKKEITSFAFEHTTKAWLQPIAVAQTGWANTNPSYEEHYQMNIPVDTVSPSPAGWVFP
ALFKANKHWLLITEAGMNGDYHASRLHAESPNGEYSLGIPMAAEVFEQDGNKGALLAQ
SNTAFHSPWRVILVGGLDTIIASTLGTDLADPAIAKMDFVKPGTASWSWALLKDESVN
YETSLEFIDYAAEMGWDYTLVDADWDRRIGYERTAQLAAYAQSKNVGLLVWYNSSGDW
NTTEYSPKSALLDRDKRRAEFARLQNMGVKGVKIDFFPGDGKSVMAYYNDLAKDAADY
NLLVNYHGSSLPRGLHRTYPNIMTMESVHGFEMITFMQPSADKAATHMAILPFTRNAF
DPMDFTPTTFSDIPNIERRTSNGFELALPVLFLSGLQHIAETAQGMATNAPDYVKAYM
RDIPVLWDESKLIDGMPGEHVVIARKHGERWFVAGINATNEAINLEMNFDFALGKQGT
LITDSNINTKGVESFTSHTITATKNNALTVKANGGFVIVFN"
   gene 1..1860
      /gene="Mdeg2393"
   CDS 1..1860
      /gene="Mdeg2393"
      /codon_start=1
      /transl_table=11
      /product="COG3940: Predicted beta-xylosidase"
      /protein_id="ZP 00066994.1"
      /db_xref="GI:23028601"
      /db_xref="COG:COG3940" 
/translation="MAAGQIISLEVKVKKIEEIMKHTARTIALGATGAALLTGLIACN
GTNVNTNGDTQQASIKKAPEGMFANPLFANGADPWLEYYDGNYYLTTTTWTSQLVMRK
SPTLDGLSTALPVNVWSDSDLTRCCNFWAFEFHRLNGPNGWRWYLMYTSGQHGTLDHQ
HLSVLESVGDDPMGPYTYKGEMMPNTWNIDGSYLEHNGQLYLLWSEWVGDEQQNFISK
MTTPWSIEGPRALLTRPEAEWEKSGRKVNEGPEILKKDGRTFLIYSASYCDTPDYKLA
MKELTGDDPMNSEHWTKYDKPVFERGNGVFAPGHNGFFKSPDGTEDWIVYHGNSKEEH
GCGATRSVRAQKFTWNTDGTPNFGEPIPEGQFLPLPSGENGPLVTALQGARIQLRNGE
SCLLAEGKELKQGSCQAEASLWVMDNTADNHYRFGNVASNLFLTADEGLSQSAWVNTA
SQRWALNAGEGNFVAFTNKYTGDALMQNNWQILPVGKVAISSIQSGRVLQACDKNSAN
VNQGGWQGRACQAWQFNPASEGHVQIKTGNQCLTVENKSIVPGTNVIAGECESTSSQW
LYQLDKEGRATFTNRESKQRLDLANCGLADGTNFAQAPALDTICQAFQVRYLP"
ORIGIN 
1 gtggccgcag gccaaataat ttcactggag gttaaagtga aaaagataga agaaataatg
61 aaacacacag cgcgcactat agcgctaggt gcaacaggtg ccgccttgct aacggggtta
121 attgcctgta acggtaccaa tgtgaataca aacggggata cccaacaagc aagcattaaa
181 aaagcgccag aaggcatgtt tgccaacccg ttgttcgcca atggcgcaga cccttggtta
241 gagtattacg atggcaatta ctacctcact accaccacat ggacatcgca acttgttatg
301 cgtaaatcac ccacgttgga tggtttgtct actgcgctgc cggtgaatgt atggtccgat
361 tcagatttaa cccgctgctg taacttttgg gcgtttgaat tccatcgctt aaacggccct
421 aacggctggc gttggtattt aatgtacacc tcgggccagc acggcacttt agatcaccaa
481 cacttaagcg tattagaaag tgtgggtgac gaccctatgg ggccatacac ctacaaaggt
541 gaaatgatgc ccaatacatg gaatatagac ggcagttatt tagagcataa tggccaatta
601 tatttgttgt ggtctgaatg ggtaggtgac gagcagcaaa actttatatc taaaatgacc
661 accccatgga gcattgaagg cccgcgagca ctgttaactc ggccggaagc agagtgggaa
721 aaaagcggtc gcaaagttaa cgaaggccca gagattctaa aaaaagatgg tcgtaccttt
781 ttgatttact cggcgagcta ttgcgatacg ccagattata aactggcaat gaaagagcta
841 acgggcgacg acccaatgaa ctccgagcac tggactaaat acgataagcc cgtgtttgaa
901 agagggaacg gtgtgtttgc cccaggccac aatggtttct tcaaatcgcc cgatggcaca
961 gaagattgga ttgtgtacca cggaaattcg aaagaagagc acggctgcgg tgcaacgcgc
1021 tctgtgcgcg cacagaaatt tacttggaac acagacggca ccccaaattt tggtgagcca
1081 ataccagaag gccaattttt gcccttacct tctggcgaaa acggtccttt agtgactgca
1141 ctgcaaggtg cacgtattca gttgcgcaat ggcgaaagct gtttattagc cgaaggtaaa
1201 gagctgaagc agggcagttg ccaagcagaa gccagtttat gggtaatgga taacacggca
1261 gataatcact accgctttgg caatgtagcc agcaatttat ttttaacggc agacgaaggc
1321 cttagtcaga gtgcctgggt taatactgca agccagcgct gggcacttaa tgcaggcgaa
1381 ggtaactttg ttgcgtttac aaacaagtac accggcgatg cgcttatgca aaataattgg
1441 caaatactac cggttggcaa agtggcaatt agcagcattc aaagtggccg cgtattacag
1501 gcgtgcgata aaaacagcgc gaatgtaaac caaggcggct ggcagggtag ggcttgtcaa
1561 gcatggcaat ttaacccagc aagtgaaggc catgtgcaaa ttaaaacggg caaccaatgt
1621 ttaaccgttg agaataaatc catagtgcct ggcaccaatg ttattgccgg cgagtgtgaa
1681 tcaactagca gccaatggct ttatcaatta gataaagaag gccgcgcaac cttcacaaac
1741 cgcgaaagca aacagcgttt agatttagca aactgcggcc tagccgacgg caccaacttc
1801 gcacaagccc ctgcgttaga tactatttgc caagcattcc aagtgcgtta tttaccgtaa
LOCUS NZ_AAB103000006 1605 bp DNA linear BCT 17-JUN-2004
DEFINITION Microbulbifer degradans 2-40 Mdeg03_6, whole genome shotgun sequence.
ACCESSION NZ AABI03000006 REGION: 149813..151417
VERSION NZ_AABI03000006.1 GI:48861946
KEYWORDS WGS.
SOURCE Microbulbifer degradans 2-40
ORGANISM Microbulbifer degradans 2-40
   Bacteria; Proteobacteria; Gammaproteobacteria; Alteromonadales;
   Alteromonadaceae; Microbulbifer.
REFERENCE 1 (bases 1 to 1605)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
FEATURES Location/Qualifiers
   source 1..1605
      /organism="Microbulbifer degradans 2-40"
      /mol type="genomic DNA"
      /strain="2-40"
      /db_xref="taxon:203122"
   gene 1..1605
      /locus_tag="Mdeg02002711"
   CDS 1..1605
      /locus_tag="Mdeg02002711"
      /codon_start=1
      /product="COG3534: Alpha-L-arabinofuranosidase"
      /protein_id="ZP 00315975.1"
      /db_xref="GI:48862077"
      /db_xref="COG:COG3534" 
/translation="MNPASTLSVKTTNKTTNHLKKVALTVAAIVAPLTSWADVKVSLN
PQNTGETISKYIYGQFAEHLGSGIYGGIWVGEDSPIPNKNGFRNDVIKALQELQVPVI
RWPGGCFADEYRWRDGIGPREQRPIRVNTHWGGVEEPNTFGTHEFFELVELLNTEAYV
AGNLGTGSPQEMAEWLEYIVSNSNSTVVAERKKNGREEPWEVAFWGVGNESWGCGGNL
TPEYYTNLYRHFSTFVKATGAKRPKLVASGSYDDDETWTTPLSKLKNNIDGISHHYYT
LPTSDWSIKGAATGFDEKEWILTLERTLKIDSYLATQTGILKKNNPEGNIGLYLDEWG
TWYDAEPGTNPGFLYQQNTVRDAIVAAVNLNIFHNYADRLHMANIAQMVNVLQAMILT
DNEKMLLTPTYHVFKMYIPFQDATHIPLDIKGQRDYSAHKTTVPGFSASAAKTPNGNI
VVSLVNLNPNEAEEVSIALQGIKVKTITGELLTSQKMDAHNTFDKPNNVQPRALNQSD
YSISKNGKTLTVKLPAKAVVVLQLNK"
ORIGIN 
1 atgaacccag cttcaaccct aagtgtgaaa acaactaata aaacaacaaa ccacctcaaa
61 aaagttgccc ttaccgtagc cgctatagtt gccccgctaa caagctgggc cgatgtgaaa
121 gttagcctaa acccacaaaa cacaggcgaa accataagta aatatatcta cggccaattc
181 gcagagcacc ttggcagcgg catatacggc ggcatatggg tgggcgaaga ctccccaata
241 cccaacaaaa acggctttcg taacgatgta atcaaagccc tgcaagagct acaagtacct
301 gttataagat ggcccggtgg ctgctttgcc gacgaatatc gctggcgtga tggcattggc
 
361 ccacgtgagc aacgccctat ccgcgtaaat acccactggg gcggtgtgga agaacccaat
421 acctttggta ctcacgaatt cttcgaatta gttgagctac ttaatacgga agcctatgtg
481 gcaggtaacc taggcactgg ctcaccacaa gaaatggccg aatggctgga atatattgtt
541 tccaactcca actctactgt agtggcagag cgcaaaaaaa atggccgcga agagccttgg
601 gaagtggcct tttggggtgt gggtaatgaa tcttggggtt gcggtggcaa cttaacgccc
661 gagtactaca ccaatttata tcgtcatttt tccacttttg ttaaagccac tggcgctaag
721 cgcccgaagt tagttgcaag cggctcgtat gatgatgacg aaacttggac aacgccacta
781 agtaagctca aaaataatat agatggtata agccaccact actacacctt acccaccagc
841 gactggagca taaaaggtgc ggctacaggc tttgatgaaa aagaatggat tcttaccctt
901 gagcgcacat tgaaaataga cagctacctt gcaactcaaa cgggtattct taaaaagaat
961 aaccccgaag gcaatatagg gttgtattta gatgaatggg gtacgtggta cgatgcagag
1021 cccggtacaa accccggctt tttataccaa caaaataccg tgcgcgacgc catagtagca
1081 gcagtaaact taaatatatt ccacaactat gccgaccgct tacacatggc gaacatcgcc
1141 caaatggtaa acgtactaca ggcaatgata ctgaccgaca acgaaaaaat gttgctcaca
1201 cccacgtatc acgtttttaa gatgtacata ccatttcaag atgctaccca tataccgtta
1261 gatataaaag gccagcgcga ctacagcgca cataaaacta ccgtaccagg gttctcggcc
1321 tcggcagcta aaaccccaaa tggcaatatt gtagtttcac ttgttaacct taacccaaac
1381 gaagcggaag aagtgagcat tgcactacaa ggcatcaagg taaaaaccat tactggtgaa
1441 ttacttacca gccaaaaaat ggatgcgcat aacaccttcg acaagccaaa caatgtgcag
1501 ccacgcgcac ttaaccaaag cgactacagc attagcaaaa acggcaaaac ccttaccgtt
1561 aagctacccg ctaaagccgt agttgtttta cagcttaata aataa
LOCUS NZ_AAB102000036 1194 bp DNA linear BCT 23-SEP-2003
DEFINITION Microbulbifer degradans 2-40 Mdeg_35, whole genome shotgun sequence.
ACCESSION NZ AABI02000036 REGION: 4764..5957
VERSION NZ_AABI02000036.1 GI:28878266
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1194)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029800. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1194
      /organism="Microbulbifer degradans 2-40"
      /mol type="genomic DNA"
      /strain="2-40"
      /db_xref="taxon:203122"
   gene 1..1194
      /gene="Mdeg3529"
   CDS 1..1194
      /gene="Mdeg3529"
      /codon_start=1
      /transl_table=11
      /product="COG3867: Arabinogalactan
      endo-1,4-beta-galactosidase"
      /protein_id="ZP 00068112.1"
      /db_xref="GI:23029804"
      /db_xref="COG:COG3867" 
/translation="MLLAQLPVKKYFVLLAIFSFMLGCNSAGVQQSAKSIQVAGTHSK
PARFFAGADLSYVNEMEDCGATYRVNGVTTDPYQAFADAGANLVRVRLWHNPTWTEYS
DFADVKKTIRKAKQNNQTVLLDFHYSDTWADPEKQFVPAAWEHMVDDTPALAQALAQY
TTDVLEKLQAENLLPDMVQVGNETNAEVLQLEAHMKHGEIDWQRNAALLNSGLAAVAE
FNQNNNTYIERVLHIAQPENALWWFDDAAQAGITDFEIIGLSYYAKWSTYKLDSIGEA
IRALRTAFNKDVLVVETSYPWTMQNFDQANNVLDATSLQQGYPATAEGQKKYMMDLAK
QIMYAGGIGIAYWEPAWVSTPCKTLWGTGSHWENAVFFDSGNNNEALPALSFYTDIMA
LFKQD"
ORIGIN

1 atgttattag ctcaattgcc agttaaaaaa tattttgtct tattagctat tttctcgttt
61 atgctggggt gtaatagtgc tggcgtacaa caaagtgcta aatcaattca ggttgctggc
121 acgcacagta aacccgctcg tttttttgct ggtgccgacc tttcttacgt aaacgaaatg
181 gaagattgcg gagcaacata ccgcgtaaac ggtgtaacta ccgaccctta ccaagccttt

241 gccgatgccg gcgcaaattt agtgcgcgtg cgcttatggc acaaccctac ttggacagaa
301 tattccgact ttgccgacgt taaaaaaact atccgcaaag ccaaacaaaa taatcaaacg
361 gtattgttag attttcatta ttcagatacc tgggccgacc cagaaaaaca atttgttcca
421 gccgcttggg aacatatggt ggatgacacc ccagcactag cgcaagcctt agcgcaatac
481 acaaccgatg tattagaaaa gctgcaagca gaaaacctat tgccagatat ggtgcaagta
541 ggtaacgaaa caaacgcaga agtcttacag ctagaagcgc acatgaaaca cggcgaaata
601 gattggcagc gcaatgcagc gctactaaac agtgggttag cagccgttgc tgaatttaac
661 caaaacaaca acacctatat tgaacgcgta ttacatatcg cccagccaga aaatgctttg
721 tggtggtttg acgatgccgc gcaggctggc ataaccgatt ttgaaattat aggtcttagc
781 tactatgcca aatggtcaac gtataaatta gattccatcg gcgaagctat acgcgccttg
841 cgaaccgcat tcaataaaga tgtgttggtg gtagaaacct catacccctg gactatgcaa
901 aatttcgatc aagccaataa cgtgctcgat gctaccagct tgcagcaggg ctaccctgca
961 acggccgaag gccaaaaaaa atacatgatg gatttagcta aacaaattat gtacgccggt
1021 ggaattggta ttgcctactg ggaaccagct tgggtaagca ccccttgcaa aactctatgg
1081 ggtacaggtt ctcactggga aaatgccgtg ttttttgact ctggcaacaa caacgaagcg
1141 ctacccgcgc ttagtttcta cacagacata atggctcttt ttaagcaaga ttaa

LOCUS NZ_AABI02000005 1263 bp DNA linear BCT 23-SEP-2003
DEFINITION Microbulbifer degradans 2-40 Mdeg_5, whole genome shotgun sequence.
ACCESSION NZ AABI02000005 REGION: complement(76912..78174)
VERSION NZ_AABI02000005.1 GI:28878297
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1263)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027361, Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1263
      /organism="Microbulbifer degradans 2-40"
      /mol_type="genomic DNA"
      /strain="2-40"
      /db_xref="taxon:203122"
   gene 1..1263
      /gene="Mdeg1250"
   CDS 1..1263
      /gene="Mdeg 1250"
      /codon_start=1
      /transl_table=11
      /product="COG3867: Arabinogalactan
      endo-1,4-beta-galactosidase"
      /protein_id="ZP_00065874.1"
      /db_xref="GI:23027428"
      /db_xref="COG:COG3867" 
/translation="MLQILKDHGMDSIRLRVWVNPAGGWYSSINDVIEKAQRAKAAGM
RIMIDFHYSDSWADPGKQYKPAAWTNYTLDGLMSAVWWHTYDSLVALKNAGITPEWVQ
VGNETNNGMLWEEGRASANMQNYAWLVNSGYDAVKEVFPNTKAVVHLANCHDNANFRW
IFDGLQANGGKWDVIGASIYPTNASGYSWSQANSLCEANLNDMQSRYGSEVLIAEVGA
PWDHPEAKAIVSDVIAKAQNAGATGVFYWEPQASNWQGYTLGAWNPNTMRPTEALDAF
IDGSSNVTTARLQSRNSNRCIDVNGRSTADGADIIQWSCHSNANQQWTFEDMGNNYVR
LRVGHSNKCLDVLGAGTADGDNVVQWACHNNANQQWLKEDMGDGYFRLKSRASGKCVD
VNAGGANNGDSIIQWSCHTGWNQQWMVY"
ORIGIN 
1 gtgctgcaaa ttctaaaaga tcacggtatg gattccatcc gtctgcgcgt gtgggtaaac
61 cccgccggtg gatggtatag cagcattaat gacgtaatag aaaaagctca gcgcgccaaa
121 gctgcgggca tgcgtattat gatcgatttt cactacagcg actcttgggc tgacccaggc
181 aagcaataca agcccgccgc gtggaccaac tataccttag acggtttaat gtctgcggtg
241 tggtggcaca cctacgattc cctcgtggcc ctaaagaatg cgggtattac ccctgaatgg
 
301 gtgcaagtgg gcaacgaaac aaacaacggt atgttatggg aagaggggcg cgcatccgcc
361 aatatgcaaa actatgcgtg gttggtgaat agtggctacg atgccgttaa agaagtgttc
421 cctaatacca aggcagtggt gcacttggca aactgccacg acaacgcaaa cttccgctgg
481 atatttgacg gcttacaagc caatggtggt aagtgggatg taataggtgc ctctatttac
541 cctaccaacg caagcggtta tagctggagc caagccaaca gtttgtgcga ggcaaactta
601 aacgatatgc aatcgcgcta tgggtccgag gtgctaattg ccgaggttgg tgcgccgtgg
661 gatcacccag aagcgaaagc aatcgtgagc gatgtaattg ctaaggcgca aaacgccggt
721 gcaacagggg tattttattg ggagccgcag gcatcaaact ggcagggcta cacgctaggt
781 gcatggaacc caaacaccat gcgccccacc gaagcattag acgcgtttat tgacggcagc
841 tcgaatgtga caaccgcgcg tttgcaatcg cgcaatagca accgctgtat agatgttaat
901 ggccgcagta cagcagatgg tgccgatatc attcagtgga gttgccacag caacgccaac
961 cagcaatgga cttttgaaga tatgggcaat aactacgtgc gattgcgcgt gggccacagt
1021 aataagtgct tagatgtact gggtgcaggc actgccgatg gcgataacgt agtgcagtgg
1081 gcatgccaca ataacgccaa tcagcaatgg ctaaaagaag acatgggcga tggctacttc
1141 cgcttaaaat ctcgcgccag cggtaaatgc gtagatgtaa acgcaggcgg tgctaacaac
1201 ggtgattcta ttattcaatg gagttgccac actggttgga accagcaatg gatggtttat
1261 tag
LOCUS NZ_AABI02000044 1770 bp DNA linear BCT 23-SEP-2003
DEFINITION Microbulbifer degradans 2-40 Mdeg_43, whole genome shotgun sequence.
ACCESSION NZ AABI02000044 REGION: <2..1771
VERSION NZ_AABI02000044.1 GI:28878258
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1770)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029947. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1770
      /organism="Microbulbifer degradans 2-40"
      /mol_type="genomic DNA"
      /strain="2-40"
      /db_xref="taxon:203122"
   gene <1..1770
      /gene="Mdeg3654"
   CDS <1..1770
      /gene="Mdeg3654"
      /codon_start=1
      /transl_table=11
      /product="COG3867: Arabinogalactan
      endo-1,4-beta-galactosidase"
      /protein_id="ZP_00068237.1"
      /db_xref="GI:23029948"
      /db_xref="COG:COG3867" 
/translation="NDLSYVNEMEDCGAVYKDAGSVVDPYEVIANHGGNLVRVRHWND
PYWQALITQPESVAANWKANYSGLEDVTETIRRSKAAGMEVLLDFHFSDIWADPGRQT
TPRAWENDFGDEDAMAAHIYDYVTSVLTGLNDEGLMPELIQIGNESNSGMMTTQNLII
EMNDAGTGLNVSKGGQTNYSDQYVARMYNSAISAVRDISEGMTNAPRIAIHVAGADKA
VAFFDKLKSIGVTDIDIAGFSFYYGWEQAPIEDVASMIATLKERHPNLDPLMLETGYL
WDEENIDSLGNIIGIADPAYLPVSKQNQLKYLTDLSQAVADAGGIGVVFWEPSWVSTE
CRTPWGQGSSHEHVAYFDHRDGLNFHIGGQWMEVTKLSETPEAGLATTFRVDMTGQDT
SAGVFIRGAFTEDTLQPMLYEGENIYSYTTHIQAAQSGSYHYAIGLKNGTRETVPSEC
ANPEDTLNRLYTVGENGEQLVTAVWASCDVFDPQAAGPTTLTLNVDMTGVDVSGGVYV
AGDLNAWTITELTQVGASAIYTISYDLAVGAEGGYYFLNGSDWGDRETIPEECVGYYD
ADRGFLVEEQSPQVLDLVWSSCQ"
ORIGIN 1 aacgaccttt cctacgtaaa cgaaatggaa gactgcggcg cagtgtataa agatgcaggc 
61 agtgtagtag acccctacga agtgatcgcc aatcacggcg gcaaccttgt gcgcgtgcgt
121 cactggaacg acccctactg gcaggcgctt attacccagc cagaatctgt agcggcaaat
181 tggaaagcta attacagtgg gcttgaagat gtaacagaaa caattcgacg atcaaaagcc
241 gccggtatgg aagtgctgct cgactttcat ttctcagata tttgggcaga ccccggcagg
301 caaacaacgc cgcgcgcatg ggaaaatgac tttggcgatg aagacgccat ggctgcgcat
361 atatacgatt acgtaacatc agtactcaca gggttaaacg acgaaggttt aatgcccgag
421 cttattcaaa ttggtaacga atctaactcc ggcatgatga cgactcaaaa cctcattata
481 gaaatgaatg atgcgggtac ggggttaaat gtgagtaaag gcgggcaaac aaattactca
541 gatcaatatg ttgcgcgtat gtataactct gcgatttctg ctgtgcgcga tattagtgaa
601 gggatgacca acgctccgcg tattgctatc cacgtggcag gtgcagataa agccgtcgca
661 ttttttgata agttaaaaag catcggagta acggatattg atatcgcggg cttctcgttt
721 tattacggtt gggagcaagc accaatagaa gacgttgcaa gcatgattgc aaccttaaag
781 gaacgtcacc ctaatttaga cccgttaatg cttgaaacag gctacctgtg ggatgaagaa
841 aacatcgata gcttaggcaa tattattggc attgccgacc ccgcatattt acctgtgagc
901 aaacagaacc aacttaaata tcttaccgat ttatcgcaag cggttgctga tgctggtggt
961 attggagtgg tgttttggga gccatcttgg gtatcaaccg aatgtcgcac accttggggg
1021 cagggctcat ctcacgagca tgttgcttac ttcgatcacc gcgacggctt aaactttcat
1081 attggtggcc aatggatgga ggttaccaag ttaagcgaaa ccccagaagc ggggctagct
1141 actaccttta gagtggatat gactggccaa gacaccagcg caggggtatt tattcgcggg
1201 gcgtttaccg aagacacatt gcagcccatg ctatatgaag gcgaaaacat ttatagttac
1261 accacgcata tccaagcagc gcaaagcgga agctaccact atgcgattgg cttaaaaaat
1321 ggtacgcgcg aaacggttcc tagcgaatgt gcaaacccag aagatacgtt aaatcgttta
1381 tatacggtgg gcgaaaatgg ggagcagtta gttaccgcag tttgggcaag ctgtgatgtt
1441 ttcgatccgc aagcggctgg gccaacaacc ttaacgctta atgtagatat gactggtgta
1501 gatgtaagtg gtggtgtgta tgttgcaggc gacttaaatg cttggacaat caccgagctt
1561 acacaagttg gcgctagcgc aatttatacc attagttacg atttagccgt aggtgcagaa
1621 ggtggctact acttcttgaa tggcagcgat tggggcgata gggaaacaat accagaagaa
1681 tgtgtgggct actatgatgc agaccgcggc tttttggtgg aagagcaaag cccacaggta
1741 ttggatttag tgtggagtag ttgtcaataa
LOCUS NZ_AABI02000005 1020 bp DNA linear BCT 23-SEP-2003
DEFINITION Microbulbifer degradans 2-40 Mdeg_5, whole genome shotgun sequence.
ACCESSION NZ AABI02000005 REGION: 56447..57466
VERSION NZ_AABI02000005.1 GI:28878297
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1020)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027361. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1020
      /organism="Microbulbifer degradans 2-40"
      /mol_type="genomic DNA"
      /strain="2-40"
      /db_xref="taxon:203122"
   gene 1..1020
      /gene="Mdeg1232"
   CDS 1..1020
      /gene="Mdeg1232"
      /codon_start=1 1
      /transl_table=11
      /product="COG4124: Beta-mannanase"
      /protein_id="ZP 00065857.1"
      /db_xref="GI:23027411"
      /db_xref="COG:COG4124" 
/translation="MERTQTAMSDTPVNPNANTTTKAVYTYLKQQWGSKMLTGQMDLT
WKDSIDEYQRVINDTGKAPAIMGYDYMNYGIESSFISGLEQTEEAIVHWQRGGLVTFA
WHWRDPNVSGSNIGEFYTADTSFQIPIANGQLDESSQSFINMQADIDMIAAELQKLED
 
AGAVVLWRPLHEASGGWFWWGRTRTDSVSAAYAQVLLWRHMYTRLTDHHGLDNLLWVW
NGQNSAWYPGDEYADIVSHDIYDGAKNYESQLAVYNDTKNTPMQTKMVALSENSNIPD
PDAMQADGAWWLWFMVWNDSDTAEGVTHENNFWTGEYYNSNAHKQHVYNHELVITLDE
LPSFD"
ORIGIN 
1 atggagcgca ctcaaacggc catgagcgat acgccggtaa accctaacgc caataccacc
61 accaaggctg tttacactta ccttaagcag caatggggca gcaaaatgct aaccgggcag
121 atggatttga cctggaagga cagcattgat gagtaccagc gcgtaattaa cgataccggc
181 aaagcccccg caattatggg ctacgactat atgaattacg gtattgagag cagttttatt
241 agtggccttg agcaaacaga agaagctatt gtccactggc agcgcggcgg cttagtgact
301 tttgcttggc actggcgaga cccgaacgta agcggtagta acattggcga gttttatacg
 
361 gctgatacga gctttcaaat cccaattgcc aatggtcaac tggatgaaag cagtcagagc
421 tttataaata tgcaggccga tatcgatatg atagcggcag agctgcaaaa gctagaagat
481 gccggcgctg tggtgctgtg gcgccccttg cacgaagctt ctggcggttg gttctggtgg
541 ggccgtacgc gtaccgattc ggtatctgcc gcttatgcac aagtgctgtt gtggcgccac
601 atgtacaccc gccttactga tcatcacggt ttagataatt tactttgggt gtggaacggg
661 caaaacagcg cttggtaccc aggcgatgaa tatgccgata ttgtaagtca cgacatttac
721 gacggcgcga aaaattacga gtcgcaatta gccgtatata acgatacgaa aaacaccccc
781 atgcaaacca aaatggtggc gctaagcgag aacagtaata ttcccgaccc agatgccatg
841 caggccgatg gcgcttggtg gttgtggttt atggtgtgga acgactcgga taccgcggaa
901 ggcgtaaccc acgaaaacaa cttttggaca ggtgaatatt acaactctaa cgcccataag
961 cagcatgttt acaatcatga gctggttatt acgctggatg agttacctag ctttgactag
LOCUS NZ_AABI02000003 1686 bp DNA linear BCT 23-SEP-2003
DEFINITION Microbulbifer degradans 2-40 Mdeg_3, whole genome shotgun sequence.
ACCESSION NZ AABI02000003 REGION: 276126..277811
VERSION NZ_AABI02000003.1 GI:28878299
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1686)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026886. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1686
      /organism="Microbulbifer degradans 2-40"
      /mol_type="genomic DNA"
      /strain="2-40"
      /db_xref="taxon:203122"
   gene 1..1686
      /gene="Mdeg0943"
   CDS 1..1686
      /gene="Mdeg0943"
      /codon_start=1
      /transl_table=11
      /product="COG2730: Endoglucanase"
      /protein_id="ZP 00065570.1"
      /db_xref="GI:23027107"
      /db_xref="COG:COG2730" 
/translation="MFNKISTPALSGWSKAARYLCHTATGALMLAASTVNAGFSVSGT
QLLDDNGQAFIMRGVNHPHAWYANQTSSFADIASVGANTVRVVLSDGQQWTRNSASDV
ANVISLCKANKLVCVLEVHDVTGSGEASAAGTLANAAQYWVDIANVLKGQEDYVIINI
ANEPFGNNVPASNWINQHKAAIQTLRAAGLTHTLMIDAANWGQDWQQVMLNNASEVAQ
ADSLSNTMFSVHMYQVYNNLSTVENYVSTFLSSHNLPLIVGEFGADHQGEEVDEDAIL
SVAEQYGIGYLGWSWSGNGSCCGTLDITNNFNVNSLTSWGNRLINGTNGIKATSVIAS
VYGGSSSSSSSSSSSSTSSSSSSSSTSSSSSSSGSSGGAQQCNWYGSVYPLCNNQASG
WGWENQQSCIGRTTCESQSGNGGVIGGSSSSSSSSSSSSSSSSSSSSSSSSSSSSSSS
SSSSSSSSSSSSGGSGATCEHIITNSWNSGFQGAVRITNNGSSAINGWQVSWSYSDGTT
IGSVWNANQSGSNPYTASNLGWNATVNPGQSVEFGFTANGGGAASAVTGSVCN"
   gene complement(1227..1412)
      /gene="Mdeg0944"
   CDS complement(1227..1412)
      /gene="Mdeg0944"
      /codon_start=1 1
      /transl_table=11
      /product="hypothetical protein"
      /protein_id="ZP_00065571.1"
      /db_xref="GI:23027108" 
/translation="MCSQVAPLPPDELLEEEELEDDELLLDELELLLDELLEELLDEL
LEELLDEDPPITPPLPD"
ORIGIN 
1 atgtttaaca agatatctac acccgcgtta agcggttgga gcaaggctgc gcgctattta
61 tgccatacgg ctactggcgc attaatgttg gctgcaagta cggtaaatgc ggggtttagc
121 gtttcgggta cgcagctgtt agatgataac ggccaagcgt ttattatgcg cggtgttaat
181 catccgcatg cgtggtacgc caatcaaacc agctcgtttg ctgatatagc ttcagtaggt
241 gctaatacgg ttcgagtggt actaagcgat ggccagcaat ggacgcgcaa cagcgcatct
301 gatgtggcga atgttatctc gctatgtaaa gcaaacaagt tagtgtgtgt tcttgaggta
361 cacgatgtaa ccggttcagg cgaggccagt gcggcgggta ccttggcaaa tgcggcccag
421 tattgggtag atattgccaa cgtgcttaag gggcaagaag attacgtaat aatcaatatt
481 gccaacgaac cgttcggcaa caatgtgcct gccagtaatt ggattaacca gcacaaagcg
541 gctattcaaa cgctgcgagc ggccggttta acccacacgc taatgataga tgcggcaaac
601 tgggggcaag attggcaaca agtaatgcta aacaatgctt cagaggtggc acaggccgat
661 agcctaagta acaccatgtt tagcgtgcat atgtatcagg tttacaacaa cttaagcacc
721 gtagaaaact atgtgtctac gtttttaagt agccataact tgccgttaat agtgggggag
781 tttggtgcag atcaccaagg tgaagaggtt gatgaagatg caattttatc tgtggcagag
841 cagtatggca ttggctactt aggctggagt tggtcgggca atggcagttg ttgcggcacc
901 ttagatataa ccaacaactt taacgttaac agtttaacca gttggggtaa ccgcctaata
961 aacggtacca atggtattaa agcaacctcg gtaattgcat ctgtatacgg tggctcgtcg
1021 agcagttcta gttcatctag tagctcttca actagctcta gcagttccag ttcttctacc
1081 agttcatcca gcagttcgtc cggttctagt ggtggtgcgc aacagtgtaa ttggtacggc
1141 tcggtttacc cattgtgtaa taatcaagcc agtggttggg gctgggaaaa tcagcaaagc
1201 tgtattggcc gtactacctg cgaaagtcag tctggtaatg gcggagttat tggcgggtct
1261 tcgtcgagca gttcttctag tagttcatca agcagttctt ctagcagctc atccagtagc
1321 agttctagct catctagcag cagctcgtcg tcctcaagtt cttcttcttc tagtagttca
1381 tcgggcggca gtggtgcaac ttgcgaacac ataattacaa atagttggaa cagcggtttt
1441 caaggggcgg tgcgcattac caataatggc agcagtgcca ttaacggctg gcaagtaagt
1501 tggagctaca gtgatggcac aaccattggc agtgtatgga atgccaatca aagcggtagc
1561 aacccttaca cagccagcaa tttagggtgg aacgccacgg ttaaccctgg gcagtcggta
1621 gagtttggtt ttactgccaa cggcggcggt gctgcatcag cagtgacggg gtcggtttgt
1681 aactaa
LOCUS NZ_AABI02000004 1545 bp DNA linear BCT 23-SEP-2003
DEFINITION Microbulbifer degradans 2-40 Mdeg_4, whole genome shotgun sequence.
ACCESSION NZ AABI02000004 REGION: 268515..270059
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1545)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1545
      /organism="Microbulbifer degradans 2-40"
      /mol_type="genomic DNA"
      /strain="2-40"
      /db_xref="taxon:203122"
   gene 1..1545
      /gene="Mdeg1159"
   CDS 1..1545
      /gene="Mdeg 1159"
      /codon_start=1
      /transl_table=11
      /product="hypothetical protein"
      /protein_id="ZP 00065784.1"
      /db_xref="GI:23027329" 
/translation="MNNEEVNMRIFSIALALCAVLCAGQSLAGLSIQGTRLVDGNGST
VVLRGVNHPHAWYAGETAAAIPKIAATGANSVRVVMAMGTKWSRTSAAEIQTIIDLCK
QNNMIAVLEFHDGTGWGEESGTAHISDIADYWVSSDVMAVVKGEEDYVIINIANEPFG
NGVSASTYTNDTIAAIQKLRNAGYTHTLMVDAANWGQDWQNLMRDNAQTIFNGDSLNN
TMFSVHMYQVYNTSAKVQSYMQAFSDKGLALVVGEFAADHFSEDVAEAAIMQYAEQFG
FGYMGWSWTGNSSDLASLDIVKSFSDNTYTTWGNRLINGSNGIATTSRIASVYTGTSS
GGSSSSSSSSNSSSSGGTSACSTGGSCDWSGTSFPLCENGNTNDWGYENGQSCVGVGL
CDSNPDATTHCGTSSGGGSTSCGTTSDGHPVCCDAASDPDGDGWGWENEASCVVESGS
SSSSSSSSTSSSSSSSSSSSSGGSSATCNWYGTQYPMCTSTSSGWGWENNQSCISP
STCSGQ"
   gene complement(912..1070)
      /gene="Mdeg1160"
   CDS complement(912..1070)
      /gene="Mdeg1160"
      /codon_start=1 1
      /transl_table=11
      /product="hypothetical protein"
      /protein_id="ZP_00065785.1"
      /db_xref="GI:23027330" 
/translation="MEHADVPPLLLELDDDEELELPPLDVPVYTLAIREVVAIPLLPF
IKRLPQVV"
ORIGIN 
1 atgaataacg aggaagtcaa catgagaata ttttctatag cgcttgcgct atgcgccgtg
61 ctttgcgcgg gtcaatcttt agctgggtta tctattcagg gtacgcgact tgtggatggc
121 aatggcagca cggttgtgct gcgcggcgtg aaccatcccc atgcgtggta tgccggtgaa
181 acggcggcgg ctatccctaa aattgctgca acaggtgcta actcggtacg ggtagtgatg
241 gctatgggca ctaagtggag ccgcaccagt gccgcagaga ttcaaaccat tattgacctg
301 tgcaaacaaa acaacatgat tgctgtactg gaatttcacg atggcactgg ctggggggaa
361 gagagcggta ctgcgcatat atctgatatt gccgattact gggtgagcag tgatgtaatg
421 gcggtagtta agggtgagga agactacgta attataaata ttgccaacga accctttggc
481 aacggtgtat cggcaagcac ctacaccaac gacacgattg cggctattca aaaactgcgc
541 aacgccggtt atacccacac ccttatggtt gatgcggcaa attgggggca agattggcag
601 aacctaatgc gtgataacgc acaaaccatt tttaatggcg actcacttaa caacactatg
661 tttagcgtgc atatgtatca ggtgtataac acctcggcaa aagtgcaaag ctacatgcaa
721 gcctttagcg acaaaggttt agcgttagtt gtaggcgaat tcgcagcaga tcattttagt
781 gaagatgttg cagaagcagc cattatgcaa tacgccgagc aattcggttt tggttacatg
841 ggttggagtt ggacgggtaa tagctcggat ttagcatcgc tagacatagt aaaaagcttt
901 agcgataaca cctatacaac ctggggcaac cgcttaataa acggcagcaa cggtattgcg
961 accacttcgc gtatagccag tgtgtacaca ggaacgtcta gcggcggcag ctctagttct
1021 tcgtcatcgt ctaattcaag tagcagtggc ggtacatcag cgtgttccac tggcggaagt
1081 tgtgattgga gtggtacaag ctttccattg tgtgaaaacg gtaatactaa cgactggggt
1141 tacgagaatg gccaaagttg tgtaggtgta ggtttatgcg attctaaccc cgatgctacc
1201 actcactgcg gtacttcttc tggcggcggt tctacaagtt gcggcactac cagcgacggc
1261 caccctgttt gctgtgatgc agcctcagac ccagatggcg atggctgggg ttgggagaac
1321 gaagccagtt gtgtcgttga aagcggtagc tcttcgagtt catcaagttc tagctctacg
1381 tctagttcaa gttcgtcgag ctcttcaagc agctcaagtg gcggtagcag cgccacgtgt
1441 aattggtatg gcactcaata cccaatgtgt acttctacca gtagcggctg gggtgggag
1501 aataaccaaa gttgtatttc gccttctacg tgtagtgggc aatga
LOCUS NZ_AABI02000004 1374 bp DNA linear BCT 23-SEP-2003
DEFINITION Microbulbifer degradans 2-40 Mdeg_4, whole genome shotgun sequence.
ACCESSION NZ_AABI02000004 REGION: 56484..57857
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1374)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1374
      /organism="Microbulbifer degradans 2-40"
      /mol_type="genomic DNA"
      /strain="2-40"
      /db_xref="taxon:203122"
   gene 1..1374
      /gene="Mdeg1015"
   CDS 1..1374
      /gene="Mdeg1015"
      /codon_start=1 1
      /transl_table=11
      /product="COG3934: Endo-beta-mannanase"
      /protein_id="ZP 00065642.1"
      /db_xref="GI:23027187"
      /db_xref="COG:COG3934" 
/translation="MVNYLKRAALCMLALVAVACAKSQPETKVEELPATKTAANAGVA
ATEFVQVNGGRFTLRGQDYAYIGTNMWFAAYIGSTNPEYGDRERLIKELDLLKSLGVT
NLRILGASEKSPLRDSMKPAISERGEINQHDILEGLDFALAEMAKRDMKAVIFLNNFW
EWSGGMATYLSWVNGGEIVDMADPTKPWPAFALFSAGFYSNEEAKQLFNNYLTKVVSR
RNTITGELYANDPTIMSWQLANEPRPGNGDVSKSNLPAYYDWISKTTQLIKSIAPKQL
VSIGSEGTMGCLELDECVITAHKETGIDYVTFHMWLKNWGWFDVQNAEQTYDSAVATA
DKYIDHHIKLANELNMPVVLEEFGMERDGGEFSPESAVTYRDKFYAYVFDRQIKSIRS
GGPFVGSNFWAWGGYGKAMHDDAVWRKGDKTFVGDPPQEPQGLNAVFASDTSTLEVLK
QAADAIR"
ORIGIN 
1 atggtgaact acttaaagcg cgctgccctg tgcatgcttg cgctcgttgc tgtggcttgc
61 gccaaatcgc agccagaaac aaaagttgaa gagctgcctg ccaccaaaac tgcggcaaat
121 gccggtgtgg cagcgacaga gtttgtgcaa gtaaatggtg gccgctttac attgcgcggg
181 caggactacg cctacatagg cactaatatg tggtttgccg cgtatattgg ttctaccaac
241 cctgaatacg gcgatcgcga gcgcctaatt aaagaactcg atttattaaa atccttgggt
 
301 gtgaccaacc tgcgtatttt aggagcatcc gaaaaatctc cattgcgcga ttccatgaaa
361 ccggcaatta gtgagcgcgg tgaaattaac cagcacgata ttttagaggg cttagatttt
421 gcgttagctg aaatggctaa gcgcgatatg aaagccgtga tatttctgaa taacttttgg
481 gaatggtccg gcggtatggc gacttaccta agctgggtta acggtggcga aattgttgat
541 atggcagacc ccaccaaacc atggccagct tttgcacttt tttcagcggg gttttactct
601 aatgaagagg cgaaacaact attcaataat taccttacaa aagttgtaag ccgccgcaat
661 accattaccg gcgagttgta cgctaacgac cccactatta tgtcttggca gctcgctaac
721 gagccgcgcc caggtaacgg cgatgtgagc aagtctaact tacccgccta ttacgattgg
781 ataagtaaaa ccactcagct aattaaaagc attgccccta agcagttggt gtcgattggt
841 agcgaaggta ccatgggctg cttagagttg gatgagtgcg ttattaccgc ccacaaagaa
901 acaggtatcg actacgttac attccatatg tggcttaaaa actggggctg gttcgatgtg
961 caaaacgcag agcaaaccta cgacagcgca gttgctactg cagataaata tatcgatcac
1021 cacattaaac tggctaacga gttaaatatg ccggtggtgc tggaagagtt tggtatggag
1081 cgcgacggag gtgaattctc cccagaaagc gcggtaacct atcgcgataa attctatgcc
1141 tatgtgttcg atcgccaaat taaaagtatt cgctctggcg ggccgtttgt agggtccaac
1201 ttttgggcgt ggggcggtta tggcaaagcc atgcacgatg atgctgtatg gcgcaaaggc
1261 gataaaacct ttgttggtga cccgccgcag gagccgcaag gtttaaatgc ggtatttgct
1321 tccgatactt caacgctgga agtgttgaag caagcggcgg acgctattcg gtaa
LOCUS NZ_AABI02000001 2553 bp DNA linear BCT 23-SEP-2003
DEFINITION Microbulbifer degradans 2-40 Mdeg_1, whole genome shotgun sequence.
ACCESSION NZ AABI02000001 REGION: complement(355661..358213)
VERSION NZ_AABI02000001.1 GI:28878301
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2553)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026153. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2553
      /organism="Microbulbifer degradans 2-40"
      /mol_type="genomic DNA"
      /stain="2-40"
      /db_xref="taxon:203122"
   gene 1..2553
      /gene="Mdeg0255"
   CDS 1..2553
      /gene="Mdeg0255"
      /codon_start=1
      /transl_table=11
      /product="hypothetical protein"
      /protein_id="ZP_00064894.1"
      /db_xref="GI:23026408" 
/translation="MNTKIKLLSFLASAMLLQACGGDMLGTSDSEDYKLIPEEVTEDP
TKARPSENAPVLKTSGTTIQLPDGTPVLLRGINLQFGDNPIEQIDGIQAIRETGSNVV
RIQLLADTSTANLEAVLNKVVEHNLIAVLSLYDEALHCKEDDEAFTDAVKQVWLTDFL
PIIAQDRYQANIMINIASGWGPEAIFDGYSVGYKTYTDNYKAAIRQFRKAGFNVPLVI
DAPGCGADFNAFLSNRGKELMAADDKDNLVLSVHGYGSQWNTATKVTDAISQLAAQNI
PVLMSEFGGSGVGEKPVKHMAILDKGAGDYAAEIILPWASATDKVAMNVPFSAPINLT
NTDVSFDVKLDEAYVTDGQMGVVMYLRDVNGEYANLAWHSASEFPAGEWATKSYAIQN
NASFGWASEAFDITAVAKVGVELVANGKLAEVAGSVVIDNLRVAEGSGAVELYSQSFD
DDIAGWGVPWTGTVAAHADGALSLTHDNGEIIAQLDGLGGVVDFAQPVVISGRFFVPA
DYAGSWMYAKFFNNGEAWTEVGITGLTPGEWTEISVETEFPAAATSVGIQIGNIGIAD
GATITDSTEPFLLDDFAISGVAANDSFELGTQYMASFDESEDGWAYLSWGASATVEAI
DGALNFYPNANDAVRIVLYKTDLSAIDDLDLQDPFTIKTRVLIPDSYTGQAFEYQLFL
QDANWQNHFAAKIWNQDELIPGEWMDLVVEVEFPAEFDRAGIPQYLGFDLSSEVALPQ
DPILIDEIVFEGMVPVEKEVVIIDQVDFFYTNHFTDFAIDYIEGEILEDDILELAYIH
QRSEPFSWIAWSWYGNDIENSDWDMTTIVGDATALTERGEDIVNGKGGIAGN"
ORIGIN 
1 atgaacacaa aaataaaatt actttcattt cttgcgagtg caatgctgtt gcaggcatgt
61 ggtggtgaca tgctgggcac ctctgacagc gaagactata aattgattcc agaagaagta
121 acggaagatc caactaaagc taggccgtct gaaaatgccc ccgtgttaaa aacgagtggt
181 actactatac agctaccaga tggtacacca gtgctgctgc gaggtattaa ccttcaattt
241 ggcgacaatc ctattgagca aatagatggc atacaggcta ttcgcgaaac aggttcaaac
301 gttgtgcgaa ttcaattgtt agcagataca tctacagcaa atttagaggc ggtgctaaat
361 aaagttgttg agcataattt aatagctgta cttagtctct acgacgaagc tttacattgt
421 aaagaagatg atgaggcatt tacggatgca gttaagcaag tatggcttac cgattttctt
481 ccaattattg ctcaagatcg ttatcaagca aatattatga ttaacatcgc aagtggctgg
541 gggcctgaag ccatttttga tgggtatagt gtaggttaca aaacgtatac cgataactat
601 aaagcagcta ttcgtcagtt ccgaaaggct gggtttaatg tgcctttggt aattgatgcc
661 cctggatgtg gtgctgactt taacgctttt ttaagtaatc gcggtaaaga gcttatggct
721 gcggatgata aagacaatct tgtgttgtcc gtgcacggtt atggttcgca gtggaacacg
781 gctactaaag ttacggatgc aattagccag cttgctgcgc aaaatatccc tgtgctaatg
841 agcgagtttg gtggctctgg tgtcggtgaa aagcctgtta aacatatggc gatccttgat
901 aaaggtgcag gagattacgc cgcagaaatt attctgccat gggcgagcgc tacagataaa
961 gtggctatga atgtgccatt ctcagcacct atcaatctga caaacactga tgtgagcttt
1021 gacgttaagc tggatgaagc atatgtcacg gatggtcaaa tgggggttgt aatgtacctc
1081 cgagatgtaa atggagaata tgcaaactta gcttggcact ctgcctctga atttccggct
1141 ggtgaatggg ctactaaatc ttatgcgatt cagaataatg catctttcgg ctgggcaagt
1201 gaggcgtttg atattactgc cgtcgctaaa gttggtgttg agttggttgc aaatggcaag
1261 ctcgccgagg ttgctggcag cgttgttatc gacaaccttc gagtggctga aggtagtggt
1321 gcagtcgagt tatacagtca aagctttgat gatgatattg caggttgggg cgtgccttgg
1381 actggcacag ttgctgcgca cgctgacggt gctttatcgc taacccatga caatggagaa
1441 attattgccc agctggacgg gcttggtggt gtagttgatt ttgcccagcc ggttgttatt
1501 agtggcaggt tttttgtgcc tgcggattat gctggttcat ggatgtacgc taaatttttc
1561 aataacggcg aggcatggac agaggttgga attactgggc ttactcctgg agagtggaca
1621 gaaatttccg tcgaaaccga atttcctgca gctgctacca gtgtaggcat tcagattggc
1681 aacataggta ttgctgatgg ggcaaccatt acagattcca cagagccttt tttgctggac
1741 gactttgcta taagtggcgt agcagctaat gactcgttcg aacttggtac gcagtatatg
1801 gcgtcgttcg atgaatctga agatggctgg gcttacttga gttggggggc gtctgctact
1861 gtagaggcca ttgatggcgc tttgaatttc tatccaaatg ccaatgatgc ggtgagaata
1921 gtactttata aaaccgactt aagtgctata gacgacttgg atttgcagga cccgttcacc
1981 attaaaacgc gagtattgat tccagatagc tacacaggac aggcgttcga gtatcagctg
2041 tttctacaag atgctaactg gcaaaatcat tttgcagcga aaatttggaa ccaagatgag
2101 cttatccctg gtgagtggat ggacttggtg gttgaggttg agtttcctgc tgaattcgat
2161 cgggctggaa ttcctcaata cctcggtttt gacctgtcct ccgaagttgc tttaccacaa
2221 gacccaatac taattgatga aatagttttt gaaggcatgg ttccagtaga aaaagaagtt
2281 gtgatcattg atcaggtaga tttcttctac actaaccact ttacagattt tgctatcgac
2341 tatattgagg gtgaaatatt agaggacgac attctggagc ttgcttatat tcatcagcgc
2401 agcgagccat tctcttggat agcttggtct tggtacggaa atgatatcga aaattctgac
2461 tgggatatga ccaccatagt tggcgacgca acagccttga ctgaacgtgg tgaagatatc
2521 gtaaatggta aaggtgggat tgcaggtaac tag
LOCUS NZ_AABI02000011 1227 bp DNA linear BCT 23-SEP-2003
DEFINITION Microbulbifer degradans 2-40 Mdeg_9, whole genome shotgun sequence.
ACCESSION NZ AABI02000011 REGION: complement(88053..89279)
VERSION NZ_AABI02000011.1 GI:28878291
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1227)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028020. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1227
      /organism="Microbulbifer degradans 2-40"
      /mol_type="genomic DNA"
      /strain="2-40"
      /db_xref="taxon:203122"
   gene 1..1227
      /gene="Mdeg1904"
   CDS 1..1227
      /gene="Mdeg1904"
      /codon_start=1
      /transl_table=11
      /product="hypothetical protein"
      /protein_id="ZP 00066516.1"
      /db_xref="GI:23028098" 
/translation="MQKITRLAALVVAGLCLLGTQAQAKKFEHLAQTPPMGWNSWNNF
GCDVDEKLIKETADYMVSSGMKDAGYEYVNIDDCWHGERDANGFIQADPERFPSGIKA
LADYVHSKGLKFGIYSDAGWTTCGGKPGSRGYEFQDAQMYAKWGVDYLKYDWCATDGL
KAEGAYQTMREAIHKAGRPMVFSICEWGDNQPWEWAKPIGHLWRTTGDIYNCFDCEYD
 
HGTWSSWGVLQILDMQDDLRQYAGPGHWNDPDMMEVGNGMTEAEDRSHFSMWAMLAAP
LIAGNDIRNMSEATRKILTNKAVIAVDQDELGVQGFKYSSKNGVEVWFKPLANDEWAM
AVLNRNKGEVKFEFKWRNEVVKDELTHRTITFNEQKFDWQDLWNKSNKGHTKKFLKTK
IAGHDTLMFRLTPAKN"
ORIGIN 
1 atgcaaaaaa ttacacgttt agcggcgtta gtcgtcgctg gcttgtgcct gctaggtacg
61 caggcgcagg cgaaaaagtt tgaacactta gcccaaaccc cgcctatggg gtggaatagc
121 tggaacaatt ttggctgtga tgtagatgaa aagctcatca aggaaacggc tgattatatg
181 gtttcttccg gcatgaaaga cgcgggatat gagtatgtaa acatagacga ttgctggcat
241 ggtgagcgcg atgctaatgg ttttattcag gcagaccccg agcggtttcc ttccggtatt
301 aaagcgcttg ccgactatgt tcattcgaag ggcctgaagt ttggtattta ttctgacgcg
 
361 ggttggacca cctgcggcgg taaaccgggc agtcgtgggt acgagtttca agatgcacaa
421 atgtatgcga aatggggcgt ggattatctt aaatacgatt ggtgcgctac agatggctta
481 aaggccgaag gcgcatatca aacaatgcgt gaagccattc ataaagccgg tagaccaatg
541 gtatttagca tttgcgagtg gggcgacaac cagccttggg agtgggccaa acctattggg
601 catctatggc gtaccacggg tgatatctac aactgtttcg attgcgaata tgaccacggt
661 acttggtctt cctggggtgt attgcaaatt ttggatatgc aagacgacct aaggcagtac
721 gctgggccag gccattggaa cgaccccgat atgatggagg tgggcaatgg catgactgaa
781 gctgaagacc gttcgcattt ttcaatgtgg gctatgttgg cagcgcccct tattgctggt
841 aacgatatac gcaatatgtc ggaagctacc agaaaaattc tcaccaataa ggccgtgata
901 gcagtcgatc aggacgagct aggcgtacag ggctttaaat attccagtaa aaatggcgta
961 gaggtttggt ttaaaccgtt ggcgaatgat gagtgggcaa tggctgtact caaccgaaat
1021 aaaggtgaag ttaaattcga gtttaagtgg cgtaatgaag tggttaaaga tgagctgacg
1081 catcgcacta ttacgttcaa cgagcaaaaa tttgactggc aagatttgtg gaataaatcc
1141 aacaagggcc ataccaaaaa attcctaaaa acaaaaatag ctgggcacga tacgctgatg
1201 tttaggctaa cgccggctaa aaactag
LOCUS NZ_AABI02000013 5202 bp DNA linear BCT 23-SEP-2003
DEFINITION Microbulbifer degradans 2-40 Mdeg_12, whole genome shotgun sequence.
ACCESSION NZ AABI02000013 REGION: complement(66951..72152)
VERSION NZ_AABI02000013.1 GI:28878289
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 5202)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028387. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..5202
      /organism="Microbulbifer degradans 2-40"
      /mol_type="genomic DNA"
      /strain="2-40"
      /db_xref="taxon:203122"
   gene 1..5202
      /gene="Mdeg2235"
   CDS 1..5202
      /gene="Mdeg2235"
      /codon_start=1
      /transl_table=11
      /product="COG3488: Predicted thiol oxidoreductase"
      /protein_id="ZP 00066839.1"
      /db_xref="GI:23028440"
      /db_xref="COG:COG3488" 
/translation="MSCTPCIRSKASSLNFDNKLIKSEFIMSNQKRLSAGLIAKLALA
SGLTLVATAATHADVTNPVIGNLIFEENFNTLNSDRWNVIEGDGCQYGPNLCGWGNQE
LQYYQDSNVTIEDVPGEPGNKAVVFEARNETVNGSAFTSGKIDSEDGIAIKYGMIEFR
 
LRVPNMGVGYWPAVWMLGTSTESWPSKGEIDMMEMGHRAQGMADAGHPGTNLNNYTAA
NAIFYAEAACVPENPTCAAMTAWQTDNAYVSQTSMGERFVIYRTYWTDTQLRFTVIDN
GVEYDMYDDPITIGEEATELQQPFYLLANLAVGGNFTDASTPAEVTAQLPGKMYLDYI
RVYQLDGMGEIFEGSIAQKEYGTFGVFTDDTPTSNKLVAGDTSQIYIWNQNSLSEGTL
APAEGDNVIAWNYTAPEWFGAGIQAVHARDMSNFENGEFKFKIKIPANVSFKVGFADT
YTNENWLTFPANQTTYGLVRNGEWAEATIPVADLRGSLIALQSMAGMFYIASVDGQIP
TSNFEFAIDDVRWEGGGAGPVDSDGDGVADELDQCPNTPAGTAVDSVGCQIGLPQPVA
VTVEAEDYEAYYDTTSGNSGNAYRSDDVDIEAASEGGFNVGWTDAGEWMDYTLNLAAG
TYDVTARVASNTDTGVYSVSLDGTTIGSNGVATGGWQNWVTQVVGQITVNGGQQTLRI
STDVAGYNINWVHFEPVPDADNDGVPDSQDNCPNTPAGTEVDANGCAIVVDPVPVHIE
 
AEDYAAYHDLSAGNNGGQYRSDDVDIEAASEGGFNVGWTDTGEWLEYSVELIEGVYDL
TARVASLSGNGAYSVSISGQAVGGSNAVATGGWQNWETQHVARFVAGTGTYTIRVNAD
AGGFNLNWLHLEPVNDPDSDNDGVPDSQDNCANTPAGTEVDASGCPVVVQPFGVTQSD
SNSAQFYVNGADWAVLHYSVNGGGQVNVSMSLENGKHVYTVPDLAPGDSISYFVTYWD
PELGGARDSETVSYSVVAAGSDSDGDGVGDSADQCPNTPAGTAVDSVGCPVTQPPSDS
DNDGVTDANDQCPNTPAGTSVDSVGCPVVQPPSDSDNDGVDDSSDQCPNTPAGTSVNA
VGCPVTQTNIVPLYNASTNLEGAISFDRGDALVTRISDRGRDRHAKENHFQAYDHYLT
FYWEDRTIAIEIVDYVAKGGSSIRMNIVSQMRLDDTEAENRWFYIGNNTLAEYCGNGV
MNEVDHTHYWKESSFNCREGRPIQIGDKMEFEISQFLDPALLPRGRSNYYGTTYLYIV
GEGLVPWDVTDKVAFQGGNRLQRDSIPVPEHARLGGDTTLHVQMTAEPDGHFQQMATN
LGFDNGQPFVLGRRVHHTSYVDGTHDESAENGVFDGMPGKAGPHYVNDRCSDCHERNG
RAPVVGIGEPLDRWVFKVGDANGNPHPDMGRVLQPEANNGAASEGTPTIAFFSEENGL
RKPNYAFSGITPDTFSARIAPQLNGIGLLEAIPESAILAQEDVNDANGDGISGKAQRS
IDPVTGETRLGRFGYKAATSSVKHQVAAAFNTDIGVRTSVMPNPDCGSNQNDCGPSGA
ELADEHLDNLVKYVSLLGVRPQRDYNDAQVLQGKQVFNDAGCVSCHTDTYQTSQYHPL
AELRSQTIHPYTDLLLHDMGPGLADNLGEGDATGAEWRTAPLWGLGLSACVTGGVEGG
RGWDDFGLDGYETCTPHHSYLHDGRARTIEEAILWHGGEGENSKQAYQNLSNSERDAL
LAFLNSL"
ORIGIN

1 gtgagttgta caccctgtat tagatcaaaa gcttcatcac ttaacttcga taataaactt
61 ataaaaagtg agttcatcat gagtaaccaa aagcgattgt ccgctgggtt aattgcaaag
121 ttagcgttgg cttcaggcct tacgctagtc gccactgcgg ccacccacgc cgacgttacc
181 aatcctgtta ttggtaattt aatctttgaa gagaatttca ataccctcaa cagcgatcgt
241 tggaatgtga ttgagggtga tggctgccaa tacggcccaa acctttgcgg ttggggcaac
301 caagaattgc agtattacca agatagcaat gtaaccatcg aagatgtgcc cggagaaccg
361 ggcaacaaag cggttgtttt tgaagcgcgc aacgaaactg taaatggcag tgcgtttaca
421 tccggcaaaa ttgattcgga agacggaata gccattaagt acggcatgat tgagttccgt
481 ttgcgagtac caaatatggg cgttggctat tggcctgcgg tttggatgct tggcacaagc
541 acagaaagct ggccgagcaa aggcgaaata gacatgatgg aaatgggcca tcgtgcccaa
601 ggcatggccg atgcgggcca cccaggcaca aacctaaaca actacaccgc ggccaatgca
661 attttttatg cagaagcagc ctgtgtaccc gaaaacccca cctgtgcagc catgaccgca
721 tggcaaacag ataacgccta tgtttctcaa acgtctatgg gcgagcgatt tgttatttac
781 cgcacttatt ggaccgacac ccagttgcgt tttaccgtaa ttgataatgg cgttgagtac
841 gatatgtacg atgatccaat tactatcggc gaagaggcta ctgagctgca acagccgttt
901 tatttgttgg caaacttagc agttggcggc aattttaccg atgcgtctac acctgccgaa
961 gttaccgcac agctgcccgg taaaatgtat ttagactata ttcgtgtgta ccaattagac
1021 ggtatgggcg aaatattcga aggctcaatt gcccaaaaag aatacggcac atttggtgtg
1081 tttaccgatg acacgcccac cagcaataag ctggtagccg gtgatacgtc acaaatttat
1141 atttggaatc aaaactccct aagtgaagga acgcttgcgc ccgccgaagg cgacaatgta
1201 atagcctgga actacaccgc gccagagtgg ttcggtgctg gcattcaagc tgtgcacgcg
1261 cgcgatatga gtaattttga gaatggcgag tttaaattca aaataaaaat acccgctaac
1321 gtctcattta aagttggttt tgcagatact tacaccaacg aaaactggct aactttccct
1381 gctaatcaaa caacctacgg tttggtgcgc aatggcgaat gggctgaggc gacaattcca
1441 gtagcggact tgcgcggcag tttaattgca ttgcagtcta tggctggtat gttttatatc
1501 gccagtgttg acggccaaat tccaacgtct aattttgaat ttgccatcga cgatgtgcgc
1561 tgggaaggcg gtggtgcagg gcctgtagac agcgacggcg acggcgtagc tgatgaactt
1621 gatcagtgcc ccaacacacc agccggtact gcagtagata gcgttggctg ccaaataggt
1681 ttgccacagc ctgtggctgt aacggtagaa gcggaagact acgaagctta ctacgataca
1741 accagtggca actctggcaa tgcgtatcgc agcgatgatg tagatataga agctgcaagc
1801 gaaggtggtt ttaacgtcgg ttggactgat gctggcgagt ggatggatta cactttaaac
1861 ctcgctgcgg gcacctacga cgttacagct cgtgttgctt caaataccga tactggtgtg
1921 tacagtgtta gtttagatgg caccacaatc ggctctaacg gagttgcaac tggcggttgg
1981 caaaactggg ttactcaagt tgtcggccaa attacggtaa atggcggtca acaaactctg
2041 cgcattagta ctgatgtggc tggatataac attaactggg tgcattttga gcccgtgcca
2101 gacgcagata acgatggcgt acctgacagc caagataact gccccaatac accagcgggt
2161 acagaggtgg atgctaatgg ctgtgcgatt gtcgtagacc cagtacccgt gcacatagaa

2221 gccgaagatt acgcggccta tcacgaccta tctgcaggta acaatggcgg tcaataccgc
2281 tctgatgatg tggatattga agccgctagc gaaggtggat tcaacgtagg ttggacagat
2341 actggcgagt ggttggaata ctcggttgaa ttaattgaag gtgtttatga cctaactgca
2401 cgcgtggcat cgttaagcgg caatggcgct tacagcgttt ctatttctgg tcaagctgtg
2461 ggtgggtcta atgctgtagc gactggtggt tggcaaaatt gggaaaccca gcacgtagcg
2521 cgttttgttg caggcacggg tacctacact attcgtgtaa atgcagatgc cggcggcttt
2581 aaccttaatt ggttgcactt agagccagta aacgaccccg atagcgataa cgacggtgtg
2641 ccagacagcc aagacaactg cgccaatacc cctgccggca ccgaagtgga cgccagcggt
2701 tgcccggtag ttgtacagcc atttggtgtg actcagtcag attccaacag cgcgcaattt
2761 tatgtaaatg gcgccgactg ggcagtgttg cattacagtg ttaatggcgg tgggcaagtg
2821 aatgtgtcca tgagtttgga aaacggtaag catgtatata ccgtgccaga cttggcccca
2881 ggtgactcca ttagttactt tgttacctat tgggacccag aactaggcgg tgcacgcgac
2941 agtgaaacgg taagctacag cgtagttgct gcgggtagcg atagtgatgg cgacggcgta
3001 ggtgatagtg ctgaccaatg cccaaataca cccgccggca cagctgtcga ttctgttggt
3061 tgcccagtaa cacagccacc atccgatagt gacaacgatg gcgttacgga tgctaatgat
3121 cagtgcccaa atacacctgc aggtacatcg gttgattccg ttggctgccc agtggttcag
3181 ccaccatcag atagcgataa cgacggtgtg gacgattcaa gtgatcaatg tccaaatacc
3241 cctgcgggta ccagtgtaaa tgcagtgggt tgccctgtaa cgcaaactaa tattgtgcct
3301 ttatataatg ccagcacaaa cttagaaggc gctatttctt ttgatcgcgg tgatgcgctt
3361 gttacgcgaa tttcagatcg cggtcgcgac cgtcacgcaa aagaaaacca cttccaagct
3421 tacgatcact accttacttt ctattgggaa gatcgcacta ttgctattga aatagtggat
3481 tacgttgcga aaggtggcag cagcattcgc atgaatatcg taagtcaaat gcgtttggat
3541 gacaccgaag cagaaaaccg ctggttttat attggtaaca acacgctcgc tgaatactgt
3601 ggcaatggcg tgatgaacga agtggatcac acgcactact ggaaggaatc tagctttaac
3661 tgtcgtgagg gtcgtcccat tcaaattggc gataaaatgg agtttgaaat cagccaattt
3721 ttagaccccg cgctattacc tcgcggtcgc tctaattatt acggcaccac ttacctttac
3781 attgtgggtg agggcttagt gccttgggat gttaccgata aagttgcttt ccaaggcggc
3841 aaccgtttgc agcgagattc cattcctgtg cccgagcacg cgcgtttagg tggcgataca
3901 actttgcatg tgcaaatgac tgccgagcca gatggccact tccagcaaat ggcaaccaac
3961 ctgggttttg ataatggcca gccgtttgta ctcggtcgcc gtgtgcacca cacatcttac
4021 gtagatggta cgcacgatga gagcgcagaa aacggcgtat ttgatggtat gccaggtaaa
4081 gcaggcccgc actatgtgaa tgaccgctgc tcggattgtc acgagcgcaa tggtcgtgca
4141 ccggttgtgg gtattggtga accgttagac cgttgggtgt ttaaagtggg cgacgcaaat
4201 ggtaacccgc atccagatat ggggcgcgta ttgcagccag aagcgaacaa cggtgccgct
4261 agcgagggca cgccaaccat cgcattcttt agcgaagaaa acggtttgcg caaaccaaac
4321 tatgcattta gcggcataac gccagacact ttctctgcgc gtattgcccc acagctaaat
4381 ggcattggtt tgctcgaagc gattccagaa agtgcaattt tagcgcaaga agatgtgaac
4441 gatgcgaacg gtgatggtat ctccggtaaa gcgcagcgct ctatcgaccc tgtaaccggt
4501 gaaacccgct taggtcgctt tggctacaaa gcagcaacca gcagtgtaaa gcatcaagtg
4561 gctgcggcgt ttaatacgga tataggtgta cgtacctctg taatgcccaa cccagattgc
4621 ggctcgaatc aaaacgattg tggcccaagt ggtgcggaac tagcagatga gcatttagat
4681 aaccttgtta agtatgtttc tttactgggt gttcgcccac agcgagacta caacgatgcg
4741 caagtactac agggtaagca agtgtttaac gatgctggct gtgtgagttg tcataccgac
4801 acttaccaaa catcgcaata tcacccgctt gcagagttac gcagccaaac tattcacccc
4861 tacacagatc tcttgctgca cgatatgggc ccaggtttgg ccgataactt aggtgaaggc
4921 gatgcaacgg gtgccgaatg gcgtaccgcg ccgctttggg gcttaggttt gtctgcttgt
4981 gttacgggtg gtgtagaagg tggtcgcggc tgggatgatt ttggcttaga tggctacgaa
5041 acctgtactc cacatcacag ctacttacac gatggtcgcg cacgcaccat tgaagaagct
5101 attttgtggc acggtggcga aggtgaaaac tcgaagcaag cgtaccaaaa cttaagcaat
5161 agcgagcgcg atgctctact cgcattctta aattccctct aa

LOCUS NZ_AABI02000006 4326 bp DNA linear BCT 23-SEP-2003
DEFINITION Microbulbifer degradans 2-40 Mdeg_6, whole genome shotgun sequence.
ACCESSION NZ_AABI02000006 REGION: 86580..90905
VERSION NZ_AABI02000006.1 GI:28878296
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 4326)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027577. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..4326
      /organism="Microbulbifer degradans 2-40"
      /mol_type="genomic DNA"
      /strain="2-40"
      /db_xref="taxon:203122"
   gene 1..4326
      /gene="Mdeg1461"
   CDS 1..4326
      /gene="Mdeg1461"
      /codon_start=1
      /transl_table=11
      /product="COG2273: Beta-glucanase/Beta-glucan synthetase"
      /protein_id="ZP 00066081.1"
      /db_xref="GI:23027643"
      /db_xref="COG:COG2273" 
/translation="MTTRNTFKLSKLLLSLGVLTASSQLVAQDLIWSDEFEGDKIDRS
IWSYNVGGSGNGNGELQYYTANATNSRIEDGNLVIEARREEMEGKQFTSARLHTNGRF
SFKYGTLEARIKLPKLDDGLWPAFWTLGDNFGVDGWPKSGEIDILEAGYKAAREAGTT
NTAVSGAVHWWHESGDWSDWLQADAHAETHVTTPMNEAYHTYRLEWTPSELSISVNDN
TYFTMDITDPNMSEFHGAQHLLLNLAVGGWNFVEIEDPALITADFPAQMLVDYVRLYS
NEFTEVFDANENLPTGDFGVMTDLTPVFNELNWGDRMHLYIWNNMEVAGIDPYEGTSV
LAYDVAPDAWWGMGLLHKDYNMRNYKHGYLHFMMKTTATSDISINMASTSGGEGAVVL
ANGGEEYGLERDGEWHEVNIPLAKFGAMDFETIKTFFSMSGPGQAEAFQIAVDDIYLK
SSIALPRPEFGSFGIYTESPENMSAGNFGFGVEGDLFLWADTLELLPGEVVEGNASLH
LKSTGQGWFGMGLTAREGFNLSAFDNADAKLHLSMKTTDQTDFQVGIKSGSVNDIGQV
WIKFTPGNDPYGFARDGQWHDLVIPMSDIAQDLDMFDVRQVFQLLGFGEIESLAIDNI
YISGGGASTPEIVNPSEPVNRAPMAAIKPSVNGGPATLSVDFDASQSGDVNGDALTYT
WDFGDGTTATGVQVSHDFEKEGNYRVSLIVSDGQATDETAAIITVDDNYGLSRSDKRG
LGFGHHSVADFEAISQGISWWYNWSIKPDSLIQDVYQNYGVEFVPMAWNGGFDDQAMR
DYINAHRDDVKYILAFNEPNFLEQANMTPSQAAAEWPRLEAIADEFGLKIVSVAMNFC
 
GNCVTENGTTYYDPIDYFDDFFEVCPDCRVDALSIHAYMGGVGGIEWYIDLFAKYNLP
IWMTEFSAWDDTTTEEEQIFFMTQAVDYMEQHENVERYAWFTGRRNGHPYNGLFDYRQ
SGVLTELGSAYINMPVHGEDSIHTLPKHIEAEAYAYQSGGRVAPTTDANGFLQMGENA
AGSWVEYNVINPATRSYNVAVRVNSETGGTITVLVDGVEKGQIPVPAAADAQAWATVD
TDLEITAGEHDVRLVFGASVNLNWLNIGDALSPELEPEPEPEPEPEPEPEPEPEPEPEPE
PEPEPEPEPEPEPEPEPEPEPEPEPEPEPEPEPEPEVDPTAPLACTATASSQEGALAPSFVCDG
DAGTRWSSEWNDNETITLDLGDVYEISSINLTWENAYGSKYDILVSDDNVHWTLAYSE
QAGDGGIDNLAVTATGQYVRLLGLERGIGYGFSLFSFDVYGALKPVVVVPEGDLALGK
PTSASSSEWAEPTAANATDGNTGTRWASAWTDSEWISVDMGEVYPVGRVVLNWEGAYG
KGYNIQTSNNGADWTTVYTETAGDGGTDEIILPFASGRYIRVLGTERGTGYGYSLWDF
EIYAD"
ORIGIN 
1 atgacaacgc gaaatacttt caagctgagt aagctcctgc tttcgctagg tgtacttacc
61 gcttcttcgc aactcgtcgc ccaagaccta atttggtccg acgaattcga aggcgataaa
121 attgaccgca gcatatggtc ttacaatgtg ggtggtagtg gtaacggcaa tggtgagttg
181 cagtactaca ccgccaatgc caccaattca cgcattgaag acggcaacct cgttattgag
241 gcgcgccgcg aagagatgga aggtaagcag tttacttctg cacgtctcca tacaaatggt
301 cgcttttctt ttaaatacgg taccttagag gcaagaatta aattgcctaa gttagacgat
361 ggcctgtggc cagcgttttg gactttgggt gataactttg gtgtagacgg ctggccaaaa
421 tctggtgaga tcgatatttt agaagccggc tacaaggctg cgcgcgaagc gggcaccacc
481 aatactgcgg tgtctggtgc tgtgcactgg tggcacgaaa gtggcgattg gagtgattgg
541 ttgcaggccg atgctcatgc cgaaacgcat gttacaacgc ctatgaacga ggcgtatcac
601 acctatcgtt tagagtggac tcctagcgag ctatctattt ctgtaaacga caatacctat
661 ttcaccatgg atattaccga cccaaacatg tcggaatttc acggtgctca gcatttgctg
721 cttaacttgg ctgtaggtgg ttggaacttt gtagaaattg aagacccagc gcttattact
781 gcagatttcc ctgcacaaat gcttgtggac tacgtgcgct tatacagcaa cgaattcacc
841 gaagtgtttg acgcaaacga aaacttacca accggtgact tcggcgtaat gaccgatttg
901 acccccgtgt tcaatgaatt gaactggggt gatcgtatgc acctgtacat ttggaacaat
961 atggaagtag cgggcataga cccgtacgaa ggcacttctg tattggctta tgatgtggcg
1021 ccagatgcgt ggtggggcat gggtcttttg cataaagact acaacatgcg caactacaaa
1081 cacggctacc tgcacttcat gatgaagaca accgctacct ccgatatcag cattaacatg
1141 gccagtacct ctggcggtga aggtgctgtt gtattggcaa acggcggtga agagtacggt
1201 ttagagcgcg acggcgaatg gcatgaggtg aatattcctc tggctaaatt cggcgcgatg
1261 gatttcgaaa ccatcaaaac tttcttcagc atgtctggcc caggccaagc agaagcattc
1321 caaattgctg tggacgacat ttatctaaaa agcagtattg ctttacctcg cccagagttt
1381 ggcagcttcg gtatttacac cgaaagccca gaaaatatga gcgcaggtaa ctttggtttt
1441 ggtgtagaag gtgatttatt tctttgggct gataccttag agctattgcc aggcgaagtg
1501 gttgaaggca atgcatcact gcatttgaaa tcaaccggtc aaggttggtt cggtatgggc
1561 ttaacggctc gcgaaggctt taacctttct gcgtttgata acgccgatgc gaaattacac
1621 ctctcaatga aaaccacaga tcaaactgat ttccaagtgg gcattaagag cggcagcgta
1681 aacgacatcg gtcaagtgtg gattaagttt acccctggta acgacccata cggtttcgca
1741 cgcgacggtc agtggcacga cttagttata ccgatgtctg atatcgcaca agatttagat
1801 atgttcgatg tgcgtcaagt atttcaattg ttaggttttg gcgaaataga aagcctcgca
1861 attgacaata tttatattag cggtggtggt gcatctacac cggaaattgt taacccaagt
1921 gaacctgtaa accgcgcacc gatggctgca attaagcctt ctgtaaatgg cggccctgca
1981 acattgagtg ttgatttcga cgcgagccaa tctggcgacg taaacggcga tgcgcttacc
2041 tatacctggg actttggtga cggtactact gcaaccggcg tacaagtaag tcacgacttt
2101 gaaaaagaag gtaactaccg cgttagctta attgtaagcg acggccaagc gacagacgaa
2161 actgcagcca ttattactgt tgacgataac tacggtttat cgcgcagcga taagcgcgga
2221 cttggttttg gtcaccactc agttgccgac ttcgaagcta tttcgcaggg tatttcttgg
2281 tggtacaact ggtctattaa gccagattca ctgattcaag atgtttacca aaactacggc
2341 gtagagtttg tacctatggc gtggaacggt ggttttgacg atcaagcaat gcgcgattac
2401 atcaatgcgc accgcgacga cgttaaatac attcttgcgt tcaacgagcc taacttctta
2461 gagcaagcca acatgacgcc ttcgcaggcg gcagccgagt ggccacgttt agaagccata
2521 gccgatgagt ttggtttgaa aattgtttcc gttgctatga acttctgtgg caactgtgta
2581 acagaaaatg gcactaccta ttacgacccc attgattact ttgacgattt cttcgaagtg
 
2641 tgccctgact gccgtgtaga tgcgctttct atccatgctt acatgggtgg tgttggcggc
2701 atcgagtggt atatcgatct gtttgcaaaa tacaaccttc caatttggat gaccgagttt
2761 tctgcatggg acgataccac cacagaagaa gagcaaatct tctttatgac ccaagcagta
2821 gactacatgg agcaacacga aaatgtagaa cgctatgcgt ggttcactgg tcgtcgcaat
2881 ggtcacccgt acaacggttt gtttgattac cgccagtcgg gtgtacttac cgagctaggc
2941 agcgcgtaca tcaacatgcc tgtgcacggt gaagacagca ttcacacttt accaaagcat
3001 attgaagcag aagcttacgc ttaccaaagc ggtggacgtg tagcacctac tactgatgca
3061 aatggcttct tgcaaatggg cgagaacgct gctggctctt gggttgaata caacgtaata
3121 aacccagcga ctcgcagtta caacgtagcg gtacgtgtaa acagcgaaac cggcggcacc
3181 attaccgtat tggtagacgg tgttgagaaa ggccaaattc ctgtacctgc agcagctgat
3241 gcgcaagctt gggcaactgt ggataccgat ttggaaatta ctgcaggtga gcacgatgtg
3301 cgcttggtat ttggtgcaag tgttaacctt aactggttaa acattggcga tgcgttaagc
3361 ccagaactag aaccagaacc agaaccagaa ccagaacctg aaccagagcc tgaaccagaa
3421 cctgaaccag agccagaacc agaaccagaa ccagaaccag aaccagagcc tgagccagag
3481 ccagagccag agccagagcc agagcctgag cctgagccag agccagaagt agacccaact
3541 gcaccattag cgtgtaccgc tacagcctca tcgcaagaag gcgcgttagc accaagcttt
3601 gtgtgtgatg gtgatgccgg cacccgttgg tcatccgagt ggaacgataa cgaaaccatt
3661 acgttagacc taggtgatgt ttatgaaata tctagcatta acctaacgtg ggaaaatgcc
3721 tacggaagca agtacgacat attggtttcg gacgataacg tacactggac gcttgcctac
3781 agcgaacaag ctggcgatgg cggtatagat aacttagcag taacggctac tggccaatac
3841 gtgcgcctgc ttggtttaga gcgcggcatt ggttacggct tctctctgtt tagtttcgat
3901 gtttacggtg cactaaaacc tgtagtggtt gtaccagaag gtgatttggc gttaggtaag
3961 cctacgagtg cttcttcatc ggaatgggca gagccaaccg ccgctaatgc aaccgatggc
4021 aacaccggca cgcgttgggc cagtgcttgg acagacagtg agtggatttc tgtagatatg
4081 ggtgaagtgt atcctgttgg tcgagtggta cttaactggg aaggtgcata cggtaaaggc
4141 tataacattc aaacctcaaa caatggggct gattggacta ccgtttacac cgaaaccgca
4201 ggcgatggcg gtacagatga aattattctg ccattcgcgt ctggccgcta tattcgtgta
4261 ctaggtaccg agcgtggcac aggttacggt tactcactgt gggattttga aatctacgca
4321 gattaa
LOCUS NZ_AABI02000013 3624 bp DNA linear BCT 23-SEP-2003
DEFINITION Microbulbifer degradans 2-40 Mdeg_12, whole genome shotgun sequence.
ACCESSION NZ_AABI02000013 REGION: complement(2677..6300)
VERSION NZ_AABI02000013.1 GI:28878289
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3624)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028387. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..3624
      /organism="Microbulbifer degradans 2-40"
      /mol_type="genomic DNA"
      /strain="2-40"
      /db_xref="taxon:203122"
   gene 1..3624
      /gene="Mdeg2183"
   CDS 1..3624
      /gene="Mdeg2183"
      /codon_start=1
      /transl_table=11
      /product="COG2273: Beta-glucanase/Beta-glucan synthetase"
      /protein_id="ZP 00066789.1"
      /db_xref="GI:23028390"
      /db_xref="COG:COG2273" 
/translation="MQPSAYGLYCAACYSQKSKCEFIMIKNNLIKYAVRCAALGALST
SAVHVSATDYNDPYWTNPEVNSDFVDNFNQTQIDRSKWLVETNIFVNGEDIDYQDVEY
PQADWTIGVGQDDPAAIDGKALILKARYMDGEVQDYYGGDPANPGKPLFIRSGRIESQ
ITDDTTFTYGKFEARLKMPPARNGEFPAWWLLGNFPDVGWTACQELDIMEFTGNNGMN
IPQTYWTAPYAVHGGTTVGLAGLGITNPQEQYVTYGIIKTPSKVEWYINGVLTNTFSR
DNQGDDQPWPYVTPMRMILNHAITHVEWPDVGNYNAYSPDANRPSATGWSYVDNNGVT
QYEYIDVDAMNANIGRAGTDFVVDYVAHWPLPTSDAEHKYVDDSKSSFFRESGNTKGF
YNLKGWLAPVSVTADGFDQPGWDTDVRDNGADNAADGYVGSKWATPNDDGIHWVEVDY
GQDKQINYLWLEWAWNLPADYDIYGKSSTGAWEFITSSQQEVATWATHVFDVNRTYRY
LKLVTKGRIDKSSPIWLLELMAFEDVPNMYPKPAGTTMPNRSVNVLNNGNFSQGLDSW
GTEAFDGANPTYNVQNGAAVIALTNDGGLSGSVQLHTSGFGLKRDYRYHISFDARADV
ARNLMVRLAENNLNPSAAGTYHVETVAVGTSFNTYSFTYDYTGQGEPARLAFLLGGMG
TATTYIDNVVIREGDFIGSGEPLVAAISSTNFVSASNGWETEWWGAPARAVDGNLGNK
ASGNDGEAEGMDLDITVRIDEHYDVRAVLVAGDNSPERSLDQFRVEFGNGTPLMGWTD
 
STTEGVYEEFANFNNTPAAGERDFKFFFRPPAGQLVEVADVQLLAVDLMPHRISAIAL
DEGGTISPAGTTRYSRNNTDDATYTFTAPQGQSVSNVIVDGVSMGPLNSYTFTDINAD
HTLAVSFGGEVEQPETGDAINYAPEAYATASSELQTAALANDGDAGSRWESEHGAGPS
WLALELNDTVRVSKVVIDWEAANAGTYEIQGSLNGIDWNTLEVVSGGAFGNRTDTVLL
DGSASNSVNHIRIYGVERSAGNNWGYSIFNVEVWGEAGDTSMEEEVEIEPVSAAASSD
FQAAANAIDADAGSRWESAHADATAHLTLDLASTYKLVSVAIDWEAANAGAYTLEGSS
DGVNWTTIASFTGGAFGNRTDTLTVSGSHRFVRINCTEKSAGNNWGYSIYDVRLTALL
PTP"
ORIGIN 
1 atgcaaccgt ctgcgtacgg tttgtattgt gctgcgtgtt attcacaaaa atcaaagtgt
61 gagtttatca tgattaaaaa taacctaatt aaatatgccg ttagatgtgc agccttaggc
121 gcactctcta caagtgcagt acatgttagt gctacagatt acaacgaccc atattggaca
181 aaccccgaag tgaattctga ctttgtggat aatttcaacc aaacccaaat tgaccgaagt
241 aagtggttgg ttgaaacaaa tatttttgta aatggcgaag atatcgatta tcaagatgtg
301 gagtacccac aagccgattg gacaattggc gtagggcagg acgaccccgc cgcaatagac
361 ggcaaggcgc ttatattaaa agcgcgctac atggatggag aagtgcaaga ttactacggc
421 ggtgacccgg caaacccagg caagccattg tttattcgtt cggggcgtat tgagtcgcaa
481 ataaccgacg atacaacttt tacctatggg aaattcgaag cccgattaaa aatgccgcct
541 gcacgcaacg gcgaattccc cgcgtggtgg ctattaggta actttcctga tgtgggttgg
601 accgcttgcc aagagttaga cattatggaa tttaccggca acaacggtat gaatattcca
661 caaacctatt ggaccgcccc ctatgctgta cacggtggta caacagtcgg tttagcaggc
721 cttggtatta ctaatccgca agagcagtat gttacttacg gtattattaa aacgccgtca
781 aaagtagagt ggtatatcaa cggtgtgctc accaatactt tttctcgcga caaccaaggt
841 gacgatcaac cttggccgta cgtcaccccc atgcgcatga ttttaaacca cgcaattact
901 catgtggaat ggccggatgt aggtaactac aacgcttatt ccccagatgc caacaggccc
961 agcgccacag gttggagcta tgtggataac aatggcgtaa cccaatacga atatattgat
1021 gtagatgcaa tgaacgcaaa tatcggtcgg gcgggtaccg attttgttgt ggattatgtt
1081 gcccattggc cgctacctac tagcgatgcc gaacacaaat atgtagacga tagtaaatcc
1141 agctttttcc gcgaatcggg taacactaaa ggctttacaa atttaaaagg ctggttagcg
1201 ccagtgtcag tgactgctga tggatttgat caaccaggtt gggataccga tgtgcgcgac
1261 aatggcgccg ataatgcagc tgatggctat gtaggctcta agtgggcaac acccaacgac
1321 gacggtattc attgggttga agtggattac ggccaagaca agcaaattaa ttacttgtgg
1381 ttagagtggg cgtggaacct acctgccgat tacgatatat acggtaaaag cagtactggt
1441 gcttgggaat ttatcacctc gtcacaacaa gaagtggcca cgtgggctac gcacgttttt
1501 gatgtaaatc gcacttaccg ttacctaaag ctcgtaacaa aaggccgcat agataaatct
1561 agccctattt ggttgttaga gcttatggca tttgaagatg tgccaaatat gtaccctaaa
1621 cctgcgggta ccacaatgcc taaccgatcc gttaacgtgc taaataatgg caatttttcg
1681 caagggttag atagctgggg aaccgaggcc tttgatggcg caaacccaac ttacaacgtg
1741 caaaatggtg cggcagtcat tgcgcttaca aacgatggtg gcttatcggg ctctgtgcaa
1801 ttgcacactt caggttttgg tttaaagcgt gattatcgct atcacataag tttcgatgct
1861 cgcgccgatg ttgcacgaaa tttaatggtg cgcttagcag aaaacaacct taacccaagc
1921 gcagctggca cctaccatgt agaaaccgtt gctgtgggga caagctttaa cacctatagt
1981 tttacttacg attacacggg gcaaggtgaa cctgctcgac tcgcattttt actgggcgga
2041 atgggtactg cgaccaccta tatcgataat gtggttattc gtgaaggtga ctttattggc
2101 agtggtgaac cgcttgtagc ggctattagc tctaccaact ttgttagcgc cagtaatggc
2161 tgggaaaccg aatggtgggg tgcgcctgct cgtgcggtag atggaaactt aggtaataaa
2221 gcgagcggta acgatggtga agccgaaggc atggatcttg atattaccgt gcgtattgac
2281 gagcattacg acgttagagc ggtattggtt gcgggcgaca attcacctga gcgaagtttg
2341 gatcagttcc gcgttgagtt tggcaatggc acacctttaa tgggctggac cgacagcaca
2401 acagagggtg tatacgaaga gttcgcaaac tttaataaca cgcctgctgc tggcgagcgc
2461 gattttaaat tcttcttccg cccacctgca gggcaactgg tagaagtggc cgatgtgcaa
2521 ttgctagcgg tagatttaat gccccataga atttcagcca tagcgttaga cgagggcggc
2581 actatttcgc cagcaggcac tacgcgctac agcagaaaca acaccgatga tgcaacctac
2641 acgtttaccg ccccacaagg gcaatcggta agtaatgtga ttgtggatgg cgtgtcgatg
2701 ggaccgttaa atagttatac ttttaccgat atcaatgctg atcacacctt agcggtaagc
2761 tttggtggtg aagttgagca accagaaact ggcgatgcta tcaactatgc gcccgaggct
 
2821 tacgcaactg cgagttccga gttacaaacg gcggcgttag ccaacgacgg cgatgcaggt
2881 tcgcgctggg agagtgagca cggtgcaggc ccttcgtggt tagcgctaga gttaaatgac
2941 acagtgcgcg tatccaaagt ggttattgat tgggaagcgg caaacgctgg cacctatgaa
3001 attcaaggct cgctaaacgg tatagattgg aacaccttag aagtcgttag tggtggagcc
3061 tttggtaatc gtacggatac ggttttacta gatggctctg ctagtaattc tgtcaaccat
3121 attcgtatat acggtgtaga acgcagtgct ggcaacaact gggggtattc aatctttaac
3181 gttgaagtat ggggcgaagc aggtgatacc tcgatggagg aggaagtaga aattgagcct
3241 gtatccgcag cagctagcag tgattttcaa gcggcagcga atgcaataga tgccgatgct
3301 ggtagtcgct gggaaagcgc gcacgcagat gctaccgcac acttaacatt ggatttagca
3361 tcaacctata aactcgtgtc agtcgctata gattgggaag ctgccaatgc tggagcttac
3421 accctagaag gttctagcga tggtgtaaat tggacaacca ttgcaagctt caccggcggt
3481 gctttcggca atagaaccga tactttaacg gtgagtggca gccatagatt tgtacgcatt
3541 aactgtacag aaaaaagtgc aggcaacaat tggggctatt ccatttatga cgtacgtcta
3601 actgcattat tacctacgcc ttaa
LOCUS NZ_AABI02000010 2124 bp DNA linear BCT 23-SEP-2003
DEFINITION Microbulbifer degradans 2-40 Mdeg_14, whole genome shotgun sequence.
ACCESSION NZ_AABI02000010 REGION: complement(50649..52772)
VERSION NZ_AABI02000010.1 GI:28878292
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2124)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028613. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2124
      /organism="Microbulbifer degradans 2-40"
      /mol_type="genomic DNA"
      /strain="2-40"
      /db_xref="taxon:203122"
   gene 1..>98
      /gene="Mdeg2431"
   CDS 1..>98
      /gene="Mdeg2431"
      /codon_start=1
      /transl_table=11
      /product="COG2273: Beta-glucanase/Beta-glucan synthetase"
      /protein_id="ZP 00067028.1"
      /db_xref="GI:23028640"
      /db_xref="COG:COG2273" 
/translation="MKKVFSLIIGLSLSLPVWAGWERQWIDTFDGDSVDWTNWTAQVE
ANYNNEVQCYTADETSANKNYDVSDGTLKIIARKQSVECAGLGGQNKTWTSGRLNSKD
KQEFLYGRIESRIRFHNLEGGTWPAFWMLENRISETPRKYDDDYEQWPNPGAGEIDVW
EWFSNGPNIYIINFFNANNCGDRHDYVYPNGGSDVLNWHNYAMEWDANNISFFIDGSL
ITSFDVSSCPQYKEKMFVLLNLAVGGNLGGTIDPNLSLATLEVDYVGYCTATNANDYA
SCDETTPAALQGEPSTSWVLSTVNATATLANAAQNSXXXXXXXXXXXXXXXXXKL"
   gene 1..2124
      /gene="Mdeg2429"
   CDS 1..2124
      /gene="Mdeg2429"
      /codon_start=1
      /transl_table=11
      /product="COG2273: Beta-glucanase/Beta-glucan synthetase"
      /protein_id="ZP 00067027.1"
      /db_xref="GI:23028639"
      /db_xref="COG:COG2273" 
/translation="MLPPRLPMQRKIAXXXXXXXXXXXXXXXXXXNYNNEVQCYTADET
SANKNYDVSDGTLKIIARKQSVECAGLGGQNKTWTSGRLNSKDKQEFLYGRIESRIRF
HNLEGGTWPAFWMLENRISETPRKYDDDYEQWPNPGAGEIDVWEWFSNGPNIYIINFF
NANNCGDRHDYVYPNGGSDVLNWHNYAMEWDANNISFFIDGSLITSFDVSSCPQYKEK
MFVLLNLAVGGNLGGTIDPNLSLATLEVDYVGYCTATNANDYASCDETTPAALQGEPS
TSWVLSTVNATATLANAAQNSGAIAFETSAFGENASDPMIYQAGQNITAGKEYTLAFD
VRSNTAGRAFRAFVAASAEASQGILDQEVVVANADAWQFVSLNFTSEQTFNDAVIAFQ
SGTAALGNGELLFKNIVLTEHSTLYSTTVVTNAGGSINPPGPTVKTVSGYSSHFTITA
NVTHAIGDVFIDGVSVGPVAEYTFDNIQGDHEIEVQFVALPPPEGTEILTPVAATASS
SLSVASRAIDGDYATRWESRHGIEATWLELDLGTAVDLHSILINWEAANAKAYTLEGS
NDGENWTVLASVTNGTFGDRADELSLTGSYSHLRINATERSAGNDWGYSIWDIVLYAY
TQDQNASSSSSSSSNSTTSSSSSSSTSSSSSSSSSSSSSSSSSGGSEPASSGSGRMD
GVVFLFLMVLFGLVTQRVVLAKLVS"
ORIGIN 
1 atgctaccgc cacgcttgcc aatgcagcgc aaaatagcgn nnnnnnnnnn nnnnnnnnnn
61 nnnnnnnnnn nnnnnnnnnn nnnnnnnnna aattataaca acgaagtgca gtgttatacc
121 gcagatgaaa catccgctaa taaaaactac gacgtatctg acggtacgct aaaaataata
181 gcaagaaagc aatccgtcga gtgtgcaggc ctaggtggcc aaaacaaaac atggacatca
241 gggcgattaa acagcaaaga taaacaagag ttcttgtatg gccgtataga atcccgcatt
301 cgcttccaca acctagaggg cggcacgtgg ccggcatttt ggatgctcga aaaccgcata
361 tccgaaaccc cgcgtaaata cgatgatgat tacgagcaat ggcccaaccc cggtgccggt
421 gaaatagatg tgtgggagtg gttttcgaac ggccctaata tttacatcat aaacttcttt
481 aatgctaata actgcggcga taggcacgat tacgtttacc caaatggcgg cagtgatgta
541 ctaaattggc ataactatgc catggagtgg gatgcaaata acattagctt ttttatagat
601 ggcagcctga ttacctcgtt tgatgtgtct tcgtgcccgc agtacaaaga gaaaatgttt
661 gtactactca acttggccgt tggcgggaat ctaggtggta ctatcgaccc caatttatcg
721 ctcgcaacat tagaagtgga ttacgtaggc tactgcacag ctaccaatgc taacgactac
781 gcaagctgtg atgaaacaac acccgctgca ttgcaaggtg agcccagcac atcgtgggtg
841 ttaagcacag taaatgctac cgccacgctt gccaatgcag cgcaaaatag cggagccatt
901 gcgtttgaaa caagcgcttt cggcgaaaat gcatcagacc ctatgattta ccaagcaggg
961 caaaacatca cagccggtaa agaatatacc ttagcgtttg atgtgcgatc caatactgcc
1021 ggccgcgcct ttcgtgcatt tgtggcggct agtgccgaag cctcgcaggg tatattagat
1081 caagaagttg tggttgcaaa tgcagacgca tggcaattcg taagtttaaa cttcacgtca
1141 gaacaaacct ttaacgatgc ggttatagcg tttcaatccg gcactgcagc actgggtaac
1201 ggtgagttgt tgtttaaaaa tatagtgctt accgagcatt ccaccttgta ctcaacaacg
1261 gttgttacca atgctggtgg ttcaataaac cctcctgggc ctacagtaaa aaccgtaagc
1321 ggttacagta gccactttac aattactgca aatgtaaccc atgctattgg cgatgtgttt
1381 atcgatggcg taagcgttgg ccctgttgcc gaatatacgt tcgacaatat acaaggcgac
1441 cacgaaatag aagtgcagtt tgttgcactg ccgccccccg aaggtaccga gatacttacc
1501 cctgtagccg ccactgcgag cagttcactt agtgttgcga gcagagcaat cgatggcgac
1561 tacgcaaccc gctgggaatc gcgccacggt atagaggcta cgtggctaga gttagattta
1621 ggcacagccg tagatttgca ctccatactc ataaactggg aagccgcaaa cgcaaaagcg
1681 tacaccctag aaggctctaa cgatggcgaa aattggacag tactcgcaag cgtaacgaat
1741 ggcacctttg gcgaccgcgc cgatgagcta tcactcactg gcagctactc acacctgcga
1801 ataaacgcaa ccgaaagaag cgcgggtaat gattggggct attccatttg ggatatagtg
1861 ctatacgcat acacgcaaga tcaaaacgca agtagcagct cgtctagttc ttcaaacagt
1921 acaacctcta gttcttctag ctcatccact tcttcgagta gctcttcctc aagctcttct
1981 agttcatctt caagttctag ctctggcggc agtgaacctg cttcgagcgg tagcggcaga
2041 atggatggtg ttgtttttct tttcttgatg gtgttgtttg gtttggttac acaacgcgtc
2101 gtgttagcta aattagttag ttaa
LOCUS NZ_AABI02000004 1722 bp DNA linear BCT 23-SEP-2003
DEFINITION Microbulbifer degradans 2-40 Mdeg_4, whole genome shotgun sequence.
ACCESSION NZ_AABI02000004 REGION: complement(261777..263498)
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1722)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1722
      /organism="Microbulbifer degradans 2-40"
      /mol_type="genomic DNA"
      /strain="2-40"
      /db_xref="taxon:203122"
   gene 1..1722
      /gene="Mdeg1155"
   CDS 1..1722
      /gene="Mdeg1155"
      /codon_start=1
      /transl_table=11
      /product="COG2273: Beta-glucanase/Beta-glucan synthetase"
      /protein_id="ZP 00065780.1"
      /db_xref="GI:23027325"
      /db_xref="COG:COG2273" 
/translation="MEATMIKTCKTTPAKWAAALSLGCALLLPSGVNAATFQAEDYSA
FYDTTAGNTGGAYRNDNVDIEATNDNGGGYNVGWIDANEWLVYPGVNITTTGDYVINV
RVASASGGALSVDFNAGSIPLGQFDIPNTGGWQNWVTVSKTVTLTAGTYDMGVFASTG
GWNFNWIEVTPVNNGGGNGGGSATFFQAEDYSNYSDTTPENIGGAYRNDGVDIETTSD
ANGGHNIGWMENGEWLAYEGLSIPSNGNYIIKARVASPNGGALSFDLNGGGQVLGTMN
IPATGGWQNWQTVELSTNINAGTYTLGAFVSTSGFNLNWIEVVAGGDGGNNGGGGGNI
TWRDEFDTINRDVWNFETGGGGWGNNELQYYTDGQNASIQFDPQAGSNVLVIEARKET
 
GGQCWWGGNCGYTSSRMNTRFKKSFQYGRIEARMKLPRTQGIWPAFWMLGDNFNNVGW
PQGGELDIMEHVGTNNITSGALHGPGYSGNTPITGHLEHGASIDSGYRVYAVEWDTNG
IRWFVDGTNFYSVEKWQVQQYGEWVYDQPFWILLNLAVGGNWPGDPDHANFTTQRFYI
DYVRVIQ"
ORIGIN 
 1 atggaggcta caatgataaa aacatgcaaa accacaccag ccaagtgggc agcagccctt
61 agcttaggct gcgccctact tttaccctca ggcgtcaatg ctgccacctt ccaagcagaa
 
121 gattacagcg ccttctacga caccaccgcg ggtaacactg gcggcgccta ccgcaatgat
181 aacgtcgata tagaagccac caatgataac ggcggcggct ataacgtggg ctggatagat
241 gccaacgaat ggctggtata cccaggcgta aatatcacca ccaccggcga ctatgtaatt
301 aatgtacgcg tggcgagcgc aagcggcggt gcactttctg tcgactttaa tgcaggctct
361 attccactgg gtcagttcga tattcccaac actggcggtt ggcaaaactg ggtaaccgtt
421 tcaaaaacag ttactttaac tgccggcacc tacgatatgg gcgtattcgc ctctaccggc
481 ggctggaact ttaactggat agaagtcaca cccgttaata acggtggtgg caatggcggc
541 ggttcagcca cgttctttca agcggaagac tacagcaact actccgacac cacacccgaa
601 aacattggcg gcgcctatcg caatgacggt gtagatatag aaaccacaag cgatgccaac
661 ggcggccaca atataggctg gatggaaaac ggcgaatggc ttgcctacga aggtttatct
721 attcccagta acggcaacta catcattaaa gcgcgtgtag ctagccccaa cggcggcgca
781 ctatcgtttg atttaaacgg cggcggccaa gtgcttggca caatgaacat acccgctact
841 ggcggttggc aaaattggca aaccgtagaa ttgagcacca acattaatgc cggaacctat
901 acgctcggcg cctttgttag tactagtggc tttaacctaa attggataga agtcgttgcc
961 ggtggcgacg gcggcaataa tggtggcggt ggtggcaaca tcacttggcg cgatgaattc
1021 gacacaatca accgcgatgt ttggaatttt gaaaccggtg gcggcggctg ggtaacaac
1081 gaattgcaat actacaccga cggccaaaac gcttccattc agttcgaccc gcaagccggt
1141 agtaatgtgt tagttatcga agcccgcaaa gaaaccggtg gccaatgttg gtggggcggc
1201 aattgcggtt acacctctag ccgcatgaac acgcggttta aaaaatcgtt ccaatacggc
1261 cgtatagaag cacgtatgaa gctgccgcgt acccaaggta tttggccagc gttttggatg
1321 ctaggtgaca actttaataa tgtaggctgg cctcaaggtg gcgaactaga cattatggag
1381 cacgtaggca ccaacaatat cacatcgggt gcattgcacg gcccaggcta cagcggtaat
1441 acgcccatta ccggccattt ggagcacggt gcaagcattg attctggcta ccgtgtttac
1501 gcagtagaat gggataccaa tggtattcgc tggtttgttg acggcaccaa cttctacagc
1561 gtagaaaaat ggcaagtgca acagtacggc gaatgggttt acgaccaacc tttctggata
1621 ctgctaaacc tagcggtggg aggcaactgg cccggcgacc cagaccacgc caactttact
1681 acccaacgtt tttacattga ctatgtgcgg gtgattcagt aa
LOCUS NZ_AABI02000008 2229 bp DNA linear BCT 23-SEP-2003
DEFINITION Microbulbifer degradans 2-40 Mdeg_7, whole genome shotgun sequence.
ACCESSION NZ_AABI02000008 REGION: 137194..139422
VERSION NZ_AABI02000008.1 GI:28878294
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2229)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027735. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2229
      /organism="Microbulbifer degradans 2-40"
      /mol_type="genomic DNA"
      /strain="2-40"
      /db_xref="taxon:203122"
   gene 1..2229
      /gene="Mdeg1670"
   CDS 1..2229
      /gene="Mdeg 1670"
      /codon_start=1
      /transl_table=11
      /product="COG2273: Beta-glucanase/Beta-glucan synthetase"
      /protein_id="ZP 00066288.1"
      /db_xref="GI:23027856"
      /db_xref="COG:COG2273" 
/translation="MKKLKLLELSLVVLVALGLASCGGSDDKKTPDPVEEPVGEPESE
PESEPESEPESEPEPENAWQLLWEDNFDSEISASNWGFEVNCTGGGNNEKQCYTDRAD
NAYVDEAGILHIVAKEEAFSGPAIQDDDPNYNPDDTSAARNYTSARLRTLDKFDFKYG
RVEIRAQIPGGQGSWPALWMLPSDKVYGGWPASGEIDIMEAVNLDTDAANAVHGTLHY
GLQWPQWSTIGASYETNDDFTGEFHTYAIEWEADQIRWFVDGVHTQTQVSDNWYNFVW
GGQESGFAVANPRAPYDQEFHLIMNVAIGGNWPGDPDTGWASDREMLVDYVRVYQCDS
EADDGTGCANATDAVDIAISPTADVGAPSQVEYDLYSDGLQTPAFTNAGVTLAANVWQ
ETEGNVVTTTGNLGDDHGDAWEITFNGTGNVSIAIAEQDGVESYVRLNGGTAWSNYGV
LAFDMYVDSVDAETGFVVKMDSVYPNVGAVDIATPAAGEWTRVHVKVADILANPIAGG
GGLNVAQAVNLFVLEPTGTKTAHVYVDNISISCAYNSTAKSWQGDKTCDVAAVPTADT
SGIDLSGNELVIFDEQEPDFWGFGQFSAGGAEVAMSFLADPEDASHGNVLGFSYPDSQ
NVAYLQSATPLNLSDWAGGTIQFDMYVISEPANVNWMMKVDCVHPCSSGDIPLTTNID
GVVPAVGVWQTYRFNLDALVAGNPGLDLTKVDTPLVIFPAWDNQTGANFRIDNIKFVR
AN"
ORIGIN 
1 atgaaaaaac taaagcttct agaactttcg ctggtggtgt tggtagccct agggctagcg
61 agctgcggcg gttctgacga caaaaaaacg ccagacccag tagaagaacc cgtgggtgaa
121 ccagaatcag agcctgaatc agagccagaa tctgaacccg aatctgagcc agaaccagaa
181 aacgcatggc aattgctgtg ggaagataat ttcgacagcg aaataagcgc atctaactgg
241 ggcttcgaag ttaactgcac cggcgggggt aacaacgaga aacagtgtta taccgataga
301 gccgacaacg cttatgtaga tgaggcaggc atactgcaca tagttgccaa agaggaagca
361 tttagcggac ccgctattca ggatgatgac cccaactaca accccgatga cacatcggct
421 gcgcgcaatt atacctctgc gcgtttgcgt acattagata agtttgactt caaatatggt
481 cgagtcgaaa ttcgtgcgca aataccaggt gggcagggct catggcctgc attgtggatg
541 ttgccaagtg acaaagtata tggtggttgg ccagccagtg gcgaaatcga cattatggaa
601 gcggttaatt tagatactga tgcggccaat gccgtgcatg gtactttgca ctacggttta
661 cagtggccac aatggtcgac gataggcgct tcttacgaaa caaatgatga tttcaccggt
721 gagttccaca cttatgccat tgagtgggaa gcagaccaaa ttcgctggtt tgtggatggc
781 gtacacactc aaacacaggt gtccgataac tggtacaact ttgtatgggg cgggcaagag
841 tccggctttg ctgtggctaa cccgcgtgca ccttacgatc aagagtttca tttaatcatg
901 aacgtcgcta taggcggcaa ctggccgggt gacccggaca cgggttgggc atctgatcgc
961 gaaatgcttg tagattatgt gcgtgtgtac cagtgtgatt ctgaagccga tgatggaaca
1021 ggctgtgcaa acgcaaccga cgccgttgat attgctattt caccgaccgc agatgtaggt
1081 gcgccatcac aagtagaata cgacctatat agtgacggtt tacaaacgcc tgcatttacc
1141 aacgccggcg taactcttgc tgcaaatgtt tggcaagaaa cagaaggcaa cgtagtaact
1201 accactggca acttgggcga cgaccatggc gatgcttggg aaattacctt taatggtact
1261 ggtaatgtat ctattgctat tgccgagcaa gacggcgtgg aaagctatgt gcgattaaat
1321 ggcggaacag cttggagtaa ctacggcgta ctagcgtttg atatgtatgt agatagcgtg
1381 gatgccgaaa ctggctttgt tgttaaaatg gacagtgttt accccaatgt aggtgctgta
1441 gatattgcta cacctgccgc aggtgagtgg acgcgagtgc acgtaaaagt ggctgatatt
1501 ttagctaacc caattgcagg tggcggtggc cttaacgttg cgcaagcggt taacttgttt
1561 gtacttgagc caacaggtac taaaacagca catgtatatg tagataatat ttctattagc
1621 tgcgcatata actctactgc aaaatcttgg cagggtgata aaacctgtga tgttgcagcg
1681 gtgcctactg ccgatacctc aggtatagat ttaagtggta acgagcttgt gatttttgat
1741 gagcaagagc cggatttttg ggggtttggg cagttttctg ctggtggtgc tgaagtagca
1801 atgagcttct tagctgaccc agaagatgcg agtcacggta atgtgttggg ctttagctac
1861 ccagatagcc aaaacgttgc ctacttacaa tctgctacac cgcttaattt aagcgattgg
1921 gctgggggta ctatccaatt cgacatgtac gttattagcg agcctgctaa tgtaaattgg
1981 atgatgaaag tggattgtgt acacccttgt tcatctggcg atataccgct caccaccaat
2041 attgatggtg tagtgcctgc tgtgggtgta tggcaaacct atcgctttaa cttagatgca
2101 ttagttgctg gcaacccagg gttagatttg accaaagtgg atacgccgct agtaatattc
2161 cctgcatggg ataaccaaac cggtgctaac ttccgtatag ataacattaa atttgttcgg
2221 gctaactag
LOCUS NZ_AAB102000048 2646 bp DNA linear BCT 23-SEP-2003
DEFINITION Microbulbifer degradans 2-40 Mdeg_49, whole genome shotgun sequence.
ACCESSION NZ_AABI02000048 REGION: 1591..4236
VERSION NZ_AAB102000048.1 GI:28878254
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2646)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23030011. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2646
      /organism="Microbulbifer degradans 2-40"
      /mol_type="genomic DNA"
      /strain="2-40"
      /db_xref="taxon:203122"
   gene 1..2646
      /gene="Mdeg3707"
   CDS 1..2646
      /gene="Mdeg3707"
      /codon_start=1
      /transl_table=11
      /product="hypothetical protein"
      /protein_id="ZP 00068290.1"
      /db_xref="GI:23030013" 
/translation="MGLTMVKNKLYLALSIAAATSLTACGGGGDAADDTVKRNVFAVG
DVFKTQEDGDAVEADVSENDFGRGLTFALESGSTTANGELVFNADGSFTYTPNADFSG
KDSFTYVATHTASGDTASALVTINVISDFETIEESGWTLTWSDEFDSLDSMAWDAMNA
SASEGVLSVSAVEGQTSYVKSTAALGQAGRIEASIQLPDGKSLYSGFGLMPMADMFDG
KNALMAIESANNKATAGGHWGIGLVNGVEINEPTNAVVRAEFHTYAIEWNESLIRWYI
DDIHIHTVDTLNTWSYNLSGDTVVADTTTKPFSQDLQIMMELTAASSGLPNAMLVDFV
KVYECDTSVTDQIESCAFAADENVDKLASNRIESVGEIVTPLFTDELTSLSWHYSDAE
EAVTFVTEQESAVPTHRGIVSQPDVMEPVAEVAPVDPVEEGEEGYEEYQAYLAYVDYL
NYLETLAFLDTVDPSRERGAVIRYQSDASTWSNFSLNTPSLGLVGKDSALQFDMYIDS
ASTTTETIEIRMETGWPFLGTVLLNVADLQLDTWVTYNIPVSDFLANPFITPDWAVGQ
DWFLGGNGVEGQPLYLDTNSITKAIVVQLAAPGHLVFDNVAITCVSNESCFQGPLAKQ
PIVKAGPAPIIYEAEAYTAVTGEVQTEDTQDAGGGQNVGYIDAGEALEYTIVAPIDGT
YKFQYRLASGLESASEFDVSIDDMLIDGQSLPGTGGWQVWTTLESGEFDLTAGEHAIV
FNFAGGMNFNWFAIVPPPIAIFIEAEDYSSMAGVQLEDTADEGGGQNVGYIDAGDFLQ
YNVEVPADGTYFIELRVASSGGSDGFTITSNGITTSTIPVADTGGWQNWTTQTVEMQL
 SAGQQTLRFDFIGGAINFNWINVTN"
ORIGIN 
1 atggggttaa caatggttaa gaataaattg tatctagcgc tatctatagc ggctgctaca
61 agcctaacgg catgtggtgg tggcggggat gcggctgatg acactgttaa acgcaacgta
121 tttgccgtag gcgatgtgtt taaaacgcaa gaagatggcg atgcggttga ggcggatgta
181 agtgaaaacg attttggcag aggcctcacg ttcgcattag aaagcggtag caccacggct
241 aatggcgagc tggtattcaa tgccgatggc tcttttacgt atacacccaa tgcggacttc
301 tctggtaaag actcctttac ctatgtggcc acccacaccg catcgggcga tacagccagt
361 gccttggtga ctattaacgt aataagtgac tttgaaacca tcgaggaatc tgggtggacg
421 ctaacgtggt ccgacgaatt cgatagttta gatagcatgg cgtgggatgc gatgaacgca
481 tctgcttctg aaggtgtgtt gtcggtaagc gctgtagaag ggcaaacatc ttacgttaaa
541 agcaccgctg cactggggca ggctgggcgt attgaggcga gcattcagtt gcccgatggt
601 aaaagcttgt actctggctt tggcttaatg ccaatggccg acatgttcga tggtaaaaat
661 gcattaatgg ccatcgagag cgcgaacaat aaggcaactg ctggtggtca ttggggtatt
721 ggattagtaa atggtgttga aattaatgaa cccactaacg cagtagtacg tgcggaattt
781 catacctatg ccatagagtg gaatgagagt ctgattcgct ggtatataga tgatatacat
841 attcataccg tagataccct taatacctgg tcatacaatt taagcggcga taccgtggtt
901 gccgatacta ccactaagcc gtttagccaa gacctgcaaa taatgatgga gttaacggca
961 gcaagttcag gtttgccaaa tgcaatgctg gtagattttg taaaggttta tgagtgtgac
1021 acgtctgtta ccgatcaaat agaaagctgc gcattcgcgg ctgacgaaaa tgtggataag
1081 cttgcttcca accgtattga gtcggttggt gaaattgtta ccccattgtt cacagatgag
1141 cttacatcgt taagctggca ttactctgat gccgaagaag ctgttacttt tgttacagag
1201 caagaaagcg ctgtgcctac tcatagaggt attgtttctc agcccgacgt tatggagcct
1261 gtagcagaag ttgcaccagt ggaccctgta gaagaaggtg aagagggcta cgaggaatat
1321 caagcttact tagcttatgt ggactacctg aactatttag aaactctcgc atttttagat
1381 acagtggacc caagccgcga acgtggcgct gttattcggt atcaatcgga tgcaagcaca
1441 tggtctaatt ttagtttgaa tactccgagc ttaggcttag ttggaaaaga ttccgcgctg
1501 cagtttgata tgtatatcga tagcgcatct acaactaccg aaaccatcga aatacgcatg
1561 gaaaccggtt ggccattcct tggcactgtt ctgctaaacg ttgcagactt gcagctagat
1621 acttgggtga cgtacaacat tcccgttagc gactttttag ccaatccatt tattacaccc
1681 gattgggcag tgggccaaga ctggttcttg ggcggtaacg gtgttgaagg gcagcctctc
1741 tatttagata ccaattcaat tactaaagcc attgtggttc agctagcagc accagggcat
1801 ttagttttcg ataacgttgc aattacttgt gtttcgaatg aaagctgctt ccaggggcca
1861 ttagccaaac agcccatcgt aaaggccggt cctgctccca ttatttacga agcagaagct
1921 tacacggcgg taacgggtga agtgcaaacg gaagataccc aagacgcggg cggcggccaa
1981 aacgttggct atatcgacgc aggtgaagca ttggaataca ccattgttgc acctatcgac
2041 ggtacctata aattccaata tcgcttggca agtggcttag aaagcgcttc tgagtttgat
2101 gtttctatcg acgatatgct tatcgatggc caaagcctac ctggtactgg tggctggcaa
2161 gtgtggacca cattggagtc tggtgagttt gatctaactg ctggtgaaca cgctattgtg
2221 tttaattttg ctggcggtat gaactttaac tggtttgcaa ttgttccgcc ccctattgcg
2281 atttttatcg aagctgaaga ttactcgtca atggctggtg ttcagcttga agacaccgca
2341 gatgaaggcg gcggtcaaaa tgttgggtat atcgacgcag gtgatttcct tcaatacaac
2401 gtcgaagtgc cggctgatgg tacctacttc attgaattgc gtgtagccag cagtggtggt
2461 agcgatggct ttaccattac ttccaatggc attactacaa gcactattcc tgtagcggat
2521 accggtggtt ggcaaaattg gactactcaa actgtagaga tgcaattgtc tgctggtcaa
2581 caaacacttc gtttcgactt tatcggtggg gccattaact ttaactggat caatgtcacc
2641 aactaa
LOCUS NZ_AAB102000054 3558 bp DNA linear BCT 23-SEP-2003
DEFINITION Microbulbifer degradans 2-40 Mdeg_54, whole genome shotgun sequence.
ACCESSION NZ_AABI02000054 REGION: complement(<2..3559)
VERSION NZ_AABI02000054.1 GI:28878248
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3558)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23030054. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..3558
      /organism="Microbulbifer degradans 2-40"
      /mol_type="genomic DNA"
      /strain="2-40"
      /db_xref="taxon:203122"
   gene <1..3558
      /gene="Mdeg3739"
   CDS <1..3558
      /gene="Mdeg3739"
      /codon_start=1
      /transl_table=11
      /product="COG3979: Uncharacterized protein contain
      chitin-binding domain type 3"
      /protein_id="ZP 00068322.1"
      /db_xref="GI:23030055"
      /db_xref="COG:COG3979" 
/translation="MILRLIRAAIYIVAFVSLIACGGSSTSKPAQPDIPDTDLPDTGT
PEPENSDPVVQSTPATAIAAGNVYSYQIMASDADESDVISYAAVTLPSWLAFDPDTGI
LTGTPQQAHAGNAEVVLSYSDGNVTLQQQFTIAVSASYTEEPPTPMSRPTVNAADTST
YTITAYGAGSIADAINPASYGCVYDYGNWIYNAGVVEPGVSGCDPIGAPTYRTPQVVG
EAASVPTPTHKWWGSVSFLGEMKIGDPAGAGYITPDPITARISNKGVRIMGIPNGLGA
QGNQFIYSVPDPFSEVFDGIAVANSEYANLEAYLKSYSDGTATVQWQSGNLPVMQATF
VHGSPYVFFKAYRGNMVLRTKAADGGEKGTFYNENNSLGIWTSVAGNKNDFLITGEGE
TVFNNIETDTITLTNAANEFTLTLLPTAGAGTPSSTVIQAFEDSARAVVAKVDIQYSV
DRTNNMVTVTHTYKNESNTPVQTLAGLLPMHWKYSDTALSGYKTRSARGMVQFAHIDS
FSYTIPYVGVLPYLPSSVGDFDSSVLAGLVQAFVAEGPENWNPHTDTYWSGKAFNKVA
ELSAIARSVGMTSEADTLLNWLKAELQDWFSANTNGSLDEKKYFVYDAEWNTLLGLEE
SFAAHQQLNDHHFHYGYFVRAAAEICRVDASWCGADQYGPMVELLIRDYAGAKDDTMF
PYVRNFDPANGFSWASGSANFVLGNNNESTSEAANAYGAIILYGLITGDNELVERGMY
 
LHASSSVAYWEYWNNIDRYLGADADRDNFPSGYDKLTTSIIWGHGGVFSTWFSGAYAH
ILGIQGLPTNPLIFHVGLHPEYMEDYVALGLSESSNNKPSGLIDDQWRDIWWNLWALT
DAEAAIADYNTVGSNYAPEFGETKAHTYHWLHTWNALGHLKTGTGELTVNDPAALVFE
KDGIKTYVAYNFSGTPKTILASDGFEFIAQPNDFTVVTTADNNPDDTQPPTLPANLQA
LNLTQTSLDVKWDASTDNYRMAGYVVQVLQADTLIEETSSIASIASFNNLTASTSYTI
QVKAKDRSGNETAWVSITVTTPSETDDLLPTLDGGVYSANVGPNSADLSWAAATDDRG
IASYTIEVQVGGAVFVTETVFDTSYALSGLTEATEYNVAVYATDTGGQQSATISGIVN
TTSNPFGSGCELICASATSSSSVTFTVHQAGAVDIHYLVN"
ORIGIN 
1 atgatactgc gactgatacg tgccgcaatt tacattgtgg cgtttgtttc tcttatcgcc
61 tgtgggggct caagcacatc caagcccgcg caaccggata ttcccgatac ggatctgcct
121 gacacgggaa cacccgaacc cgaaaacagc gatccagttg ttcaatctac gccggctaca
181 gccattgcgg cgggcaacgt gtatagctat caaataatgg ctagcgacgc agacgaatcg
241 gatgtcatta gctatgcggc ggttactttg cctagctggc tggcgttcga cccagatacc
301 ggaatactta ccggcacgcc ccagcaagct catgctggta atgctgaagt ggtgcttagc
361 tatagtgacg gcaacgtaac cctgcagcag caatttacaa ttgcagttag cgcgagctac
421 accgaagagc caccaacacc gatgagtcgc ccaaccgtaa atgcggcgga taccagtact
481 tacactatta ctgcctacgg cgcgggcagt attgcggatg cgattaaccc tgctagctat
541 ggctgtgtgt acgattacgg taattggatt tataacgccg gcgttgtgga gccaggagta
601 agcggctgtg accctatagg cgcaccaacc taccgtacac cgcaggtggt tggggaagcg
661 gcaagcgtgc caaccccaac gcataaatgg tggggctcgg tttctttttt aggtgaaatg
721 aaaataggtg acccggcagg cgcaggttac ataacgccag acccaataac tgcacgtatc
781 tcgaataaag gcgtgcgtat tatgggcata cccaacggct tgggcgcgca aggcaaccag
841 tttatttact ctgtacccga cccttttagc gaggtattcg atggaatagc ggtagcgaat
901 agcgaatatg ccaacctaga agcgtattta aaatcctaca gcgatggcac cgctaccgtg
961 caatggcaga gtggcaacct gccggttatg caggccacat ttgtacacgg ctccccctat
1021 gtgtttttta aagcctatcg cggcaacatg gtgttgcgca ccaaagctgc agacggcggt
1081 gaaaaaggca cgttttataa tgaaaataat agtttaggta tttggaccag tgtagcgggt
1141 aacaaaaatg actttttaat taccggtgaa ggcgaaacgg tttttaacaa tatcgaaacc
1201 gataccatta cgttaaccaa tgcagcgaac gaatttacct taacgttatt acctacagcg
1261 ggcgccggta caccttcaag tactgttatt caggcgtttg aagatagcgc ccgtgcagtg
1321 gttgccaaag tagatattca atactcggta gaccgtacca ataacatggt aacggttact
1381 catacctata aaaatgaaag caatacgcca gtgcaaaccc tcgcggggtt actgcccatg
1441 cattggaagt attccgatac agcattaagc ggctacaaaa cccgaagtgc gcgcgggatg
1501 gtgcaatttg cccatattga ttcgtttagc tacaccattc cttatgtggg agtattaccg
1561 tatttaccgt catcggtagg ggatttcgat tcctctgtgc tcgcaggctt agtgcaggcg
1621 tttgtagctg aagggccaga aaattggaac ccgcacaccg atacctattg gtcgggcaaa
1681 gcgtttaata aagttgccga gttatcggca atagcgcgct cggtaggtat gacaagcgaa
1741 gcagatacgc tgttaaattg gcttaaagcc gaactgcaag attggtttag cgccaacacc
1801 aacggcagtt tggatgagaa aaaatacttt gtgtacgatg ccgagtggaa taccttgctt
1861 ggcctagaag aatcttttgc tgcacaccaa caactaaacg atcaccactt tcactacggc
1921 tattttgtac gcgccgctgc agaaatatgc cgagtagatg caagctggtg tggcgcagac
1981 cagtatggtc ccatggtgga actgcttatt cgcgattacg ccggtgccaa agacgatact
2041 atgttccctt acgtgcgtaa cttcgacccc gccaatggct tttcttgggc atcgggtagt
2101 gccaactttg tgctgggtaa caacaacgaa tccacatccg aagcggccaa tgcctatggt
2161 gcaattattc tgtacgggtt aataactggc gataatgagt tagtggagcg cggtatgtat
2221 ttgcacgcgt cttcgtcggt ggcctactgg gaatattgga acaacatcga ccgctacttg
2281 ggggccgatg ccgaccgcga taattttcca tcgggttacg acaaactaac tacctctatt
2341 atttgggggc atggcggcgt gttctctacc tggttcagtg gcgcttacgc tcatattctt
2401 ggtattcaag ggctgcctac caacccgctt atttttcatg tgggcttaca ccccgagtat
2461 atggaagact atgtggcact gggtttaagt gaatcgagca acaataaacc ttcggggtta
2521 atcgacgatc agtggcgcga tatttggtgg aacctatggg ccttgactga tgcagaagcg
2581 gccatcgccg attacaacac ggtaggcagt aactatgcgc cagagtttgg tgaaaccaag
2641 gcgcacacct accactggtt gcacacgtgg aatgcgctcg gccatttaaa aaccggcact
2701 ggcgagctaa cggtaaacga ccccgctgca ttagtgtttg aaaaggacgg aataaaaacc
2761 tacgtagcgt ataactttag cggtacacca aaaactattt tggctagcga tggctttgag
 
2821 tttattgcgc agcccaatga ttttaccgtg gtgaccaccg ccgataacaa ccccgacgac
2881 acgcaaccgc ctacactacc ggcaaacttg caagcgctta accttaccca aacgagcttg
2941 gatgttaaat gggatgcatc taccgataac taccgtatgg caggttatgt agtgcaggta
3001 ttacaagccg atacattaat agaagaaacg tcttctatag ccagcatcgc ctcgtttaat
3061 aacttaacgg ccagcacaag ttacaccatt caagttaaag ctaaggatcg ctctggcaat
3121 gaaaccgcat gggtaagcat tacggtaacc acgcccagcg aaaccgacga tttactacca
3181 acacttgatg gcggtgtata tagcgcaaac gtggggccta actctgcgga tttaagctgg
3241 gcggcagcaa cagatgatcg cggtatagca agttacacca tagaggtgca ggttggcggc
3301 gcagtatttg taactgaaac agtgtttgat acttcgtatg cgcttagcgg attgaccgaa
3361 gcaaccgaat acaatgtggc ggtttatgct acagataccg gcggtcaaca atctgccact
3421 attagcggta ttgttaatac caccagcaat ccatttggca gcggttgcga attgatttgt
3481 gccagcgcta cctctagctc atctgttacg tttactgtgc atcaagcggg cgcggtagat
3541 attcactact tagtgaac
LOCUS NZ_AABI02000023 1116 bp DNA linear BCT 23-SEP-2003
DEFINITION Microbulbifer degradans 2-40 Mdeg_24, whole genome shotgun sequence.
ACCESSION NZ_AABI02000023 REGION: complement(49214..50329)
VERSION NZ_AABI02000023.1 GI:28878279
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1116)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029379. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1116
      /organism="Microbulbifer degradans 2-40"
      /mol_type="genomic DNA"
      /strain="2-40"
      /db_xref="taxon:203122"
   gene 1..1116
      /gene="Mdeg3181"
   CDS 1..1116
      /gene="Mdeg3181"
      /codon_start=1
      /transl_table=11
      /product="COG5297: Cellobiohydrolase A
      (1,4-beta-cellobiosidase A)"
      /protein_id="ZP 00067766.1"
      /db_xref="GI:23029425"
      /db_xref="COG:COG5297" 
/translation="MGSSFLTCYKPSKEVITVKRAAINLVLIPGLAVSMGTLSSSAFA
QSTCSVDYRVESDWGAGATHKVLVTNTGAPINGWQMSWTFSGNEQITNLWNGMFTQSS
QSVIVDSLSWNAQLNTGATAEVGFNINSPSGQLPDVYLNGVNCSNPGEPEPEPEPEPEPE
PEPEPEPEPEPEPEPEPEPEPEPEPEPEPEPEPEPEPEPELAYSLDATASFLNFVSSKKT
HVLETHRFDVLSGGISTAGEAQLVIDLNSVNTGIDVRNGRMRDYLFETATYSVATVTV
PVDLAAVAGLAVGEDMLVDVSATLDLHGVPGVIDTQLNVQRLSATRIMVQNQSPLLIK
AADYSLEAGIETLRNLASLNVISTTVPVDFVLFYEAP"
ORIGIN 
1 atgggaagca gttttctcac ctgttataaa cctagcaaag aggtaataac cgtgaagcga
61 gcagcaataa acttggtgct gatacccggg ctagccgtaa gtatggggac gttgtcgtct
121 tctgcttttg cacaaagcac ttgtagtgtg gactatcgag tagagtccga ctggggtgca
181 ggggctacgc acaaagtatt agttactaac actggagccc ccataaacgg gtggcaaatg
241 tcatggactt tttctggcaa cgagcaaatc actaaccttt ggaatggcat gttcacccaa
 
301 agctctcaaa gtgtgattgt tgatagtctg tcgtggaatg cccagctaaa taccggtgcc
361 accgcagaag tggggtttaa tattaactct ccttcagggc agttgccgga tgtgtatttg
421 aacggcgtaa attgtagtaa ccctggtgag ccagagccag agccagagcc agagccagag
481 ccagagccag agccagagcc agagccagaa cctgagcctg agccagaacc tgagccagaa
541 cctgagccag aacctgagcc agaacctgag ccagagccag agccagagcc agaattggcc
601 tattcattag atgcgactgc atctttctta aactttgtta gctcaaagaa aactcacgtt
661 cttgaaacac accgttttga cgtgctttct ggcggtatta gtactgcggg tgaggcacaa
721 cttgttatcg atcttaacag tgtgaacaca ggtatagacg tgcgcaacgg acgtatgcgc
781 gattacctgt ttgagactgc aacctattct gtagcaaccg tgactgtgcc tgtagatcta
841 gccgcagtag caggtttagc cgtgggcgaa gatatgctgg tggatgtatc cgcaactttg
901 gatcttcacg gtgtgccagg cgttatcgat acgcagctaa atgtacagcg cttatcggca
961 actcgcatta tggttcagaa ccaatctcca ctattaataa aggcagccga ttattcgctt
1021 gaggcgggta tcgagacatt gcgtaatctc gcgagcctaa atgttattag caccacggtg
1081 ccagtggatt ttgttttgtt ctacgaagcg ccttaa
LOCUS NZ_AABI02000023 3129 bp DNA linear BCT 23-SEP-2003
DEFINITION Microbulbifer degradans 2-40 Mdeg_24, whole genome shotgun sequence.
ACCESSION NZ_AABI02000023 REGION: complement(46057..49185)
VERSION NZ_AABI02000023.1 GI:28878279
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3129)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029379. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..3129
      /organism="Microbulbifer degradans 2-40"
      /mol_type="genomic DNA"
      /strain="2-40"
      /db_xref="taxon:203122"
   gene 1..3129
      /gene="Mdeg3180"
   CDS 1..3129
      /gene="Mdeg3180"
      /codon_start=1
      /transl_table=11
      /product="hypothetical protein"
      /protein_id="ZP 00067765.1"
      /db_xref="GI:23029424"

/translation="MKKIFKISALSLGFSIAGAASAADLCNVTYEAVNSWGSGAQQAV
TVVNNGPALNAWQLSWTFNGSENIDNLWDGVLSQTGANVTVNNAGYNGSVGTGGQFSF
GFTVSGWSENFPTEFYLNGEACSGAVDPNPNPNPEPTDGAVWELNSADSVFSFVTVKK
EHVAEVQTFTAYNATVDSDGVATLAIDLNSAETNIDIRNERFRNVLFETAFLPTLYYS
VQLDMASLSALAVGDAQTQTLGGTLTLHGVQAVVEAEVLVVKTSATDLTVSTSKPILI
KAADFDLVSGVESLRALASLSSIGQTVPVYFRLDFDAADPQVTNAVAVPATPAAPTSL
TADFTESSGIAALNWNDASNNETEFLVRRREASTGYWSRLTEVNANSTLLDDLLLEED
TYDYKVIALNNGVPSAPSPVATVVATTNPNPEPLTGEEHYQAKCASCHGDDASGGVVG
VALNTERDLTVMLNTIVTRMPPGEADNCDQECAEAIGGYIQTTFWNGGEPEPELACDT
VTYGARQLKLLTKAEYQRSVEDLVGIDYNVASGLAEDNIIGYFVNNTTKVVVPTVYDQ
YLTVAEEIAQWSADRNFAGALTCGTNFNQTCANQFVNNFAPKVFRRALSSDEAAAYLA
IANGSATNGDVKAGIQLAMEGLFSSPQFVYRHELGEANPNNNAIDSDAFELTSYEMAT
WLSYTYAGTTPDAIAMQKAANNQLRTDAEIRAEAQRLLEGAGAKQKMGDFVASWLGTD
HIANAPKDASVWPGFDALIPHLQTEIREMFSYVMLEPTESFASVYNANYTFVNGPLAQ
HYGINGVSGNEFQKVTTTDRGGILANGAFMARWGESVESSPIRRSVRVRRRMLCQDQP

DPPGNVNIGRENAADEFHEALADPTTTNRERYELLTSGETCATCHQEWINPLGFGMED
FTAVGTRRVTDLNGNTIDASGQLYAPENLNDKDVFINFNGTQGLGALLTTLPSAQSCL
PQNLFRYSVGVGVEGLDDNPEGNELVPAERDGYACEVKNLTSTMLEQSPRAMLEGMGS
MQAVRYRKAWAR"

ORIGIN 
1 atgaaaaaaa tattcaagat ttcagcgctc tcgctaggtt tctcaattgc tggcgctgca
61 tctgcagctg atttgtgcaa tgtaacctac gaagcggtaa atagctgggg ctccggtgcc
121 cagcaagcgg taaccgttgt aaataacggc cctgctttaa atgcctggca gttaagctgg
181 acatttaacg gcagcgaaaa tatagacaac ctatgggatg gcgtgctttc gcaaactggt
241 gcgaatgtta ccgttaacaa tgctggctat aacggcagtg ttggtactgg cggtcaattt
301 agcttcggtt ttaccgtaag cggttggagc gaaaactttc ccacagagtt ttacctaaat
361 ggtgaagcgt gtagtggagc tgttgaccct aatcccaatc ctaacccaga gccaactgat
421 ggcgctgtgt gggagttaaa ctcagccgat tcagtgttta gctttgtaac tgttaaaaaa
481 gagcacgttg ccgaagttca aacgtttact gcctacaacg caacagttga tagcgatggc
541 gttgcaacgc ttgctataga cttaaacagt gcagaaacga acattgatat acgcaacgag
601 cgcttccgca atgtattgtt tgaaacagct ttccttccta ctctgtacta cagtgttcag
661 ctagatatgg cttcactgtc tgcattggcg gttggcgatg ctcaaacgca aacgcttggt
721 ggcacgctaa cactacacgg tgtacaagca gtggttgagg ctgaagtgtt ggttgttaaa
781 actagcgcta ccgacttaac cgtatcaacg tctaagccta ttttaattaa ggctgccgat
841 tttgatttgg taagcggtgt agagtctttg cgagccttgg ctagtctttc aagtattggt
901 cagacggtac ctgtttactt ccgtttagac ttcgatgctg cagacccgca agtaactaat
961 gcggttgctg ttcctgctac tcctgcggcg ccaacgtctc taactgcaga cttcacagaa
1021 agcagtggta ttgccgcgtt gaactggaat gatgcaagca ataacgaaac cgagtttttg
1081 gttcgtcgtc gtgaagcgtc aaccggttac tggtcaagac tgaccgaagt taacgcgaac
1141 agtactttgt tagatgatct tctgttagaa gaagacacct acgactacaa agtgattgca
1201 ttgaacaacg gtgtgccttc tgcgccatcg ccagttgcaa ctgttgtagc tactaccaac
1261 cctaacccag aaccattgac tggcgaagag cactatcaag ctaagtgcgc aagctgtcac
1321 ggtgatgacg ctagcggtgg tgtagttggt gttgctttga atactgagcg cgatttaacc
1381 gttatgctga acaccattgt tactcgcatg cctccaggcg aagcggataa ctgtgatcaa
1441 gagtgtgcgg aagcaattgg tggttatatt caaactactt tctggaacgg cggtgagcca
1501 gagccagaat tagcttgtga cactgttact tacggtgctc gtcagttgaa gctacttacc
1561 aaagctgagt atcaacgttc tgttgaagac ttagttggta ttgattacaa cgtagcaagc
1621 ggcttagcag aagataacat tattggttac ttcgttaata acaccaccaa agtggttgtg
1681 ccaacggttt acgatcagta tctcacagta gctgaagaaa tcgcgcagtg gtctgctgac
1741 agaaactttg cgggtgcatt aacttgtggc actaacttta accaaacctg tgcgaatcag
1801 tttgttaaca acttcgctcc aaaagtattc cgacgcgcgc tttctagcga tgaagctgcg
1861 gcttacttgg cgattgctaa cggcagcgct accaacggtg atgttaaagc gggtattcaa
1921 cttgcgatgg aaggcttgtt ctcttctccg caatttgtat atagacacga gcttggtgaa
1981 gccaatccaa ataacaatgc tatcgactct gacgcgtttg aattaacgtc ttatgaaatg
2041 gctacttggt tgtcttacac ttatgccggt accacaccag atgcaattgc tatgcaaaaa
2101 gctgcaaaca atcagttgcg cacagacgcg gaaattcgcg ccgaagctca acgtttgtta
2161 gaaggtgctg gtgcgaagca aaaaatgggt gacttcgttg ctagctggtt aggtactgac
2221 cacattgcaa acgcacctaa agatgcttct gtatggcctg gttttgatgc gttaattccg
2281 catttacaaa ctgaaatacg cgaaatgttc tcgtacgtaa tgttagagcc aactgaaagc
2341 tttgcttcgg tttacaacgc taactatacc ttcgtgaacg gaccattggc gcaacactac
2401 ggcattaacg gtgtaagcgg taatgaattc caaaaagtaa caactaccga tcgcggtggc
2461 attttggcga acggtgcgtt tatggcgcgc tggggtgaaa gtgtagagtc ttcaccaatt
2521 cgtcgttctg tgcgcgtacg ccgtcgtatg ctttgtcagg atcaaccaga tccccctggc
2581 aacgtaaaca tcggtcgtga aaacgctgca gacgaattcc acgaggcgtt ggcagatcct
2641 actacgacta accgtgagcg ttatgagctg ttaacatcag gtgaaacttg tgctacttgt
2701 caccaagagt ggattaaccc tctcggcttt ggtatggaag atttcactgc ggttggtacg
2761 cgtcgtgtga cggatctaaa tggcaatact attgatgcaa gcggtcaatt gtacgcgcca
2821 gaaaacctca acgataaaga cgtctttatt aactttaacg gtactcaagg tttaggtgcg
2881 ttactgacta ccttgccaag tgcgcagtct tgtttgccac aaaacttgtt ccgttattcc
2941 gtgggcgttg gtgtagaagg gttggatgat aatccagaag gcaacgagct agttcctgca
3001 gaacgggatg gctatgcctg tgaagttaaa aacctgacga gcactatgtt agagcaaagt
 
3061 ccgcgagcaa tgctggaagg catgggttcc atgcaagcgg tacgttaccg caaagcgtgg
3121 gcgcgttaa
LOCUS NZ_AABI02000007 2802 bp DNA linear BCT 23-SEP-2003
DEFINITION Microbulbifer degradans 2-40 Mdeg_8, whole genome shotgun sequence.
ACCESSION NZ_AABI02000007 REGION: 111940..114741
VERSION NZ_AABI02000007.1 GI:28878295
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2802)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027895. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson microbes@cuba.jgi-psf.org.
   COG assignment was automatically updated on Sep 23, 2003.
FEATURES Location/Qualifiers
   source 1..2802
      /organism="Microbulbifer degradans 2-40"
      /mol_type="genomic DNA"
      /strain="2-40"
      /db_xref="taxon:203122"
   gene 1..2802
      /gene="Mdeg1781"
   CDS 1..2802
      /gene="Mdeg1781"
      /codon_start=1
      /transl_table=11
      /product="COG4625: Uncharacterized protein with a
      C-terminal OMP (outer membrane protein) domain"
      /protein_id="ZP 00066396.1"
      /db_xref="GI:23027971"
      /db_xref="COG:COG4625" 
/translation="MYPLPFNSKIVISLGAMTLALATQQAQALSCTVSADSWNSGYTA
NVTVVNDSSYSINSWDVTLGFNQPPSVSAGWNANVSTVGTTVMASNVGYNGNLSPGQS
TSFGFQGAHNGNFELPSCAGGMTSSSSSSSSSSSSSSSSSSSSSSSSSSSSSSSSSS
SSSNSSSSSSSSSSSSTSSSSGSGGANTVTIELENLSGQNGFSPFSVQNDSSASGGQY
IVWPNNGDQLLSGASDGQSGTLAVSFELSQTANVSFDIRASLANGNDDSFYYKLDSGV
WSTQNNTSTSGFETLSPTTFNGVSAGVHTLYIQRREDGAKLDSLSLTASVGNIISSHA
NSSSSSSSSGSSSSSSSSSSSSGGNELVIAINAGGGATSLDGVNFVADVHSLGGSTGST
TDSIAGATSSTLYQTERYGSYSYAVPVTNATYSLKLHFAEIYHTEAGARSFNLAVEGQ
QEMASVDLYSLSGHDGAYTYEVNDFPVDDGEVTISLESLTDNGTLSGFELSSSDGGEY
VEPTPIPTPEPGEGGRYRVIHTTDMGADPDDEQSLVRQLVMANEYDLEGIITTTGCWK
KSTSNTAYVDRILNAYSQAYPNLSKHAEGFPTPAYLDSINVMGQRGYGMGDVGSGKDS
AGSNLIIAAVDKDDPRPVWATCWGGCNTIAQAVWKVQNTRSQAQLDAFISKLRVYDIL
GQDNAGTWLAKNFPNLIYIRARSVYSWQPSDSYLDNHIQSHGALGAVYPNRRYATEGD
TPAFLHMANPGLNDPSVVSMGGWGGRFPSKQAGVRGMSCMSGEDAVYDTYYMYTENGE
 
SIKRWSTAIHNDFQARMDWAIESNYSAANHHPVPVVNNDANEAVMYLNASAGSTVSLD
ASGSSDPDGDSLNYSWSHYGEADSYSGSVSISNSSSASANVQIPSNAGGKDIHILLTL
RDNGSPNLYAYRRVVINVQ"
   gene complement(330..656)
      /gene="Mdeg1782"
   CDS complement(330..656)
      /gene="Mdeg1782"
      /codon_start=1
      /transl_table=11
      /product="hypothetical protein"
      /protein_id="ZP 00066397.1"
      /db_xref="GI:23027972" 
/translation="MYWPPLALLSFCTLNGEKPFWPLKFSSSIVTVLAPPLPEEELVL
ELDELLLELEELEELEELEELEELEELEELEELEELEELEELEELEELEEDDEVIPPAQEG
NSKLPL"
ORIGIN 
1 atgtatccct tgccatttaa ttcaaagata gttatttcgc ttggtgcaat gacgctcgcg
61 ctggcgacgc agcaagctca ggcgcttagt tgtacggttt cggccgacag ctggaatagc
121 ggttatacgg ccaatgtaac agttgtcaac gatagtagtt acagtattaa tagttgggac
181 gttacgcttg gttttaatca gccgccaagc gtgagcgccg gttggaatgc gaatgtgtct
241 actgttggca ccacagtcat ggccagcaac gtgggttaca atggaaactt gtcgcccggg
301 cagtctacat cctttggttt tcagggggct cacaacggca attttgaatt gccttcttgc
361 gctggaggta tgacttcatc atcctcttct agttcttcta gttcttctag ttcttctagt
421 tcttctagtt cttctagttc ttctagttct tctagttctt ctagttcttc tagttcttct
481 agttcttcta attcttctag ctcgagcagc agctcgtcga gttctagtac cagctcttct
541 tccggcagcg ggggcgctaa tacagtgact attgagctag agaatttgag cggccagaat
601 ggcttttcac catttagcgt gcagaatgac agtagtgcca gtggcggcca gtacatcgtt
661 tggcccaata atggtgacca actgctaagc ggagcctccg atggccaatc tggtactttg
721 gcagtgtcgt ttgaattgtc gcaaacggca aatgtgtcct tcgatataag agcgagcttg
781 gcaaatggca atgatgattc gttttattac aaacttgact ctggtgtttg gagtacccag
841 aacaacactt ctaccagcgg ttttgaaact ctctcgccaa ctacctttaa cggagtgtct
901 gctggtgttc atactcttta tattcagcgc cgagaagatg gagctaagct cgatagtctg
961 agcctgactg cctctgtggg caatattatc agcagccatg ctaatagcag ttctagttct
1021 tcttccagcg gctcaagttc gtctagctct tcttccagtt caagtggtgg taatgagcta
1081 gtaattgcta taaacgctgg gggcggtgcc acatctctag atggtgtgaa ttttgtagct
1141 gatgttcatt cattgggcgg ctccaccggt tccaccaccg acagcatcgc aggcgctact
1201 agcagtactt tgtatcaaac agagcgctac ggcagctaca gctacgctgt tcccgtaact
1261 aatgccactt attcgttaaa gctgcatttt gctgagattt accatactga agcgggggcc
1321 cgctcgttta atcttgctgt ggaaggtcaa caggagatgg ctagtgtaga tctgtattca
1381 ctgtcagggc acgatggtgc ttatacctac gaggtgaatg atttcccggt ggatgatggc
1441 gaggttacga tttcgcttga atcacttacc gataacggca cgcttagtgg tttcgagctt
1501 tcctcctctg acggtggtga atatgtggaa ccaacaccta tacctacgcc agagccgggt
1561 gaaggcggtc gttaccgagt gattcacacc acggatatgg gggcagaccc agacgatgag
1621 cagtcgttgg ttcgccagtt ggtaatggcc aacgagtatg acctagaggg cattattacg
1681 acaacaggtt gttggaaaaa atccaccagt aacacggcct atgttgacag gatcctcaac
1741 gcctacagcc aggcatatcc caatttgagc aagcatgcgg aaggattccc aactccggcg
1801 tatctggatt ccattaatgt gatggggcag cgtggctacg gtatgggtga tgtcggctct
1861 ggcaaagact ctgccgggtc caacctgatt attgctgcag tggataaaga tgatccacgt
1921 cctgtatggg caacttgttg gggtggatgt aatactattg ctcaggcagt gtggaaagtg
1981 caaaacacgc gctcgcaagc gcaattggat gcttttatca gcaagttacg tgtatacgat
2041 attctcgggc aagacaatgc gggcacttgg ctggctaaaa actttccaaa ccttatctac
2101 attcgcgcgc gatctgttta cagctggcaa ccctctgata gttatctgga taatcacatt
2161 cagagtcacg gtgcactggg tgctgtatac ccaaatcgtc gatatgcaac agagggggat
2221 acaccagcat ttctgcatat ggctaatccg ggattgaacg acccatcagt ggtttccatg
2281 gggggctggg gtgggcgttt ccctagcaaa caagctggtg tacgtggtat gtcgtgcatg
2341 agtggtgaag atgccgtcta cgatacctac tacatgtata ctgagaatgg cgaatccatt
 
2401 aagcggtgga gcactgcaat tcacaacgac tttcaggcac gtatggattg ggccattgag
2461 agtaactatt ctgcagcgaa ccaccaccca gtacccgttg ttaataatga tgctaacgag
2521 gcagtaatgt atctcaatgc gtctgcgggt tcgacagttt cgctggatgc tagcggctcg
2581 agtgacccgg atggagatag ccttaattat tcgtggtctc actacggcga agcggattct
2641 tacagtggtt ccgtgagcat tagtaatagt agttcagcca gcgccaatgt tcagattccg
2701 tcgaatgctg gtgggaagga tatccacatt ttgctaaccc tgcgtgataa cggttcccct
2761 aacctctatg cctaccgccg cgtggttatt aacgtgcaat aa
LOCUS NZ_AABI02000004 1755 bp DNA linear BCT 23-SEP-2003
DEFINITION Microbulbifer degradans 2-40 Mdeg_4, whole genome shotgun sequence.
ACCESSION NZ AABI02000004 REGION: 257640..259394
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1755)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1755
      /organism="Microbulbifer degradans 2-40"
      /mol_type="genomic DNA"
      /strain="2-40"
      /db_xref="taxon:203122"
   gene 1..1755
      /gene="Mdeg1152"
   CDS 1..1755
      /gene="Mdeg1152"
      /codon_start=1
      /transl_table=11
      /product="COG0724: RNA-binding proteins (RRM domain)"
      /protein_id="ZP 00065777.1"
      /db_xref="GI:23027322"
      /db_xref="COG:COG0724" 
/translation="MRCVLVKNYQIIKLKNYSHHVCWRKFMSTQGKPCLKKLWPALAL
AAAVASPNAFSACEYVVSNQWDSGYSATIKIHNDTNSTINGWNVNWQYSGDNRVTNLW
NAAYVGSNPYSASNLSWNSTVGAGQTIEFGFQGAKNGGSAEVPVVTGDVCSGGGSSSG
SSSSSSSSSSTGSSTSSSSSSSSSSSTGGSTSSSSSSSSTGGNGGAQQCNWYGEIRPL
CNNQDSGWGWENQQSCIGRDTCSNQSGNGGIIGGSSSSSSSSTGSSSSSSSSSSSS
SSSSSTSSSSSSSSSTSSSSSSSSSSSSSSSTSSSSSSSSSSSSSSSSSTSSSSSSSS
SSSSSSSSSSSSSSSSSSSSSGGGVIFSADFESDSDSTQPAGWDNFIGWRQNGPNPNGST
FALVESGRAHSGNNAVHFSGGASPAMIARALPADLDTLYVRAWVYMTRQLGMNPGDNH
ETLIALRGSAGNANNEVRFGEIKGVIGTNEVPSDNIAPTMASWGGGPAYASDTWHCIE
VAFLSQPAYDTVNAWVNDALVHTIDEGSDWNNGALEADWLSNKYVELAFGWHSFSGND
VDVWMDDIVVSTTPIGCD"
   gene complement(378..572)
      /gene="Mdeg1153"
   CDS complement(378..572)
      /gene="Mdeg1153"
      /codon_start=1
      /transl_table=11
      /product="hypothetical protein"
      /protein_id="ZP 00065778.1"
      /db_xref="Gl:23027323" 
/translation="MEPPVELLEELLEELLVELPVELLDEELDDEPEELPPPLHTSPV
TTGTSALPPFLAPWKPNSIV"
ORIGIN 
1 gtgcggtgtg tattagtaaa aaattatcaa ataataaaat taaaaaatta tagccatcat
61 gtttgctgga gaaagtttat gtcaacccaa ggtaaacctt gtttaaaaaa attgtggccg
121 gcgttagcgc ttgctgctgc tgtagctagt ccgaacgcat tttctgcgtg tgagtatgtt
181 gtatccaatc aatgggattc agggtattcg gcgactatta aaattcataa cgatacaaat
241 tcaaccatta atggttggaa tgtgaattgg caatatagcg gcgataaccg cgtaaccaat
301 ttatggaatg cggcgtatgt aggtagtaac ccatactctg caagtaacct ttcgtggaat
361 agcaccgttg gtgccggtca aactatcgag tttggtttcc agggggccaa aaacggcggc
421 agtgccgaag tgcccgttgt tacgggtgat gtgtgtagtg gtggtggcag ttcttctggt
481 tcatcatcta gctcttcatc gagtagctct acaggtagtt ctacaagcag ttcttcaagt
541 agctcttcaa gtagttccac aggcggttcc acaagcagtt catcaagctc gtcttctacc
601 ggtggcaatg gtggtgcaca gcaatgcaat tggtacggtg aaattcgccc actgtgtaac
661 aatcaagata gcggctgggg ttgggaaaat cagcaaagct gtatcggtcg cgacacctgt
721 tccaaccaaa gtggcaatgg cggtataatt ggcggtagtt cttctagctc gtccagttct
781 tcaaccggtt catcgagcag ctcttcgagt tcttcgtcaa gctcatccag ctcttctagt
841 tcaacgtcaa gctcatccag ctcttctagt tcaacgtctt caagttcttc gtcgagttca
901 tcatctagct cttcaagttc tacaagtagc tcgtcgtctt cgtctagctc atcgagtagt
961 tcttcaagca gttcgacgtc tagcagtagt tcttcatctt cgagctctag cagttcatct
1021 agctcttcaa gcagttcttc tagtagctct tcttcaagtg gtggcggtgt tatttttagc
1081 gcagacttcg aaagcgatag cgatagtaca cagccagcag gttgggataa ttttattggc
1141 tggcgtcaaa acgggccaaa cccaaatggt tcgacatttg cgttggtgga atcaggtcgc
1201 gcacacagtg gtaacaacgc tgtgcacttc agtggtggcg caagcccagc tatgattgct
1261 cgcgctttgc ctgcagattt agatacttta tatgtgcgtg cttgggtgta tatgactcgc
1321 caattgggta tgaaccccgg tgataaccat gaaacgctca tcgccttgcg tggctctgct
1381 ggcaatgcaa acaacgaagt gcgttttggt gaaattaaag gggtaattgg tactaacgaa
1441 gtaccgagcg ataacattgc acctactatg gcaagctggg gtggtggccc tgcctatgca
1501 tcagatactt ggcattgtat cgaagtggca tttttatcgc agccagcgta cgacactgta
1561 aatgcatggg ttaacgacgc gcttgtacat actattgatg aaggctctga ctggaacaat
1621 ggtgccttag aggcggattg gttaagtaat aaatacgtag agctagcctt cggctggcac
1681 agctttagcg gcaacgatgt agatgtatgg atggatgata tcgttgtatc cacaacccca
1741 atcggttgcg actaa
LOCUS NZ_AABI02000004 2367 bp DNA linear BCT 23-SEP-2003
DEFINITION Microbulbifer degradans 2-40 Mdeg_4, whole genome shotgun sequence.
ACCESSION NZ AABI02000004 REGION: complement(138007..140373)
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2367)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2367
      /organism="Microbulbifer degradans 2-40"
      /mol_type="genomic DNA"
      /strain="2-40"
      /db_xref="taxon:203122"
   gene 1..2367
      /gene="Mdeg1069"
   CDS 1..2367
      /gene="Mdeg 1069"
      /codon_start=1
      /transl_table= 11
      /product="hypothetical protein"
      /protein_id="ZP 00065695.1"
      /db_xref="GI:23027240" 
/translation="MFTRITFMLINKARGRLAAAMLATAASAWGSTALAECSYQVTNN
WGSGFTAAIRITNTQTSSINDWQVSWTYENNTLVNAWNANVSGNYTATNMGWNGSLTS
GQSVEFGLQGTTTGGVVEIPLLTGNVCNSNTSSSSSSSTSSASSSSSSSGAAAVNLAG
IANASTSYVSAWETLAAVNDGNTPANSNDKSNGAYGNWNNPNSIQWVQYDWPQNYTLS
STQIYWFDDNGGVLVPDVAYIEYWSNGTWVKVGDVPRQENTFNTLNLNNIVTNRLRVS
ISNTLQSTGILEWRVEGTEPSGSSSSSSSSSSSSSSTSSSSGGPEQCDAYVWPEYEPNL
NYDFRQDYADINPEEFEVFLGCDPSQVAGVKTSGWYAFIWGHNRNPAITDEDIDRVLA
NLNEDMAYARGEMGWPPDKLPQEGYYSNVYLYGSGLCTDNAANTERGGWQSSIAGYPM
VLLSYYPVITPSERGGITHEAIHTIMASMGNKAAWFNEGGNTWLQMNMEASRVGDYGV
GFLDGAPFLAPHMPIENYSGWLQDGSFGGPNAEGVHRELNGQQIATWRDYLGGHQYNS
VFSHFLAQYVSSGANAWIWKNGPYNHILASLAAGLGDDQTRHLIMQYRARQAMVDFGP
WTNGFKQPINNNWQRTIGAEETAAGKWMEPEPHQLTFYAATSQEGNTLIPAQNTLPGW
SGANQIPLQVTGNKVRVDFEPFGNNMRLQLAYRAQDGSAVYSQPIESGEACLTLEKTP
KNGVVVAIVSNTDYTYAGDETRKQKYDYRVHIQEGVSGTASLYSKHYE"
ORIGIN 1 atgtttacga ggattacctt tatgttaatt aacaaagcga gaggtcgctt agctgcagca 
61 atgttagcca cagcagcctc agcttggggt agcacggctc tagccgagtg cagctatcaa
121 gtcacaaata attggggcag cggatttact gccgccattc gcatcactaa tacccaaacg
181 agtagtatta atgactggca agttagctgg acgtacgaaa ataatacact tgtaaatgct
241 tggaacgcga atgttagcgg caattacacc gctacaaaca tgggctggaa tggctcactt
301 acctcgggcc aatctgtcga gtttggcctg caaggcacca caactggcgg tgtggttgaa
361 atcccccttt taaccggtaa tgtgtgcaac tcaaatacaa gtagtagctc ttctagtagt
421 acaagttcag cctcaagttc aagttcgagt tccggcgctg ccgcagtaaa tctcgctggt
481 atagccaatg cgagcacttc ctatgtttct gcatgggaaa cattggcagc agtgaatgat
541 ggtaacaccc cggccaattc caacgacaag tccaacggag cttacggcaa ctggaacaat
601 ccaaattcga tacagtgggt acagtacgat tggccacaaa actatacgtt atcctcaacg
661 caaatatatt ggtttgatga taatggcggc gtactggtac ccgatgttgc ttacattgaa
721 tattggagca atggcacatg ggtaaaagta ggtgatgtac cgcggcaaga aaatacattt
781 aacacgctaa atttaaacaa tattgttaca aaccgcctac gtgtttctat aagcaacaca
841 ttgcaatcta caggcatact ggagtggcgc gtagagggaa cagagccaag cggcagctca
901 tctagcagca gctcaagctc atcatccagc tcaacgtcta gtagtagtgg cggaccagaa
961 cagtgtgatg cgtatgtgtg gcccgaatac gagccgaatt taaattacga ctttagacag
1021 gactacgcag atataaaccc cgaagaattt gaggtatttt taggttgcga cccaagccaa
1081 gttgcaggtg ttaaaacttc tggctggtac gcgtttattt gggggcataa tcgcaacccc
1141 gcaattaccg atgaagatat agatagagta ttggccaatt taaatgaaga tatggcatac
1201 gctcgcggcg aaatgggctg gccacccgat aagcttcctc aggaaggcta ctacagcaat
1261 gtgtatttgt acggctctgg tttatgtacc gataacgcag ccaataccga gcgaggtggc
1321 tggcaaagta gcattgccgg ctaccccatg gtactgcttt cgtattaccc agtaataaca
1381 cctagcgagc gcggtggtat aacccacgaa gctatccaca ctattatggc ttcgatggga
1441 aacaaagctg catggtttaa cgaaggcggc aacacttggc tacaaatgaa tatggaggca
1501 tctcgcgttg ggattatggg cgttggcttt ttagatggtg ccccctttt agcgccgcac
1561 atgccaatcg aaaattacag tggttggcta caagatggct ccttcggtgg gccgaatgct
1621 gaaggcgtac accgggaatt aaatggtcag caaatagcaa cttggcgaga ttacttaggc
1681 ggccatcaat acaactctgt gttttctcac tttttagcgc aatatgtttc tagcggtgca
1741 aatgcgtgga tatggaaaaa cgggccatat aaccatattc tcgcctcgct agcagcgggc
1801 ttgggcgacg accaaactcg ccacttaatt atgcaatacc gcgcacggca agcaatggtt
1861 gattttggcc cttggacgaa tggatttaaa caaccaatca ataacaattg gcaacgcacc
1921 attggtgcag aagaaaccgc tgctggcaaa tggatggagc ctgaacccca tcaattaacg
1981 ttttatgctg caacaagcca ggaaggtaat actttgatac cagcacaaaa tacattgccg
2041 ggctggtctg gtgccaatca aataccatta caagtaaccg gcaacaaagt gcgtgttgat
2101 tttgaaccat tcggcaacaa tatgcgcttg caattagcct atcgcgcaca agacggttca
2161 gcggtataca gccagcctat agaaagtggc gaagcgtgct taacactaga aaaaacaccc
2221 aaaaatggcg tagtggtggc aattgtatcc aacaccgatt acacctacgc aggggatgaa
2281 actcgcaaac aaaaatacga ttacagagtg catattcaag aaggcgtaag cggtacggca
2341 tcactttata gcaagcacta tgaatag
LOCUS NZ_AABI02000006 2364 bp DNA linear BCT 23-SEP-2003
DEFINITION Microbulbifer degradans 2-40 Mdeg_6, whole genome shotgun sequence.
ACCESSION NZ AABI02000006 REGION: 67047..69410
VERSION NZ_AABI02000006.1 GI:28878296
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2364)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027577. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2364
      /organism="Microbulbifer degradans 2-40"
      /mol_type="genomic DNA"
      /strain="2-40"
      /db_xref="taxon:203122"
   gene 1..2364
      /gene="Mdeg1448"
   CDS 1..2364
      /gene="Mdeg1448"
      /codon_start=1
      ftrans_table=11
      /product="COG4880: Secreted protein containing C-terminal
      beta-propeller domain distantly related to WD-40 repeats"
      /protein_id="ZP 00066068.1"
      /db_xref="GI:23027630"
      /db_xref="COG:COG4880" 
/translation="MLRKLNFILAPLGMVLGVNTYADVSCAVSGSVWNNGYVANVAVT
NAGESLQEGWSVALLFDNTPTINNSWSAELAVDGNVLTASNVAWNANLAAGQSAHFGF
VGSYSGEFELPECFVGALDDDTALEDYLKQGVHNHLNLRIANSASGSSSSSSSSGASS
SSSSSSASSASSTSSGSPVAEPDTQPDDITNTQEDGVDEADIIESDGNHFFVVRSSLY
FTGTSSSTSGSSSSSSASSSGSGGYGDYTQGVLIEAYSKDSAAGTTQHVGQVTLPYEEN
YLHVSGAYFRKTPQGNRLAIVSNTRESQPYYWSYGYGYYPYYQISNKVAISVANIDTP
ELMDEAERITFDGDLVSSRRIGSKLYIASRYSPNLNRLGFDFSSSASNEENAQRLDEA
PLADLLPHVYDENGVGTPLVTAADCTVPEWPESVDVYAGSLLVITQIDLDNNLAISSR
CLPASATEIYSSADAIYAFSNAFYAGVKVHKFTLKNDAQESEISYKGSVKLPGFIACS
NQSYCFGEQDGALRVLYRVSNYSDATPYRIAVIKQSPSSAEGLDIIATLPNAERPASI
GKPGEIVYAMRSFGDHAYVVTFDRIDPLYAIDFSNPADPYIAGELEVTGVSDYLHPVG
ENLLLGVGRDAIFDEARNLTWFQGVKVELFDVSDPTNLRSLGSEVIGKRESSTTLSFD
ARGIAFSHDENGIRFAIPVKMHDKPIGPANTDALNQYYSWTHTGLYVYQIETQPNTDA
SLSRLGILKTDIGEASVRYDRGVIDGDAVYYLHNYHMFSSLINYLAQ"
ORIGIN 
1 atgttacgga aattaaattt tattttagcg ccgcttggta tggtgttagg cgttaacacc
61 tatgcggatg tgtcgtgcgc tgtttctggc agcgtgtgga acaatggcta tgtagctaat
121 gttgcagtta ctaacgcggg tgaatctctg caagagggtt ggagtgttgc tttgttgttc
181 gataacactc caactataaa caatagttgg agtgcggagc ttgctgtaga cggtaatgtg
241 ctaaccgcga gtaatgtggc ttggaacgct aatttggcag cggggcaatc tgctcatttt
301 ggttttgttg gctcatattc aggtgagttt gaattgcctg aatgtttcgt tggcgcacta
361 gatgatgaca ctgcactcga agattattta aaacagggcg tgcataatca tttaaattta
421 cgcattgcaa attcggcgtc tggttcatct agctcttcga gctcttccgg tgcgtccagt
481 tcttccagtt catccagtgc ttctagcgca tcgagtacat cttctggttc gcccgtggcc
541 gagcccgata ctcagcctga tgatattacc aatacccaag aagatggcgt ggatgaggcg
601 gatataattg aatccgacgg taaccacttc tttgtggtgc gttcttcttt atattttacc
661 ggcactagct catcaacctc tggctctagt tctagttcgg cttcatctag cggttcaggt
721 gggtatggcg attacacaca gggagtgttg attgaagcct acagtaaaga ttcagcagcc
781 ggaacaactc aacatgtggg gcaggtgacg ttgccctatg aagaaaatta tcttcacgtt
841 tctggtgctt actttcgcaa aacaccgcaa ggcaatagat tagctattgt aagtaacacc
901 agagaatcac agccttatta ttggagctat gggtatggtt attaccccta ttatcaaatt
961 tctaataagg tagctatttc tgttgctaat attgataccc ctgaattaat ggacgaggca
1021 gagcgaatta cttttgatgg tgatttagta tcgagcagac gtattggtag caaattatat
1081 attgccagcc gttatagccc gaacctaaat cgtttagggt ttgatttttc tagcagcgcg
1141 agcaatgagg aaaatgcaca gcgtttagat gaagcgcccc ttgcggattt attgccacat
1201 gtttacgacg aaaatggcgt aggtacgcct ttggttactg ctgctgactg caccgtaccg
1261 gagtggccag aaagcgtgga tgtatatgcc gggtcactgt tggttattac tcaaattgac
1321 ttggataata acttagcaat tagctcgcgt tgtttacctg cttcggcaac agaaatatac
1381 agttctgcgg atgcaattta tgctttctcc aatgcatttt atgctggggt aaaagtgcat
1441 aaattcacat taaaaaatga tgcccaagaa agtgaaatta gctataaggg tagtgttaag
1501 ttgccgggtt ttatagcttg cagtaatcaa tcttattgct ttggcgagca agacggggca
1561 ttgcgcgttt tgtatagagt gtccaattac agtgatgcta ctccctatcg tattgcggta
1621 attaagcaat cgccaagctc tgcagaaggt ttagacatta tagctacctt gccaaatgct
1681 gaacgcccag cctctattgg taagccgggc gagattgttt atgccatgcg tagttttggt
1741 gatcacgctt acgtcgttac tttcgataga attgacccgt tatatgctat tgatttctct
1801 aacccagcag acccttacat tgccggtgag cttgaggtta ctggtgtgtc tgattactta
1861 catccagtgg gggagaattt attgttgggc gtgggccgag atgcaatatt cgacgaagcg
1921 cgcaatttaa cttggtttca gggtgttaaa gtggagctgt ttgatgtgag tgaccctaca
1981 aacctgcgca gcttaggcag tgaagtaata ggtaagcgcg agtcgtcaac gacacttagt
2041 tttgatgccc gtggtattgc atttagccat gatgaaaacg gtattcgttt tgctattcct
2101 gtgaaaatgc atgataagcc aattggaccg gcaaataccg atgcgttaaa ccaatattat
2161 agttggaccc atacggggtt gtatgtttac caaatagaaa cgcagccaaa caccgatgct
2221 agtttgtctc gcttaggtat actaaaaact gatatcggcg aggccagtgt gcgctatgat
2281 cgtggcgtaa ttgatggtga tgctgtttac tatttgcata attaccacat gttttcgagc
2341 ttaatcaatt atttggcgca atag
LOCUS NZ_AABI02000040 2046 bp DNA linear BCT 23-SEP-2003
DEFINITION Microbulbifer degradans 2-40 Mdeg_40, whole genome shotgun sequence.
ACCESSION NZ AABI02000040 REGION: 9019..11064
VERSION NZ_AAB102000040.1 GI:28878262
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2046)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029901. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2046
      /organism="Microbulbifer degradans 2-40"
      /mol_type="genomic DNA"
      /strain="2-40"
      /db_xref="taxon:203122"
   gene 1..2046
      /gene="Mdeg3624"
   CDS 1..2046
      /gene="Mdeg3624"
      /codon_start=1
      /transl_table=11
      /product="hypothetical protein"
      /protein_id="ZP 00068207.1"
      /db_xref="GI:23029911" 
/translation="MRISTAITQFLIVIATLILAACSGSGGGANSGGSNPGASSSSSS
SSSSSNSSSSSTSSSSSSGGSSGAAVSLPSTIAAMDFVAAYDTDNANSGDCGNGPVDM
QTSTDTQGAACTVGWTKAGEWLAYDVSVAVTQKMDIVFRVATNQNSRALKVQLNNKTL
GVLNVSGTAFDEWQTVSLKDIEIPAGTHQLKLVVWMTGAINVNTLSFTAHSAYGQTTNL
WGSNGAEHDPSGVLSDWSYAGYHWGEEEPPVKSPTINVVTDHGAIADDDSDDSAAFIA
ALTAANNGDVVYVPEGRFILTQVLSIPNGVVLQGAGSELTTLYIPTNLNEATGIDPSF
TGGFIEMKGSSSDGSKLSTITAAAARGSNQITVESVAGLTVGDWVQIQQTDVNGNFLL
EALYAGYTAGLSDYSNLVNDAELEFFSRITNISGNTLTLERPLPITVSPNYMAELHSV
SVAYGEVGFEGMTLEFPETTYPGHFNELGYNGIDMRAQHSWVRDVVVKNIDYGINLRG
SHFVSILDFTVINTNDRSGHHGISVGSSTDCLIRGFDIQAELVHDLTVEWYAYGNVFT
QGRGKNITLDHHRAAAYANLFTQIDLGEATRAWKTGGRSDRGYKTAVYSTFWNMTAEQ
AIDWPANDFGPRMVFMGLTMDGSHSSVLDWVVEDISADDIYPPNLWLSQREKRLGRQ"
   gene complement(24..224)
      /gene="Mdeg3625"
   CDS complement(24..224)
      /gene="Mdeg3625"
      /codon_start=1
      /transl_table=11
      /product="hypothetical protein"
      /protein_id="ZP 00068208.1"
      /db_xref="GI:23029912" 
/translation="MLGSDTAAPDEPPLEDDELVLLELLLDEEDEELELELAPGLEPP
EFAPPPEPLQAARISVAITIRN"
ORIGIN 
1 atgcgtattt ctactgctat cactcaattc ctaattgtaa ttgctacact tattctagct
61 gcctgcagtg gctcgggcgg aggggcgaat tctggtggct ctaatcctgg tgctagttct
121 agctccagtt cttcgtcttc ttcatctaat agcagttcga gtagcactag ttcgtcgtcc
181 tctagtggtg gttcatccgg tgccgcggta tcgctcccaa gcactattgc cgcaatggat
241 tttgtcgcag cttacgatac agacaacgca aattcaggtg attgtggtaa tggccctgtc
301 gatatgcaaa cgtcaaccga tacgcaaggt gcggcctgca cggtaggttg gacaaaagct
361 ggcgagtggt tggcttatga tgtgagtgtt gctgttaccc aaaaaatgga tattgtgttt
421 cgtgttgcta ctaaccaaaa ctcacgtgcc ttaaaagtac agctaaataa caaaacattg
481 ggtgtgttaa atgtttctgg tacagcgttt gatgaatggc aaactgtatc gctaaaagac
541 atagaaattc ctgcaggcac gcatcaatta aaactcgtgt ggatgaccgg ggcaataaac
601 gtcaataccc ttagttttac cgcgcacagc gcctacggcc aaactaccaa tttgtggggc
661 agtaacggtg ccgaacacga cccgagcggc gtgttatctg attggtctta tgctggttat
721 cactggggtg aagaagagcc gccggtaaaa tcgccaacca ttaatgtggt taccgatcac
781 ggtgcgatag ccgatgacga tagcgacgat agcgctgcgt ttattgctgc attaaccgca
841 gcaaataatg gtgatgtagt ttacgtaccc gaggggcgtt ttattcttac gcaggtgcta
901 agcataccga atggtgttgt gttgcagggc gcaggcagcg aattaactac tttatatatt
961 cccaccaact taaatgaagc aacgggtata gatccatctt ttactggtgg gtttattgaa
1021 atgaaaggtt cgtcgagtga cggcagtaaa ctttctacta ttactgcagc ggcggcacga
1081 ggcagtaatc aaataacagt agaaagtgtt gcaggtttaa ccgtaggcga ttgggtgcaa
1141 atacaacaaa ccgatgtgaa cggtaacttt ttattagagg ccttgtatgc agggtacacc
1201 gcaggcttaa gtgattacag taatttggtt aacgatgcag agttagagtt tttcagccgc
1261 ataacaaata ttagtggcaa cacacttacc ctagagcgac cgctacccat taccgtaagc
1321 cctaactata tggcggaatt acacagcgta tcggttgcct acggcgaggt gggtttcgaa
1381 ggaatgactt tagagtttcc agaaacaacc tacccaggtc acttcaatga gttagggtac
1441 aacggcatag atatgcgcgc tcagcactct tgggtgcgag atgttgtagt aaaaaatata
1501 gattatggta ttaatttaag aggctcgcac tttgtaagca ttttagattt tactgtaatt
1561 aacacaaatg atcgcagtgg ccaccacggg ataagtgtgg ggtcgtccac cgattgttta
1621 attcgcggtt tcgatattca agccgaacta gtccacgatt taactgttga atggtatgcg
1681 tatggcaatg tatttacgca agggcgaggc aaaaatatta ccctcgatca tcaccgcgca
1741 gcagcctacg ccaacttatt tacccaaatt gatttaggtg aagcaacacg cgcatggaaa
1801 acaggtggcc gaagcgatcg cggatacaaa accgcggtat acagtacgtt ttggaacatg
1861 accgccgaac aggcgatcga ttggcccgcc aatgatttcg gcccacgtat ggtgtttatg
1921 gggttaacca tggacggcag tcacagctct gttttagatt gggtggtaga agatatttct
1981 gcagatgata tatatccacc aaacctttgg ttgtcgcaaa gagaaaaaag attggggcgg
2041 cagtag
LOCUS NZ_AABI02000007 1965 bp DNA linear BCT 23-SEP-2003
DEFINITION Microbulbifer degradans 2-40 Mdeg_8, whole genome shotgun sequence.
ACCESSION NZ AABI02000007 REGION: 160746..162710
VERSION NZ_AABI02000007.1 GI:28878295
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1965)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027895. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1965
      /organism="Microbulbifer degradans 2-40"
      /mol_type="genomic DNA"
      /strain="2-40"
      /db_xref="taxon:203122"
   gene 1..1965
      /gene="Mdeg1805"
   CDS 1..1965
      /gene="Mdeg1805"
      /codon_start=1
      /transl_table=11
      /product="hypothetical protein"
      /protein_id="ZP 00066418.1"
      /db_xref="GI:23027993" 
/translation="MRFQYALCTDSGGTIKNNQSWVSIMLRIHLAMPNMAFSLLQHYL
LNTFSGVSIWVLCGLLMGAYVNAATQEINGNQLQVTCQSETFCDIHYRLNNGRELNIA
MASLGNGEYAHTISNLISGDVIVYYLTYQQNGLAYDSERLTHIYGGASSSAGADPYLG
FRAPIPGTILAVNYDTGGEGIAFHDATIGNSGGQYRDESVDIESASIGGFNVGWVASG
EWLQYSVDVARAGSFDIVAQIASPNSGGSLHYEITGATNVQSDAVQFANTGGWQVWAH
TAAAKVQLNAGAHTIRLVFESAGFNIHSLLVSEASSSFGKVEAESFDSMSGVVSEATS
VGYFDRGDWMKYSAVSFGNLAKSITLSVAGEYNNGIAELRLDSVSGPVIGTYSMESTG
GWASFKPKSFNIVHTLGVHDLYIVGKNGSGIFNLDYFQLSADVVEETNPATAVKAMSL
NIYGWATMPQNADKYAALIRSRGVDVVGIQEGVEDWLIGPGFPTNYSKADALGAALGA
CWQQRYQIFINICEGNSFVSNRRFDMTDGPNATRTGESARINKNGFEYAVLTVHWDHQ
SGAAKVANAHETAAEVNYYGALPTVVVGDFNTGCTSHEVNTLMHEAGMVLIGNAGIDC
ILAKRFNGTAQTFDAAPSDHPGLDASLSTN"
ORIGIN 
1 gtgcgttttc agtatgcgtt gtgcacagac agtggcggta caattaaaaa taatcaaagt
61 tgggtatcta ttatgttgcg aattcatcta gccatgccaa atatggcctt ttcactatta
121 cagcactatc ttttaaacac attttctggg gtatccatat gggtactttg tggtttattg
 
181 atgggggcat acgttaatgc cgccacacaa gagatcaacg gcaaccagtt acaagtgaca
241 tgccaaagcg aaaccttttg cgatattcat tatcgtttga ataatggcag agaattaaat
301 atagcgatgg cgtcgttagg taatggtgag tacgcacata cgatttctaa tcttatttct
361 ggcgatgtga ttgtttacta ccttacgtac caacaaaacg gtttggccta tgactctgag
421 cgcctgacgc acatatatgg gggggctagc tccagcgctg gcgcagatcc ctatttgggg
481 tttagagcac ctattccagg gactattctc gctgtgaact acgatactgg aggcgaaggc
541 atcgcttttc atgatgccac aataggaaac tctggtggtc aataccgcga cgaaagtgtg
601 gatatagaga gcgcttcgat tggtggcttt aatgtggggt gggttgcttc cggcgagtgg
661 ctgcaatact ccgtggatgt ggcgagagct ggcagtttcg atattgttgc gcaaatcgcc
721 tctcctaatt ctgggggcag ccttcattat gaaattacag gggcaaccaa tgttcaatct
781 gacgcggtgc aatttgcgaa tacaggtggt tggcaggttt gggcacacac agcggctgcg
841 aaggtacagc tgaatgcagg tgctcatacc attcggttag tgtttgaatc agcagggttt
901 aatattcatt ctctgctggt aagcgaagcg agctctagtt tcgggaaagt tgaggcagaa
961 agttttgatt ccatgagtgg cgtagtgtcg gaggcgacaa gcgtaggcta ttttgatcgc
1021 ggtgattgga tgaaatacag cgcggtcagc tttggtaatc ttgccaagag cataacgtta
1081 tccgtcgctg gtgaatacaa taatggtata gcagaactta gattagacag tgtcagtggg
1141 ccggtgattg gcacctactc aatggaatcg acaggtggtt gggctagttt taagccaaag
1201 agttttaata tcgttcatac cttgggagtg catgatcttt atattgttgg taaaaatggc
1261 agtggcattt ttaatttaga ctattttcaa ttatctgcag atgtcgtaga agagaccaac
1321 cctgctacag cggtaaaagc gatgtcgctg aatatatatg ggtgggcaac aatgcctcaa
1381 aatgctgata agtatgcagc gcttattcgt tctcggggtg ttgatgttgt gggtattcaa
1441 gagggggtag aagattggct tattgggccg ggttttccca caaattactc caaagcagat
1501 gctcttggtg ctgccttagg agcttgttgg cagcagcgct atcaaatttt tattaatatt
1561 tgtgaaggaa atagcttcgt gtcgaatcgt cgattcgaca tgaccgatgg gcctaatgca
1621 acgcgcacag gcgagtctgc acgaataaac aaaaatgggt tcgaatacgc agtcttaacg
1681 gtgcactggg atcaccaaag tggcgcagct aaagttgcta atgcacatga aacagccgcc
1741 gaggttaatt actacggtgc attgcctacc gttgttgtgg gcgatttcaa cactggctgt
1801 acaagccatg aagtaaatac acttatgcat gaagctggaa tggttttgat tggtaacgcc
1861 ggcattgatt gcattttagc taagcgattc aatggtacgg cacaaacatt tgatgctgca
1921 ccatctgatc accctggctt ggatgcgtca ctcagtacca actag
LOCUS NZ_AABI02000003 1653 bp DNA linear BCT 23-SEP-2003
DEFINITION Microbulbifer degradans 2-40 Mdeg_3, whole genome shotgun sequence.
ACCESSION NZ AABI02000003 REGION: 51474..53126
VERSION NZ_AABI02000003.1 GI:28878299
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1653)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026886. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1653
      /organism="Microbulbifer degradans 2-40"
      /mol_type="genomic DNA"
      /strain="2-40"
      /db_xref="taxon:203122"
   gene 1..1653
      /gene="Mdeg0757"
   CDS 1..1653
      /gene="Mdeg0757"
      /codon_start=1
      /transl_table=11
      /product="hypothetical protein"
      /protein_id="ZP 00065386.1"
      /db_xref="GI:23026923" 
/translation="MKSCCIKFFTTLCTAVYVLGCGALAHAQTGPAGYNYAAAENETV
YLNGTTNVAYGANGSFYYAYNQTGSVNCSNQTFGDPIFGVRKACYTQQVASNNPPSVS
FASPTGNLTVDEGYALSVTVNASDSDGSIASVELFINNQLVRQELYAPYEWGAAAEPD
ELNGLPVGTHTIKAVATDNDGDTKQASFKLTVRGAAVDVPGLVQAEDYTGFYDTTNGN
TGGAYRNDNVDVETTSDSNGGYDVGWFAANEWLEYPINVTEAGNYVLEARVASAVGGG
MFTAEINGNNSSTFSIGNTGGWQNWQTLNNNIGNLSTGKKTLRIQAQSGNFNLNWLRL
KRATTSVCTLNTPAENIPTPFNLFTVIDTDLNRYEFCKASKWFEESNGKQVFKLFTGD
NLADNVPGARVHARTEAGQGLKFKAGSTWHTFEARMKPSKKLDYTYTIAQLFAGCCGP
QLRIEVKSNGRIHMGSRGNGNIRISDDQDYANGSRSFKIKIRTNGDQFEVYFNSSKKF
SGRTDEAKNGNTSALYHFRWGVYSNEVMSEDLSNTVTEIIRN"
ORIGIN 
1 atgaaatcat gttgtatcaa gttttttacc acactctgta ctgctgttta tgtactgggt
61 tgtggcgcgt tagcgcatgc gcaaactggc ccggcgggtt ataactacgc tgctgccgaa
121 aacgaaacgg tgtatttaaa cggaaccacc aacgttgcct acggcgcgaa tggctcgttt
181 tattacgctt acaatcaaac cggttcggtt aattgctcta atcaaacctt tggcgaccct
241 atttttgggg tgcgcaaagc gtgttacacc cagcaagtgg cgagcaataa cccgccgtcg
 
301 gtttcgtttg ctagcccaac gggcaaccta acagtcgacg aaggttatgc tctttctgta
361 accgtgaacg ccagcgatag cgacggcagc attgccagtg tagagctgtt tattaacaac
421 caattagtac gacaagagct ttacgcacct tacgagtggg gcgcagctgc tgagccagac
481 gaattaaatg gcctacccgt tggtacacac acaataaaag cggtagccac cgataacgac
541 ggcgacacca aacaagctag ctttaagcta acggtacgcg gtgcggcggt agatgttcct
601 ggcttggtac aagcggagga ttacacaggc ttttacgaca caaccaatgg caacactggt
661 ggtgcgtatc gcaacgacaa tgtagacgta gaaaccacaa gcgatagtaa cggtggttac
721 gacgtaggtt ggtttgcggc aaacgaatgg ctggaatacc ccattaacgt taccgaagcg
781 ggcaattatg tattagaagc acgcgtcgca tctgctgtag gcggtggtat gtttaccgca
841 gaaataaatg gcaacaacag cagtacattt agcataggca ataccggcgg ctggcaaaat
901 tggcaaaccc tgaataacaa tattggcaat ttaagcaccg gtaaaaaaac gttacgcatt
961 caagcgcaaa gcggaaactt taatttaaac tggctgcgcc tcaagcgtgc cacaacctct
1021 gtgtgcacac ttaatactcc tgccgaaaac attccaacgc catttaattt atttaccgtt
1081 atcgatacag atttaaaccg ctacgaattc tgtaaagcgt ctaaatggtt tgaggaatct
1141 aacggtaaac aagtgtttaa attatttacc ggcgacaacc tagcagataa cgtacctggc
1201 gcccgcgtac atgctcgcac agaagctggc caagggctta agtttaaagc aggctccaca
1261 tggcacacct ttgaagccag aatgaaaccc agcaaaaagt tagactacac ttacaccatt
1321 gctcaattgt ttgccggctg ttgcgggccg cagttgcgca ttgaagtaaa atctaacgga
1381 cgcatccaca tggggtcgcg cggtaacggc aatattcgta ttagtgacga ccaagattac
1441 gccaacggct ctagatcgtt caaaattaaa attcgcacca atggcgatca gttcgaagtg
1501 tattttaaca gcagcaaaaa gttcagcggc cgcacagacg aagccaagaa cggcaatacc
1561 agtgcgcttt accacttccg ctggggtgta tattccaacg aagtaatgag cgaagattta
1621 tctaacactg ttacagaaat tattcgaaat taa
LOCUS NZ_AABI02000037 1503 bp DNA linear BCT 23-SEP-2003
DEFINITION Microbulbifer degradans 2-40 Mdeg_37, whole genome shotgun sequence.
ACCESSION NZ AABI02000037 REGION: complement(7812..9314)
VERSION NZ_AABI02000037.1 GI:28878265
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1503)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029842. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1503
      /organism="Microbulbifer degradans 2-40"
      /mol_type="genomic DNA"
      /stain="2-40"
      /db_xref="taxon:203122"
   gene 1..1503
      /gene="Mdeg3569"
   CDS 1..1503
      /gene="Mdeg3569"
      /codon_start=1
      /transl_table=11
      /product="hypothetical protein"
      /protein_id="ZP 00068152.1"
      /db_xref="GI:23029850" 
/translation="MFASNLEDPTYYIKYKGVYMNTNRKQVNQNLIALLGLMLLMLFT
PHGYAQDQCNTTLECKILHGDTATDCKNSRSDNSICMCGSTECAVDNPQPEPSNSAAV
PGLIQAEDFTDYYDVTAANHGGAYRSTGVDIQVTTDTNGGYNVGWIAANEWLEYNINV
LQAGNYTANIRVASNNGVGMYSLAVDGVTVSGTNTVNGTGGWQVWITQTANLGYLTQG
EHTLRIAVQAGNFNINWLELLLAGTQQPDMLGVFNKSRDLLLANFDSRPDPDDIHSVA
ALATMLKDSRFSNVQYHAVSGAYGIQGGDYIEATHLFNLAFGAGNWSNAHTNRDVALT
TVYNKVAATLTNGGDIWVQEAGQSDFSADLVRRIKQQLPAINTQTRIHIVQHSNWNQD
KTTPADLTYVKNQTDYKKIADGNSTGNGTPGFNSSSSANWNRALNHAQVGAIWQEAKR
IADNAIANHQGWQNPNIRDGGMDFSDAVEDCWIFGFNSLTNINSFFDEFL"
ORIGIN 
1 atgtttgcta gcaacctcga agaccctact tactatataa aatataaagg tgtttacatg
61 aatactaata ggaaacaagt caaccaaaac ctcatcgcgc tattgggctt aatgttgcta
121 atgctattta cacctcatgg ctatgcgcaa gaccaatgca acaccacgct tgagtgcaaa
181 atactccacg gcgatacagc cacggattgt aaaaatagtc gctcagataa cagtatttgc
241 atgtgtggaa gtactgaatg tgcagtagat aatccacaac ccgaacctag caattccgcc
 
301 gcagtaccgg gcctaataca agcagaagac tttaccgatt actacgatgt aacggcggct
361 aatcacggcg gcgcttaccg ctccacaggc gtagacattc aagttaccac agacaccaat
421 ggcgggtaca acgtaggctg gatagccgct aatgaatggc tagagtacaa cataaacgta
481 ctgcaagctg gtaactacac cgcgaatatt cgtgttgcat ctaataatgg tgttggcatg
541 tatagcttgg cggtagatgg tgtaacggta agtggcacca acaccgttaa tggcacgggt
601 ggctggcaag tttggattac ccaaaccgca aaccttggct acttaaccca aggcgagcat
661 acgttgcgta tagcagtaca ggctggcaat tttaatatta actggttaga gctgctgcta
721 gcgggtactc agcaaccaga tatgcttggt gtatttaata aaagccgcga cctgttgtta
781 gcaaatttcg atagccgccc agacccagac gatattcatt cagtagcggc cctggcaaca
841 atgctaaaag attcccgttt tagcaatgtg caataccacg cagtatcggg tgcttacggt
901 atacaaggtg gtgattacat tgaagctaca catttgttta acttagcctt tggtgccggt
961 aattggtcca acgcgcacac caatagagat gtggcgctta caactgttta caacaaagtt
1021 gccgcaacgt taaccaatgg tggtgatatt tgggttcaag aggccggtca atccgacttt
1081 agcgccgatt tggtacgcag aatcaaacaa cagttacccg ctattaatac gcaaacgcga
1141 attcatatag tgcagcatag taactggaac caagacaaaa ccactccagc cgatttaacc
1201 tatgtaaaaa accaaaccga ctacaaaaaa attgctgatg gcaattccac aggcaacggc
1261 acgcctgggt ttaactcatc cagcagcgcc aactggaacc gcgcactaaa ccacgcgcaa
1321 gtaggcgcaa tttggcaaga agcaaaacgc atagccgaca atgccatcgc caaccaccaa
1381 ggctggcaaa accccaatat tcgagacggc ggtatggact tctcggatgc agtcgaagac
1441 tgctggatat tcggcttcaa ctcactcaca aatataaaca gcttttttga tgagttttta
1501 tag
LOCUS NZ_AABI02000013 1422 bp DNA linear BCT 23-SEP-2003
DEFINITION Microbulbifer degradans 2-40 Mdeg_12, whole genome shotgun sequence.
ACCESSION NZ AABI02000013 REGION: complement(886..2307)
VERSION NZ_AABI02000013.1 GI:28878289
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1422)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028387. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1422
      /organism="Microbulbifer degradans 2-40"
      /mol_type="genomic DNA"
      /strain="2-40"
      /db_xref="taxon:203122"
   gene 1..1422
      /gene="Mdeg2182"
   CDS 1..1422
      /gene="Mdeg2182"
      /codon_start=1
      /transl_table=11
      /product="hypothetical protein"
      /protein_id="ZP 00066788.1"
      /db_xref="GI:23028389" 
/translation="MQLIQLLKTMGISRLCIFLFGAVLASTMLAGCGGSSSSEKSSGQ
VVTEPESEPESEPESEPESEPESEPESEPESEPESEPDPAPDTAQDMRSEKRGLAYGYHSEN
DLKAMQGKVKWWYNWDTQADANVKENYASYGYDFVPMAWDENFNEEALRSFLDNHPDV
KYLLGWNEPNFMEQANLTPAEAAAHWPVLEAIAQDYNLKLVAPAVNYSPGNVDIPGTD
DDYDPWLYLDAFFEACEGCQVDYIAVHCYMKYESAFSWYVGEFERYNKPIWVTEWAGW
DDGGPANMGEQMNFLSDTVRWMESNDNIYRYSWFLGRSSEGYDQFPYLDVLLADGELT
PLGSVYTSIPSNDFRYKIPARIEAEGAHSLTGFKHLATTDTTGLAKLIAASNEVAEYK
LNVEEGGDYTLALRLASSANSDIAIRVDGLLVYTFEDINTGGVEAWMTFSSTPISLTA
GDHILRVESKSSRFGFNWLELTN"
ORIGIN

1 atgcagttaa ttcagctact aaagacgatg ggtattagtc ggctatgtat tttttattt
61 ggtgccgtac tcgctagcac aatgctagct ggttgcggtg gttcttcaag ttcagaaaag
121 tcctcaggcc aagtggtgac tgagcccgaa tctgaaccag agtcagaacc agagtcagaa
181 ccagagtcgg agccagaatc tgaaccagag tcggagccag aatccgagcc ggacccagcg
241 cccgacacag cgcaagatat gcgcagcgaa aaacgcggct tagcctatgg ctaccacagt

301 gaaaacgacc tcaaagctat gcagggtaaa gtgaagtggt ggtacaactg ggatacccaa
361 gcggacgcga acgtaaaaga gaattacgcc agttatggct acgattttgt tcctatggcg
421 tgggacgaaa actttaacga agaagcgctg cgcagctttt tagataatca ccccgatgtg
481 aaatacctgc tgggctggaa tgaaccaaac ttcatggaac aggccaacct cacaccagcg
541 gaagccgcag cccattggcc cgtgctagag gccatcgcgc aggattacaa cctaaaatta
601 gtcgcccctg cggtgaacta cagccccggt aatgttgata ttccaggtac cgatgatgat
661 tacgaccctt ggctatacct cgatgccttc tttgaagcgt gtgaaggttg ccaagtagat
721 tacattgccg tgcattgcta tatgaaatac gaaagcgctt tcagttggta tgtgggtgaa
781 tttgagcgct acaacaaacc tatttgggta accgagtggg cagggtggga cgatggcggc
841 ccagcgaata tgggtgagca aatgaacttc ttgtccgata ctgtgcgctg gatggagagc
901 aacgataata tatatcgcta ttcttggttt ttggggcgca gtagtgaagg ctacgatcag
961 ttcccatacc tagatgtttt actggccgat ggcgaactaa caccgttggg tagtgtgtat
1021 acctctattc cgtccaacga ttttcgctac aaaatacccg cgcgtattga ggccgaaggc
1081 gcccatagct taacgggctt caaacactta gccacaaccg atactacagg tttagctaag
1141 ttaatagccg cgtctaacga agtagcagag tacaaattaa acgtggaaga gggggcgat
1201 tacaccttag ctttacgttt ggcttcatct gcaaacagtg atattgccat ccgtgtggat
1261 ggcttattgg tgtacacctt cgaagatatt aataccggcg gtgttgaggc gtggatgacc
1321 tttagctcaa cccctattag tttaaccgcg ggtgatcaca tattacgtgt agagtctaaa
1381 tcgtcgcgtt ttggctttaa ttggttagag cttactaatt ag

LOCUS NZ_AAB102000003 1398 bp DNA linear BCT 23-SEP-2003
DEFINITION Microbulbifer degradans 2-40 Mdeg_3, whole genome shotgun sequence.
ACCESSION NZ AABI02000003 REGION: 85570..86967
VERSION NZ_AABI02000003.1 GI:28878299
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1398)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23026886. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..1398
      /organism="Microbulbifer degradans 2-40"
      /mol_type="genomic DNA"
      /strain="2-40"
      /db_xref="taxon:203122"
   gene 1..1398
      /gene="Mdeg0792"
   CDS 1..1398
      /gene="Mdeg0792"
      /codon_start=1
      /transl_table=11
      /product="hypothetical protein"
      /protein_id="ZP 00065421.1"
      /db_xref="GI:23026958" 
/translation="MKLLSITHTLKRAIASAVFVASAATASIANAVTVDILVLYDNYS
ANYFGGDPQTAMNGWANDMNSALKASQIDMKFRIVGVRHHEEDGAGMGDVLGNLRVDG
GAIALRDQLGADMVSQLHEKGACGVGYVAVDKNYTWNVTHPGCGPMVMLHEFGHNMGV
THSRKQGDQGGTRYRYGVGYGVQDVFVDIMAYEGVFNTSRVNVFSNPNLNCRGLPCGK
PVGDSEEAHASLAIHNVRNELANFRNTVNSGGPVRLFEHCYYTGYTVGLGEGSYRLAD
LMNRGLVNDDLSSLQVDAGYRVEMFQHDNFTGNVVTRTGSDDCLVDEGMNDDISSLRI
TRVSGGFSQTIQAENFFANNGVQLENTTDSGGGQNVGWIDANDWMAFSNITIPTTGNY
RIEYRVAGFGGTLSLDLNGGAIVLGQINLPNTNGWQNWQTASHTVHINAGTYNFGIFA
NAPGWNINWFRIVQL"
ORIGIN 
1 atgaaattac tctcaatcac tcacacacta aagcgggcaa ttgcgagcgc agtttttgtc
61 gcgtccgctg ctaccgcctc tatagccaat gcagtaacgg tagatattct tgtgctttac
121 gataattatt cggcaaatta ttttggcggc gacccgcaga cggccatgaa cggttgggct
181 aacgatatga actctgcgtt aaaagccagc caaattgata tgaagttccg cattgttggt
241 gtgcgtcatc acgaagaaga tggcgcgggc atgggcgacg tacttggtaa tttgcgcgtt
301 gacggtggcg caatagccct gcgcgatcaa cttggtgcag atatggtgtc acaactccac
 
361 gaaaagggtg cctgtggcgt ggggtatgtt gccgtagata aaaattacac atggaacgtt
421 actcacccag gctgcgggcc aatggtaatg ctgcatgaat ttggccataa catgggggtt
481 actcactcgc gtaaacaggg tgatcaaggt ggtacccgct atcgctatgg cgtaggttat
541 ggtgtgcagg atgttttcgt cgatatcatg gcttacgaag gcgtttttaa caccagccgc
601 gttaatgtat tttctaaccc taacctcaat tgcagaggcc ttccgtgtgg taaacccgtt
661 ggcgattccg aagaagccca cgcttcactc gctattcaca atgtgcgtaa cgaacttgcc
721 aattttcgca ataccgtaaa ttctggtgga ccggttcgat tgtttgaaca ctgttattac
781 acgggttata cagttggctt aggcgaaggt agctatcgct tagccgattt gatgaaccgc
841 ggtttggtaa acgacgattt gtcatcgttg caagttgatg caggctatag agtggaaatg
901 tttcaacacg acaactttac cggcaatgtt gtaacgcgca ccggcagcga cgattgttta
961 gtcgatgaag gcatgaacga cgatataagt tcactgcgta taacacgagt aagtggcggg
1021 tttagtcaaa ctatccaagc agagaatttt ttcgctaaca acggcgtgca actagagaat
1081 acaaccgata gcggtggcgg ccaaaacgta ggttggattg atgctaacga ttggatggct
1141 ttcagcaaca ttaccattcc aaccaccggt aactaccgta tcgaataccg cgttgcaggt
1201 tttggtggca cgctttcgct cgacttaaat ggcggcgcta tagtgctagg ccaaattaat
1261 ttacccaata ccaatggctg gcagaattgg caaaccgcat cgcacactgt acacattaat
1321 gctggcactt ataatttcgg tatttttgct aacgcacccg gctggaatat caattggttc
1381 cgtatagtac agctttaa
LOCUS NZ_AABI02000007 3075 bp DNA linear BCT 23-SEP-2003
DEFINITION Microbulbifer degradans 2-40 Mdeg_8, whole genome shotgun sequence.
ACCESSION NZ AABI02000007 REGION: 24366..27440
VERSION NZ_AABI02000007.1 GI:28878295
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3075)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027895. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..3075
      /organism="Microbulbifer degradans 2-40"
      /mol_type="genomic DNA"
      /strain="2-40"
      /db_xref="taxon:203122"
   gene 1..3075
      /gene="Mdeg1720"
   CDS 1..3075
      /gene="Mdeg1720"
      /codon_start=1
      /transl_table=11
      /product="hypothetical protein"
      /protein_id="ZP 00066336.1"
      /db_xref="GI:23027911" 
/translation="MKKPPSRYSTLAITLCMSLSQAAIAKDIYVAPTGDDAGAGSFSS
PYQTLAKAAQTAQAGDVVYLREGTYQETLRPANSGTASHPIVFQSYQNEKVIISAMEA
LSGWQQDTSNIYKTTVNWDLGQENFVMHKSTALDLARWPNNTDADPFTLNSKRNTGGS
GPEVGQGAYIEYAAGLPNINWTGGTVFYYGDKPGGGWLAWRETIVSHTQTRINIDLSK
KNPAWVRTAHDPASGGEFYLMGVKGALDYQNEWYFDSNTRELFVQLPNGARPQNGDIQ
FRKRLQTINLANRSHIHIKNIAVFGGAIEITNNANSNLLSGVSSFYGNATLGVHTGFS
APSYSVKIQGSDNRIENSEIAYGSGTGIYDSGTRSQIVNNYIHDFNTLGDYNAPVNAR
GGSNTLVKNNRISRGGRDTIQAFNRDSEWSYNDVSHSNLIADDCGLFYTV.GGPHNVEI
HHNWFHDAYSHGNKNKAAGIYLDNDARGFKVHHNVVWNTEWTGIQINWNGTDIDVFNN
 
TLWNNSAAMGAWHKAGTAFSDVRVWNNLSNSNKWEEQANKQNNLTGTGDPFVNSQAGD
FRLKANTAPIDYGRTIAGTTEGHSGANPDAGAYEYGATAWKAGVTWDITKGAANRCYQ
LPGEVCFDDTDTGGDIGELPGKVEAENYAHYYDTTPGNIGGAYRNQDVDIQPTTDTLG
GYNVGWINAGEWLEYDIDVTQAGRYDAELRVASKLGAGQVAIAIDGVARGEALTIQST
GDWQNWATLTTQLGYLEAGLHTLRVSAMSGGFNLNWYNFTKQVSFGETAVGFTNTPPT
RIPATRHQSFSVDYVANEPRELFLLFFNPDWSWVASTKTTVEPGKASTTLQLNLPFVP
 
TAVQAFNVKLENRPIGANWDNPNNVEAHAAVTTQAAPLQHNGGFENGGTSGWNGYGSH
ALSTEAHSGSYAGKVTGGPSAFSQTIPNLTPNTTYSLSAFVKARAGHTGFLGVKEYGG
QETSLIVNSTHYQKKTITFTTGPNASSAKIYLYVRDNNHVAFIDELVIVKVY"
ORIGIN 
1 atgaaaaaac cacctagccg ttacagtacg ctggccataa ccctgtgtat gagtttatcg
61 caggccgcta tagcaaagga tatctacgtc gcccctacag gagatgacgc tggagccgga
121 tcgtttagca gcccctatca aacgctggcg aaagcggcac aaaccgcaca agctggtgat
181 gtcgtttacc tgcgcgaagg cacctatcag gaaacgcttc gcccagcgaa ttcaggcaca
241 gccagtcacc ccattgtgtt tcaatcctat cagaatgaaa aagtgatcat cagtgcaatg
301 gaagccttaa gcggctggca gcaagacacc tcaaatattt ataaaaccac ggtgaactgg
361 gatctaggcc aagaaaattt tgtgatgcat aaatctacag cactggattt agcccgctgg
421 ccaaacaata ccgatgcaga ccccttcacg ttaaattcta aaagaaacac agggggaagt
481 ggccccgagg taggccaagg tgcttatata gaatacgcag caggattgcc caatattaat
541 tggactgggg gtaccgtatt ttattatggc gataaacccg gtggcggttg gctagcgtgg
601 cgcgagacga ttgtaagcca cactcaaacc cgtataaata tagacctttc taaaaagaat
661 cccgcgtggg tacgcaccgc acacgacccg gcaagcggcg gagaattcta tttaatgggc
721 gtaaaaggtg ctttagatta tcagaacgaa tggtacttcg attcaaatac gcgagagctt
781 tttgtccaac tgcccaacgg cgcgcgccca caaaatggcg atattcaatt tagaaaacgc
841 ctgcagacca ttaacttggc aaatagaagc cacatacaca ttaaaaacat tgctgttttt
901 ggtggcgcta tagaaataac caataacgct aattctaacc tgctttctgg ggtatccagt
961 ttttatggca atgctacgct cggtgtacac accggatttt cggcgccaag ctacagcgta
1021 aaaattcaag gcagcgataa ccgtatagaa aacagtgaaa ttgcctacgg ctccggtact
1081 gggatatacg acagcggcac ccgcagccaa attgtgaata actacattca cgattttaat
1141 acccttggcg actacaacgc gccggttaac gcgcgcggcg gaagtaatac gttagtaaaa
1201 aataatcgaa tctcccgcgg aggccgcgac accatacaag cttttaatcg agacagcgaa
1261 tggtcgtata acgatgtttc ccacagtaat ttaatagccg atgactgcgg cttgttttac
1321 accgttggcg gaccacacaa tgtggaaatc catcacaact ggtttcacga cgcatattca
1381 cacggcaaca aaaacaaagc cgctggtatt tatttagata acgatgcccg aggttttaaa
1441 gtacaccaca acgtggtgtg gaacaccgag tggacaggca ttcaaataaa ttggaacggc
1501 acggacatag acgtatttaa caacacatta tggaacaaca gtgccgctat gggagcatgg
1561 cacaaagccg ggactgcgtt ttcggatgtt cgagtttgga acaacctctc caacagtaat
1621 aagtgggagg agcaagccaa taagcaaaac aaccttacgg gaacaggcga tccgtttgtg
1681 aacagccaag ccggagattt tagattaaaa gccaatacgg cccctatcga ctatggccgc
1741 actatagcgg gtacaaccga ggggcacagt ggcgccaacc cagatgctgg tgcgtatgaa
1801 tacggcgcaa ccgcttggaa agcaggcgtc acatgggata taaccaaagg cgcagccaac
1861 cgctgctatc aacttcccgg tgaggtttgc tttgatgaca ccgataccgg cggagacatt
1921 ggtgagctac ccggtaaagt agaagccgaa aactacgccc actactacga caccaccccc
1981 ggtaatattg gcggcgccta ccgcaaccaa gatgtagata ttcagcccac caccgataca
2041 ctaggtggct acaacgtagg ctggattaac gctggcgagt ggttagaata cgacatcgat
2101 gtcactcagg cggggcgcta tgatgccgag cttagagtag cctctaaact gggtgcgggc
2161 caagtagcca tagccataga tggggtcgct agaggcgaag cgttaacgat tcagtcgacg
2221 ggcgattggc aaaattgggc aaccctaaca actcaactag ggtatttaga agcagggcta
2281 catacgctgc gggtatccgc tatgagcggt ggcttcaatc taaattggta taacttcact
2341 aaacaagtaa gctttgggga aaccgccgta ggcttcacca acactcctcc tacacgtata
2401 ccagctacaa ggcatcagag cttttctgtg gattatgttg ccaacgagcc gcgagaactg
2461 ttcttattat ttttcaaccc cgactggagt tgggtagcct cgacaaaaac aaccgtagag
2521 cccggtaaag cttctaccac gttgcaatta aaccttccct ttgtgccaac cgctgtgcaa
2581 gcatttaacg ttaaattaga aaatcgcccc ataggcgcca actgggataa cccgaacaat
2641 gtcgaagcgc acgctgccgt taccacgcaa gcagcaccgc tacaacataa cggtggcttc
2701 gaaaacggtg gcacctcggg ttggaatgga tacggtagcc atgcacttag cacagaagcc
2761 cacagcggca gctatgcagg caaagtaacc ggtggcccct ccgcgttttc gcaaactatt
2821 cccaacctta ccccaaacac cacctacagc ctctcggctt ttgttaaagc acgcgctggt
2881 cacacggggt ttttaggcgt taaagaatat ggaggccaag aaacgagcct gatagtaaac
2941 agcacccact atcaaaagaa aaccatcacc ttcaccaccg gccccaacgc cagctcggca
3001 aaaatttatt tatacgttag ggataacaac catgtggcgt ttattgatga gttagtcatt
3061 gtgaaagtgt actag
LOCUS NZ_AABI02000009 2739 bp DNA linear BCT 23-SEP-2003
DEFINITION Microbulbifer degradans 2-40 Mdeg_10, whole genome shotgun sequence.
ACCESSION NZ AABI02000009 REGION: complement(131825..134363)
VERSION NZ_AABI02000009.1 GI:28878293
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2739)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23028157. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2739
      /organism="Microbulbifer degradans 2-40"
      /mol_type="genomic DNA"
      /strain="2-40"
      /db_xref="taxon:203122"
   gene 1..2739
      /gene="Mdeg2055"
   CDS 1..2739
      /gene="Mdeg2055"
      /codon_start=1
      /transl_table=11
      /product="hypothetical protein"
      /protein_id="ZP 00066663.1"
      /db_xref="GI:23028252" 
/translation="MKKILFLALGLLISQASFAQQTISSLAEFIALQDGSNQNIKMAP
GTYHISSSSKSLFPGGDWRANVEGNWPGLFKFSGNNNTFDLTGVTFTFDSTILLEMPN
LVHANLMEFGGSGNVVKGLNIQEKPNSKGEYGAFIHTSGGTIAVFTGDNHKVSDFTLK
TRFSRPYGLGSLYGKTGNSSSTLPGVRLSKKTAMFLISLDDSYFENVLIDHSGFGHTL
AFNGVDNVVFNTVEIIAESRSTDDLYANGIGGTDRNGVPFNVLFNGDELIGTNFADAD
YFLNLFDTDNFNQCQNMSGGVQYSPIRKGYQYSLTEDSFRGYYSAGSLGNIEIYNATV
TGSRAGVVMEYASEGMIVDGMTVRGIAGHGVPACDGAWNSANGGEGDASAYGPPSNSV
LKRAKADAAYSTVLEIPNFVDNVTADIEVLDPLNGYNRPSASNALALIKGDDHDIRLW
KRDNQALTRDLVVKVSDADNLLLCNMTKQGVTVASSVTNSTIYSVGSISNSSGSSNTV
VKLSSAADEPAICKALETNEVITDCGDFDAFAGIQAEAYCDMAGVEIESSSDDGGEQV
GYINNNEWIAFNDVDFGNGASGFEARVSSATSGGNIELRLDSQTGALIGTCSVNGTGS
WTTYETVSCDIGGVSGVQDLYLVFTGNSGYLMNVNWFNFTQAATNCSLPWSDSDFSVE
KEIVNYSSGAIDISCASNVEISMNLEGVGAMEDADYLNVYYRVDGGAQQVISENVNAF
SEKTVSVSGINGSSLEIIANVYTSYGAEIYTISDMSVKADSQTAYSLEVVSVLASADD
GNVPANTRDGDLGTRWSANGDSQWITYDLGSSKTVTDVGIAFFRGDQRTAFISIETST
 NNSTWQTVYSDEQSSSTTEIQNFDVTDSNARYVRIIGYGNSVNNWNSFTEVEIIGR"
ORIGIN 
1 atgaaaaaaa tcctgttttt ggccttaggc cttttaataa gtcaggcttc gtttgcccag
61 cagacgattt caagccttgc cgagtttatt gctcttcaag atggcagcaa tcaaaacata
121 aaaatggccc ccggcaccta tcatatatca tcatcgtcta aatcactgtt ccccggtggg
181 gattggcggg cgaacgtcga aggtaattgg ccgggcctgt ttaagtttag tggcaacaat
241 aatacgtttg acctgaccgg cgttaccttc accttcgact ccacgatctt gttagaaatg
301 cctaatcttg ttcacgcaaa cctcatggag tttgggggtt ccggtaacgt ttggaagggg
361 ctgaatattc aggagaagcc caacagtaag ggcgagtacg gcgcttttat tcatacctct
421 ggcggcacca tcgctgtatt cactggggat aatcacaaag tttctgattt tactttaaag
481 acacgttttt ctcggcccta tggtttaggt tctctctatg gaaaaacggg taactcgtct
541 agcacgctgc ccggcgttcg cctgtccaag aaaacggcca tgtttcttat tagtttagat
601 gatagttact ttgaaaatgt tctgattgat cacagtggct ttggtcatac gcttgccttt
661 aacggtgtcg ataacgtggt attcaatacc gttgaaatca tcgcagaatc tcgctctacc
721 gatgatttgt atgctaacgg cattggcgga acagaccgca atggtgtgcc ttttaacgtc
781 ctctttaatg gcgatgaact cataggaacg aattttgccg atgccgatta ctttcttaac
841 ctgtttgata cggataactt caatcagtgt caaaacatga gcggtggcgt gcaatattcg
901 cccatccgaa aaggttacca atatagcctg actgaagatt cgtttcgcgg gtactacagt
961 gctggttccc ttggcaatat cgagatttat aacgccaccg ttacgggttc aagagcgggc
1021 gtcgtcatgg aatatgcgag cgagggtatg attgtagacg gtatgacggt tagaggtatt
1081 gccgggcatg gcgtgccagc gtgcgacggc gcgtggaatt cagctaacgg tggtgaaggc
1141 gatgcttccg cctatggtcc tccatcaaac agtgtactta agcgtgcgaa agccgatgcc
1201 gcctattcca ctgttttaga aatccccaat tttgtcgata acgtgacggc cgatattgaa
1261 gtgttggacc cgttaaacgg ttacaaccgt ccttcggcat caaacgcttt ggcgcttatt
1321 aaaggtgatg atcacgatat tcgactttgg aaacgtgata accaagcctt aacccgtgac
1381 ttggtggtta aggtgagtga tgcggataat ctattgctct gtaacatgac aaaacaaggc
1441 gtgactgtcg ccagcagcgt gaccaactcc accatttatt ctgttggtag catcagtaat
1501 tcaagtggca gttctaacac ggtggtaaaa ctgtcaagcg cagctgacga gccagcgata
1561 tgtaaagctc ttgagactaa cgaagttata acggattgcg gtgattttga tgcattcgcc
1621 ggtatccaag ccgaagccta ctgcgatatg gctggcgttg aaattgaatc ctccagcgat
1681 gacggtggcg aacaggttgg ttacattaac aacaacgaat ggattgcatt caacgatgtt
1741 gattttggca atggcgcaag cggttttgaa gcgcgcgtta gtagtgctac ctccggtggc
1801 aatattgagc ttcgtttaga cagccaaaca ggcgcgttaa ttggcacgtg ctctgttaat
1861 ggaacgggca gttggacgac ctatgaaacg gtttcttgcg atatcggtgg cgtcagcggc
1921 gtgcaggatt tgtacctcgt gtttaccgga aatagtggct atttaatgaa tgttaattgg
1981 tttaacttca cgcaagctgc aacgaattgt tctctgcctt ggagtgacag tgacttcagc
2041 gttgaaaaag aaatagttaa ctacagttcc ggtgctattg atatttcatg tgcttccaac
2101 gttgaaattt ccatgaatct agaaggtgta ggcgccatgg aagatgccga ttacttgaac
2161 gtttattacc gcgttgatgg cggtgcccaa caagtgatat ctgaaaatgt gaatgcgttt
2221 tctgaaaaaa ccgtatcggt atcaggtatc aatggcagta gcttagaaat tatcgccaat
2281 gtttatacca gctatggcgc agagatttat acgatttctg atatgagtgt taaagctgat
2341 tcgcaaacag cttattctct agaggttgtt tctgtcttag ccagtgcgga cgatggcaat
2401 gtgcctgcca atacccgtga tggcgacctg ggtacacgct ggtcggctaa tggtgattcg
2461 cagtggatta cctatgatct tggcagcagt aaaactgtga ccgatgtggg catcgccttt
2521 tttagaggcg accaacgcac ggcatttatc agcatcgaaa cctcaaccaa taatagtacc
2581 tggcaaaccg tttattctga tgagcagtca agcagcacaa cagagattca aaattttgat
2641 gtgaccgata gcaatgctcg ctatgtaaga attattggct acggaaatag cgtaaataac
2701 tggaacagtt ttacggaggt cgagattatt ggtcgttaa
LOCUS NZ_AABI02000051 1626 bp DNA linear BCT 23-SEP-2003
DEFINITION Microbulbifer degradans 2-40 Mdeg_51, whole genome shotgun sequence.
ACCESSION NZ AABI02000051 REGION: complement(8184..>9809)
VERSION NZ_AABI02000051.1 GI:28878251
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1626)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23030027. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jdi.doe.gov
   Contact: Paul Richardson
   microbes@cuba.jgi-psf.org.
   COG assignment was automatically updated on Sep 23, 2003.
FEATURES Location/Qualifiers
   source 1..1626
      /organism="Microbulbifer degradans 2-40"
      /mol_type="genomic DNA"
      /strain="2-40"
      /db_xref="taxon:203122"
   gene 1..>1626
      /gene="Mdeg3723"
   CDS 1..>1626
      /gene="Mdeg3723"
      /codon_start=1
      /trans_table=11
      /product="hypothetical protein"
      /protein_id="ZP 00068306.1"
      /db_xref="GI:23030033" 
/translation="LALSASLTQAATISNSGFESGFDGWTDTDPSALSSDANNGSRSA
KITGSAGRVDQDVAVTPNTNYQLTAYVLGSGRVGVNTGTAVYDEAVNTSSWSKVTVNF
NSGSANSVEVFGKYNSGTGRFDDFSLVETGTPTPTPTPTPTPTPTPAGCNSLNTIDIS
SATDDGSHDGHGPHLAVDGDLSADSRWSSKGDGKAITLDLGAEATVRQLKTAWYKGDS
RTAYFDVETSTDKSNWSTALSNVQSQGSTGLKSNSIDDVTARYVRIVGHGNSSNTWNS
LIEAQVLGCAGTVTPTPTPTPTPTPTPTPTPTPTPSGSKIPESITNSDVWDLEGENPH
PLVDPYTLEFVPLEARVTTPNGNGWRHEYKIASSERTAMTATYEDFSATIKVDLSTGG
KTIVAQHHAGDTGTIMKLYVSDTSESGFFDSVAANGIFDVYVRIRNTSGVEEKKPLGT
IRSGDSFSFHVLNNYGVVKVSAFGKNLETEVEDDSASYLKFGNYLQSQYPQGSKDCGS
HGDSDSFRACYEDIGITEAKITMTNVSYTRITK"
ORIGIN 
1 ctcgcgctta gtgcatcttt aacacaggct gcgaccattt ctaactcagg cttcgaaagt
61 ggctttgacg gttggacaga cactgaccct tcggcacttt ctagcgatgc aaataacggc
121 agccgatctg ccaaaattac tggctccgcc ggccgtgtag atcaagacgt tgcggtaacc
181 ccaaacacca actaccagtt aactgcatat gtacttggca gtggacgcgt gggtgttaac
 
241 accggcactg cagtttatga cgaagccgta aacacaagca gctggagcaa agtaactgtt
301 aactttaact ctggctctgc aaactctgta gaagtgtttg gtaagtacaa tagtggtact
361 ggccgctttg acgatttcag cttagttgag acgggaacac cgactcctac gccaacacct
421 actcctacgc ccacgcctac accagcgggc tgtaacagct taaataccat cgacattagc
481 tctgctaccg atgatggctc tcacgatggt cacggcccac acctagctgt ggacggcgat
541 ttatcagctg attcacgctg gtcttctaaa ggcgacggca aagcgattac tttagactta
601 ggcgccgaag ccacagttcg tcaactaaaa acggcttggt acaaaggtga ctcacgtacc
661 gcttacttcg atgtagaaac gtctaccgat aagagcaact ggtctaccgc tttaagcaac
721 gtgcagtcac aaggtagcac tggccttaaa tcaaacagca ttgacgacgt aacagcgcgt
781 tacgtacgta ttgttggtca cggtaactcg tcgaatactt ggaacagctt gattgaagca
841 caagtattgg gttgtgctgg cactgtaacg ccaacaccta ctcctacgcc tacaccaact
901 ccgaccccaa cgcctactcc tacgccaact ccatctggct caaaaattcc tgaaagcatt
961 acaaacagcg atgtttggga tttggaaggc gagaacccac acccattggt agacccttac
1021 acgttggaat tcgtacctct tgaagcacgc gtaacgactc caaacggtaa cggctggcgc
1081 cacgagtaca aaatcgcttc tagcgagcgt actgcgatga ctgctaccta cgaagatttc
1141 tctgcaacta ttaaagtaga cctatctact ggcggtaaga caattgttgc acagcatcac
1201 gcaggcgaca ctggcaccat catgaaacta tacgtttccg acacaagcga atctggcttt
1261 ttcgatagcg tagcggcaaa cggcattttc gacgtgtatg ttcgtattcg taacaccagc
1321 ggtgttgaag agaagaaacc attgggcaca atccgctctg gcgactcttt cagcttccac
1381 gtacttaaca actacggcgt tgtaaaagta tctgcctttg gtaaaaacct agagacagaa
1441 gtagaagacg attctgcatc ttacttgaag tttggtaact acctacaatc gcaataccca
1501 caaggtagca aagattgtgg ttcacatggc gactccgatt cgttccgtgc ctgctacgaa
1561 gatataggca ttaccgaagc gaaaatcacc atgactaacg tttcttacac gcgtatcact
1621 aagtaa
LOCUS NZ_AABI02000004 3102 bp DNA linear BCT 23-SEP-2003
DEFINITION Microbulbifer degradans 2-40 Mdeg_4, whole genome shotgun sequence.
ACCESSION NZ AABI02000004 REGION: 20681..23782
VERSION NZ_AABI02000004.1 GI:28878298
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 3102)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027141. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..3102
      /organism="Microbulbifer degradans 2-40"
      /mol_type="genomic DNA"
      /strain="2-40"
      /db_xref="taxon:203122"
   gene 1..3102
      /gene="Mdeg0984"
   CDS 1..3102
      /gene="Mdeg0984"
      /codon_start=1
      /transl_table=11
      /product="COG3488: Predicted thiol oxidoreductase"
      /protein_id="ZP 00065611.1"
      /db_xref="GI:23027156"
      /db_xref="COG:COG3488" 
/translation="MGEAPVKKQAYTTTPVKPNALGLAIRTLALGGLAAGLVNVAHAD
LLSVNKTATASSEMQAAAYAFDNNQNTRWESAHAVDPTSISVDLGETYDLDSIVVHWE
AANAASYTIEGSDNGVNWTQIGTYTGGTFGNRTDTVNVDGNYRHVRLNGTQRSDGNAW
GYSIWELEVHGTEVTEPPVVEPPTEPGENLAIYGTATASSGNADVAIDNNAGTRWESD
HGIDPSSFTLDLGATYSLNQVVIDWEAANAKVYAIQGSNDGTNFTTLANYSGGEFGTR
TDTLNIAGDYRYVRLLGTERSDGNAWGYSIWEFKVYGGGKTEPPVTEPPVTEPPVVEP
PIFTDLNYQPLFNNTHSPDTAQEWYTKPDGTVVTIASGRARSRHESEDIFYTFPTHYF
EHRTFEIEIHDHTPKGQNLVEVFYHPEYANYVPPGCRSSYSNVWRADFNNNAGMDEKL
QTATPDGKGERWVCRIQRDAHNGDDGILDVGSWMEFELQQFLGLYEGDPNVRGQAVYY
TDTYRFKLGQPGIYIVGDEAMEEKIRAGGRATAPYVKGGDSVPVNEVISVNGDNTLTY
KVMANGKWTQKDNPNGTVVTFPIRDGIEVYDNYVVASGVADWTTYFREALNIQWDTHN
AFMQGRRVFHTRMDTGVHEEVGNPDFPELANIADGLMVKNSCLGCHVNNGRGIAPQNG
ALLDTLVVKVGSGAFDNLGQPQPHSYYGGVLQNLSLDAAVPAEGSVRVTYTAQNGTFN
DGTGYSLQVPTYSLEMNDTNGGAIQHISPRMPQNITGLGLLEALPENEILAWHDPDDS
NGDGISGRANVVTSPETGQQFIGRFGWKASSASLRDFAATALSGDMGVNTSVLPNADC
 
GAQQTACIQNSGQGVELSDLRLNELVVYLQALGAPSRRPETVDQPMVVAGEQRFTDIG
CASCHRPEMNTGHKHDLAELRGNVIRPYTDMLLHDMGPGLADSLTQAPELNREWRTAP
LWGLGMNLAVNGHDNLLHDGRARSIEEAILWHGGEAQASNNAYKALSAQQRAELIAFL
RSL"
ORIGIN 
1 gtgggagaag cacctgtgaa aaaacaagct tatacaacta caccagttaa accaaatgcg
61 ttaggtttag ccattcgcac actggccctt ggtggcctag ctgcgggact tgtaaacgta
121 gcgcatgcag atttattgtc ggtaaataaa actgctacgg ccagcagtga aatgcaagct
181 gcagcttacg cattcgataa caatcaaaat acgcgctggg aaagtgcgca cgcggttgat
241 ccaaccagca taagtgtaga cttgggcgaa acctacgatt tagatagcat tgtcgttcac
301 tgggaagctg ccaacgctgc aagctacacc atagagggct ccgataacgg tgttaactgg
361 acgcaaatag gcacctatac cggtggtacc tttggcaata gaaccgatac cgtaaacgta
421 gatggtaatt accgtcacgt gcgattaaat ggtactcagc gcagcgatgg caatgcatgg
481 ggttactcca tatgggagct ggaagttcac gggacagaag taaccgaacc acctgtagtg
541 gaacctccta cagagcctgg cgagaatttg gcaatatatg gcacggcaac cgccagctct
601 ggtaacgccg atgtagctat tgataataac gctggtacgc gctgggagag tgaccacggc
661 atcgaccctt ctagttttac tttagaccta ggcgcaacct acagcttgaa ccaagtggtt
721 atagattggg aggccgccaa cgccaaggtg tatgctatcc aaggctctaa cgatggcact
781 aactttacta cccttgctaa ttacagcggc ggtgaattcg gcacgcgtac cgatacatta
841 aatattgctg gtgattatcg ctatgtacgc ctgttgggta ctgagcgcag cgatggcaac
901 gcatggggct attctatttg ggaatttaaa gtatatggcg gtggaaaaac tgagccaccg
961 gttaccgagc cacctgtgac agaaccgcca gtggtagagc ccccccattt taccgaccta
1021 aattatcagc cactgtttaa caacacacac agcccagata ctgcgcaaga gtggtacacc
1081 aagcccgacg gtacagtggt aacaattgca agtggccgcg cgcgttcacg tcacgaatcg
1141 gaagatattt tttacacttt cccaacgcat tattttgagc accgtacatt tgaaatagaa
1201 attcacgacc atacccctaa gggccaaaat ttggtggagg ttttctacca cccagaatac
1261 gccaactatg tgccgccggg ttgtcgctct tcttacagca atgtttggcg tgcagacttt
1321 aacaacaacg ccggcatgga tgaaaaactg caaaccgcca ccccagacgg taaaggtgag
1381 cgttgggtat gccgcataca gcgcgacgcg cacaatggcg acgacggcat tttagatgtg
1441 ggttcatgga tggagtttga attacaacaa ttcttaggct tatatgaagg cgaccctaat
1501 gtgcgtggcc aggccgttta ctacaccgat acataccgtt ttaaattagg ccagcccggt
1561 atttatattg tgggcgatga agccatggag gaaaaaattc gtgcgggtgg ccgcgctaca
1621 gcgccttatg taaaaggcgg cgactcggta cctgttaacg aagttatttc ggttaatggc
1681 gacaacacac ttacctacaa agtaatggca aatggtaagt ggacgcaaaa agataacccc
1741 aacggcactg tagttacttt ccctattcgc gatggcattg aggtatacga taactacgta
1801 gtggcgagtg gcgtggccga ttggaccacc tatttccgcg aagcgttaaa tattcagtgg
1861 gatacgcaca acgcatttat gcaggggcgg cgcgtgtttc atacgcgtat ggataccggc
1921 gtacacgaag aagtgggtaa cccagacttt cccgaattgg caaatattgc cgatggttta
1981 atggtgaaaa attcgtgctt gggttgtcat gtaaacaacg gccgcggtat tgctccgcaa
2041 aacggcgcgt tattagatac gctagtcgtt aaagtaggct cgggtgcgtt cgataacctt
2101 ggccagccac aaccacacag ttattacggt ggtgtactgc aaaacctatc actagatgct
2161 gctgtgcctg cagagggttc ggttcgtgta acttacaccg cgcaaaacgg aacctttaac
2221 gacggcacag gctacagctt gcaagtgcct acctattcgt tagaaatgaa cgataccaac
2281 ggcggggcta ttcagcatat ttcaccgcgc atgccacaaa acataactgg tttaggctta
2341 ttagaagcct tacccgaaaa tgaaattttg gcatggcacg acccagatga tagcaatggc
2401 gatggtattt ctggccgcgc taatgttgtt acctcaccag aaacggggca acagtttata
2461 ggtcgctttg gctggaaagc gtcatcagca agtttgcgtg attttgctgc aacggcttta
2521 agtggcgata tgggtgtaaa tacctctgtg ttacccaatg cagattgtgg cgcacaacaa
2581 accgcctgta ttcaaaatag cggtcagggc gtagagctaa gcgacctgcg tttgaatgaa
2641 ctggtggttt acttgcaagc gctgggcgcg ccttcgcgca ggccagaaac tgtagaccag
2701 ccaatggtag tggccggtga gcagcgtttt accgatatag gctgtgcctc gtgtcaccga
2761 ccagaaatga acaccggtca taagcacgat ttagctgagt tgcgtggcaa cgttattcgc
2821 ccctataccg atatgctgtt acacgatatg ggcccaggtt tggcagatag cttaactcaa
2881 gcgccagagc taaatcgcga atggcgtaca gcgccgctgt ggggcttggg catgaacttg
2941 gctgtaaacg gtcacgacaa cttgctccac gatggtcgcg cacgcagtat tgaagaagcc
3001 attttatggc acggtgggga agcgcaagcc agtaataatg cctataaagc actaagtgcg
 3061 cagcagcgag cagagttaat agcgttttta cgctcactct aa
LOCUS NZ_AABI02000005 1674 bp DNA linear BCT 23-SEP-2003
DEFINITION Microbulbifer degradans 2-40 Mdeg_5, whole genome shotgun sequence.
ACCESSION NZ AABI02000005 REGION: complement(228218..229891)
VERSION NZ_AABI02000005.1 GI:28878297
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 1674)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027361. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.go
   Contact: Paul Richardson
   microbes@cuba.jgi-psf.org.
   COG assignment was automatically updated on Sep 23, 2003.
FEATURES Location/Qualifiers
   source 1..1674
      /organism="Microbulbifer degradans 2-40"
      /mol_type="genomic DNA"
      /strain="2-40"
      /db_xref="taxon:203122"
   gene 1..1674
      /gene="Mdeg1383"
   CDS 1..1674
      /gene="Mdeg1383"
      /codon_start=1
      /transl_table=11
      /product="hypothetical protein"
      /protein_id="ZP 00066007.1"
      /db_xref="GI:23027561" 
/translation="MLHISVQPNLTRSISGFLLGLAGVVSGSAYAQWNPAPNWEDSYS
VNGVCYCNSSNYDHGLSAKTAPTPIGELNVVDICTDIKAVLGEGATNGRIPFNDIQCG
NGPANDAADEAGCPGRVDIGSAGCDVIGPKWDLVSVYGPWPDGGLNRDAWEVSASNGS
GSAQLALDGLASTRWATGVFQSPGQYFDIDFQDALTFDSIVLATTENPEDYPRAYEVY
ISSNGSDWGVPVVTGAGNGSTTTIELDTVTTRYLRILQTGSSSNRWWSIHEFNIFNSG
PIPPEPEYPALDRSDWTVSASVNGAEAEFAIDSSPNTRWDTAQSQRAGQSFEVDLGEL
NTLAAIELDSAGSANDYPRGYAVYVSNDGSNWGSAIASGAAVSASTTIEFTPVSARFV
KIEQTGGDGHYWWSIHNLNIFGEPSDNPQPTIELLDSTPWSLAANRRNSAAGNAIDNN
QSTRWTTGQTQRDGQTFEIDLSTVQTFSRIVLDSAASDDDHPRNYELYVSNDGSNWGS
PVATGAGDSSGVTVIDFPSVTARYVLIAQAGSDSSHWWSIHELSIYNVQ"
ORIGIN 
1 atgctacaca tctctgttca accaaatctt acccgctcta tttctgggtt cttgttgggc
61 ctagcgggcg ttgtctcggg ctcggcttac gcgcagtgga acccagcacc caactgggag
121 gacagctatt ctgtgaatgg agtttgctac tgtaattcca gtaattatga tcatgggcta
181 agcgccaaaa cagcacctac tcccattggc gagcttaacg ttgtcgatat atgtaccgac
241 atcaaagccg tgcttggtga gggggcaacg aatggacgta ttccatttaa cgacattcag
 
301 tgtggtaacg ggcccgccaa cgatgctgcc gatgaagcgg ggtgcccagg ccgagtggat
361 attggctcgg cagggtgtga tgtgattggg cctaaatggg atttggttag cgtttacggg
421 ccatggccag atggcgggct taatagggat gcttgggaag tgagtgcctc aaacggttct
481 ggtagcgcgc aactggcgct tgatggcttg gcgagtacgc gctgggcgac gggtgtgttt
541 caatcgccag gtcagtattt tgatattgat tttcaagacg cgctaacttt cgattcaata
601 gtacttgcta ccaccgagaa cccagaagat tacccaaggg cttacgaggt ttacatttca
661 tcaaatggca gcgactgggg agtaccagtg gtgacgggag ccggtaacgg ctccactaca
721 actatcgagc tagatactgt taccactcgc tacttaagaa ttttacaaac cggcagctca
781 tctaaccgtt ggtggtccat tcatgaattt aacatattca attctgggcc aattccgcca
841 gagccagagt acccggcatt agatagaagt gactggacag tttctgcatc ggtaaatggc
901 gcagaagccg aattcgctat cgactcttca cccaatacgc ggtgggatac agcacaaagc
961 caacgagcag ggcagagttt tgaggtagat ttaggagaac tcaacacgct agcggccatt
1021 gagctggatt cagcaggcag tgccaatgac tatccgcggg gttacgccgt ttatgtatcg
1081 aacgatggct caaactgggg gagcgctatc gcttctggcg ctgcagtaag cgcgtccaca
1141 acaatagaat ttactccagt aagcgctcgc ttcgttaaaa tagagcagac gggaggtgac
1201 ggacactatt ggtggtctat acacaactta aatatttttg gtgagccttc agataaccca
1261 caaccgacta tagagctact cgatagcact ccctggtctc tagccgcgaa caggcgaaat
1321 agcgcggctg gcaatgccat agataataac cagtctacac gatggaccac gggccagacc
1381 cagcgcgatg gtcagacgtt cgaaattgac ctgagcactg tgcaaacctt tagccgtatt
1441 gttttagatt ccgcagcgag tgacgatgat caccctcgca attatgaact ttatgtctct
1501 aacgatggtt ctaattgggg cagcccagtg gcaaccggtg caggagatag tagcggagta
1561 acggtaatag atttccccag cgtgaccgct cgctatgtac ttatagctca ggctggctcg
1621 gatagctcac actggtggtc cattcacgag ttgagtattt acaacgtaca ataa
LOCUS NZ_AABI02000025 2025 bp DNA linear BCT 23-SEP-2003
DEFINITION Microbulbifer degradans 2-40 Mdeg_25, whole genome shotgun sequence.
ACCESSION NZ AABI02000025 REGION: complement(50063..52087)
VERSION NZ_AABI02000025.1 GI:28878277
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2025)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23029437. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom_table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2025
      /organism="Microbulbifer degradans 2-40"
      /mol_type="genomic DNA"
      /strain="2-40"
      /db_xref="taxon:203122"
   gene 1..2025
      /gene="Mdeg3228"
   CDS 1..2025
      /gene="Mdeg3228"
      /codon_start=1
      /transl_table=11
      /product="hypothetical protein"
      /protein_id="ZP 00067813.1"
      /db_xref="GI:23029476" 
/translation="MKIIRLLVLCLGVVSSVVAVASSSEPHDEGNALESALWDTLTIP
VCWENPEAFPVEEQAWVRKAVERTWEQESLVRFTGWGECGANDDGIRILVDDVGPHVK
QLGSRLDGYVNGMVLNHTFQNWGTSCSYRRQYCAEVIAVHEFGHALGFAHEQNRDDTD
DMCAAEAQGTDGDIYVGAWDLDSVLNYCNPEWNGAGNLSDTDIEMVQLFYGQPIETSG
NEPVAICSVSASSSDGNIAENTLDGDYATRWSANGDGEWIQFNLCESQVADRVELAWY
KGDTRSSTFSIEYITTDGYVWYTTPVRRYSSGASLGLESASFTAAEIQSLRITGYGNS
SNTWNSITEAVIYTPSAGLDYPNLVAPTDVAATVTGQSQITISWADTNSEEESYFVEA
RIGGNDFFAIGLTEANATSYTHTDLIAEGLYEYRVTAVSGIIRSNFAAASVYFQPAGG
SQANLVRPENFTVTANAQGQIALSWVDVSEGEEGYTLEYKLSSEAEFTVIELAANADS
AIVSDLAAGSYYFRVSSYFGNNASEYSELLVTVFASEATIVPVDVYASSDDGNVASNV
FDNDYSTRWSAFGVGENLTIALGDNYLVTDMRIAWYKGDQRQTRFQVEVSDDNQTWVQ
VFDGINSGESLSLETTFGGDYRASYIRIIGLGNEFNNWNSITEVSIKGHL"
ORIGIN 
1 gtgaaaatca tacggttgct tgttttgtgt ttgggggtag taagttctgt ggttgcggta
61 gctagtagct cagagccaca tgatgaaggt aatgcattag agagtgcatt gtgggatacg
 
121 ttaactattc ctgtgtgctg ggagaatcca gaggcttttc ctgtagaaga gcaagcttgg
181 gtaagaaaag cggttgagcg tacttgggag caggagtcat tagtccgctt caccggttgg
241 ggcgagtgtg gggcgaacga tgatgggata cgtattttag tggatgatgt tgggccgcat
301 gttaagcagc taggctcgcg tttggatggc tatgttaatg gcatggtact aaaccataca
361 ttccaaaatt ggggcaccag ctgcagttac cgtcgtcaat attgtgcaga agtgattgca
421 gttcatgagt ttggtcatgc tctaggcttt gcccatgaac aaaacagaga tgacaccgat
481 gatatgtgcg cggccgaggc gcagggtact gacggcgaca tttatgtcgg cgcctgggac
541 ttggattcgg ttttaaacta ttgcaaccct gagtggaatg gcgcgggcaa tctaagcgat
601 accgatatag agatggttca gctgttttac ggtcagccca ttgaaacgtc tggtaacgag
661 cctgtagcta tttgtagtgt tagcgccagt tcaagcgatg gcaatattgc agaaaatacg
721 ctagacggcg attacgctac gcgttggtct gcaaacggcg acggcgagtg gatacaattt
781 aatttgtgtg aaagccaagt cgcggatcgt gtcgagttag cttggtataa gggagatact
841 cgctcaagta ccttctctat tgagtatata actaccgatg gttacgtttg gtataccacg
901 cctgttcgtc gatattcttc cggtgcttcg ctaggcttgg aatctgcgtc atttactgca
961 gctgaaattc agtccttgcg tattactggc tacggcaact cgtctaacac ttggaatagt
1021 attaccgaag cggtaattta tactccaagc gctgggctag attaccctaa cttagttgca
1081 cctacagatg tggcggctac ggtaacaggg caatcgcaaa ttactatttc atgggcagat
1141 actaatagcg aagaagaaag ctatttgtt gaggcgcgta ttggtggcaa cgattttttc
1201 gctataggtc ttactgaggc gaacgcaaca tcttatacgc ataccgacct tattgctgaa
1261 ggcctttacg agtatcgtgt tactgccgta agcggcatta ttcgctcaaa ctttgcagct
1321 gcaagtgtat attttcaacc tgcaggcggc tcgcaggcta atcttgtgcg cccagaaaac
1381 ttcactgtaa cagctaatgc gcaagggcaa atagcactaa gttgggtgga tgtaagtgaa
1441 ggcgaagagg gttatacctt agagtataag cttagtagtg aagctgaatt tactgtaatt
1501 gagctagctg cgaatgccga ttctgcaatt gttagcgatt tggctgcggg tagttactat
1561 tttcgtgtaa gcagttattt cggcaacaat gcttctgaat acagcgagct tttggttact
1621 gtgtttgcat cagaagcgac tattgtgccg gttgatgttt atgcatctag tgatgatggc
1681 aatgtggcaa gcaatgtttt tgataatgat tactctacgc gatggtcggc ttttggtgta
1741 ggtgagaatt taaccattgc gctgggggat aattatctcg ttaccgatat gcgcatagcg
1801 tggtacaaag gtgatcaacg tcaaacccgc tttcaggtag aggtgagtga tgataaccaa
1861 acctgggtgc aagtgtttga tgggattaac tcgggtgagt cgttaagttt agagacgacg
1921 tttggcggtg attatcgcgc aagctatatt cgaattatcg gtttgggtaa tgagtttaat
1981 aactggaaca gtattactga ggtaagcatt aaaggcacc tctag
LOCUS NZ_AABI02000005 2457 bp DNA linear BCT 23-SEP-2003
DEFINITION Microbulbifer degradans 2-40 Mdeg_5, whole genome shotgun sequence.
ACCESSION NZ AABI02000005 REGION: complement(73784..76240)
VERSION NZ_AABI02000005.1 GI:28878297
KEYWORDS WGS.
SOURCE Microbulbifer degradans 2-40
ORGANISM Microbulbifer degradans 2-40
   Bacteria; Proteobacteria; gamma subdivision; Alteromonadaceae;
   Microbulbifer.
REFERENCE 1 (bases 1 to 2457)
AUTHORS NCBI Microbial Genomes Annotation Project.
TITLE Direct Submission
JOURNAL Submitted (17-JUN-2004) National Center for Biotechnology Information, NIH, Bethesda, MD 20894, USA
COMMENT On Mar 7, 2003 this sequence version replaced gi:23027361. Protein-coding genes were predicted using GeneMark program (kindly provided by M. Borodovsky). Functional annotation is based on CDD (Conserved Domain Database) and COG (Clusters of Orthologous Groups) assignments, it has not yet been subject to manual review. DNA sequence and predicted proteins are available for BLAST at
   http://www.ncbi.nlm.nih.gov/sutils/genom table.cgi.
   URL -- http://www.jgi.doe.gov
   Contact: Paul Richardson
   microbes@cuba.jgi-psf.org.
   COG assignment was automatically updated on Sep 23, 2003.
FEATURES Location/Qualifiers
   source 1..2457
      /organism="Microbulbifer degradans 2-40"
      /mol_type="genomic DNA"
      /strain="2-40"
      /db_xref="taxon:203122"
   gene 1..2457
      /gene="Mdeg1249"
   CDS 1..2457
      /gene="Mdeg 1249"
      /codon_start=1
      /transl_table=11
      /product="hypothetical protein"
      /protein_id="ZP 00065873.1"
      /db_xref="GI:23027427" 
/translation="MIKLSHLKHCRKFCISLLCALGMANAHAALNVTASADDGNVPAN
TLDDNIDTRWSANGSGQWIEYDLGATHTVDAVQIAFFRGDVRDATIDIQVSNDGGNWQ
TLFSGTPPTRTLAQQHFELDDTSARYVRIVGYGNSQNNWNSITEFDVVTLASGENIAL
GKATSQSSTGYEGVSSRAVDGNTNGNWNQGSITHTNNEYQPWWQVDLGSVRSIDQVNL
WNRTNCCSSRLSAFYVLVSDVPFTSQTLSGALSQAGVSAYYFNDTAGSPTEINIDRTG
RYVRVQLSGTNPLSLAEVEVIEGSEIVPPAPTGPDASWTYCAAEREQCAFSNIKEVAY
GAGDSWNYSVELDGVTCNNTNLGDPVRGTVKSCWVRNAQQNYVAVRNLAELQNAISNS
NQHIRMKRGVYEATALMSDNTTVFRFDGANNVLDFTGVTIQVPTKLLNSMSTQPIHSQ
VTYDVMGDNITFLNGTFENTYPNGQHDVTDFTAHNKNPDYWPARQMTEFRVWGNGVQF
LNNTITVRGSYPYGYGDMLGKGAGSAVYLRKHAGVQISGDNVLIDGMKLTVLAFGHGI
FMQGADNTVIKNSVVQGRMRLGADMYNDGPDSLMGPFNFEQQYPDHFVGLPIVRDLMY
NLTEDGIRAYTQGTKLDGSVVRTGAITVEDTKVINMRGCITTPLASKPSYIKNVEIQG
CSVGYALANNSDVINSRGDAGYGPLLHSTYDTRNNANVEITVTNIPSTGSHAFAYIAG
SGHNITFLSDGSNPDVPREIRVGDTGDRWAGDPSYQDAANILLINETNQPVVLTETSR
NITGESVGEVTDNGTGNSLK"
ORIGIN

1 atgataaaac tatctcacct caaacactgc aggaaatttt gtatttcttt gctttgcgcg
61 ctgggcatgg ctaatgctca cgccgcatta aatgtaactg catctgcgga tgacggcaat
121 gtgcccgcga ataccttaga tgacaatata gatacgcgct ggtcggcgaa tggatctggg
181 cagtggattg aatatgattt gggcgcgaca cacactgtgg atgctgtgca aatagcattt
241 tttcgcggag atgtgcgcga tgcaaccatc gacattcaag tgtcgaacga tggcggcaat
301 tggcaaacac ttttttcggg tacgccacca acccgaacct tagcgcagca acattttgag
361 ttggatgata cttctgcgcg ctatgtgcgt attgtaggtt atggtaacag ccaaaataat
421 tggaacagta ttaccgagtt cgatgtggtt acgcttgcaa gtggagaaaa tattgctctt
481 ggtaaagcta catcccaatc atccactggc tatgagggtg tatctagccg tgcggtagat
541 ggcaacacca atggtaactg gaatcaaggc tcgattaccc acaccaataa cgagtatcaa
601 ccttggtggc aagtggatct aggctcagtt agatctatcg accaagttaa cttgtggaat
661 cgtaccaact gctgcagctc gcgtttatcg gcgttttatg tgttggtgtc cgatgtgccc
721 tttacatcgc aaaccttaag cggtgcgctt agtcaagcgg gtgtaagtgc ttattatttt
781 aatgatactg cgggcagccc aaccgaaata aatatagatc gcacaggtcg ctatgtgcgt
841 gtacaacttt ctggcacaaa cccattaagc ctagcggaag ttgaagttat tgaaggcagc
901 gaaattgttc caccagcacc aaccggccca gatgcatcat ggacttattg tgccgccgag
961 cgcgagcaat gtgcattttc taatataaaa gaagtggcct acggcgctgg cgatagctgg
1021 aactactctg tcgagctaga cggcgtaact tgtaataata ctaacttggg tgaccccgtt
1081 cgcggcacgg ttaaatcgtg ctgggtacgc aacgcacaac aaaattatgt ggcggtgcga
1141 aaccttgctg aattgcaaaa tgctatttcc aatagcaatc aacacattcg catgaagcgc
1201 ggtgtgtatg aagctacagc gttaatgtct gataacacca ccgtatttcg attcgacggt
1261 gcgaataatg tattggattt taccggtgta accattcagg tacccactaa actcttaaac
1321 agtatgagta cccagcctat tcactcgcaa gtaacctacg atgtgatggg cgataacatt
1381 acttttttaa acggtacttt cgaaaatacc tatccgaatg gccagcacga tgtaacagat
1441 tttaccgccc ataataaaaa cccagactat tggcccgcgc gtcaaatgac ggagtttaga
1501 gtgtggggga acggcgtaca gtttttaaat aacaccatta ctgtgcgcgg ttcatacccg
1561 tatggctacg gtgatatgtt aggtaagggc gccggttctg cagtgtattt acgcaaacat
1621 gctggtgtac aaatttctgg cgacaatgta ttaattgacg gcatgaaatt aaccgtactt
1681 gcattcggcc acggcatttt tatgcagggt gcagataaca cagtaattaa aaactctgtt
1741 gtacaagggc gtatgcgctt aggcgccgat atgtataacg acgggccaga ctccttaatg
1801 gggccgttta attttgagca gcaataccca gatcactttg tgggcctgcc aattgtgcgc
1861 gacctaatgt acaaccttac cgaagatggc atacgcgctt atacacaagg caccaaatta
1921 gatggtagtg tggtgcgtac tggtgcaatt actgtagaag acaccaaagt aattaatatg
1981 cgcggttgta ttactacgcc attggcttct aagccaagtt atataaaaaa tgtagaaatt
2041 caaggttgta gtgtgggtta cgcgctggcg aataatagcg atgtaatcaa ttcgcgcggt
2101 gatgcaggct acggtccgct attgcactct acgtacgata cgcgcaacaa cgccaatgtc
2161 gaaataaccg ttaccaatat tccatctacc ggctcacacg cttttgcgta tattgcaggc
2221 tctggtcata acattacctt cctaagtgat ggcagcaacc cagatgtgcc gcgcgaaata
2281 cgcgtaggcg atactggcga ccgctgggct ggcgacccaa gctatcaaga tgcagcaaat
2341 atattactca taaacgaaac caaccaaccg gtggtgttaa ctgaaaccag tagaaatatt
2401 actggtgaaa gtgtgggtga ggtgactgat aatgggacgg gaaatagctt gaaatag

## Claims

1. A method for producing ethanol from lignocellulosic material, comprising:
contacting lignocellulosic material with *Saccharophagus degradans* expressing an effective saccharifying amount of one or more compounds listed in Figures 4-11 to obtain saccharides; and
converting the saccharides to produce ethanol.

2. The method of claim 1, wherein the *Saccharophagus degradans* expresses an effective saccharifying amount of all of the compounds listed in Figures 4-11.

3. The method of claim 1, wherein the one or more compounds listed in Figures 4-11 are present in dry form, in a buffer, or in the form of a paste, paint, or micelle.

4. The method of claim 1, wherein the one or more compounds listed in Figures 4-11 are in a system consisting essentially of one or more compounds listed in Figures 4-11.

5. The method of claim 1, wherein the one or more compounds listed in Figures 4-11 are present in a system further comprising metal ions, chelators, detergents, organic ions, inorganic ions, or one or more additional proteins.

6. The method of claim 5, where the one or more additional proteins are biotin and/or albumin.

7. The method of claim 1, wherein the treating is conducted in a marine environment.

8. The method of claim 7, wherein the treating is conducted under water.

9. The method of claim 1, wherein the one or more compounds listed in Figures 4-11 is cellulase cel5A listed in Figure 4.

10. The method according to claim 1 wherein *Saccharophagus degradans* is subjected to ultrasound.

11. Use of *Saccharophagus degradans* expressing an effective saccharifying amount of one or more compounds listed in Figures 4-11 for the production of ethanol from lignocellulosic material.

12. A method for producing saccharides from lignocellulosic material, comprising:
contacting lignocellulosic material with *Saccharophagus degradans* expressing an effective saccharifying amount of one or more compounds listed in Figures 4-11 to obtain saccharides.

13. The method according to claim 12 wherein *Saccharophagus degradans* is subjected to ultrasound.

14. Use of *Saccharophagus degradans* expressing an effective saccharifying amount of one or more compounds listed in Figures 4-11 to obtain saccharides from lignocellulosic material.

## Patentansprüche

1. Verfahren zur Herstellung von Ethanol aus Lignocellulose-Material, umfassend:
Kontaktieren von Lignocellulose-Material mit *Saccharophagus degradans,* das eine effektive saccharifizierende Menge von einer oder mehreren der Verbindungen, die in den Figuren 4-11 aufgeführt sind, exprimiert, um Saccharide zu erhalten; und
Umsetzung der Saccharide, um Ethanol herzustellen.

2. Verfahren nach Anspruch 1, wobei das *Saccharophagus degradans* eine effektive saccharifizierende Menge von allen der Verbindungen, die in den Figuren 4-11 aufgeführt sind, exprimiert.

3. Verfahren nach Anspruch 1, wobei die eine oder mehreren der Verbindungen, die in den Figuren 4-11 aufgeführt sind, in trockener Form, in einem Puffer oder in Form einer Paste, eines Lacks oder einer Mizelle vorliegt/en.

4. Verfahren nach Anspruch 1, wobei die eine oder mehreren der Verbindungen, die in den Figuren 4-11 aufgeführt sind, in einem System vorliegen, das im Wesentlichen aus einer oder mehreren der Verbindungen, die in den Figuren 4-11 aufgeführt sind, besteht.

5. Verfahren nach Anspruch 1, wobei die eine oder mehreren der Verbindungen, die in den Figuren 4-11 aufgeführt sind, in einem System vorliegen, das ferner Metallionen, Chelatbildner, Detergenzien, organische Ionen, anorganische Ionen oder ein oder mehrere zusätzliche(s) Protein(e) enthält.

6. Verfahren nach Anspruch 5, wobei das eine oder die mehreren zusätzliche(n) Protein(e) Biotin und/oder Albumin ist bzw. sind.

7. Verfahren nach Anspruch 1, wobei die Behandlung in einer marinen Umgebung durchgeführt wird.

8. Verfahren nach Anspruch 7, wobei die Behandlung unter Wasser durchgeführt wird.

9. Verfahren nach Anspruch 1, wobei es sich bei der einen oder mehreren der Verbindungen, die in den Figuren 4-11 aufgeführt sind, um Cellulase ce15A, aufgeführt in Fig. 4, handelt.

10. Verfahren nach Anspruch 1, wobei *Saccharophagus degradans* einer Ultraschallbehandlung unterworfen wird.

11. Verwendung von *Saccharophagus degradans,* das eine effektive saccharifizierende Menge von einer oder mehreren der Verbindungen, die in den Figuren 4-11 aufgeführt sind, exprimiert, zur Herstellung von Ethanol aus Lignocellulose-Material.

12. Verfahren zur Herstellung von Sacchariden aus Lignocellulose-Material, umfassend:
Kontaktieren von Lignocellulose-Material mit *Saccharophagus degradans,* das eine effektive saccharifizierende Menge von einer oder mehreren der Verbindungen, die in den Figuren 4-11 aufgeführt sind, exprimiert, um Saccharide zu erhalten.

13. Verfahren nach Anspruch 12, wobei *Saccharophagus degradans* einer Ultraschallbehandlung unterworfen wird.

14. Verwendung von *Saccharophagus degradans,* das eine effektive saccharifizierende Menge von einer oder mehreren der Verbindungen, die in den Figuren 4-11 aufgeführt sind, exprimiert, um Saccharide aus Lignocellulose-Material zu erhalten.

## Revendications

1. Procédé de production d'éthanol à partir de matière lignocellulosique, comprenant:
la mise en contact de la matière lignocellulosique avec le *Saccharophagus degradans* exprimant une quantité saccharifiante efficace d'un ou de plusieurs composés cités dans les Figures 4-11 pour obtenir des saccharides; et
la conversion des saccharides pour produire de l'éthanol.

2. Procédé selon la revendication 1, dans lequel le *Saccharophagus degradans* exprime une quantité saccharifiante efficace de tous les composés cités dans les Figures 4-11.

3. Procédé selon la revendication 1, dans lequel le ou les composés cités dans les Figures 4-11 sont présents sous forme sèche, dans un tampon, ou sous la forme d'une pâte, d'une peinture ou d'une micelle.

4. Procédé selon la revendication 1, dans lequel le ou les composés cités dans les Figures 4-11 sont dans un système constitué essentiellement d'un ou de plusieurs composés cités dans les Figures 4-11.

5. Procédé selon la revendication 1, dans lequel le ou les composés cités dans les Figures 4-11 sont présents dans un système comprenant en outre des ions métalliques, des agents chélatants, des détergents, des ions organiques, des ions inorganiques, ou une ou plusieurs protéines supplémentaires.

6. Procédé selon la revendication 5, où la ou les protéines supplémentaires sont la biotine et/ou l'albumine.

7. Procédé selon la revendication 1, dans lequel le traitement est effectué dans un environnement marin.

8. Procédé selon la revendication 7, dans lequel le traitement est effectué sous l'eau.

9. Procédé selon la revendication 1, dans lequel le ou les composés cités dans les Figures 4-11 représentent la cellulase ceI5A citée dans la Figure 4.

10. Procédé selon la revendication 1, dans lequel le *Saccharophagus degradans* est soumis aux ultrasons.

11. Utilisation du *Saccharophagus degradans* exprimant une quantité saccharifiante efficace d'un ou de plusieurs composés cités dans les Figures 4-11 pour la production d'éthanol à partir de matière lignocellulosique.

12. Procédé de production de saccharides à partir de matière lignocellulosique, comprenant:
la mise en contact de la matière lignocellulosique avec le *Saccharophagus degradans* exprimant une quantité saccharifiante efficace d'un ou de plusieurs composés cités dans les Figures 4-11 pour obtenir des saccharides.

13. Procédé selon la revendication 12, dans lequel le *Saccharophagus degradans* est soumis aux ultrasons.

14. Utilisation du *Saccharophagus degradans* exprimant une quantité saccharifiante efficace d'un ou de plusieurs composés cités dans les Figures 4-11 pour obtenir des saccharides à partir de matière lignocellulosique.
